# EUROPEAN PATENT APPLICATION

(11) **EP 1 433 851 A2**
(43) Date of publication of application: **30.06.2004**
(21) Application number: 04075919.3
(22) Date of filing: 30.12.1999
(51) Int. Cl.: C12N 15/49, C12N 5/10, C12N 15/86, A61K 39/21

(54) **Improved expression of HIV polypeptides and production of virus-like particles**

(30) Priority: 31.12.1998 US 114495 P; 01.12.1999 US 168471 P
(62) Divisional of application: 99966727.2
(71) Applicant: CHIRON CORPORATION, Emeryville, California 94608 (US)
(72) Inventor: Barnett, Susan, c/o Chiron Corporation, Emeryville, CA 94608 (US); Zurmegede, Jan, c/o Chiron Corporation, Emeryville, CA 94608 (US); Srivastava, Indresch, c/o Chiron Corporation, Emeryville, CA 94608 (US); Lian, Ying, c/o Chiron Corporation, Emeryville, CA 94608 (US); Hartog, Karin, c/o Chiron Corporation, Emeryville, CA 94608 (US); Liu, Hong, c/o Chiron Corporation, Emeryville, CA 94608 (US); Greer, Catherine, c/o Chiron Corporation, Emeryville, CA 94608 (US); Selby, Mark, c/o Chiron Corporation, Emeryville, CA 94608 (US); Walker, Christopher, c/o Chiron Corporation, Emeryville, CA 94608 (US)
(74) Representative: Hallybone, Huw George

(57) **Abstract**

The present invention relates to the efficient expression of HIV polypeptides in a variety of cell types, including, but not limited to, mammalian, insect, and plant cells. Synthetic expression cassettes encoding the HIV Gag-containing polypeptides are described, as are uses of the expression cassettes in applications including DNA immunization, generation of packaging cell lines, and production of Envtat- or Gag-containing proteins. The invention provides methods of producing Virus-Like Particles (VLPs), as well as, uses of the VLPs including, but not limited to, vehicles for the presentation of antigens and stimulation of immune response in subjects to whom the VLPs are administered.

SEQUENCE LISTING

<110> Chiron Corporation

<120> IMPROVED EXPRESSION OF HIV POLYPEPTIDES AND PRODUCTION
      OF VIRUS-LIKE PARTICLES

<130> 1621.100

<140>
<141>

<160> 90

<170> PatentIn Ver. 2.0

<210> 1
<211> 1509
<212> DNA
<213> Human immunodeficiency virus

<400> 1
atgggtgcga gagcgtcggt attaagcggg ggagaattag ataaatggga aaaaattcgg 60
ttaaggccag ggggaaagaa aaaatataag ttaaaacata tagtatgggc aagcagggag 120
ctagaacgat tcgcagtcaa tcctggcctg ttagaaacat cagaaggctg cagacaaata 180
ttgggacagc tacagccatc ccttcagaca ggatcagaag aacttagatc attatataat 240
acagtagcaa ccctctattg tgtacatcaa aggatagatg taaaagacac caaggaagct 300
ttagagaaga tagaggaaga gcaaaacaaa agtaagaaaa aggcacagca agcagcagct 360
gcagctggca caggaaacag cagccaggtc agccaaaatt accctatagt gcagaaccta 420
caggggcaaa tggtacatca ggccatatca cctagaactt taaatgcatg ggtaaaagta 480
gtagaagaaa aggctttcag cccagaagta atacccatgt tttcagcatt atcagaagga 540
gccaccccac aagatttaaa caccatgcta aacacagtgg ggggacatca agcagccatg 600
caaatgttaa aagagactat caatgaggaa gctgcagaat gggatagagt gcatccagtg 660
catgcagggc ctattgcacc aggccaaatg agagaaccaa ggggaagtga catagcagga 720
actactagta cccttcagga acaaatagga tggatgacaa ataatccacc tatcccagta 780
ggagaaatct ataaaagatg gataatcctg ggattaaata aaatagtaag aatgtatagc 840
cctaccagca ttctggacat aagacaagga ccaaaggaac cctttagaga ttatgtagac 900
cggttctata aaactctaag agccgaacaa gcttcacagg atgtaaaaaa ttggatgaca 960
gaaaccttgt tggtccaaaa tgcaaaccca gattgtaaga ctattttaaa agcattggga 1020
ccagcagcta cactagaaga aatgatgaca gcatgtcagg gagtgggggg acccggccat 1080
aaagcaagag ttttggctga agccatgagc caagtaacaa atccagctaa cataatgatg 1140
cagagaggca attttaggaa ccaaagaaag actgttaagt gtttcaattg tggcaaagaa 1200
gggcacatag ccaaaaattg cagggcccct aggaaaaagg gctgttggag atgtggaagg 1260
gaaggacacc aaatgaaaga ttgcactgag agacaggcta attttttagg gaagatctgg 1320
ccttcctaca agggaaggcc agggaatttt cttcagagca gaccagagcc aacagcccca 1380
ccagaagaga gcttcaggtt tggggaggag aaaacaactc cctctcagaa gcaggagccg 1440
atagacaagg aactgtatcc tttaacttcc ctcagatcac tctttggcaa cgacccctcg 1500
tcacaataa                                                         1509

<210> 2
<211> 1845
<212> DNA
<213> Human immunodeficiency virus

<400> 2
atgggtgcga gagcgtcggt attaagcggg ggagaattag ataaatggga aaaaattcgg 60
ttaaggccag ggggaaagaa aaaatataag ttaaaacata tagtatgggc aagcagggag 120
ctagaacgat tcgcagtcaa tcctggcctg ttagaaacat cagaaggctg cagacaaata 180

ttgggacagc tacagccatc ccttcagaca ggatcagaag aacttagatc attatataat 240
acagtagcaa ccctctattg tgtacatcaa aggatagatg taaaagacac caaggaagct 300
ttagagaaga tagaggaaga gcaaaacaaa agtaagaaaa aggcacagca agcagcagct 360
gcagctggca caggaaacag cagccaggtc agccaaaatt accctatagt gcagaaccta 420
caggggcaaa tggtacatca ggccatatca cctagaactt taaatgcatg ggtaaaagta 480
gtagaagaaa aggctttcag cccagaagta atacccatgt tttcagcatt atcagaagga 540
gccaccccac aagatttaaa caccatgcta aacacagtgg ggggacatca agcagccatg 600
caaatgttaa aagagactat caatgaggaa gctgcagaat gggatagagt gcatccagtg 660
catgcagggc ctattgcacc aggccaaatg agagaaccaa ggggaagtga catagcagga 720
actactagta cccttcagga acaaatagga tggatgacaa ataatccacc tatcccagta 780
ggagaaatct ataaaagatg gataatcctg ggattaaata aaatagtaag aatgtatagc 840
cctaccagca ttctggacat aagacaagga ccaaaggaac cctttagaga ttatgtagac 900
cggttctata aaactctaag agccgaacaa gcttcacagg atgtaaaaaa ttggatgaca 960
gaaaccttgt tggtccaaaa tgcaaaccca gattgtaaga ctattttaaa agcattggga 1020
ccagcagcta cactagaaga aatgatgaca gcatgtcagg gagtgggggg acccggccat 1080
aaagcaagag ttttggctga agccatgagc caagtaacaa atccagctaa cataatgatg 1140
cagagaggca attttaggaa ccaaagaaag actgttaagt gtttcaattg tggcaaagaa 1200
gggcacatag ccaaaaattg cagggcccct aggaaaaagg gctgttggag atgtggaagg 1260
gaaggacacc aaatgaaaga ttgcactgag agacaggcta attttttagg gaagatctgg 1320
ccttcctaca agggaaggcc agggaatttt cttcagagca gaccagagcc aacagcccca 1380
ccagaagaga gcttcaggtt tggggaggag aaaacaactc cctctcagaa gcaggagccg 1440
atagacaagg aactgtatcc tttaacttcc ctcagatcac tctttggcaa cgacccctcg 1500
tcacaataag gatagggggg caactaaagg aagctctatt agatacagga gcagatgata 1560
cagtattaga agaaatgaat ttgccaggaa aatggaaacc aaaaatgata gggggaattg 1620
gaggttttat caaagtaaga cagtacgatc agatacctgt agaaatctgt ggacataaag 1680
ctataggtac agtattagta ggacctacac ctgtcaacat aattggaaga aatctgttga 1740
ctcagattgg ttgtacttta aatttcccca ttagtcctat tgaaactgta ccagtaaaat 1800
taaagccagg aatggatggc ccaaaagtta agcaatggcc attga 1845

<210> 3
<211> 4313
<212> DNA
<213> Human immunodeficiency virus

<400> 3
atgggtgcga gagcgtcggt attaagcggg ggagaattag ataaatggga aaaaattcgg 60
ttaaggccag ggggaaagaa aaaatataag ttaaaacata tagtatgggc aagcagggag 120
ctagaacgat tcgcagtcaa tcctggcctg ttagaaacat cagaaggctg cagacaaata 180
ttgggacagc tacagccatc ccttcagaca ggatcagaag aacttagatc attatataat 240
acagtagcaa ccctctattg tgtacatcaa aggatagatg taaaagacac caaggaagct 300
ttagagaaga tagaggaaga gcaaaacaaa agtaagaaaa aggcacagca agcagcagct 360
gcagctggca caggaaacag cagccaggtc agccaaaatt accctatagt gcagaaccta 420
caggggcaaa tggtacatca ggccatatca cctagaactt taaatgcatg ggtaaaagta 480
gtagaagaaa aggctttcag cccagaagta atacccatgt tttcagcatt atcagaagga 540
gccaccccac aagatttaaa caccatgcta aacacagtgg ggggacatca agcagccatg 600
caaatgttaa aagagactat caatgaggaa gctgcagaat gggatagagt gcatccagtg 660
catgcagggc ctattgcacc aggccaaatg agagaaccaa ggggaagtga catagcagga 720
actactagta cccttcagga acaaatagga tggatgacaa ataatccacc tatcccagta 780
ggagaaatct ataaaagatg gataatcctg ggattaaata aaatagtaag aatgtatagc 840
cctaccagca ttctggacat aagacaagga ccaaaggaac cctttagaga ttatgtagac 900
cggttctata aaactctaag agccgaacaa gcttcacagg atgtaaaaaa ttggatgaca 960
gaaaccttgt tggtccaaaa tgcaaaccca gattgtaaga ctattttaaa agcattggga 1020
ccagcagcta cactagaaga aatgatgaca gcatgtcagg gagtgggggg acccggccat 1080
aaagcaagag ttttggctga agccatgagc caagtaacaa atccagctaa cataatgatg 1140
cagagaggca attttaggaa ccaaagaaag actgttaagt gtttcaattg tggcaaagaa 1200
gggcacatag ccaaaaattg cagggcccct aggaaaaagg gctgttggag atgtggaagg 1260
gaaggacacc aaatgaaaga ttgcactgag agacaggcta attttttagg gaagatctgg 1320
ccttcctaca agggaaggcc agggaatttt cttcagagca gaccagagcc aacagcccca 1380
ccagaagaga gcttcaggtt tggggaggag aaaacaactc cctctcagaa gcaggagccg 1440

atagacaagg aactgtatcc tttaacttcc ctcagatcac tctttggcaa cgacccctcg 1500
tcacaataag gatagggggg caactaaagg aagctctatt agatacagga gcagatgata 1560
cagtattaga agaaatgaat ttgccaggaa aatggaaacc aaaaatgata gggggaattg 1620
gaggttttat caaagtaaga cagtacgatc agatacctgt agaaatctgt ggacataaag 1680
ctataggtac agtattagta ggacctacac ctgtcaacat aattggaaga aatctgttga 1740
ctcagattgg ttgtacttta aatttcccca ttagtcctat tgaaactgta ccagtaaaat 1800
taaagccagg aatggatggc ccaaaagtta agcaatggcc attgacagaa gaaaaaataa 1860
aagcattagt agagatatgt acagaaatgg aaaaggaagg gaaaatttca aaaattgggc 1920
ctgaaaatcc atacaatact ccagtatttg ctataaagaa aaaagacagt actaaatgga 1980
gaaaactagt agatttcaga gaacttaata aaagaactca agacttctgg gaagttcagt 2040
taggaatacc acaccccgca gggttaaaaa agaaaaaatc agtaacagta ttggatgtgg 2100
gtgatgcata cttttcagtt cccttagata aagactttag aaagtatact gcatttacca 2160
tacctagtat aaacaatgag acaccaggga ttagatatca gtacaatgtg ctgccacagg 2220
gatggaaagg atcaccagca atattccaaa gtagcatgac aaaaatctta gagcctttta 2280
gaaaacagaa tccagacata gttatctatc aatacatgga tgatttgtat gtaggatctg 2340
acttagaaat agggcagcat agaacaaaaa tagaggaact gagacagcat ctgttgaggt 2400
ggggatttac cacaccagac aaaaaacatc agaaagaacc tccattcctt tggatgggtt 2460
atgaactcca tcctgataaa tggacagtac agcctataat gctgccagaa aaagacagct 2520
ggactgtcaa tgacatacag aagttagtgg gaaaattgaa ttgggcaagt cagatttatg 2580
cagggattaa agtaaagcag ttatgtaaac tccttagagg aaccaaagca ctaacagaag 2640
taataccact aacagaagaa gcagagctag aactggcaga aaacagggag attctaaaag 2700
aaccagtaca tgaagtatat tatgacccat caaaagactt agtagcagaa atacagaagc 2760
aggggcaagg ccaatggaca tatcaaattt atcaagagcc atttaaaaat ctgaaaacag 2820
gaaagtatgc aaggatgagg ggtgcccaca ctaatgatgt aaaacagtta acagaggcag 2880
tgcaaaaagt atccacagaa agcatagtaa tatggggaaa gattcctaaa tttaaactac 2940
ccatacaaaa ggaaacatgg gaagcatggt ggatggagta ttggcaagct acctggattc 3000
ctgagtggga gtttgtcaat acccctccct tagtgaaatt atggtaccag ttagagaaag 3060
aacccatagt aggagcagaa actttctatg tagatggggc agctaatagg gagactaaat 3120
taggaaaagc aggatatgtt actgacagag gaagacaaaa agttgtctcc atagctgaca 3180
caacaaatca gaagactgaa ttacaagcaa ttcatctagc tttgcaggat tcgggattag 3240
aagtaaacat agtaacagac tcacaatatg cattaggaat cattcaagca caaccagata 3300
agagtgaatc agagttagtc agtcaaataa tagagcagtt aataaaaaag gaaaaggtct 3360
acctggcatg ggtaccagca cacaaaggaa ttggaggaaa tgaacaagta gataaattag 3420
tcagtgctgg aatcaggaaa gtactatttt tgaatggaat agataaggcc caagaagaac 3480
atgagaaata tcacagtaat tggagagcaa tggctagtga ttttaacctg ccacctgtag 3540
tagcaaaaga aatagtagcc agctgtgata aatgtcagct aaaaggagaa gccatgcatg 3600
gacaagtaga ctgtagtcca ggaatatggc aactagattg tacacatcta gaaggaaaaa 3660
ttatcctggt agcagttcat gtagccagtg gatatataga agcagaagtt attccagcag 3720
agacagggca ggaaacagca tattttctct taaaattagc aggaagatgg ccagtaaaaa 3780
caatacatac agacaatggc agcaatttca ccagtactac ggttaaggcc gcctgttggt 3840
gggcagggat caagcaggaa tttggcattc cctacaatcc ccaaagtcaa ggagtagtag 3900
aatctatgaa taatgaatta aagaaaatta taggacaggt aagagatcag gctgaacacc 3960
ttaagacagc agtacaaatg gcagtattca tccacaattt taaaagaaaa ggggggattg 4020
ggggatacag tgcaggggaa agaatagtag acataatagc aacagacata caaactaaag 4080
aactacaaaa gcaaattaca aaaattcaaa attttcgggt ttattacagg gacaacaaag 4140
atcccctttg gaaaggacca gcaaagcttc tctggaaagg tgaaggggca gtagtaatac 4200
aagataatag tgacataaaa gtagtgccaa gaagaaaagc aaaaatcatt agggattatg 4260
gaaaacagat ggcaggtgat gattgtgtgg caagtagaca ggatgaggat tag 4313

<210> 4
<211> 1515
<212> DNA
<213> Artificial Sequence

<220>
<223> Description of Artificial Sequence: synthetic
      HIV-Gag

<400> 4

gccaccatgg gcgcccgcgc cagcgtgctg agcggcggcg agctggacaa gtgggagaag 60
atccgcctgc gccccggcgg caagaagaag tacaagctga agcacatcgt gtgggccagc 120
cgcgagctgg agcgcttcgc cgtgaacccc ggcctgctgg agaccagcga gggctgccgc 180
cagatcctgg gccagctgca gcccagcctg cagaccggca gcgaggagct gcgcagcctg 240
tacaacaccg tggccaccct gtactgcgtg caccagcgca tcgacgtcaa ggacaccaag 300
gaggccctgg agaagatcga ggaggagcag aacaagtcca agaagaaggc ccagcaggcc 360
gccgccgccg ccggcaccgg caacagcagc caggtgagcc agaactaccc catcgtgcag 420
aacctgcagg gccagatggt gcaccaggcc atcagccccc gcaccctgaa cgcctgggtg 480
aaggtggtgg aggagaaggc cttcagcccc gaggtgatcc ccatgttcag cgccctgagc 540
gagggcgcca ccccccagga cctgaacacg atgttgaaca ccgtgggcgg ccaccaggcc 600
gccatgcaga tgctgaagga gaccatcaac gaggaggccg ccgagtggga ccgcgtgcac 660
cccgtgcacg ccggccccat cgcccccggc cagatgcgcg agccccgcgg cagcgacatc 720
gccggcacca ccagcaccct gcaggagcag atcggctgga tgaccaacaa cccccccatc 780
cccgtgggcg agatctacaa gcggtggatc atcctgggcc tgaacaagat cgtgcggatg 840
tacagcccca ccagcatcct ggacatccgc cagggcccca aggagccctt ccgcgactac 900
gtggaccgct tctacaagac cctgcgcgct gagcaggcca gccaggacgt gaagaactgg 960
atgaccgaga ccctgctggt gcagaacgcc aaccccgact gcaagaccat cctgaaggct 1020
ctcggccccg cggccaccct ggaggagatg atgaccgcct gccagggcgt gggcggcccc 1080
ggccacaagg cccgcgtgct ggccgaggcg atgagccagg tgacgaaccc ggcgaccatc 1140
atgatgcagc gcggcaactt ccgcaaccag cggaagaccg tcaagtgctt caactgcggc 1200
aaggagggcc acaccgccag gaactgccgc gccccccgca agaagggctg ctggcgctgc 1260
ggccgcgagg gccaccagat gaaggactgc accgagcgcc aggccaactt cctgggcaag 1320
atctggccca gctacaaggg ccgccccggc aacttcctgc agagccgccc cgagcccacc 1380
gccccccccg aggagagctt ccgcttcggc gaggagaaga ccacccccag ccagaagcag 1440
gagcccatcg acaaggagct gtaccccctg accagcctgc gcagcctgtt cggcaacgac 1500
cccagcagcc agtaa 1515

<210> 5
<211> 1853
<212> DNA
<213> Artificial Sequence

<220>
<223> Description of Artificial Sequence: synthetic
      HIV-Gag-protease

<400> 5
gccaccatgg gcgcccgcgc cagcgtgctg agcggcggcg agctggacaa gtgggagaag 60
atccgcctgc gccccggcgg caagaagaag tacaagctga agcacatcgt gtgggccagc 120
cgcgagctgg agcgcttcgc cgtgaacccc ggcctgctgg agaccagcga gggctgccgc 180
cagatcctgg gccagctgca gcccagcctg cagaccggca gcgaggagct gcgcagcctg 240
tacaacaccg tggccaccct gtactgcgtg caccagcgca tcgacgtcaa ggacaccaag 300
gaggccctgg agaagatcga ggaggagcag aacaagtcca agaagaaggc ccagcaggcc 360
gccgccgccg ccggcaccgg caacagcagc caggtgagcc agaactaccc catcgtgcag 420
aacctgcagg gccagatggt gcaccaggcc atcagccccc gcaccctgaa cgcctgggtg 480
aaggtggtgg aggagaaggc cttcagcccc gaggtgatcc ccatgttcag cgccctgagc 540
gagggcgcca ccccccagga cctgaacacg atgttgaaca ccgtgggcgg ccaccaggcc 600
gccatgcaga tgctgaagga gaccatcaac gaggaggccg ccgagtggga ccgcgtgcac 660
cccgtgcacg ccggccccat cgcccccggc cagatgcgcg agccccgcgg cagcgacatc 720
gccggcacca ccagcaccct gcaggagcag atcggctgga tgaccaacaa cccccccatc 780
cccgtgggcg agatctacaa gcggtggatc atcctgggcc tgaacaagat cgtgcggatg 840
tacagcccca ccagcatcct ggacatccgc cagggcccca aggagccctt ccgcgactac 900
gtggaccgct tctacaagac cctgcgcgct gagcaggcca gccaggacgt gaagaactgg 960
atgaccgaga ccctgctggt gcagaacgcc aaccccgact gcaagaccat cctgaaggct 1020
ctcggccccg cggccaccct ggaggagatg atgaccgcct gccagggcgt gggcggcccc 1080
ggccacaagg cccgcgtgct ggccgaggcg atgagccagg tgacgaaccc ggcgaccatc 1140
atgatgcagc gcggcaactt ccgcaaccag cggaagaccg tcaagtgctt caactgcggc 1200
aaggagggcc acaccgccag gaactgccgc gccccccgca agaagggctg ctggcgctgc 1260
ggccgcgaag gacaccaaat gaaagattgc actgagagac aggctaattt tttagggaag 1320

atctggcctt cctacaaggg aaggccaggg aattttcttc agagcagacc agagccaaca 1380
gccccaccag aagagagctt caggtttggg gaggagaaaa caactccctc tcagaagcag 1440
gagccgatag acaaggaact gtatccttta acttccctca gatcactctt tggcaacgac 1500
ccctcgtcac agtaaggatc ggcggccagc tcaaggaggc gctgctcgac accggcgccg 1560
acgacaccgt gctggaggag atgaacctgc ccggcaagtg gaagcccaag atgatcggcg 1620
ggatcggggg cttcatcaag gtgcggcagt acgaccagat ccccgtggag atctgcggcc 1680
acaaggccat cggcaccgtg ctggtgggcc ccacccccgt gaacatcatc ggccgcaacc 1740
tgctgaccca gatcggctgc accctgaact tccccatcag ccccatcgag acggtgcccg 1800
tgaagctgaa gccggggatg gacggcccca aggtcaagca gtggcccctg taa 1853

<210> 6
<211> 4319
<212> DNA
<213> Artificial Sequence

<220>
<223> Description of Artificial Sequence: synthetic
      HIV-Gag-polymerase

<400> 6
gccaccatgg gcgcccgcgc cagcgtgctg agcggcggcg agctggacaa gtgggagaag 60
atccgcctgc gccccggcgg caagaagaag tacaagctga agcacatcgt gtgggccagc 120
cgcgagctgg agcgcttcgc cgtgaacccc ggcctgctgg agaccagcga gggctgccgc 180
cagatcctgg gccagctgca gcccagcctg cagaccggca gcgaggagct gcgcagcctg 240
tacaacaccg tggccaccct gtactgcgtg caccagcgca tcgacgtcaa ggacaccaag 300
gaggccctgg agaagatcga ggaggagcag aacaagtcca agaagaaggc ccagcaggcc 360
gccgccgccg ccggcaccgg caacagcagc caggtgagcc agaactaccc catcgtgcag 420
aacctgcagg gccagatggt gcaccaggcc atcagccccc gcaccctgaa cgcctgggtg 480
aaggtggtgg aggagaaggc cttcagcccc gaggtgatcc ccatgttcag cgccctgagc 540
gagggcgcca ccccccagga cctgaacacg atgttgaaca ccgtgggcgg ccaccaggcc 600
gccatgcaga tgctgaagga gaccatcaac gaggaggccg ccgagtggga ccgcgtgcac 660
cccgtgcacg ccggccccat cgcccccggc cagatgcgcg agccccgcgg cagcgacatc 720
gccggcacca ccagcaccct gcaggagcag atcggctgga tgaccaacaa cccccccatc 780
cccgtgggcg agatctacaa gcggtggatc atcctgggcc tgaacaagat cgtgcggatg 840
tacagcccca ccagcatcct ggacatccgc cagggcccca aggagccctt ccgcgactac 900
gtggaccgct tctacaagac cctgcgcgct gagcaggcca gccaggacgt gaagaactgg 960
atgaccgaga ccctgctggt gcagaacgcc aaccccgact gcaagaccat cctgaaggct 1020
ctcggccccg cggccaccct ggaggagatg atgaccgcct gccagggcgt gggcggcccc 1080
ggccacaagg cccgcgtgct ggccgaggcg atgagccagg tgacgaaccc ggcgaccatc 1140
atgatgcagc gcggcaactt ccgcaaccag cggaagaccg tcaagtgctt caactgcggc 1200
aaggagggcc acaccgccag gaactgccgc gccccccgca agaagggctg ctggcgctgc 1260
ggccgcgaag gacaccaaat gaaagattgc actgagagac aggctaattt tttagggaag 1320
atctggcctt cctacaaggg aaggccaggg aattttcttc agagcagacc agagccaaca 1380
gccccaccag aagagagctt caggtttggg gaggagaaaa caactccctc tcagaagcag 1440
gagccgatag acaaggaact gtatccttta acttccctca gatcactctt tggcaacgac 1500
ccctcgtcac agtaaggatc ggcggccagc tcaaggaggc gctgctcgac accggcgccg 1560
acgacaccgt gctggaggag atgaacctgc ccggcaagtg gaagcccaag atgatcggcg 1620
ggatcggggg cttcatcaag gtgcggcagt acgaccagat ccccgtggag atctgcggcc 1680
acaaggccat cggcaccgtg ctggtgggcc ccacccccgt gaacatcatc ggccgcaacc 1740
tgctgaccca gatcggctgc accctgaact tccccatcag ccccatcgag acggtgcccg 1800
tgaagctgaa gccggggatg gacggcccca aggtcaagca gtggcccctg accgaggaga 1860
agatcaaggc cctggtggag atctgcaccg agatggagaa ggagggcaag atcagcaaga 1920
tcggccccga gaacccctac aacacccccg tgttcgccat caagaagaag gacagcacca 1980
agtggcgcaa gctggtggac ttccgcgagc tgaacaagcg cacccaggac ttctgggagg 2040
tgcagctggg catcccccac cccgccggcc tgaagaagaa gaagagcgtg accgtgctgg 2100
acgtgggcga cgcctacttc agcgtgcccc tggacaagga cttccgcaag tacaccgcct 2160
tcaccatccc cagcatcaac aacgagaccc ccggcatccg ctaccagtac aacgtgctgc 2220
cccagggctg gaagggcagc cccgccatct tccagagcag catgaccaag atcctggagc 2280
ccttccgcaa gcagaacccc gacatcgtga tctaccagta catggacgac ctgtacgtgg 2340

gcagcgacct ggagatcggc cagcaccgca ccaagatcga ggagctgcgc cagcacctgc 2400
tgcgctgggg cttcaccacc cccgacaaga agcaccagaa ggagcccccc ttcctgtgga 2460
tgggctacga gctgcacccc gacaagtgga ccgtgcagcc catcatgctg cccgagaagg 2520
acagctggac cgtgaacgac atccagaagc tggtgggcaa gctgaactgg gccagccaga 2580
tctacgccgg catcaaggtg aagcagctgt gcaagctgct gcgcggcacc aaggccctga 2640
ccgaggtgat ccccctgacc gaggaggccg agctggagct ggccgagaac cgcgagatcc 2700
tgaaggagcc cgtgcacgag gtgtactacg accccagcaa ggacctggtg gccgagatcc 2760
agaagcaggg ccagggccag tggacctacc agatctacca ggagcccttc aagaacctga 2820
agaccggcaa gtacgcccgc atgcgcggcg cccacaccaa cgacgtgaag cagctgaccg 2880
aggccgtgca gaaggtgagc accgagagca tcgtgatctg gggcaagatc cccaagttca 2940
agctgcccat ccagaaggag acctgggagg cctggtggat ggagtactgg caggccacct 3000
ggatccccga gtgggagttc gtgaacaccc cccccctggt gaagctgtgg taccagctgg 3060
agaaggagcc catcgtgggc gccgagacct tctacgtgga cggcgccgcc aaccgcgaga 3120
ccaagctggg caaggccggc tacgtgaccg accgcggccg ccagaaggtg gtgagcatcg 3180
ccgacaccac caaccagaag accgagctgc aggccatcca cctggccctg caggacagcg 3240
gcctggaggt gaacatcgtg accgacagcc agtacgccct gggcatcatc caggcccagc 3300
ccgacaagag cgagagcgag ctggtgagcc agatcatcga gcagctgatc aagaaggaga 3360
aggtgtacct ggcctgggtg cccgcccaca agggcatcgg cggcaacgag caggtggaca 3420
agctggtgag cgccggcatc cgcaaggtgc tgttcctgaa cggcatcgac aaggcccagg 3480
aggagcacga gaagtaccac agcaactggc gcgccatggc cagcgacttc aacctgcccc 3540
ccgtggtggc caaggagatc gtggccagct gcgacaagtg ccagctgaag ggcgaggcca 3600
tgcacggcca ggtggactgc agccccggca tctggcagct ggactgcacc cacctggagg 3660
gcaagatcat cctggtggcc gtgcacgtgg ccagcggcta catcgaggcc gaggtgatcc 3720
ccgccgagac cggccaggag accgcctact tcctgctgaa gctggccggc cgctggcccg 3780
tgaagaccat ccacaccgac aacggcagca acttcaccag caccaccgtg aaggccgcct 3840
gctggtgggc cggcatcaag caggagttcg gcatccccta caacccccag agccagggcg 3900
tggtggagag catgaacaac gagctgaaga agatcatcgg ccaggtgcgc gaccaggccg 3960
agcacctgaa gaccgccgtg cagatggccg tgttcatcca caacttcaag cgcaagggcg 4020
gcatcggcgg ctacagcgcc ggcgagcgca tcgtggacat catcgccacc gacatccaga 4080
ccaaggagct gcagaagcag atcaccaaga tccagaactt ccgcgtgtac taccgcgaca 4140
acaaggaccc cctgtggaag ggccccgcca agctgctgtg gaagggcgag ggcgccgtgg 4200
tgatccagga caacagcgac atcaaggtgg tgccccgccg caaggccaag atcatccgcg 4260
actacggcaa gcagatggcc ggcgacgact gcgtggccag ccgccaggac gaggactag 4319

<210> 7
<211> 2031
<212> DNA
<213> Artificial Sequence

<220>
<223> Description of Artificial Sequence: synthetic
      HIV-Gag/HCV-core fusion polypeptide

<400> 7
gccaccatgg gcgcccgcgc cagcgtgctg agcggcggcg agctggacaa gtgggagaag 60
atccgcctgc gccccggcgg caagaagaag tacaagctga agcacatcgt gtgggccagc 120
cgcgagctgg agcgcttcgc cgtgaacccc ggcctgctgg agaccagcga gggctgccgc 180
cagatcctgg gccagctgca gcccagcctg cagaccggca gcgaggagct gcgcagcctg 240
tacaacaccg tggccaccct gtactgcgtg caccagcgca tcgacgtcaa ggacaccaag 300
gaggccctgg agaagatcga ggaggagcag aacaagtcca agaagaaggc ccagcaggcc 360
gccgccgccg ccggcaccgg caacagcagc caggtgagcc agaactaccc catcgtgcag 420
aacctgcagg gccagatggt gcaccaggcc atcagccccc gcaccctgaa cgcctgggtg 480
aaggtggtgg aggagaaggc cttcagcccc gaggtgatcc ccatgttcag cgccctgagc 540
gagggcgcca ccccccagga cctgaacacg atgttgaaca ccgtgggcgg ccaccaggcc 600
gccatgcaga tgctgaagga gaccatcaac gaggaggccg ccgagtggga ccgcgtgcac 660
cccgtgcacg ccggccccat cgcccccggc cagatgcgcg agccccgcgg cagcgacatc 720
gccggcacca ccagcaccct gcaggagcag atcggctgga tgaccaacaa cccccccatc 780
cccgtgggcg agatctacaa gcggtggatc atcctgggcc tgaacaagat cgtgcggatg 840
tacagcccca ccagcatcct ggacatccgc cagggcccca aggagccctt ccgcgactac 900

gtggaccgct tctacaagac cctgcgcgct gagcaggcca gccaggacgt gaagaactgg 960
atgaccgaga ccctgctggt gcagaacgcc aaccccgact gcaagaccat cctgaaggct 1020
ctcggccccg cggccaccct ggaggagatg atgaccgcct gccagggcgt gggcggcccc 1080
ggccacaagg cccgcgtgct ggccgaggcg atgagccagg tgacgaaccc ggcgaccatc 1140
atgatgcagc gcggcaactt ccgcaaccag cggaagaccg tcaagtgctt caactgcggc 1200
aaggagggcc acaccgccag gaactgccgc gccccccgca agaagggctg ctggcgctgc 1260
ggccgcgagg gccaccagat gaaggactgc accgagcgcc aggccaactt cctgggcaag 1320
atctggccca gctacaaggg ccgccccggc aacttcctgc agagccgccc cgagcccacc 1380
gccccccccg aggagagctt ccgcttcggc gaggagaaga ccacccccag ccagaagcag 1440
gagcccatcg acaaggagct gtaccccctg accagcctgc gcagcctgtt cggcaacgac 1500
cccagcagcc agtcgacgaa tcctaaacct caaagaaaaa acaaacgtaa caccaaccgt 1560
cgcccacagg acgtcaagtt cccgggtggc ggtcagatcg ttggtggagt ttacttgttg 1620
ccgcgcaggg gccctagatt gggtgtgcgc gcgacgagaa agacttccga gcggtcgcaa 1680
cctcgaggta gacgtcagcc tatccccaag gctcgtcggc ccgagggcag gacctgggct 1740
cagcccgggt acccttggcc cctctatggc aatgagggct gcgggtgggc gggatggctc 1800
ctgtctcccc gtggctctcg gcctagctgg ggccccacag acccccggcg taggtcgcgc 1860
aatttgggta aggtcatcga tacccttacg tgcggcttcg ccgacctcat ggggtacata 1920
ccgctcgtcg gcgcccctct tggaggcgct gccagggccc tggcgcatgg cgtccgggtt 1980
ctggaagacg gcgtgaacta tgcaacaggg aaccttcctg gttgctctta g 2031

<210> 8
<211> 2025
<212> DNA
<213> Artificial Sequence

<220>
<223> Description of Artificial Sequence: synthetic
      HIV-Gag/HCV-Core fusion polypeptide

<400> 8
atgggtgcga gagcgtcggt attaagcggg ggagaattag ataaatggga aaaaattcgg 60
ttaaggccag ggggaaagaa aaaatataag ttaaaacata tagtatgggc aagcagggag 120
ctagaacgat tcgcagtcaa tcctggcctg ttagaaacat cagaaggctg cagacaaata 180
ttgggacagc tacagccatc ccttcagaca ggatcagaag aacttagatc attatataat 240
acagtagcaa ccctctattg tgtacatcaa aggatagatg taaaagacac caaggaagct 300
ttagagaaga tagaggaaga gcaaaacaaa agtaagaaaa aggcacagca agcagcagct 360
gcagctggca caggaaacag cagccaggtc agccaaaatt accctatagt gcagaaccta 420
caggggcaaa tggtacatca ggccatatca cctagaactt taaatgcatg ggtaaaagta 480
gtagaagaaa aggctttcag cccagaagta atacccatgt tttcagcatt atcagaagga 540
gccaccccac aagatttaaa caccatgcta aacacagtgg ggggacatca agcagccatg 600
caaatgttaa aagagactat caatgaggaa gctgcagaat gggatagagt gcatccagtg 660
catgcagggc ctattgcacc aggccaaatg agagaaccaa ggggaagtga catagcagga 720
actactagta cccttcagga acaaatagga tggatgacaa ataatccacc tatcccagta 780
ggagaaatct ataaaagatg gataatcctg ggattaaata aaatagtaag aatgtatagc 840
cctaccagca ttctggacat aagacaagga ccaaaggaac cctttagaga ttatgtagac 900
cggttctata aaactctaag agccgaacaa gcttcacagg atgtaaaaaa ttggatgaca 960
gaaaccttgt tggtccaaaa tgcaaaccca gattgtaaga ctattttaaa agcattggga 1020
ccagcagcta cactagaaga aatgatgaca gcatgtcagg gagtgggggg acccggccat 1080
aaagcaagag ttttggctga agccatgagc caagtaacaa atccagctaa cataatgatg 1140
cagagaggca attttaggaa ccaaagaaag actgttaagt gtttcaattg tggcaaagaa 1200
gggcacatag ccaaaaattg cagggcccct aggaaaaagg gctgttggag atgtggaagg 1260
gaaggacacc aaatgaaaga ttgcactgag agacaggcta attttttagg gaagatctgg 1320
ccttcctaca agggaaggcc agggaatttt cttcagagca gaccagagcc aacagcccca 1380
ccagaagaga gcttcaggtt tggggaggag aaaacaactc cctctcagaa gcaggagccg 1440
atagacaagg aactgtatcc tttaacttcc ctcagatcac tctttggcaa cgacccctcg 1500
tcacagtcga cgaatcctaa acctcaaaga aaaaacaaac gtaacaccaa ccgtcgccca 1560
caggacgtca agttcccggg tggcggtcag atcgttggtg gagtttactt gttgccgcgc 1620
aggggcccta gattgggtgt gcgcgcgacg agaaagactt ccgagcggtc gcaacctcga 1680
ggtagacgtc agcctatccc caaggctcgt cggcccgagg gcaggacctg ggctcagccc 1740

gggtaccctt ggcccctcta tggcaatgag ggctgcgggt gggcgggatg gctcctgtct 1800
ccccgtggct ctcggcctag ctggggcccc acagaccccc ggcgtaggtc gcgcaatttg 1860
ggtaaggtca tcgataccct tacgtgcggc ttcgccgacc tcatggggta cataccgctc 1920
gtcggcgccc ctcttggagg cgctgccagg gccctggcgc atggcgtccg ggttctggaa 1980
gacggcgtga actatgcaac agggaacctt cctggttgct cttag 2025

<210> 9
<211> 1268
<212> DNA
<213> Artificial Sequence

<220>
<223> Description of Artificial Sequence: synthetic Gag
      common region

<400> 9
gccaccatgg gcgcccgcgc cagcgtgctg agcggcggcg agctggacaa gtgggagaag 60
atccgcctgc gccccggcgg caagaagaag tacaagctga agcacatcgt gtgggccagc 120
cgcgagctgg agcgcttcgc cgtgaacccc ggcctgctgg agaccagcga gggctgccgc 180
cagatcctgg gccagctgca gcccagcctg cagaccggca gcgaggagct gcgcagcctg 240
tacaacaccg tggccaccct gtactgcgtg caccagcgca tcgacgtcaa ggacaccaag 300
gaggccctgg agaagatcga ggaggagcag aacaagtcca agaagaaggc ccagcaggcc 360
gccgccgccg ccggcaccgg caacagcagc caggtgagcc agaactaccc catcgtgcag 420
aacctgcagg gccagatggt gcaccaggcc atcagccccc gcaccctgaa cgcctgggtg 480
aaggtggtgg aggagaaggc cttcagcccc gaggtgatcc ccatgttcag cgccctgagc 540
gagggcgcca ccccccagga cctgaacacg atgttgaaca ccgtgggcgg ccaccaggcc 600
gccatgcaga tgctgaagga gaccatcaac gaggaggccg ccgagtggga ccgcgtgcac 660
cccgtgcacg ccggccccat cgcccccggc cagatgcgcg agccccgcgg cagcgacatc 720
gccggcacca ccagcaccct gcaggagcag atcggctgga tgaccaacaa cccccccatc 780
cccgtgggcg agatctacaa gcggtggatc atcctgggcc tgaacaagat cgtgcggatg 840
tacagcccca ccagcatcct ggacatccgc cagggcccca aggagccctt ccgcgactac 900
gtggaccgct tctacaagac cctgcgcgct gagcaggcca gccaggacgt gaagaactgg 960
atgaccgaga ccctgctggt gcagaacgcc aaccccgact gcaagaccat cctgaaggct 1020
ctcggccccg cggccaccct ggaggagatg atgaccgcct gccagggcgt gggcggcccc 1080
ggccacaagg cccgcgtgct ggccgaggcg atgagccagg tgacgaaccc ggcgaccatc 1140
atgatgcagc gcggcaactt ccgcaaccag cggaagaccg tcaagtgctt caactgcggc 1200
aaggagggcc acaccgccag gaactgccgc gccccccgca agaagggctg ctggcgctgc 1260
ggccgcga 1268

<210> 10
<211> 20
<212> PRT
<213> Artificial Sequence

<220>
<223> Description of Artificial Sequence: HIV-Gag
      peptide p7G

<400> 10
Gly Gly His Gln Ala Ala Met Gln Met Leu Lys Glu Thr Ile Asn Glu
  1               5                  10                  15

Glu Ala Ala Glu
             20


<210> 11
<211> 30
<212> DNA

<213> Artificial Sequence

<220>
<223> Description of Artificial Sequence: primer GAG5

<400> 11
aagaattcca tgggtgcgag agcgtcggta                                        30

<210> 12
<211> 30
<212> DNA
<213> Artificial Sequence

<220>
<223> Description of Artificial Sequence: primer
      p55-SAL3

<400> 12
attcgtcgac tgtgacgagg ggtcgttgcc                                        30

<210> 13
<211> 34
<212> DNA
<213> Artificial Sequence

<220>
<223> Description of Artificial Sequence: primer
      CORESAL5

<400> 13
atttgtcgac gaatcctaaa cctcaaagaa aaac                                   34

<210> 14
<211> 30
<212> DNA
<213> Artificial Sequence

<220>
<223> Description of Artificial Sequence: primer 173CORE

<400> 14
tattggatcc taagagcaac caggaaggtt c                                      31

<210> 15
<211> 21
<212> DNA
<213> Artificial Sequence

<220>
<223> Description of Artificial Sequence: primer MS65

<400> 15
cgaccatcat ggatgcagcg c                                                 21

<210> 16
<211> 30
<212> DNA
<213> Artificial Sequence

<220>
<223> Description of Artificial Sequence: primer MS66

<400> 16
aggattcgtc gagtcgctgc tggggtcgtt                                        30

<210> 17
<211> 26
<212> DNA
<213> Artificial Sequence

<220>
<223> Description of Artificial Sequence: primer XPANXNF

<400> 17
gcacgtgggc ccggcgcctc tagagc                                            26

<210> 18
<211> 26
<212> DNA
<213> Artificial Sequence

<220>
<223> Description of Artificial Sequence: primer XPANXNR

<400> 18
gctctagagg cgccgggccc acgtgc                                            26

<210> 19
<211> 20
<212> PRT
<213> Artificial Sequence

<220>
<223> Description of Artificial Sequence: HIV p55 Gag
      Major Homology Region

<400> 19
Asp Ile Arg Gln Gly Pro Lys Glu Pro Phe Arg Asp Tyr Val Asp Arg
  1               5                  10                  15

Phe Tyr Lys Thr
             20


<210> 20
<211> 60
<212> DNA
<213> Artificial Sequence

<220>
<223> Description of Artificial Sequence: synthetic p55
      Gag Major Homology Region

<400> 20
gacatccgcc agggccccaa ggagcccttc cgcgactacg tggaccgctt ctacaagacc 60

<210> 21
<211> 15

<212> PRT
<213> Human immunodeficiency virus

<400> 21
Ala Pro Thr Lys Ala Lys Arg Arg Val Val Gln Arg Glu Lys Arg
1               5                   10                  15


<210> 22
<211> 5
<212> PRT
<213> Human immunodeficiency virus

<400> 22
Lys Ala Lys Arg Arg
1               5


<210> 23
<211> 4
<212> PRT
<213> Human immunodeficiency virus

<400> 23
Arg Glu Lys Arg
1


<210> 24
<211> 15
<212> PRT
<213> Artificial Sequence

<220>
<223> Description of Artificial Sequence: aa of
      mut7.SF162 cleavage site

<400> 24
Ala Pro Thr Lys Ala Ile Ser Ser Val Val Gln Ser Glu Lys Ser
1               5                   10                  15


<210> 25
<211> 15
<212> PRT
<213> Artificial Sequence

<220>
<223> Description of Artificial Sequence: aa of
      mut8.SF162 cleavage site

<400> 25
Ala Pro Thr Ile Ala Ile Ser Ser Val Val Gln Ser Glu Lys Ser
1               5                   10                  15


<210> 26
<211> 15
<212> PRT

<213> Artificial Sequence

<220>
<223> Description of Artificial Sequence: aa of
      mut.SF162 cleavage site

<400> 26
Ala Pro Thr Lys Ala Lys Arg Arg Val Val Gln Arg Glu Lys Ser
  1               5                  10                  15


<210> 27
<211> 15
<212> PRT
<213> Human immunodeficiency virus

<220>
<223> Description of Artificial Sequence: aa of native
      cleavage site in US4

<400> 27
Ala Pro Thr Gln Ala Lys Arg Arg Val Val Gln Arg Glu Lys Arg
  1               5                  10                  15


<210> 28
<211> 5
<212> PRT
<213> Human immunodeficiency virus

<220>
<223> Description of Artificial Sequence: aa of second
      cleavage site in US4

<400> 28
Gln Ala Lys Arg Arg
  1               5


<210> 29
<211> 15
<212> PRT
<213> Artificial Sequence

<220>
<223> Description of Artificial Sequence: aa of mut.US4
      cleavage site

<400> 29
Ala Pro Thr Gln Ala Lys Arg Arg Val Val Gln Arg Glu Lys Ser
  1               5                  10                  15


<210> 30
<211> 1419
<212> DNA
<213> Human immunodeficiency virus

<400> 30

gtagaaaaat tgtgggtcac agtctattat ggggtacctg tgtggaaaga agcaaccacc 60
actctatttt gtgcatcaga tgctaaagcc tatgacacag aggtacataa tgtctgggcc 120
acacatgcct gtgtacccac agaccctaac ccacaagaaa tagtattgga aaatgtgaca 180
gaaaatttta acatgtggaa aaataacatg gtagaacaga tgcatgagga tataatcagt 240
ttatgggatc aaagtctaaa gccatgtgta aagttaaccc cactctgtgt tactctacat 300
tgcactaatt tgaagaatgc tactaatacc aagagtagta attggaaaga gatggacaga 360
ggagaaataa aaaattgctc tttcaaggtc accacaagca taagaaataa gatgcagaaa 420
gaatatgcac ttttttataa acttgatgta gtaccaatag ataatgataa tacaagctat 480
aaattgataa attgtaacac ctcagtcatt acacaggcct gtccaaaggt atcctttgaa 540
ccaattccca tacattattg tgccccggct ggttttgcga ttctaaagtg taatgataag 600
aagttcaatg gatcaggacc atgtacaaat gtcagcacag tacaatgtac acatggaatt 660
aggccagtag tgtcaactca attgctgtta aatggcagtc tagcagaaga aggggtagta 720
attagatctg aaaatttcac agacaatgct aaaactataa tagtacagct gaaggaatct 780
gtagaaatta attgtacaag acctaacaat aatacaagaa aaagtataac tataggaccg 840
gggagagcat tttatgcaac aggagacata ataggagata taagacaagc acattgtaac 900
attagtggag aaaaatggaa taacacttta aaacagatag ttacaaaatt acaagcacaa 960
tttgggaata aaacaatagt ctttaagcaa tcctcaggag gggacccaga aattgtaatg 1020
cacagtttta attgtggagg ggaatttttc tactgtaatt caacacagct ttttaatagt 1080
acttggaata atactatagg gccaaataac actaatggaa ctatcacact cccatgcaga 1140
ataaaacaaa ttataaacag gtggcaggaa gtaggaaaag caatgtatgc ccctcccatc 1200
agaggacaaa ttagatgctc atcaaatatt acaggactgc tattaacaag agatggtggt 1260
aaagagatca gtaacaccac cgagatcttc agacctggag gtggagatat gagggacaat 1320
tggagaagtg aattatataa atataaagta gtaaaaattg agccattagg agtagcaccc 1380
accaaggcaa agagaagagt ggtgcagaga gaaaaaaga 1419

<210> 31
<211> 1932
<212> DNA
<213> Human immunodeficiency virus

<400> 31
gtagaaaaat tgtgggtcac agtctattat ggggtacctg tgtggaaaga agcaaccacc 60
actctatttt gtgcatcaga tgctaaagcc tatgacacag aggtacataa tgtctgggcc 120
acacatgcct gtgtacccac agaccctaac ccacaagaaa tagtattgga aaatgtgaca 180
gaaaatttta acatgtggaa aaataacatg gtagaacaga tgcatgagga tataatcagt 240
ttatgggatc aaagtctaaa gccatgtgta aagttaaccc cactctgtgt tactctacat 300
tgcactaatt tgaagaatgc tactaatacc aagagtagta attggaaaga gatggacaga 360
ggagaaataa aaaattgctc tttcaaggtc accacaagca taagaaataa gatgcagaaa 420
gaatatgcac ttttttataa acttgatgta gtaccaatag ataatgataa tacaagctat 480
aaattgataa attgtaacac ctcagtcatt acacaggcct gtccaaaggt atcctttgaa 540
ccaattccca tacattattg tgccccggct ggttttgcga ttctaaagtg taatgataag 600
aagttcaatg gatcaggacc atgtacaaat gtcagcacag tacaatgtac acatggaatt 660
aggccagtag tgtcaactca attgctgtta aatggcagtc tagcagaaga aggggtagta 720
attagatctg aaaatttcac agacaatgct aaaactataa tagtacagct gaaggaatct 780
gtagaaatta attgtacaag acctaacaat aatacaagaa aaagtataac tataggaccg 840
gggagagcat tttatgcaac aggagacata ataggagata taagacaagc acattgtaac 900
attagtggag aaaaatggaa taacacttta aaacagatag ttacaaaatt acaagcacaa 960
tttgggaata aaacaatagt ctttaagcaa tcctcaggag gggacccaga aattgtaatg 1020
cacagtttta attgtggagg ggaatttttc tactgtaatt caacacagct ttttaatagt 1080
acttggaata atactatagg gccaaataac actaatggaa ctatcacact cccatgcaga 1140
ataaaacaaa ttataaacag gtggcaggaa gtaggaaaag caatgtatgc ccctcccatc 1200
agaggacaaa ttagatgctc atcaaatatt acaggactgc tattaacaag agatggtggt 1260
aaagagatca gtaacaccac cgagatcttc agacctggag gtggagatat gagggacaat 1320
tggagaagtg aattatataa atataaagta gtaaaaattg agccattagg agtagcaccc 1380
accaaggcaa agagaagagt ggtgcagaga gaaaaaagag cagtgacgct aggagctatg 1440
ttccttgggt tcttgggagc agcaggaagc actatgggcg cacggtcact gacgctgacg 1500
gtacaggcca gacaattatt gtctggtata gtgcaacagc agaacaattt gctgagagct 1560
attgaggcgc aacagcatct gttgcaactc acagtctggg gcatcaagca gctccaggca 1620
agagtcctgg ctgtggaaag atacctaaag gatcaacagc tcctagggat ttggggttgc 1680

tctggaaaac tcatttgcac cactgctgtg ccttggaatg ctagttggag taataaatct 1740
ctggatcaga tttggaataa catgacctgg atggagtggg agagagaaat tgacaattac 1800
acaaacttaa tatacacctt aattgaagaa tcgcagaacc aacaagaaaa gaatgaacaa 1860
gaattattag aattggataa gtgggcaagt ttgtggaatt ggtttgacat atcaaaatgg 1920
ctgtggtata ta 1932

<210> 32
<211> 2457
<212> DNA
<213> Human immunodeficiency virus

<400> 32
gtagaaaaat tgtgggtcac agtctattat ggggtacctg tgtggaaaga agcaaccacc 60
actctatttt gtgcatcaga tgctaaagcc tatgacacag aggtacataa tgtctgggcc 120
acacatgcct gtgtacccac agaccctaac ccacaagaaa tagtattgga aaatgtgaca 180
gaaaatttta acatgtggaa aaataacatg gtagaacaga tgcatgagga tataatcagt 240
ttatgggatc aaagtctaaa gccatgtgta aagttaaccc cactctgtgt tactctacat 300
tgcactaatt tgaagaatgc tactaatacc aagagtagta attggaaaga gatggacaga 360
ggagaaataa aaaattgctc tttcaaggtc accacaagca taagaaataa gatgcagaaa 420
gaatatgcac ttttttataa acttgatgta gtaccaatag ataatgataa tacaagctat 480
aaattgataa attgtaacac ctcagtcatt acacaggcct gtccaaaggt atcctttgaa 540
ccaattccca tacattattg tgccccggct ggttttgcga ttctaaagtg taatgataag 600
aagttcaatg gatcaggacc atgtacaaat gtcagcacag tacaatgtac acatggaatt 660
aggccagtag tgtcaactca attgctgtta aatggcagtc tagcagaaga aggggtagta 720
attagatctg aaaatttcac agacaatgct aaaactataa tagtacagct gaaggaatct 780
gtagaaatta attgtacaag acctaacaat aatacaagaa aaagtataac tataggaccg 840
gggagagcat tttatgcaac aggagacata ataggagata taagacaagc acattgtaac 900
attagtggag aaaaatggaa taacacttta aaacagatag ttacaaaatt acaagcacaa 960
tttgggaata aaacaatagt ctttaagcaa tcctcaggag gggacccaga aattgtaatg 1020
cacagtttta attgtggagg ggaatttttc tactgtaatt caacacagct ttttaatagt 1080
acttggaata atactatagg gccaaataac actaatggaa ctatcacact cccatgcaga 1140
ataaaacaaa ttataaacag gtggcaggaa gtaggaaaag caatgtatgc ccctcccatc 1200
agaggacaaa ttagatgctc atcaaatatt acaggactgc tattaacaag agatggtggt 1260
aaagagatca gtaacaccac cgagatcttc agacctggag gtggagatat gagggacaat 1320
tggagaagtg aattatataa atataaagta gtaaaaattg agccattagg agtagcaccc 1380
accaaggcaa agagaagagt ggtgcagaga gaaaaaagag cagtgacgct aggagctatg 1440
ttccttgggt tcttgggagc agcaggaagc actatgggcg cacggtcact gacgctgacg 1500
gtacaggcca gacaattatt gtctggtata gtgcaacagc agaacaattt gctgagagct 1560
attgaggcgc aacagcatct gttgcaactc acagtctggg gcatcaagca gctccaggca 1620
agagtcctgg ctgtggaaag atacctaaag gatcaacagc tcctagggat ttggggttgc 1680
tctggaaaac tcatttgcac cactgctgtg ccttggaatg ctagttggag taataaatct 1740
ctggatcaga tttggaataa catgacctgg atggagtggg agagagaaat tgacaattac 1800
acaaacttaa tatacacctt aattgaagaa tcgcagaacc aacaagaaaa gaatgaacaa 1860
gaattattag aattggataa gtgggcaagt ttgtggaatt ggtttgacat atcaaaatgg 1920
ctgtggtata taaaaatatt cataatgata gtaggaggtt tagtaggttt aaggatagtt 1980
tttactgtgc tttctatagt gaatagagtt aggcagggat actcaccatt atcatttcag 2040
acccgcttcc cagccccaag gggacccgac aggcccgaag gaatcgaaga agaaggtgga 2100
gagagagaca gagacagatc cagtccatta gtgcatggat tattagcact catctgggac 2160
gatctacgga gcctgtgcct cttcagctac caccgcttga gagacttaat cttgattgca 2220
gcgaggattg tggaacttct gggacgcagg gggtgggaag ccctcaagta ttgggggaat 2280
ctcctgcagt attggattca ggaactaaag aatagtgctg ttagtttgtt tgatgccata 2340
gctatagcag tagctgaggg gacagatagg attatagaag tagcacaaag aattggtaga 2400
gcttttctcc acatacctag aagaataaga cagggctttg aaagggcttt gctataa 2457

<210> 33
<211> 1453
<212> DNA
<213> Artificial Sequence

<220>
<223> Description of Artificial Sequence: gp120.modSF162

<400> 33
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgaccacc 480
agcatccgca acaagatgca gaaggagtac gccctgttct acaagctgga cgtggtgccc 540
atcgacaacg acaacaccag ctacaagctg atcaactgca acaccagcgt gatcacccag 600
gcctgcccca aggtgagctt cgagcccatc cccatccact actgcgcccc cgccggcttc 660
gccatcctga agtgcaacga caagaagttc aacggcagcg gcccctgcac caacgtgagc 720
accgtgcagt gcacccacgg catccgcccc gtggtgagca cccagctgct gctgaacggc 780
agcctggccg aggagggcgt ggtgatccgc agcgagaact tcaccgacaa cgccaagacc 840
atcatcgtgc agctgaagga gagcgtggag atcaactgca cccgccccaa caacaacacc 900
cgcaagagca tcaccatcgg ccccggccgc gccttctacg ccaccggcga catcatcggc 960
gacatccgcc aggcccactg caacatcagc ggcgagaagt ggaacaacac cctgaagcag 1020
atcgtgacca agctgcaggc ccagttcggc aacaagacca tcgtgttcaa gcagagcagc 1080
ggcggcgacc ccgagatcgt gatgcacagc ttcaactgcg gcggcgagtt cttctactgc 1140
aacagcaccc agctgttcaa cagcacctgg aacaacacca tcggccccaa caacaccaac 1200
ggcaccatca ccctgccctg ccgcatcaag cagatcatca accgctggca ggaggtgggc 1260
aaggccatgt acgccccccc catccgcggc cagatccgct gcagcagcaa catcaccggc 1320
ctgctgctga cccgcgacgg cggcaaggag atcagcaaca ccaccgagat cttccgcccc 1380
ggcggcggcg acatgcgcga caactggcgc agcgagctgt acaagtacaa ggtggtgaag 1440
atcgagcccc tgg 1453

<210> 34
<211> 1387
<212> DNA
<213> Artificial Sequence

<220>
<223> Description of Artificial Sequence:
      gp120.modSF162.delV2

<400> 34
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgggcgcc 480
ggcaagctga tcaactgcaa caccagcgtg atcacccagg cctgccccaa ggtgagcttc 540
gagcccatcc ccatccacta ctgcgccccc gccggcttcg ccatcctgaa gtgcaacgac 600
aagaagttca acggcagcgg cccctgcacc aacgtgagca ccgtgcagtg cacccacggc 660
atccgccccg tggtgagcac ccagctgctg ctgaacggca gcctggccga ggagggcgtg 720
gtgatccgca gcgagaactt caccgacaac gccaagacca tcatcgtgca gctgaaggag 780
agcgtggaga tcaactgcac ccgccccaac aacaacaccc gcaagagcat caccatcggc 840
cccggccgcg ccttctacgc caccggcgac atcatcggcg acatccgcca ggcccactgc 900
aacatcagcg gcgagaagtg gaacaacacc ctgaagcaga tcgtgaccaa gctgcaggcc 960
cagttcggca acaagaccat cgtgttcaag cagagcagcg gcggcgaccc cgagatcgtg 1020
atgcacagct tcaactgcgg cggcgagttc ttctactgca acagcaccca gctgttcaac 1080
agcacctgga acaacaccat cggccccaac aacaccaacg gcaccatcac cctgccctgc 1140

cgcatcaagc agatcatcaa ccgctggcag gaggtgggca aggccatgta cgcccccccc 1200
atccgcggcc agatccgctg cagcagcaac atcaccggcc tgctgctgac ccgcgacggc 1260
ggcaaggaga tcagcaacac caccgagatc ttccgccccg gcggcggcga catgcgcgac 1320
aactggcgca gcgagctgta caagtacaag gtggtgaaga tcgagcccct gggcgtggcc 1380
cccacca 1387

<210> 35
<211> 1323
<212> DNA
<213> Artificial Sequence

<220>
<223> Description of Artificial Sequence:
      gp120.modSF162.delV1V2

<400> 35
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgggcgc cggcaactgc cagaccagcg tgatcaccca ggcctgcccc 420
aaggtgagct tcgagcccat ccccatccac tactgcgccc ccgccggctt cgccatcctg 480
aagtgcaacg acaagaagtt caacggcagc ggcccctgca ccaacgtgag caccgtgcag 540
tgcacccacg gcatccgccc cgtggtgagc acccagctgc tgctgaacgg cagcctggcc 600
gaggagggcg tggtgatccg cagcgagaac ttcaccgaca acgccaagac catcatcgtg 660
cagctgaagg agagcgtgga gatcaactgc acccgcccca acaacaacac ccgcaagagc 720
atcaccatcg gccccggccg cgccttctac gccaccggcg acatcatcgg cgacatccgc 780
caggcccact gcaacatcag cggcgagaag tggaacaaca ccctgaagca gatcgtgacc 840
aagctgcagg cccagttcgg caacaagacc atcgtgttca agcagagcag cggcggcgac 900
cccgagatcg tgatgcacag cttcaactgc ggcggcgagt tcttctactg caacagcacc 960
cagctgttca acagcacctg gaacaacacc atcggcccca acaacaccaa cggcaccatc 1020
accctgccct gccgcatcaa gcagatcatc aaccgctggc aggaggtggg caaggccatg 1080
tacgcccccc ccatccgcgg ccagatccgc tgcagcagca acatcaccgg cctgctgctg 1140
acccgcgacg gcggcaagga gatcagcaac accaccgaga tcttccgccc cggcggcggc 1200
gacatgcgcg acaactggcg cagcgagctg tacaagtaca aggtggtgaa gatcgagccc 1260
ctgggcgtgg cccccaccaa ggccaagcgc cgcgtggtgc agcgcgagaa gcgctaactc 1320
gag 1323

<210> 36
<211> 2025
<212> DNA
<213> Artificial Sequence

<220>
<223> Description of Artificial Sequence: gp140.modSF162

<400> 36
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgaccacc 480
agcatccgca acaagatgca gaaggagtac gccctgttct acaagctgga cgtggtgccc 540
atcgacaacg acaacaccag ctacaagctg atcaactgca acaccagcgt gatcacccag 600

gcctgcccca aggtgagctt cgagcccatc cccatccact actgcgcccc cgccggcttc 660
gccatcctga agtgcaacga caagaagttc aacggcagcg gcccctgcac caacgtgagc 720
accgtgcagt gcacccacgg catccgcccc gtggtgagca cccagctgct gctgaacggc 780
agcctggccg aggagggcgt ggtgatccgc agcgagaact tcaccgacaa cgccaagacc 840
atcatcgtgc agctgaagga gagcgtggag atcaactgca cccgccccaa caacaacacc 900
cgcaagagca tcaccatcgg ccccggccgc gccttctacg ccaccggcga catcatcggc 960
gacatccgcc aggcccactg caacatcagc ggcgagaagt ggaacaacac cctgaagcag 1020
atcgtgacca agctgcaggc ccagttcggc aacaagacca tcgtgttcaa gcagagcagc 1080
ggcggcgacc ccgagatcgt gatgcacagc ttcaactgcg gcggcgagtt cttctactgc 1140
aacagcaccc agctgttcaa cagcacctgg aacaacacca tcggccccaa caacaccaac 1200
ggcaccatca ccctgccctg ccgcatcaag cagatcatca accgctggca ggaggtgggc 1260
aaggccatgt acgccccccc catccgcggc cagatccgct gcagcagcaa catcaccggc 1320
ctgctgctga cccgcgacgg cggcaaggag atcagcaaca ccaccgagat cttccgcccc 1380
ggcggcggcg acatgcgcga caactggcgc agcgagctgt acaagtacaa ggtggtgaag 1440
atcgagcccc tgggcgtggc ccccaccaag gccaagcgcc gcgtggtgca gcgcgagaag 1500
cgcgccgtga ccctgggcgc catgttcctg ggcttcctgg gcgccgccgg cagcaccatg 1560
ggcgcccgca gcctgaccct gaccgtgcag gcccgccagc tgctgagcgg catcgtgcag 1620
cagcagaaca acctgctgcg cgccatcgag gcccagcagc acctgctgca gctgaccgtg 1680
tggggcatca agcagctgca ggcccgcgtg ctggccgtgg agcgctacct gaaggaccag 1740
cagctgctgg gcatctgggg ctgcagcggc aagctgatct gcaccaccgc cgtgccctgg 1800
aacgccagct ggagcaacaa gagcctggac cagatctgga acaacatgac ctggatggag 1860
tgggagcgcg agatcgacaa ctacaccaac ctgatctaca ccctgatcga ggagagccag 1920
aaccagcagg agaagaacga gcaggagctg ctggagctgg acaagtgggc cagcctgtgg 1980
aactggttcg acatcagcaa gtggctgtgg tacatctaac tcgag 2025

<210> 37
<211> 1944
<212> DNA
<213> Artificial Sequence

<220>
<223> Description of Artificial Sequence:
      gp140.modSF162.delV2

<400> 37
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgggcgcc 480
ggcaagctga tcaactgcaa caccagcgtg atcacccagg cctgccccaa ggtgagcttc 540
gagcccatcc ccatccacta ctgcgccccc gccggcttcg ccatcctgaa gtgcaacgac 600
aagaagttca acggcagcgg cccctgcacc aacgtgagca ccgtgcagtg cacccacggc 660
atccgccccg tggtgagcac ccagctgctg ctgaacggca gcctggccga ggagggcgtg 720
gtgatccgca gcgagaactt caccgacaac gccaagacca tcatcgtgca gctgaaggag 780
agcgtggaga tcaactgcac ccgccccaac aacaacaccc gcaagagcat caccatcggc 840
cccggccgcg ccttctacgc caccggcgac atcatcggcg acatccgcca ggcccactgc 900
aacatcagcg gcgagaagtg gaacaacacc ctgaagcaga tcgtgaccaa gctgcaggcc 960
cagttcggca acaagaccat cgtgttcaag cagagcagcg gcggcgaccc cgagatcgtg 1020
atgcacagct tcaactgcgg cggcgagttc ttctactgca acagcaccca gctgttcaac 1080
agcacctgga acaacaccat cggccccaac aacaccaacg gcaccatcac cctgccctgc 1140
cgcatcaagc agatcatcaa ccgctggcag gaggtgggca aggccatgta cgcccccccc 1200
atccgcggcc agatccgctg cagcagcaac atcaccggcc tgctgctgac ccgcgacggc 1260
ggcaaggaga tcagcaacac caccgagatc ttccgccccg gcggcggcga catgcgcgac 1320
aactggcgca gcgagctgta caagtacaag gtggtgaaga tcgagcccct gggcgtggcc 1380
cccaccaagg ccaagcgccg cgtggtgcag cgcgagaagc gcgccgtgac cctgggcgcc 1440

atgttcctgg gcttcctggg cgccgccggc agcaccatgg gcgcccgcag cctgaccctg 1500
accgtgcagg cccgccagct gctgagcggc atcgtgcagc agcagaacaa cctgctgcgc 1560
gccatcgagg cccagcagca cctgctgcag ctgaccgtgt ggggcatcaa gcagctgcag 1620
gcccgcgtgc tggccgtgga gcgctacctg aaggaccagc agctgctggg catctggggc 1680
tgcagcggca agctgatctg caccaccgcc gtgccctgga acgccagctg gagcaacaag 1740
agcctggacc agatctggaa caacatgacc tggatggagt gggagcgcga gatcgacaac 1800
tacaccaacc tgatctacac cctgatcgag gagagccaga accagcagga gaagaacgag 1860
caggagctgc tggagctgga caagtgggcc agcctgtgga actggttcga catcagcaag 1920
tggctgtggt acatctaact cgag 1944

<210> 38
<211> 1944
<212> DNA
<213> Artificial Sequence

<220>
<223> Description of Artificial Sequence:
      gp140.modSF162.delV1/V2

<400> 38
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgggcgcc 480
ggcaagctga tcaactgcaa caccagcgtg atcacccagg cctgccccaa ggtgagcttc 540
gagcccatcc ccatccacta ctgcgccccc gccggcttcg ccatcctgaa gtgcaacgac 600
aagaagttca acggcagcgg cccctgcacc aacgtgagca ccgtgcagtg cacccacggc 660
atccgccccg tggtgagcac ccagctgctg ctgaacggca gcctggccga ggagggcgtg 720
gtgatccgca gcgagaactt caccgacaac gccaagacca tcatcgtgca gctgaaggag 780
agcgtggaga tcaactgcac ccgccccaac aacaacaccc gcaagagcat caccatcggc 840
cccggccgcg ccttctacgc caccggcgac atcatcggcg acatccgcca ggcccactgc 900
aacatcagcg gcgagaagtg gaacaacacc ctgaagcaga tcgtgaccaa gctgcaggcc 960
cagttcggca acaagaccat cgtgttcaag cagagcagcg gcggcgaccc cgagatcgtg 1020
atgcacagct tcaactgcgg cggcgagttc ttctactgca acagcaccca gctgttcaac 1080
agcacctgga acaacaccat cggccccaac aacaccaacg gcaccatcac cctgccctgc 1140
cgcatcaagc agatcatcaa ccgctggcag gaggtgggca aggccatgta cgcccccccc 1200
atccgcggcc agatccgctg cagcagcaac atcaccggcc tgctgctgac ccgcgacggc 1260
ggcaaggaga tcagcaacac caccgagatc ttccgccccg gcggcggcga catgcgcgac 1320
aactggcgca gcgagctgta caagtacaag gtggtgaaga tcgagcccct gggcgtggcc 1380
cccaccaagg ccaagcgccg cgtggtgcag cgcgagaagc gcgccgtgac cctgggcgcc 1440
atgttcctgg gcttcctggg cgccgccggc agcaccatgg gcgcccgcag cctgaccctg 1500
accgtgcagg cccgccagct gctgagcggc atcgtgcagc agcagaacaa cctgctgcgc 1560
gccatcgagg cccagcagca cctgctgcag ctgaccgtgt ggggcatcaa gcagctgcag 1620
gcccgcgtgc tggccgtgga gcgctacctg aaggaccagc agctgctggg catctggggc 1680
tgcagcggca agctgatctg caccaccgcc gtgccctgga acgccagctg gagcaacaag 1740
agcctggacc agatctggaa caacatgacc tggatggagt gggagcgcga gatcgacaac 1800
tacaccaacc tgatctacac cctgatcgag gagagccaga accagcagga gaagaacgag 1860
caggagctgc tggagctgga caagtgggcc agcctgtgga actggttcga catcagcaag 1920
tggctgtggt acatctaact cgag 1944

<210> 39
<211> 2025
<212> DNA
<213> Artificial Sequence

<220>
<223> Description of Artificial Sequence:
      gp140.mut.modSF162

<400> 39
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgaccacc 480
agcatccgca acaagatgca gaaggagtac gccctgttct acaagctgga cgtggtgccc 540
atcgacaacg acaacaccag ctacaagctg atcaactgca acaccagcgt gatcacccag 600
gcctgcccca aggtgagctt cgagcccatc cccatccact actgcgcccc cgccggcttc 660
gccatcctga agtgcaacga caagaagttc aacggcagcg gcccctgcac caacgtgagc 720
accgtgcagt gcacccacgg catccgcccc gtggtgagca cccagctgct gctgaacggc 780
agcctggccg aggagggcgt ggtgatccgc agcgagaact tcaccgacaa cgccaagacc 840
atcatcgtgc agctgaagga gagcgtggag atcaactgca cccgccccaa caacaacacc 900
cgcaagagca tcaccatcgg ccccggccgc gccttctacg ccaccggcga catcatcggc 960
gacatccgcc aggcccactg caacatcagc ggcgagaagt ggaacaacac cctgaagcag 1020
atcgtgacca agctgcaggc ccagttcggc aacaagacca tcgtgttcaa gcagagcagc 1080
ggcggcgacc ccgagatcgt gatgcacagc ttcaactgcg gcggcgagtt cttctactgc 1140
aacagcaccc agctgttcaa cagcacctgg aacaacacca tcggccccaa caacaccaac 1200
ggcaccatca ccctgccctg ccgcatcaag cagatcatca accgctggca ggaggtgggc 1260
aaggccatgt acgccccccc catccgcggc cagatccgct gcagcagcaa catcaccggc 1320
ctgctgctga cccgcgacgg cggcaaggag atcagcaaca ccaccgagat cttccgcccc 1380
ggcggcggcg acatgcgcga caactggcgc agcgagctgt acaagtacaa ggtggtgaag 1440
atcgagcccc tgggcgtggc ccccaccaag gccaagcgcc gcgtggtgca gcgcgagaag 1500
agcgccgtga ccctgggcgc catgttcctg ggcttcctgg gcgccgccgg cagcaccatg 1560
ggcgcccgca gcctgaccct gaccgtgcag gcccgccagc tgctgagcgg catcgtgcag 1620
cagcagaaca acctgctgcg cgccatcgag gcccagcagc acctgctgca gctgaccgtg 1680
tggggcatca agcagctgca ggcccgcgtg ctggccgtgg agcgctacct gaaggaccag 1740
cagctgctgg gcatctgggg ctgcagcggc aagctgatct gcaccaccgc cgtgccctgg 1800
aacgccagct ggagcaacaa gagcctggac cagatctgga acaacatgac ctggatggag 1860
tgggagcgcg agatcgacaa ctacaccaac ctgatctaca ccctgatcga ggagagccag 1920
aaccagcagg agaagaacga gcaggagctg ctggagctgg acaagtgggc cagcctgtgg 1980
aactggttcg acatcagcaa gtggctgtgg tacatctaac tcgag 2025

<210> 40
<211> 1944
<212> DNA
<213> Artificial Sequence

<220>
<223> Description of Artificial Sequence:
      gp140.mut.modSF162.delV2

<400> 40
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgggcgcc 480
ggcaagctga tcaactgcaa caccagcgtg atcacccagg cctgccccaa ggtgagcttc 540

gagcccatcc ccatccacta ctgcgccccc gccggcttcg ccatcctgaa gtgcaacgac 600
aagaagttca acggcagcgg cccctgcacc aacgtgagca ccgtgcagtg cacccacggc 660
atccgccccg tggtgagcac ccagctgctg ctgaacggca gcctggccga ggagggcgtg 720
gtgatccgca gcgagaactt caccgacaac gccaagacca tcatcgtgca gctgaaggag 780
agcgtggaga tcaactgcac ccgccccaac aacaacaccc gcaagagcat caccatcggc 840
cccggccgcg ccttctacgc caccggcgac atcatcggcg acatccgcca ggcccactgc 900
aacatcagcg gcgagaagtg gaacaacacc ctgaagcaga tcgtgaccaa gctgcaggcc 960
cagttcggca acaagaccat cgtgttcaag cagagcagcg gcggcgaccc cgagatcgtg 1020
atgcacagct tcaactgcgg cggcgagttc ttctactgca acagcaccca gctgttcaac 1080
agcacctgga acaacaccat cggccccaac aacaccaacg gcaccatcac cctgccctgc 1140
cgcatcaagc agatcatcaa ccgctggcag gaggtgggca aggccatgta cgcccccccc 1200
atccgcggcc agatccgctg cagcagcaac atcaccggcc tgctgctgac ccgcgacggc 1260
ggcaaggaga tcagcaacac caccgagatc ttccgccccg gcggcggcga catgcgcgac 1320
aactggcgca gcgagctgta caagtacaag gtggtgaaga tcgagcccct gggcgtggcc 1380
cccaccaagg ccaagcgccg cgtggtgcag cgcgagaaga gcgccgtgac cctgggcgcc 1440
atgttcctgg gcttcctggg cgccgccggc agcaccatgg gcgcccgcag cctgaccctg 1500
accgtgcagg cccgccagct gctgagcggc atcgtgcagc agcagaacaa cctgctgcgc 1560
gccatcgagg cccagcagca cctgctgcag ctgaccgtgt ggggcatcaa gcagctgcag 1620
gcccgcgtgc tggccgtgga gcgctacctg aaggaccagc agctgctggg catctggggc 1680
tgcagcggca agctgatctg caccaccgcc gtgccctgga acgccagctg gagcaacaag 1740
agcctggacc agatctggaa caacatgacc tggatggagt gggagcgcga gatcgacaac 1800
tacaccaacc tgatctacac cctgatcgag gagagccaga accagcagga gaagaacgag 1860
caggagctgc tggagctgga caagtgggcc agcctgtgga actggttcga catcagcaag 1920
tggctgtggt acatctaact cgag 1944

<210> 41
<211> 1836
<212> DNA
<213> Artificial Sequence

<220>
<223> Description of Artificial Sequence:
      gp140.mut.modSF162.delV1/V2

<400> 41
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgggcgc cggcaactgc cagaccagcg tgatcaccca ggcctgcccc 420
aaggtgagct tcgagcccat ccccatccac tactgcgccc ccgccggctt cgccatcctg 480
aagtgcaacg acaagaagtt caacggcagc ggcccctgca ccaacgtgag caccgtgcag 540
tgcacccacg gcatccgccc cgtggtgagc acccagctgc tgctgaacgg cagcctggcc 600
gaggagggcg tggtgatccg cagcgagaac ttcaccgaca acgccaagac catcatcgtg 660
cagctgaagg agagcgtgga gatcaactgc acccgcccca acaacaacac ccgcaagagc 720
atcaccatcg gccccggccg cgccttctac gccaccggcg acatcatcgg cgacatccgc 780
caggcccact gcaacatcag cggcgagaag tggaacaaca ccctgaagca gatcgtgacc 840
aagctgcagg cccagttcgg caacaagacc atcgtgttca agcagagcag cggcggcgac 900
cccgagatcg tgatgcacag cttcaactgc ggcggcgagt tcttctactg caacagcacc 960
cagctgttca acagcacctg gaacaacacc atcggcccca acaacaccaa cggcaccatc 1020
accctgccct gccgcatcaa gcagatcatc aaccgctggc aggaggtggg caaggccatg 1080
tacgcccccc ccatccgcgg ccagatccgc tgcagcagca acatcaccgg cctgctgctg 1140
acccgcgacg gcggcaagga gatcagcaac accaccgaga tcttccgccc cggcggcggc 1200
gacatgcgcg acaactggcg cagcgagctg tacaagtaca aggtggtgaa gatcgagccc 1260
ctgggcgtgg cccccaccaa ggccaagcgc cgcgtggtgc agcgcgagaa gagcgccgtg 1320
accctgggcg ccatgttcct gggcttcctg ggcgccgccg gcagcaccat gggcgcccgc 1380
agcctgaccc tgaccgtgca ggcccgccag ctgctgagcg gcatcgtgca gcagcagaac 1440

aacctgctgc gcgccatcga ggcccagcag cacctgctgc agctgaccgt gtggggcatc 1500
aagcagctgc aggcccgcgt gctggccgtg gagcgctacc tgaaggacca gcagctgctg 1560
ggcatctggg gctgcagcgg caagctgatc tgcaccaccg ccgtgccctg gaacgccagc 1620
tggagcaaca agagcctgga ccagatctgg aacaacatga cctggatgga gtgggagcgc 1680
gagatcgaca actacaccaa cctgatctac accctgatcg aggagagcca gaaccagcag 1740
gagaagaacg agcaggagct gctggagctg gacaagtggg ccagcctgtg gaactggttc 1800
gacatcagca agtggctgtg gtacatctaa ctcgag 1836

<210> 42
<211> 2025
<212> DNA
<213> Artificial Sequence

<220>
<223> Description of Artificial Sequence:
      gp140.mut7.modSF162

<400> 42
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgaccacc 480
agcatccgca acaagatgca gaaggagtac gccctgttct acaagctgga cgtggtgccc 540
atcgacaacg acaacaccag ctacaagctg atcaactgca acaccagcgt gatcacccag 600
gcctgcccca aggtgagctt cgagcccatc cccatccact actgcgcccc cgccggcttc 660
gccatcctga agtgcaacga caagaagttc aacggcagcg gcccctgcac caacgtgagc 720
accgtgcagt gcacccacgg catccgcccc gtggtgagca cccagctgct gctgaacggc 780
agcctggccg aggagggcgt ggtgatccgc agcgagaact tcaccgacaa cgccaagacc 840
atcatcgtgc agctgaagga gagcgtggag atcaactgca cccgccccaa caacaacacc 900
cgcaagagca tcaccatcgg ccccggccgc gccttctacg ccaccggcga catcatcggc 960
gacatccgcc aggcccactg caacatcagc ggcgagaagt ggaacaacac cctgaagcag 1020
atcgtgacca agctgcaggc ccagttcggc aacaagacca tcgtgttcaa gcagagcagc 1080
ggcggcgacc ccgagatcgt gatgcacagc ttcaactgcg gcggcgagtt cttctactgc 1140
aacagcaccc agctgttcaa cagcacctgg aacaacacca tcggccccaa caacaccaac 1200
ggcaccatca ccctgccctg ccgcatcaag cagatcatca accgctggca ggaggtgggc 1260
aaggccatgt acgccccccc catccgcggc cagatccgct gcagcagcaa catcaccggc 1320
ctgctgctga cccgcgacgg cggcaaggag atcagcaaca ccaccgagat cttccgcccc 1380
ggcggcggcg acatgcgcga caactggcgc agcgagctgt acaagtacaa ggtggtgaag 1440
atcgagcccc tgggcgtggc ccccaccaag gccatcagca gcgtggtgca gagcgagaag 1500
agcgccgtga ccctgggcgc catgttcctg ggcttcctgg gcgccgccgg cagcaccatg 1560
ggcgcccgca gcctgaccct gaccgtgcag gcccgccagc tgctgagcgg catcgtgcag 1620
cagcagaaca acctgctgcg cgccatcgag gcccagcagc acctgctgca gctgaccgtg 1680
tggggcatca agcagctgca ggcccgcgtg ctggccgtgg agcgctacct gaaggaccag 1740
cagctgctgg gcatctgggg ctgcagcggc aagctgatct gcaccaccgc cgtgccctgg 1800
aacgccagct ggagcaacaa gagcctggac cagatctgga acaacatgac ctggatggag 1860
tgggagcgcg agatcgacaa ctacaccaac ctgatctaca ccctgatcga ggagagccag 1920
aaccagcagg agaagaacga gcaggagctg ctggagctgg acaagtgggc cagcctgtgg 1980
aactggttcg acatcagcaa gtggctgtgg tacatctaac tcgag 2025

<210> 43
<211> 1944
<212> DNA
<213> Artificial Sequence

<220>

<223> Description of Artificial Sequence:
      gp140.mut7.modSF162.delV2

<400> 43
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgggcgcc 480
ggcaagctga tcaactgcaa caccagcgtg atcacccagg cctgccccaa ggtgagcttc 540
gagcccatcc ccatccacta ctgcgccccc gccggcttcg ccatcctgaa gtgcaacgac 600
aagaagttca acggcagcgg cccctgcacc aacgtgagca ccgtgcagtg cacccacggc 660
atccgccccg tggtgagcac ccagctgctg ctgaacggca gcctggccga ggagggcgtg 720
gtgatccgca gcgagaactt caccgacaac gccaagacca tcatcgtgca gctgaaggag 780
agcgtggaga tcaactgcac ccgccccaac aacaacaccc gcaagagcat caccatcggc 840
cccggccgcg ccttctacgc caccggcgac atcatcggcg acatccgcca ggcccactgc 900
aacatcagcg gcgagaagtg gaacaacacc ctgaagcaga tcgtgaccaa gctgcaggcc 960
cagttcggca acaagaccat cgtgttcaag cagagcagcg gcggcgaccc cgagatcgtg 1020
atgcacagct tcaactgcgg cggcgagttc ttctactgca acagcaccca gctgttcaac 1080
agcacctgga acaacaccat cggccccaac aacaccaacg gcaccatcac cctgccctgc 1140
cgcatcaagc agatcatcaa ccgctggcag gaggtgggca aggccatgta cgcccccccc 1200
atccgcggcc agatccgctg cagcagcaac atcaccggcc tgctgctgac ccgcgacggc 1260
ggcaaggaga tcagcaacac caccgagatc ttccgccccg gcggcggcga catgcgcgac 1320
aactggcgca gcgagctgta caagtacaag gtggtgaaga tcgagcccct gggcgtggcc 1380
cccaccaagg ccatcagcag cgtggtgcag agcgagaaga gcgccgtgac cctgggcgcc 1440
atgttcctgg gcttcctggg cgccgccggc agcaccatgg gcgcccgcag cctgaccctg 1500
accgtgcagg cccgccagct gctgagcggc atcgtgcagc agcagaacaa cctgctgcgc 1560
gccatcgagg cccagcagca cctgctgcag ctgaccgtgt ggggcatcaa gcagctgcag 1620
gcccgcgtgc tggccgtgga gcgctacctg aaggaccagc agctgctggg catctggggc 1680
tgcagcggca agctgatctg caccaccgcc gtgccctgga acgccagctg gagcaacaag 1740
agcctggacc agatctggaa caacatgacc tggatggagt gggagcgcga gatcgacaac 1800
tacaccaacc tgatctacac cctgatcgag gagagccaga accagcagga gaagaacgag 1860
caggagctgc tggagctgga caagtgggcc agcctgtgga actggttcga catcagcaag 1920
tggctgtggt acatctaact cgag 1944

<210> 44
<211> 1836
<212> DNA
<213> Artificial Sequence

<220>
<223> Description of Artificial Sequence:
      gp140.mut7.modSF162.delV1/V2

<400> 44
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgggcgc cggcaactgc cagaccagcg tgatcaccca ggcctgcccc 420
aaggtgagct tcgagcccat ccccatccac tactgcgccc ccgccggctt cgccatcctg 480
aagtgcaacg acaagaagtt caacggcagc ggcccctgca ccaacgtgag caccgtgcag 540
tgcacccacg gcatccgccc cgtggtgagc acccagctgc tgctgaacgg cagcctggcc 600
gaggagggcg tggtgatccg cagcgagaac ttcaccgaca acgccaagac catcatcgtg 660

cagctgaagg agagcgtgga gatcaactgc acccgcccca acaacaacac ccgcaagagc 720
atcaccatcg gccccggccg cgccttctac gccaccggcg acatcatcgg cgacatccgc 780
caggcccact gcaacatcag cggcgagaag tggaacaaca ccctgaagca gatcgtgacc 840
aagctgcagg cccagttcgg caacaagacc atcgtgttca agcagagcag cggcggcgac 900
cccgagatcg tgatgcacag cttcaactgc ggcggcgagt tcttctactg caacagcacc 960
cagctgttca acagcacctg gaacaacacc atcggcccca acaacaccaa cggcaccatc 1020
accctgccct gccgcatcaa gcagatcatc aaccgctggc aggaggtggg caaggccatg 1080
tacgcccccc ccatccgcgg ccagatccgc tgcagcagca acatcaccgg cctgctgctg 1140
acccgcgacg gcggcaagga gatcagcaac accaccgaga tcttccgccc cggcggcggc 1200
gacatgcgcg acaactggcg cagcgagctg tacaagtaca aggtggtgaa gatcgagccc 1260
ctgggcgtgg cccccaccaa ggccatcagc agcgtggtgc agagcgagaa gagcgccgtg 1320
accctgggcg ccatgttcct gggcttcctg ggcgccgccg gcagcaccat gggcgcccgc 1380
agcctgaccc tgaccgtgca ggcccgccag ctgctgagcg gcatcgtgca gcagcagaac 1440
aacctgctgc gcgccatcga ggcccagcag cacctgctgc agctgaccgt gtggggcatc 1500
aagcagctgc aggcccgcgt gctggccgtg gagcgctacc tgaaggacca gcagctgctg 1560
ggcatctggg gctgcagcgg caagctgatc tgcaccaccg ccgtgccctg gaacgccagc 1620
tggagcaaca agagcctgga ccagatctgg aacaacatga cctggatgga gtgggagcgc 1680
gagatcgaca actacaccaa cctgatctac accctgatcg aggagagcca gaaccagcag 1740
gagaagaacg agcaggagct gctggagctg gacaagtggg ccagcctgtg gaactggttc 1800
gacatcagca agtggctgtg gtacatctaa ctcgag 1836

<210> 45
<211> 2025
<212> DNA
<213> Artificial Sequence

<220>
<223> Description of Artificial Sequence:
      gp140.mut8.modSF162

<400> 45
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgaccacc 480
agcatccgca acaagatgca gaaggagtac gccctgttct acaagctgga cgtggtgccc 540
atcgacaacg acaacaccag ctacaagctg atcaactgca acaccagcgt gatcacccag 600
gcctgcccca aggtgagctt cgagcccatc cccatccact actgcgcccc cgccggcttc 660
gccatcctga agtgcaacga caagaagttc aacggcagcg gcccctgcac caacgtgagc 720
accgtgcagt gcacccacgg catccgcccc gtggtgagca cccagctgct gctgaacggc 780
agcctggccg aggagggcgt ggtgatccgc agcgagaact tcaccgacaa cgccaagacc 840
atcatcgtgc agctgaagga gagcgtggag atcaactgca cccgccccaa caacaacacc 900
cgcaagagca tcaccatcgg ccccggccgc gccttctacg ccaccggcga catcatcggc 960
gacatccgcc aggcccactg caacatcagc ggcgagaagt ggaacaacac cctgaagcag 1020
atcgtgacca agctgcaggc ccagttcggc aacaagacca tcgtgttcaa gcagagcagc 1080
ggcggcgacc ccgagatcgt gatgcacagc ttcaactgcg gcggcgagtt cttctactgc 1140
aacagcaccc agctgttcaa cagcacctgg aacaacacca tcggccccaa caacaccaac 1200
ggcaccatca ccctgccctg ccgcatcaag cagatcatca accgctggca ggaggtgggc 1260
aaggccatgt acgccccccc catccgcggc cagatccgct gcagcagcaa catcaccggc 1320
ctgctgctga cccgcgacgg cggcaaggag atcagcaaca ccaccgagat cttccgcccc 1380
ggcggcggcg acatgcgcga caactggcgc agcgagctgt acaagtacaa ggtggtgaag 1440
atcgagcccc tgggcgtggc ccccaccaag gccatcagca gcgtggtgca gagcgagaag 1500
agcgccgtga ccctgggcgc catgttcctg ggcttcctgg gcgccgccgg cagcaccatg 1560
ggcgcccgca gcctgaccct gaccgtgcag gcccgccagc tgctgagcgg catcgtgcag 1620
cagcagaaca acctgctgcg cgccatcgag gcccagcagc acctgctgca gctgaccgtg 1680

tggggcatca agcagctgca ggcccgcgtg ctggccgtgg agcgctacct gaaggaccag 1740
cagctgctgg gcatctgggg ctgcagcggc aagctgatct gcaccaccgc cgtgccctgg 1800
aacgccagct ggagcaacaa gagcctggac cagatctgga acaacatgac ctggatggag 1860
tgggagcgcg agatcgacaa ctacaccaac ctgatctaca ccctgatcga ggagagccag 1920
aaccagcagg agaagaacga gcaggagctg ctggagctgg acaagtgggc cagcctgtgg 1980
aactggttcg acatcagcaa gtggctgtgg tacatctaac tcgag 2025

<210> 46
<211> 1944
<212> DNA
<213> Artificial Sequence

<220>
<223> Description of Artificial Sequence:
      gp140.mut8.modSF162.delV2

<400> 46
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgggcgcc 480
ggcaagctga tcaactgcaa caccagcgtg atcacccagg cctgccccaa ggtgagcttc 540
gagcccatcc ccatccacta ctgcgccccc gccggcttcg ccatcctgaa gtgcaacgac 600
aagaagttca acggcagcgg cccctgcacc aacgtgagca ccgtgcagtg cacccacggc 660
atccgccccg tggtgagcac ccagctgctg ctgaacggca gcctggccga ggagggcgtg 720
gtgatccgca gcgagaactt caccgacaac gccaagacca tcatcgtgca gctgaaggag 780
agcgtggaga tcaactgcac ccgccccaac aacaacaccc gcaagagcat caccatcggc 840
cccggccgcg ccttctacgc caccggcgac atcatcggcg acatccgcca ggcccactgc 900
aacatcagcg gcgagaagtg gaacaacacc ctgaagcaga tcgtgaccaa gctgcaggcc 960
cagttcggca acaagaccat cgtgttcaag cagagcagcg gcggcgaccc cgagatcgtg 1020
atgcacagct tcaactgcgg cggcgagttc ttctactgca acagcaccca gctgttcaac 1080
agcacctgga acaacaccat cggccccaac aacaccaacg gcaccatcac cctgccctgc 1140
cgcatcaagc agatcatcaa ccgctggcag gaggtgggca aggccatgta cgcccccccc 1200
atccgcggcc agatccgctg cagcagcaac atcaccggcc tgctgctgac ccgcgacggc 1260
ggcaaggaga tcagcaacac caccgagatc ttccgccccg gcggcggcga catgcgcgac 1320
aactggcgca gcgagctgta caagtacaag gtggtgaaga tcgagcccct gggcgtggcc 1380
cccaccatcg ccatcagcag cgtggtgcag agcgagaaga gcgccgtgac cctgggcgcc 1440
atgttcctgg gcttcctggg cgccgccggc agcaccatgg gcgcccgcag cctgaccctg 1500
accgtgcagg cccgccagct gctgagcggc atcgtgcagc agcagaacaa cctgctgcgc 1560
gccatcgagg cccagcagca cctgctgcag ctgaccgtgt ggggcatcaa gcagctgcag 1620
gcccgcgtgc tggccgtgga gcgctacctg aaggaccagc agctgctggg catctggggc 1680
tgcagcggca agctgatctg caccaccgcc gtgccctgga acgccagctg gagcaacaag 1740
agcctggacc agatctggaa caacatgacc tggatggagt gggagcgcga gatcgacaac 1800
tacaccaacc tgatctacac cctgatcgag gagagccaga accagcagga gaagaacgag 1860
caggagctgc tggagctgga caagtgggcc agcctgtgga actggttcga catcagcaag 1920
tggctgtggt acatctaact cgag 1944

<210> 47
<211> 1836
<212> DNA
<213> Artificial Sequence

<220>
<223> Description of Artificial Sequence:
      gp140.mut8.modSF162.delV1/V2

<400> 47
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgggcgc cggcaactgc cagaccagcg tgatcaccca ggcctgcccc 420
aaggtgagct tcgagcccat ccccatccac tactgcgccc ccgccggctt cgccatcctg 480
aagtgcaacg acaagaagtt caacggcagc ggcccctgca ccaacgtgag caccgtgcag 540
tgcacccacg gcatccgccc cgtggtgagc acccagctgc tgctgaacgg cagcctggcc 600
gaggagggcg tggtgatccg cagcgagaac ttcaccgaca acgccaagac catcatcgtg 660
cagctgaagg agagcgtgga gatcaactgc acccgcccca acaacaacac ccgcaagagc 720
atcaccatcg gccccggccg cgccttctac gccaccggcg acatcatcgg cgacatccgc 780
caggcccact gcaacatcag cggcgagaag tggaacaaca ccctgaagca gatcgtgacc 840
aagctgcagg cccagttcgg caacaagacc atcgtgttca agcagagcag cggcggcgac 900
cccgagatcg tgatgcacag cttcaactgc ggcggcgagt tcttctactg caacagcacc 960
cagctgttca acagcacctg gaacaacacc atcggcccca acaacaccaa cggcaccatc 1020
accctgccct gccgcatcaa gcagatcatc aaccgctggc aggaggtggg caaggccatg 1080
tacgcccccc ccatccgcgg ccagatccgc tgcagcagca acatcaccgg cctgctgctg 1140
acccgcgacg gcggcaagga gatcagcaac accaccgaga tcttccgccc cggcggcggc 1200
gacatgcgcg acaactggcg cagcgagctg tacaagtaca aggtggtgaa gatcgagccc 1260
ctgggcgtgg cccccaccat cgccatcagc agcgtggtgc agagcgagaa gagcgccgtg 1320
accctgggcg ccatgttcct gggcttcctg ggcgccgccg gcagcaccat gggcgcccgc 1380
agcctgaccc tgaccgtgca ggcccgccag ctgctgagcg gcatcgtgca gcagcagaac 1440
aacctgctgc gcgccatcga ggcccagcag cacctgctgc agctgaccgt gtggggcatc 1500
aagcagctgc aggcccgcgt gctggccgtg gagcgctacc tgaaggacca gcagctgctg 1560
ggcatctggg gctgcagcgg caagctgatc tgcaccaccg ccgtgccctg gaacgccagc 1620
tggagcaaca agagcctgga ccagatctgg aacaacatga cctggatgga gtgggagcgc 1680
gagatcgaca actacaccaa cctgatctac accctgatcg aggagagcca gaaccagcag 1740
gagaagaacg agcaggagct gctggagctg gacaagtggg ccagcctgtg gaactggttc 1800
gacatcagca agtggctgtg gtacatctaa ctcgag 1836

<210> 48
<211> 2547
<212> DNA
<213> Artificial Sequence

<220>
<223> Description of Artificial Sequence: gp160.modSF162

<400> 48
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgaccacc 480
agcatccgca acaagatgca gaaggagtac gccctgttct acaagctgga cgtggtgccc 540
atcgacaacg acaacaccag ctacaagctg atcaactgca acaccagcgt gatcacccag 600
gcctgcccca aggtgagctt cgagcccatc cccatccact actgcgcccc cgccggcttc 660
gccatcctga agtgcaacga caagaagttc aacggcagcg gcccctgcac caacgtgagc 720
accgtgcagt gcacccacgg catccgcccc gtggtgagca cccagctgct gctgaacggc 780
agcctggccg aggagggcgt ggtgatccgc agcgagaact tcaccgacaa cgccaagacc 840
atcatcgtgc agctgaagga gagcgtggag atcaactgca cccgccccaa caacaacacc 900
cgcaagagca tcaccatcgg ccccggccgc gccttctacg ccaccggcga catcatcggc 960
gacatccgcc aggcccactg caacatcagc ggcgagaagt ggaacaacac cctgaagcag 1020

atcgtgacca agctgcaggc ccagttcggc aacaagacca tcgtgttcaa gcagagcagc 1080
ggcggcgacc ccgagatcgt gatgcacagc ttcaactgcg gcggcgagtt cttctactgc 1140
aacagcaccc agctgttcaa cagcacctgg aacaacacca tcggccccaa caacaccaac 1200
ggcaccatca ccctgccctg ccgcatcaag cagatcatca accgctggca ggaggtgggc 1260
aaggccatgt acgccccccc catccgcggc cagatccgct gcagcagcaa catcaccggc 1320
ctgctgctga cccgcgacgg cggcaaggag atcagcaaca ccaccgagat cttccgcccc 1380
ggcggcggcg acatgcgcga caactggcgc agcgagctgt acaagtacaa ggtggtgaag 1440
atcgagcccc tgggcgtggc ccccaccaag gccaagcgcc gcgtggtgca gcgcgagaag 1500
cgcgccgtga ccctgggcgc catgttcctg ggcttcctgg gcgccgccgg cagcaccatg 1560
ggcgcccgca gcctgaccct gaccgtgcag gcccgccagc tgctgagcgg catcgtgcag 1620
cagcagaaca acctgctgcg cgccatcgag gcccagcagc acctgctgca gctgaccgtg 1680
tggggcatca agcagctgca ggcccgcgtg ctggccgtgg agcgctacct gaaggaccag 1740
cagctgctgg gcatctgggg ctgcagcggc aagctgatct gcaccaccgc cgtgccctgg 1800
aacgccagct ggagcaacaa gagcctggac cagatctgga acaacatgac ctggatggag 1860
tgggagcgcg agatcgacaa ctacaccaac ctgatctaca ccctgatcga ggagagccag 1920
aaccagcagg agaagaacga gcaggagctg ctggagctgg acaagtgggc cagcctgtgg 1980
aactggttcg acatcagcaa gtggctgtgg tacatcaaga tcttcatcat gatcgtgggc 2040
ggcctggtgg gcctgcgcat cgtgttcacc gtgctgagca tcgtgaaccg cgtgcgccag 2100
ggctacagcc ccctgagctt ccagacccgc ttccccgccc cccgcggccc cgaccgcccc 2160
gagggcatcg aggaggaggg cggcgagcgc gaccgcgacc gcagcagccc cctggtgcac 2220
ggcctgctgg ccctgatctg ggacgacctg cgcagcctgt gcctgttcag ctaccaccgc 2280
ctgcgcgacc tgatcctgat cgccgcccgc atcgtggagc tgctgggccg ccgcggctgg 2340
gaggccctga agtactgggg caacctgctg cagtactgga tccaggagct gaagaacagc 2400
gccgtgagcc tgttcgacgc catcgccatc gccgtggccg agggcaccga ccgcatcatc 2460
gaggtggccc agcgcatcgg ccgcgccttc ctgcacatcc cccgccgcat ccgccagggc 2520
ttcgagcgcg ccctgctgta actcgag 2547

<210> 49
<211> 2466
<212> DNA
<213> Artificial Sequence

<220>
<223> Description of Artificial Sequence:
      gp160.modSF162.delV2

<400> 49
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgggcgcc 480
ggcaagctga tcaactgcaa caccagcgtg atcacccagg cctgccccaa ggtgagcttc 540
gagcccatcc ccatccacta ctgcgccccc gccggcttcg ccatcctgaa gtgcaacgac 600
aagaagttca acggcagcgg cccctgcacc aacgtgagca ccgtgcagtg cacccacggc 660
atccgccccg tggtgagcac ccagctgctg ctgaacggca gcctggccga ggagggcgtg 720
gtgatccgca gcgagaactt caccgacaac gccaagacca tcatcgtgca gctgaaggag 780
agcgtggaga tcaactgcac ccgccccaac aacaacaccc gcaagagcat caccatcggc 840
cccggccgcg ccttctacgc caccggcgac atcatcggcg acatccgcca ggcccactgc 900
aacatcagcg gcgagaagtg gaacaacacc ctgaagcaga tcgtgaccaa gctgcaggcc 960
cagttcggca acaagaccat cgtgttcaag cagagcagcg gcggcgaccc cgagatcgtg 1020
atgcacagct tcaactgcgg cggcgagttc ttctactgca acagcaccca gctgttcaac 1080
agcacctgga acaacaccat cggccccaac aacaccaacg gcaccatcac cctgccctgc 1140
cgcatcaagc agatcatcaa ccgctggcag gaggtgggca aggccatgta cgcccccccc 1200
atccgcggcc agatccgctg cagcagcaac atcaccggcc tgctgctgac ccgcgacggc 1260
ggcaaggaga tcagcaacac caccgagatc ttccgccccg gcggcggcga catgcgcgac 1320

aactggcgca gcgagctgta caagtacaag gtggtgaaga tcgagcccct gggcgtggcc 1380
cccaccaagg ccaagcgccg cgtggtgcag cgcgagaagc gcgccgtgac cctgggcgcc 1440
atgttcctgg gcttcctggg cgccgccggc agcaccatgg gcgcccgcag cctgaccctg 1500
accgtgcagg cccgccagct gctgagcggc atcgtgcagc agcagaacaa cctgctgcgc 1560
gccatcgagg cccagcagca cctgctgcag ctgaccgtgt ggggcatcaa gcagctgcag 1620
gcccgcgtgc tggccgtgga gcgctacctg aaggaccagc agctgctggg catctggggc 1680
tgcagcggca agctgatctg caccaccgcc gtgccctgga acgccagctg gagcaacaag 1740
agcctggacc agatctggaa caacatgacc tggatggagt gggagcgcga gatcgacaac 1800
tacaccaacc tgatctacac cctgatcgag gagagccaga accagcagga gaagaacgag 1860
caggagctgc tggagctgga caagtgggcc agcctgtgga actggttcga catcagcaag 1920
tggctgtggt acatcaagat cttcatcatg atcgtgggcg gcctggtggg cctgcgcatc 1980
gtgttcaccg tgctgagcat cgtgaaccgc gtgcgccagg gctacagccc cctgagcttc 2040
cagacccgct tccccgcccc ccgcggcccc gaccgccccg agggcatcga ggaggagggc 2100
ggcgagcgcg accgcgaccg cagcagcccc ctggtgcacg gcctgctggc cctgatctgg 2160
gacgacctgc gcagcctgtg cctgttcagc taccaccgcc tgcgcgacct gatcctgatc 2220
gccgcccgca tcgtggagct gctgggccgc cgcggctggg aggccctgaa gtactggggc 2280
aacctgctgc agtactggat ccaggagctg aagaacagcg ccgtgagcct gttcgacgcc 2340
atcgccatcg ccgtggccga gggcaccgac cgcatcatcg aggtggccca gcgcatcggc 2400
cgcgccttcc tgcacatccc ccgccgcatc cgccagggct tcgagcgcgc cctgctgtaa 2460
ctcgag 2466

<210> 50
<211> 2358
<212> DNA
<213> Artificial Sequence

<220>
<223> Description of Artificial Sequence:
      gp160.modSF162.delV1/V2

<400> 50
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgggcgc cggcaactgc cagaccagcg tgatcaccca ggcctgcccc 420
aaggtgagct tcgagcccat ccccatccac tactgcgccc ccgccggctt cgccatcctg 480
aagtgcaacg acaagaagtt caacggcagc ggcccctgca ccaacgtgag caccgtgcag 540
tgcacccacg gcatccgccc cgtggtgagc acccagctgc tgctgaacgg cagcctggcc 600
gaggagggcg tggtgatccg cagcgagaac ttcaccgaca acgccaagac catcatcgtg 660
cagctgaagg agagcgtgga gatcaactgc acccgcccca acaacaacac ccgcaagagc 720
atcaccatcg gccccggccg cgccttctac gccaccggcg acatcatcgg cgacatccgc 780
caggcccact gcaacatcag cggcgagaag tggaacaaca ccctgaagca gatcgtgacc 840
aagctgcagg cccagttcgg caacaagacc atcgtgttca agcagagcag cggcggcgac 900
cccgagatcg tgatgcacag cttcaactgc ggcggcgagt tcttctactg caacagcacc 960
cagctgttca acagcacctg gaacaacacc atcggcccca acaacaccaa cggcaccatc 1020
accctgccct gccgcatcaa gcagatcatc aaccgctggc aggaggtggg caaggccatg 1080
tacgcccccc ccatccgcgg ccagatccgc tgcagcagca acatcaccgg cctgctgctg 1140
acccgcgacg gcggcaagga gatcagcaac accaccgaga tcttccgccc cggcggcggc 1200
gacatgcgcg acaactggcg cagcgagctg tacaagtaca aggtggtgaa gatcgagccc 1260
ctgggcgtgg cccccaccaa ggccaagcgc cgcgtggtgc agcgcgagaa gcgcgccgtg 1320
accctgggcg ccatgttcct gggcttcctg ggcgccgccg gcagcaccat gggcgcccgc 1380
agcctgaccc tgaccgtgca ggcccgccag ctgctgagcg gcatcgtgca gcagcagaac 1440
aacctgctgc gcgccatcga ggcccagcag cacctgctgc agctgaccgt gtggggcatc 1500
aagcagctgc aggcccgcgt gctggccgtg gagcgctacc tgaaggacca gcagctgctg 1560
ggcatctggg gctgcagcgg caagctgatc tgcaccaccg ccgtgccctg gaacgccagc 1620
tggagcaaca agagcctgga ccagatctgg aacaacatga cctggatgga gtgggagcgc 1680

gagatcgaca actacaccaa cctgatctac accctgatcg aggagagcca gaaccagcag 1740
gagaagaacg agcaggagct gctggagctg gacaagtggg ccagcctgtg gaactggttc 1800
gacatcagca agtggctgtg gtacatcaag atcttcatca tgatcgtggg cggcctggtg 1860
ggcctgcgca tcgtgttcac cgtgctgagc atcgtgaacc gcgtgcgcca gggctacagc 1920
cccctgagct tccagacccg cttccccgcc ccccgcggcc ccgaccgccc cgagggcatc 1980
gaggaggagg gcggcgagcg cgaccgcgac cgcagcagcc ccctggtgca cggcctgctg 2040
gccctgatct gggacgacct gcgcagcctg tgcctgttca gctaccaccg cctgcgcgac 2100
ctgatcctga tcgccgcccg catcgtggag ctgctgggcc gccgcggctg ggaggccctg 2160
aagtactggg gcaacctgct gcagtactgg atccaggagc tgaagaacag cgccgtgagc 2220
ctgttcgacg ccatcgccat cgccgtggcc gagggcaccg accgcatcat cgaggtggcc 2280
cagcgcatcg gccgcgcctt cctgcacatc ccccgccgca tccgccaggg cttcgagcgc 2340
gccctgctgt aactcgag 2358

<210> 51
<211> 1494
<212> DNA
<213> Human immunodeficiency virus

<400> 51
acaacagtct tgtgggtcac agtctattat ggggtacctg tgtggaaaga agcaaccacc 60
actctgtttt gtgcatcaga tgctaaagca tacaaagcag aggcacataa cgtctgggct 120
acacatgcct gtgtacccac agaccccaac ccacaggaag taaatttaac aaatgtgaca 180
gaaaatttta acatgtggaa aaataacatg gtggaacaga tgcatgagga tataatcagt 240
ttatgggatc aaagcctaaa gccatgtgta aaattaaccc cactctgtgt tactttaaat 300
tgtactgata agttgacagg tagtactaat ggcacaaata gtactagtgg cactaatagt 360
actagtggca ctaatagtac tagtactaat agtactgata gttgggaaaa gatgccagaa 420
ggagaaataa aaaactgctc tttcaatatc accacaagtg taagagataa agtgcagaaa 480
gaatattctc tcttctataa acttgatgta gtaccaatag ataatgataa tgctagctat 540
agattgataa attgtaatac ctcagtcatt acacaagcct gtccaaaggt atcttttgaa 600
ccaattccca tacattattg tgccccggct ggttttgcga ttctaaagtg taaagataag 660
aagttcaatg gaacaggacc atgtaaaaat gtcagcacag tacaatgcac acatggaatt 720
agaccagtag tatcaactca actgctgtta aatggcagtc tagcagaaga agagatagta 780
cttagatctg aaaatttcac agacaatgct aaaaccataa tagtacagct gaatgaatct 840
gtagaaatta attgtataag acccaacaat aatacaagaa aaagtataca tataggacca 900
gggagagcat tttatgcaac aggtgatata ataggagaca taagacaagc acattgtaac 960
attagtaaag caaactggac taacacttta gaacagatag ttgaaaaatt aagagaacaa 1020
tttgggaata ataaaacaat aatctttaat tcatcctcag gaggggaccc agaaattgta 1080
tttcacagtt ttaattgtgg aggggaattt ttctattgta atacatcaca actatttaat 1140
agtacctgga atattactga agaggtaaat aagactaaag aaaatgacac tatcatactc 1200
ccatgcagaa taagacaaat tataaacatg tggcaagaag taggaaaagc aatgtatgcc 1260
cctcccatca gaggacaaat taaatgttca tcaaatatta cagggctgct attaactaga 1320
gatggtggta ctaacaataa taggacgaac gacaccgaga ccttcagacc tgggggagga 1380
aacatgaagg acaattggag aagtgaatta tataaatata aagtagtaag aattgaacca 1440
ttaggagtag cacccaccca ggcaaagaga agagtggtgc aaagagagaa aaga 1494

<210> 52
<211> 2007
<212> DNA
<213> Human immunodeficiency virus

<400> 52
acaacagtct tgtgggtcac agtctattat ggggtacctg tgtggaaaga agcaaccacc 60
actctgtttt gtgcatcaga tgctaaagca tacaaagcag aggcacataa cgtctgggct 120
acacatgcct gtgtacccac agaccccaac ccacaggaag taaatttaac aaatgtgaca 180
gaaaatttta acatgtggaa aaataacatg gtggaacaga tgcatgagga tataatcagt 240
ttatgggatc aaagcctaaa gccatgtgta aaattaaccc cactctgtgt tactttaaat 300
tgtactgata agttgacagg tagtactaat ggcacaaata gtactagtgg cactaatagt 360
actagtggca ctaatagtac tagtactaat agtactgata gttgggaaaa gatgccagaa 420
ggagaaataa aaaactgctc tttcaatatc accacaagtg taagagataa agtgcagaaa 480

gaatattctc tcttctataa acttgatgta gtaccaatag ataatgataa tgctagctat 540
agattgataa attgtaatac ctcagtcatt acacaagcct gtccaaaggt atcttttgaa 600
ccaattccca tacattattg tgccccggct ggttttgcga ttctaaagtg taaagataag 660
aagttcaatg gaacaggacc atgtaaaaat gtcagcacag tacaatgcac acatggaatt 720
agaccagtag tatcaactca actgctgtta aatggcagtc tagcagaaga agagatagta 780
cttagatctg aaaatttcac agacaatgct aaaaccataa tagtacagct gaatgaatct 840
gtagaaatta attgtataag acccaacaat aatacaagaa aaagtataca tataggacca 900
gggagagcat tttatgcaac aggtgatata ataggagaca taagacaagc acattgtaac 960
attagtaaag caaactggac taacacttta gaacagatag ttgaaaaatt aagagaacaa 1020
tttgggaata ataaaacaat aatctttaat tcatcctcag gaggggaccc agaaattgta 1080
tttcacagtt ttaattgtgg aggggaattt ttctattgta atacatcaca actatttaat 1140
agtacctgga atattactga agaggtaaat aagactaaag aaaatgacac tatcatactc 1200
ccatgcagaa taagacaaat tataaacatg tggcaagaag taggaaaagc aatgtatgcc 1260
cctcccatca gaggacaaat taaatgttca tcaaatatta cagggctgct attaactaga 1320
gatggtggta ctaacaataa taggacgaac gacaccgaga ccttcagacc tgggggagga 1380
aacatgaagg acaattggag aagtgaatta tataaatata aagtagtaag aattgaacca 1440
ttaggagtag cacccaccca ggcaaagaga agagtggtgc aaagagagaa aagagcagtg 1500
ggactaggag ctttgttcat tgggttcttg ggagcagcag gaagcactat gggcgcagcg 1560
tcagtgacgc tgacggtaca ggccagacaa ttattgtctg gtatagtgca acagcagaac 1620
aatttgctga gagctattga ggcgcaacag catctgttgc aactcacggt ctggggcatc 1680
aaacagctcc aggcaagaat cctggctgtg gaaagatacc taaaggatca acagctccta 1740
gggatttggg gttgctctgg aaaactcatt tgcaccacta ctgtgccttg gaactctagt 1800
tggagtaata aatctctgac tgagatttgg gataatatga cctggatgga gtgggaaaga 1860
gaaattggca attatacagg cttaatatac aatttaattg aaatagcaca aaaccagcaa 1920
gaaaagaatg aacaagaatt attggaatta gacaagtggg caagtttgtg gaattggttt 1980
gatataacaa actggctgtg gtatata 2007

<210> 53
<211> 2532
<212> DNA
<213> Human immunodeficiency virus

<400> 53
acaacagtct tgtgggtcac agtctattat ggggtacctg tgtggaaaga agcaaccacc 60
actctgtttt gtgcatcaga tgctaaagca tacaaagcag aggcacataa cgtctgggct 120
acacatgcct gtgtacccac agaccccaac ccacaggaag taaatttaac aaatgtgaca 180
gaaaatttta acatgtggaa aaataacatg gtggaacaga tgcatgagga tataatcagt 240
ttatgggatc aaagcctaaa gccatgtgta aaattaaccc cactctgtgt tactttaaat 300
tgtactgata agttgacagg tagtactaat ggcacaaata gtactagtgg cactaatagt 360
actagtggca ctaatagtac tagtactaat agtactgata gttgggaaaa gatgccagaa 420
ggagaaataa aaaactgctc tttcaatatc accacaagtg taagagataa agtgcagaaa 480
gaatattctc tcttctataa acttgatgta gtaccaatag ataatgataa tgctagctat 540
agattgataa attgtaatac ctcagtcatt acacaagcct gtccaaaggt atcttttgaa 600
ccaattccca tacattattg tgccccggct ggttttgcga ttctaaagtg taaagataag 660
aagttcaatg gaacaggacc atgtaaaaat gtcagcacag tacaatgcac acatggaatt 720
agaccagtag tatcaactca actgctgtta aatggcagtc tagcagaaga agagatagta 780
cttagatctg aaaatttcac agacaatgct aaaaccataa tagtacagct gaatgaatct 840
gtagaaatta attgtataag acccaacaat aatacaagaa aaagtataca tataggacca 900
gggagagcat tttatgcaac aggtgatata ataggagaca taagacaagc acattgtaac 960
attagtaaag caaactggac taacacttta gaacagatag ttgaaaaatt aagagaacaa 1020
tttgggaata ataaaacaat aatctttaat tcatcctcag gaggggaccc agaaattgta 1080
tttcacagtt ttaattgtgg aggggaattt ttctattgta atacatcaca actatttaat 1140
agtacctgga atattactga agaggtaaat aagactaaag aaaatgacac tatcatactc 1200
ccatgcagaa taagacaaat tataaacatg tggcaagaag taggaaaagc aatgtatgcc 1260
cctcccatca gaggacaaat taaatgttca tcaaatatta cagggctgct attaactaga 1320
gatggtggta ctaacaataa taggacgaac gacaccgaga ccttcagacc tgggggagga 1380
aacatgaagg acaattggag aagtgaatta tataaatata aagtagtaag aattgaacca 1440
ttaggagtag cacccaccca ggcaaagaga agagtggtgc aaagagagaa aagagcagtg 1500
ggactaggag ctttgttcat tgggttcttg ggagcagcag gaagcactat gggcgcagcg 1560

tcagtgacgc tgacggtaca ggccagacaa ttattgtctg gtatagtgca acagcagaac 1620
aatttgctga gagctattga ggcgcaacag catctgttgc aactcacggt ctggggcatc 1680
aaacagctcc aggcaagaat cctggctgtg gaaagatacc taaaggatca acagctccta 1740
gggatttggg gttgctctgg aaaactcatt tgcaccacta ctgtgccttg gaactctagt 1800
tggagtaata aatctctgac tgagatttgg gataatatga cctggatgga gtgggaaaga 1860
gaaattggca attatacagg cttaatatac aatttaattg aaatagcaca aaaccagcaa 1920
gaaaagaatg aacaagaatt attggaatta gacaagtggg caagtttgtg gaattggttt 1980
gatataacaa actggctgtg gtatataaga atattcataa tgatagtagg aggcttgata 2040
ggtttaagaa tagtttttgc tgtactttct atagtgaata gagttaggca gggatactca 2100
ccaatatcat tgcagacccg cctcccagct cagaggggac ccgacaggcc cgaaggaatc 2160
gaagaagaag gtggagagag agacagagac agatccaatc gattagtgca tggattattg 2220
gcactcatct gggacgatct gcggagcctg tgcctcttca gctaccaccg cttgagagac 2280
ttactcttga ttgtagcgag gattgtggaa cttctgggac gcagggggtg ggaagccctc 2340
aagtattggt ggaatctcct gcagtattgg agtcaggagc taaagagtag tgctgttagt 2400
ttgtttaatg ccacagcaat agcagtagct gaagggacag ataggattat agaaatagta 2460
caaagaattt ttagagctgt aattcacata cctagaagaa taagacaggg cttggagagg 2520
gctttactat aa 2532

<210> 54
<211> 1599
<212> DNA
<213> Artificial Sequence

<220>
<223> Description of Artificial Sequence: gp120.modUS4

<400> 54
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgaagctg 360
acccccctgt gcgtgaccct gaactgcacc gacaagctga ccggcagcac caacggcacc 420
aacagcacca gcggcaccaa cagcaccagc ggcaccaaca gcaccagcac caacagcacc 480
gacagctggg agaagatgcc cgagggcgag atcaagaact gcagcttcaa catcaccacc 540
agcgtgcgcg acaaggtgca gaaggagtac agcctgttct acaagctgga cgtggtgccc 600
atcgacaacg acaacgccag ctaccgcctg atcaactgca acaccagcgt gatcacccag 660
gcctgcccca aggtgagctt cgagcccatc cccatccact actgcgcccc cgccggcttc 720
gccatcctga agtgcaagga caagaagttc aacggcaccg gcccctgcaa gaacgtgagc 780
accgtgcagt gcacccacgg catccgcccc gtggtgagca cccagctgct gctgaacggc 840
agcctggccg aggaggagat cgtgctgcgc tccgagaact tcaccgacaa cgccaagacc 900
atcatcgtgc agctgaacga gtccgtggag atcaactgca tccgccccaa caacaacacg 960
cgtaagagca tccacatcgg ccccggccgc gccttctacg ccaccggcga catcatcggc 1020
gacatccgcc aggcccactg caacatcagc aaggccaact ggaccaacac cctcgagcag 1080
atcgtggaga agctgcgcga gcagttcggc aacaacaaga ccatcatctt caacagcagc 1140
agcggcggcg accccgagat cgtgttccac agcttcaact gcggcggcga gttcttctac 1200
tgcaacacca gccagctgtt caacagcacc tggaacatca ccgaggaggt gaacaagacc 1260
aaggagaacg acaccatcat cctgccctgc cgcatccgcc agatcatcaa catgtggcag 1320
gaggtgggca aggccatgta cgcccccccc atccgcggcc agatcaagtg cagcagcaat 1380
attaccggcc tgctgctgac ccgcgacggc ggcaccaaca acaaccgcac caacgacacc 1440
gagaccttcc gccccggcgg cggcaacatg aaggacaact ggcgcagcga gctgtacaag 1500
tacaaggtgg tgcgcatcga gcccctgggc gtggccccca cccaggccaa gcgccgcgtg 1560
gtgcagcgcg agaagcgcta agatatcgga tcctctaga 1599

<210> 55
<211> 1350
<212> DNA
<213> Artificial Sequence

<220>
<223> Description of Artificial Sequence:
      gp120.modUS4.del 128-194

<400> 55
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgaagctg 360
acccccctgt gcgtgggggc agggaactgc gagaccagcg tgatcaccca ggcctgcccc 420
aaggtgagct tcgagcccat ccccatccac tactgcgccc ccgccggctt cgccatcctg 480
aagtgcaagg acaagaagtt caacggcacc ggcccctgca agaacgtgag caccgtgcag 540
tgcacccacg gcatccgccc cgtggtgagc acccagctgc tgctgaacgg cagcctggcc 600
gaggaggaga tcgtgctgcg ctccgagaac ttcaccgaca acgccaagac catcatcgtg 660
cagctgaacg agtccgtgga gatcaactgc atccgcccca acaacaacac gcgtaagagc 720
atccacatcg gccccggccg cgccttctac gccaccggcg acatcatcgg cgacatccgc 780
caggcccact gcaacatcag caaggccaac tggaccaaca ccctcgagca gatcgtggag 840
aagctgcgcg agcagttcgg caacaacaag accatcatct tcaacagcag cagcggcggc 900
gaccccgaga tcgtgttcca cagcttcaac tgcggcggcg agttcttcta ctgcaacacc 960
agccagctgt tcaacagcac ctggaacatc accgaggagg tgaacaagac caaggagaac 1020
gacaccatca tcctgccctg ccgcatccgc cagatcatca acatgtggca ggaggtgggc 1080
aaggccatgt acgccccccc catccgcggc cagatcaagt gcagcagcaa tattaccggc 1140
ctgctgctga cccgcgacgg cggcaccaac aacaaccgca ccaacgacac cgagaccttc 1200
cgccccggcg gcggcaacat gaaggacaac tggcgcagcg agctgtacaa gtacaaggtg 1260
gtgcgcatcg agcccctggg cgtggccccc acccaggcca agcgccgcgt ggtgcagcgc 1320
gagaagcgct aagatatcgg atcctctaga                                  1350

<210> 56
<211> 2112
<212> DNA
<213> Artificial Sequence

<220>
<223> Description of Artificial Sequence: gp140.modUS4

<400> 56
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgaagctg 360
acccccctgt gcgtgaccct gaactgcacc gacaagctga ccggcagcac caacggcacc 420
aacagcacca gcggcaccaa cagcaccagc ggcaccaaca gcaccagcac caacagcacc 480
gacagctggg agaagatgcc cgagggcgag atcaagaact gcagcttcaa catcaccacc 540
agcgtgcgcg acaaggtgca gaaggagtac agcctgttct acaagctgga cgtggtgccc 600
atcgacaacg acaacgccag ctaccgcctg atcaactgca acaccagcgt gatcacccag 660
gcctgcccca aggtgagctt cgagcccatc cccatccact actgcgcccc cgccggcttc 720
gccatcctga agtgcaagga caagaagttc aacggcaccg gcccctgcaa gaacgtgagc 780
accgtgcagt gcacccacgg catccgcccc gtggtgagca cccagctgct gctgaacggc 840
agcctggccg aggaggagat cgtgctgcgc tccgagaact tcaccgacaa cgccaagacc 900
atcatcgtgc agctgaacga gtccgtggag atcaactgca tccgccccaa caacaacacg 960
cgtaagagca tccacatcgg ccccggccgc gccttctacg ccaccggcga catcatcggc 1020
gacatccgcc aggcccactg caacatcagc aaggccaact ggaccaacac cctcgagcag 1080
atcgtggaga agctgcgcga gcagttcggc aacaacaaga ccatcatctt caacagcagc 1140
agcggcggcg accccgagat cgtgttccac agcttcaact gcggcggcga gttcttctac 1200
tgcaacacca gccagctgtt caacagcacc tggaacatca ccgaggaggt gaacaagacc 1260

aaggagaacg acaccatcat cctgccctgc cgcatccgcc agatcatcaa catgtggcag 1320
gaggtgggca aggccatgta cgcccccccc atccgcggcc agatcaagtg cagcagcaat 1380
attaccggcc tgctgctgac ccgcgacggc ggcaccaaca acaaccgcac caacgacacc 1440
gagaccttcc gccccggcgg cggcaacatg aaggacaact ggcgcagcga gctgtacaag 1500
tacaaggtgg tgcgcatcga gcccctgggc gtggccccca cccaggccaa gcgccgcgtg 1560
gtgcagcgcg agaagcgcgc cgtgggcctg ggcgccctgt tcatcggctt cctgggcgcc 1620
gccgggagca ccatgggcgc cgcctccgtg accctgaccg tgcaggcccg ccagctgctg 1680
agcggcatcg tgcagcagca gaacaacctg ctgcgcgcca tcgaggccca gcagcacctg 1740
ctgcagctga ccgtgtgggg catcaagcag ctgcaggccc gcatcctggc cgtggagcgc 1800
tacctgaagg accagcagct gctgggcatc tggggctgca gcggcaagct gatctgcacc 1860
accaccgtgc cctggaacag cagctggagc aacaagagcc tgaccgagat ctgggacaac 1920
atgacctgga tggagtggga gcgcgagatc ggcaactaca ccggcctgat ctacaacctg 1980
atcgagatcg cccagaacca gcaggagaag aacgagcagg agctgctgga gctggacaag 2040
tgggccagcc tgtggaactg gttcgacatc accaactggc tgtggtacat ctaagatatc 2100
ggatcctcta ga 2112

<210> 57
<211> 2112
<212> DNA
<213> Artificial Sequence

<220>
<223> Description of Artificial Sequence:
      gp140.mut.modUS4

<400> 57
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgaagctg 360
acccccctgt gcgtgaccct gaactgcacc gacaagctga ccggcagcac caacggcacc 420
aacagcacca gcggcaccaa cagcaccagc ggcaccaaca gcaccagcac caacagcacc 480
gacagctggg agaagatgcc cgagggcgag atcaagaact gcagcttcaa catcaccacc 540
agcgtgcgcg acaaggtgca gaaggagtac agcctgttct acaagctgga cgtggtgccc 600
atcgacaacg acaacgccag ctaccgcctg atcaactgca acaccagcgt gatcacccag 660
gcctgcccca aggtgagctt cgagcccatc cccatccact actgcgcccc cgccggcttc 720
gccatcctga agtgcaagga caagaagttc aacggcaccg gcccctgcaa gaacgtgagc 780
accgtgcagt gcacccacgg catccgcccc gtggtgagca cccagctgct gctgaacggc 840
agcctggccg aggaggagat cgtgctgcgc tccgagaact tcaccgacaa cgccaagacc 900
atcatcgtgc agctgaacga gtccgtggag atcaactgca tccgccccaa caacaacacg 960
cgtaagagca tccacatcgg ccccggccgc gccttctacg ccaccggcga catcatcggc 1020
gacatccgcc aggcccactg caacatcagc aaggccaact ggaccaacac cctcgagcag 1080
atcgtggaga agctgcgcga gcagttcggc aacaacaaga ccatcatctt caacagcagc 1140
agcggcggcg accccgagat cgtgttccac agcttcaact gcggcggcga gttcttctac 1200
tgcaacacca gccagctgtt caacagcacc tggaacatca ccgaggaggt gaacaagacc 1260
aaggagaacg acaccatcat cctgccctgc cgcatccgcc agatcatcaa catgtggcag 1320
gaggtgggca aggccatgta cgcccccccc atccgcggcc agatcaagtg cagcagcaat 1380
attaccggcc tgctgctgac ccgcgacggc ggcaccaaca acaaccgcac caacgacacc 1440
gagaccttcc gccccggcgg cggcaacatg aaggacaact ggcgcagcga gctgtacaag 1500
tacaaggtgg tgcgcatcga gcccctgggc gtggccccca cccaggccaa gcgccgcgtg 1560
gtgcagcgcg agaagagcgc cgtgggcctg ggcgccctgt tcatcggctt cctgggcgcc 1620
gccgggagca ccatgggcgc cgcctccgtg accctgaccg tgcaggcccg ccagctgctg 1680
agcggcatcg tgcagcagca gaacaacctg ctgcgcgcca tcgaggccca gcagcacctg 1740
ctgcagctga ccgtgtgggg catcaagcag ctgcaggccc gcatcctggc cgtggagcgc 1800
tacctgaagg accagcagct gctgggcatc tggggctgca gcggcaagct gatctgcacc 1860
accaccgtgc cctggaacag cagctggagc aacaagagcc tgaccgagat ctgggacaac 1920
atgacctgga tggagtggga gcgcgagatc ggcaactaca ccggcctgat ctacaacctg 1980

atcgagatcg cccagaacca gcaggagaag aacgagcagg agctgctgga gctggacaag 2040
tgggccagcc tgtggaactg gttcgacatc accaactggc tgtggtacat ctaagatatc 2100
ggatcctcta ga 2112

<210> 58
<211> 2181
<212> DNA
<213> Artificial Sequence

<220>
<223> Description of Artificial Sequence: gp140TM.modUS4

<400> 58
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgaagctg 360
acccccctgt gcgtgaccct gaactgcacc gacaagctga ccggcagcac caacggcacc 420
aacagcacca gcggcaccaa cagcaccagc ggcaccaaca gcaccagcac caacagcacc 480
gacagctggg agaagatgcc cgagggcgag atcaagaact gcagcttcaa catcaccacc 540
agcgtgcgcg acaaggtgca gaaggagtac agcctgttct acaagctgga cgtggtgccc 600
atcgacaacg acaacgccag ctaccgcctg atcaactgca acaccagcgt gatcacccag 660
gcctgcccca aggtgagctt cgagcccatc cccatccact actgcgcccc cgccggcttc 720
gccatcctga agtgcaagga caagaagttc aacggcaccg gcccctgcaa gaacgtgagc 780
accgtgcagt gcacccacgg catccgcccc gtggtgagca cccagctgct gctgaacggc 840
agcctggccg aggaggagat cgtgctgcgc tccgagaact tcaccgacaa cgccaagacc 900
atcatcgtgc agctgaacga gtccgtggag atcaactgca tccgccccaa caacaacacg 960
cgtaagagca tccacatcgg ccccggccgc gccttctacg ccaccggcga catcatcggc 1020
gacatccgcc aggcccactg caacatcagc aaggccaact ggaccaacac cctcgagcag 1080
atcgtggaga agctgcgcga gcagttcggc aacaacaaga ccatcatctt caacagcagc 1140
agcggcggcg accccgagat cgtgttccac agcttcaact gcggcggcga gttcttctac 1200
tgcaacacca gccagctgtt caacagcacc tggaacatca ccgaggaggt gaacaagacc 1260
aaggagaacg acaccatcat cctgccctgc cgcatccgcc agatcatcaa catgtggcag 1320
gaggtgggca aggccatgta cgcccccccc atccgcggcc agatcaagtg cagcagcaat 1380
attaccggcc tgctgctgac ccgcgacggc ggcaccaaca acaaccgcac caacgacacc 1440
gagaccttcc gccccggcgg cggcaacatg aaggacaact ggcgcagcga gctgtacaag 1500
tacaaggtgg tgcgcatcga gcccctgggc gtggccccca cccaggccaa gcgccgcgtg 1560
gtgcagcgcg agaagcgcgc cgtgggcctg ggcgccctgt tcatcggctt cctgggcgcc 1620
gccgggagca ccatgggcgc cgcctccgtg accctgaccg tgcaggcccg ccagctgctg 1680
agcggcatcg tgcagcagca gaacaacctg ctgcgcgcca tcgaggccca gcagcacctg 1740
ctgcagctga ccgtgtgggg catcaagcag ctgcaggccc gcatcctggc cgtggagcgc 1800
tacctgaagg accagcagct gctgggcatc tggggctgca gcggcaagct gatctgcacc 1860
accaccgtgc cctggaacag cagctggagc aacaagagcc tgaccgagat ctgggacaac 1920
atgacctgga tggagtggga gcgcgagatc ggcaactaca ccggcctgat ctacaacctg 1980
atcgagatcg cccagaacca gcaggagaag aacgagcagg agctgctgga gctggacaag 2040
tgggccagcc tgtggaactg gttcgacatc accaactggc tgtggtacat ccgcatcttc 2100
atcatgatcg tgggcggcct gatcggcctg cgcatcgtgt tcgccgtgct gagcatcgtg 2160
taagatatcg gatcctctag a 2181

<210> 59
<211> 1818
<212> DNA
<213> Artificial Sequence

<220>
<223> Description of Artificial Sequence:
gp140.modUS4.delV1/V2

<400> 59
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgggcgcc 360
ggccaggcct gccccaaggt gagcttcgag cccatcccca tccactactg cgcccccgcc 420
ggcttcgcca tcctgaagtg caaggacaag aagttcaacg gcaccggccc ctgcaagaac 480
gtgagcaccg tgcagtgcac ccacggcatc cgccccgtgg tgagcaccca gctgctgctg 540
aacggcagcc tggccgagga ggagatcgtg ctgcgctccg agaacttcac cgacaacgcc 600
aagaccatca tcgtgcagct gaacgagtcc gtggagatca actgcatccg ccccaacaac 660
aacacgcgta agagcatcca catcggcccc ggccgcgcct tctacgccac cggcgacatc 720
atcggcgaca tccgccaggc ccactgcaac atcagcaagg ccaactggac caacaccctc 780
gagcagatcg tggagaagct gcgcgagcag ttcggcaaca acaagaccat catcttcaac 840
agcagcagcg gcggcgaccc cgagatcgtg ttccacagct tcaactgcgg cggcgagttc 900
ttctactgca acaccagcca gctgttcaac agcacctgga acatcaccga ggaggtgaac 960
aagaccaagg agaacgacac catcatcctg ccctgccgca tccgccagat catcaacatg 1020
tggcaggagg tgggcaaggc catgtacgcc ccccccatcc gcggccagat caagtgcagc 1080
agcaatatta ccggcctgct gctgacccgc gacggcggca ccaacaacaa ccgcaccaac 1140
gacaccgaga ccttccgccc cggcggcggc aacatgaagg acaactggcg cagcgagctg 1200
tacaagtaca aggtggtgcg catcgagccc ctgggcgtgg cccccaccca ggccaagcgc 1260
cgcgtggtgc agcgcgagaa gcgcgccgtg ggcctgggcg ccctgttcat cggcttcctg 1320
ggcgccgccg ggagcaccat gggcgccgcc tccgtgaccc tgaccgtgca ggcccgccag 1380
ctgctgagcg gcatcgtgca gcagcagaac aacctgctgc gcgccatcga ggcccagcag 1440
cacctgctgc agctgaccgt gtggggcatc aagcagctgc aggcccgcat cctggccgtg 1500
gagcgctacc tgaaggacca gcagctgctg ggcatctggg gctgcagcgg caagctgatc 1560
tgcaccacca ccgtgccctg gaacagcagc tggagcaaca agagcctgac cgagatctgg 1620
gacaacatga cctggatgga gtgggagcgc gagatcggca actacaccgg cctgatctac 1680
aacctgatcg agatcgccca gaaccagcag gagaagaacg agcaggagct gctggagctg 1740
gacaagtggg ccagcctgtg gaactggttc gacatcacca actggctgtg gtacatctaa 1800
gatatcggat cctctaga 1818

<210> 60
<211> 2031
<212> DNA
<213> Artificial Sequence

<220>
<223> Description of Artificial Sequence:
      gp140.modUS4.delV2

<400> 60
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgaagctg 360
acccccctgt gcgtgaccct gaactgcacc gacaagctga ccggcagcac caacggcacc 420
aacagcacca gcggcaccaa cagcaccagc ggcaccaaca gcaccagcac caacagcacc 480
gacagctggg agaagatgcc cgagggcgag atcaagaact gcagcttcaa catcggcgcc 540
ggccgcctga tcaactgcaa caccagcgtg atcacccagg cctgccccaa ggtgagcttc 600
gagcccatcc ccatccacta ctgcgccccc gccggcttcg ccatcctgaa gtgcaaggac 660
aagaagttca acggcaccgg cccctgcaag aacgtgagca ccgtgcagtg cacccacggc 720
atccgccccg tggtgagcac ccagctgctg ctgaacggca gcctggccga ggaggagatc 780
gtgctgcgct ccgagaactt caccgacaac gccaagacca tcatcgtgca gctgaacgag 840
tccgtggaga tcaactgcat ccgccccaac aacaacacgc gtaagagcat ccacatcggc 900
cccggccgcg ccttctacgc caccggcgac atcatcggcg acatccgcca ggcccactgc 960

aacatcagca aggccaactg gaccaacacc ctcgagcaga tcgtggagaa gctgcgcgag 1020
cagttcggca acaacaagac catcatcttc aacagcagca gcggcggcga ccccgagatc 1080
gtgttccaca gcttcaactg cggcggcgag ttcttctact gcaacaccag ccagctgttc 1140
aacagcacct ggaacatcac cgaggaggtg aacaagacca aggagaacga caccatcatc 1200
ctgccctgcc gcatccgcca gatcatcaac atgtggcagg aggtgggcaa ggccatgtac 1260
gcccccccca tccgcggcca gatcaagtgc agcagcaata ttaccggcct gctgctgacc 1320
cgcgacggcg gcaccaacaa caaccgcacc aacgacaccg agaccttccg ccccggcggc 1380
ggcaacatga aggacaactg gcgcagcgag ctgtacaagt acaaggtggt gcgcatcgag 1440
cccctgggcg tggcccccac ccaggccaag cgccgcgtgg tgcagcgcga gaagcgcgcc 1500
gtgggcctgg gcgccctgtt catcggcttc ctgggcgccg ccgggagcac catgggcgcc 1560
gcctccgtga ccctgaccgt gcaggcccgc cagctgctga gcggcatcgt gcagcagcag 1620
aacaacctgc tgcgcgccat cgaggcccag cagcacctgc tgcagctgac cgtgtggggc 1680
atcaagcagc tgcaggcccg catcctggcc gtggagcgct acctgaagga ccagcagctg 1740
ctgggcatct ggggctgcag cggcaagctg atctgcacca ccaccgtgcc ctggaacagc 1800
agctggagca acaagagcct gaccgagatc tgggacaaca tgacctggat ggagtgggag 1860
cgcgagatcg gcaactacac cggcctgatc tacaacctga tcgagatcgc ccagaaccag 1920
caggagaaga acgagcagga gctgctggag ctggacaagt gggccagcct gtggaactgg 1980
ttcgacatca ccaactggct gtggtacatc taagatatcg gatcctctag a 2031

<210> 61
<211> 1818
<212> DNA
<213> Artificial Sequence

<220>
<223> Description of Artificial Sequence:
      gp140.mut.modUS4.delV1/V2

<400> 61
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgggcgcc 360
ggccaggcct gccccaaggt gagcttcgag cccatcccca tccactactg cgcccccgcc 420
ggcttcgcca tcctgaagtg caaggacaag aagttcaacg gcaccggccc ctgcaagaac 480
gtgagcaccg tgcagtgcac ccacggcatc cgccccgtgg tgagcaccca gctgctgctg 540
aacggcagcc tggccgagga ggagatcgtg ctgcgctccg agaacttcac cgacaacgcc 600
aagaccatca tcgtgcagct gaacgagtcc gtggagatca actgcatccg ccccaacaac 660
aacacgcgta agagcatcca catcggcccc ggccgcgcct tctacgccac cggcgacatc 720
atcggcgaca tccgccaggc ccactgcaac atcagcaagg ccaactggac caacaccctc 780
gagcagatcg tggagaagct gcgcgagcag ttcggcaaca acaagaccat catcttcaac 840
agcagcagcg gcggcgaccc cgagatcgtg ttccacagct tcaactgcgg cggcgagttc 900
ttctactgca acaccagcca gctgttcaac agcacctgga acatcaccga ggaggtgaac 960
aagaccaagg agaacgacac catcatcctg ccctgccgca tccgccagat catcaacatg 1020
tggcaggagg tgggcaaggc catgtacgcc ccccccatcc gcggccagat caagtgcagc 1080
agcaatatta ccggcctgct gctgacccgc gacggcggca ccaacaacaa ccgcaccaac 1140
gacaccgaga ccttccgccc cggcggcggc aacatgaagg acaactggcg cagcgagctg 1200
tacaagtaca aggtggtgcg catcgagccc ctgggcgtgg cccccaccca ggccaagcgc 1260
cgcgtggtgc agcgcgagaa gagcgccgtg ggcctgggcg ccctgttcat cggcttcctg 1320
ggcgccgccg ggagcaccat gggcgccgcc tccgtgaccc tgaccgtgca ggcccgccag 1380
ctgctgagcg gcatcgtgca gcagcagaac aacctgctgc gcgccatcga ggcccagcag 1440
cacctgctgc agctgaccgt gtggggcatc aagcagctgc aggcccgcat cctggccgtg 1500
gagcgctacc tgaaggacca gcagctgctg ggcatctggg gctgcagcgg caagctgatc 1560
tgcaccacca ccgtgccctg gaacagcagc tggagcaaca agagcctgac cgagatctgg 1620
gacaacatga cctggatgga gtgggagcgc gagatcggca actacaccgg cctgatctac 1680
aacctgatcg agatcgccca gaaccagcag gagaagaacg agcaggagct gctggagctg 1740
gacaagtggg ccagcctgtg gaactggttc gacatcacca actggctgtg gtacatctaa 1800

gatatcggat cctctaga 1818

<210> 62
<211> 1818
<212> DNA
<213> Artificial Sequence

<220>
<223> Description of Artificial Sequence:
      gp140.modUS4.del 128-194

<400> 62
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgggcgcc 360
ggccaggcct gccccaaggt gagcttcgag cccatcccca tccactactg cgcccccgcc 420
ggcttcgcca tcctgaagtg caaggacaag aagttcaacg gcaccggccc ctgcaagaac 480
gtgagcaccg tgcagtgcac ccacggcatc cgccccgtgg tgagcaccca gctgctgctg 540
aacggcagcc tggccgagga ggagatcgtg ctgcgctccg agaacttcac cgacaacgcc 600
aagaccatca tcgtgcagct gaacgagtcc gtggagatca actgcatccg ccccaacaac 660
aacacgcgta agagcatcca catcggcccc ggccgcgcct tctacgccac cggcgacatc 720
atcggcgaca tccgccaggc ccactgcaac atcagcaagg ccaactggac caacaccctc 780
gagcagatcg tggagaagct gcgcgagcag ttcggcaaca acaagaccat catcttcaac 840
agcagcagcg gcggcgaccc cgagatcgtg ttccacagct tcaactgcgg cggcgagttc 900
ttctactgca acaccagcca gctgttcaac agcacctgga acatcaccga ggaggtgaac 960
aagaccaagg agaacgacac catcatcctg ccctgccgca tccgccagat catcaacatg 1020
tggcaggagg tgggcaaggc catgtacgcc ccccccatcc gcggccagat caagtgcagc 1080
agcaatatta ccggcctgct gctgacccgc gacggcggca ccaacaacaa ccgcaccaac 1140
gacaccgaga ccttccgccc cggcggcggc aacatgaagg acaactggcg cagcgagctg 1200
tacaagtaca aggtggtgcg catcgagccc ctgggcgtgg cccccaccca ggccaagcgc 1260
cgcgtggtgc agcgcgagaa gagcgccgtg ggcctgggcg ccctgttcat cggcttcctg 1320
ggcgccgccg ggagcaccat gggcgccgcc tccgtgaccc tgaccgtgca ggcccgccag 1380
ctgctgagcg gcatcgtgca gcagcagaac aacctgctgc gcgccatcga ggcccagcag 1440
cacctgctgc agctgaccgt gtggggcatc aagcagctgc aggcccgcat cctggccgtg 1500
gagcgctacc tgaaggacca gcagctgctg ggcatctggg gctgcagcgg caagctgatc 1560
tgcaccacca ccgtgccctg gaacagcagc tggagcaaca agagcctgac cgagatctgg 1620
gacaacatga cctggatgga gtgggagcgc gagatcggca actacaccgg cctgatctac 1680
aacctgatcg agatcgccca gaaccagcag gagaagaacg agcaggagct gctggagctg 1740
gacaagtggg ccagcctgtg gaactggttc gacatcacca actggctgtg gtacatctaa 1800
gatatcggat cctctaga 1818

<210> 63
<211> 1863
<212> DNA
<213> Artificial Sequence

<220>
<223> Description of Artificial Sequence:
      gp140.mut.modUS4.del 128-194

<400> 63
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300

cagatgcatg aggacatcat cagcctgtgg gaccagagcc tgaagccctg cgtgaagctg 360
acccccctgt gcgtgggggc agggaactgc gagaccagcg tgatcaccca ggcctgcccc 420
aaggtgagct tcgagcccat ccccatccac tactgcgccc ccgccggctt cgccatcctg 480
aagtgcaagg acaagaagtt caacggcacc ggcccctgca agaacgtgag caccgtgcag 540
tgcacccacg gcatccgccc cgtggtgagc acccagctgc tgctgaacgg cagcctggcc 600
gaggaggaga tcgtgctgcg ctccgagaac ttcaccgaca acgccaagac catcatcgtg 660
cagctgaacg agtccgtgga gatcaactgc atccgcccca acaacaacac gcgtaagagc 720
atccacatcg gccccggccg cgccttctac gccaccggcg acatcatcgg cgacatccgc 780
caggcccact gcaacatcag caaggccaac tggaccaaca ccctcgagca gatcgtggag 840
aagctgcgcg agcagttcgg caacaacaag accatcatct tcaacagcag cagcggcggc 900
gaccccgaga tcgtgttcca cagcttcaac tgcggcggcg agttcttcta ctgcaacacc 960
agccagctgt tcaacagcac ctggaacatc accgaggagg tgaacaagac caaggagaac 1020
gacaccatca tcctgccctg ccgcatccgc cagatcatca acatgtggca ggaggtgggc 1080
aaggccatgt acgccccccc catccgcggc cagatcaagt gcagcagcaa tattaccggc 1140
ctgctgctga cccgcgacgg cggcaccaac aacaaccgca ccaacgacac cgagaccttc 1200
cgccccggcg gcggcaacat gaaggacaac tggcgcagcg agctgtacaa gtacaaggtg 1260
gtgcgcatcg agcccctggg cgtggccccc acccaggcca agcgccgcgt ggtgcagcgc 1320
gagaagagcg ccgtgggcct gggcgccctg ttcatcggct tcctgggcgc cgccgggagc 1380
accatgggcg ccgcctccgt gaccctgacc gtgcaggccc gccagctgct gagcggcatc 1440
gtgcagcagc agaacaacct gctgcgcgcc atcgaggccc agcagcacct gctgcagctg 1500
accgtgtggg gcatcaagca gctgcaggcc cgcatcctgg ccgtggagcg ctacctgaag 1560
gaccagcagc tgctgggcat ctggggctgc agcggcaagc tgatctgcac caccaccgtg 1620
ccctggaaca gcagctggag caacaagagc ctgaccgaga tctgggacaa catgacctgg 1680
atggagtggg agcgcgagat cggcaactac accggcctga tctacaacct gatcgagatc 1740
gcccagaacc agcaggagaa gaacgagcag gagctgctgg agctggacaa gtgggccagc 1800
ctgtggaact ggttcgacat caccaactgg ctgtggtaca tctaagatat cggatcctct 1860
aga 1863

<210> 64
<211> 2634
<212> DNA
<213> Artificial Sequence

<220>
<223> Description of Artificial Sequence: gp160.modUS4

<400> 64
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgaagctg 360
acccccctgt gcgtgaccct gaactgcacc gacaagctga ccggcagcac caacggcacc 420
aacagcacca gcggcaccaa cagcaccagc ggcaccaaca gcaccagcac caacagcacc 480
gacagctggg agaagatgcc cgagggcgag atcaagaact gcagcttcaa catcaccacc 540
agcgtgcgcg acaaggtgca gaaggagtac agcctgttct acaagctgga cgtggtgccc 600
atcgacaacg acaacgccag ctaccgcctg atcaactgca acaccagcgt gatcacccag 660
gcctgcccca aggtgagctt cgagcccatc cccatccact actgcgcccc cgccggcttc 720
gccatcctga agtgcaagga caagaagttc aacggcaccg gcccctgcaa gaacgtgagc 780
accgtgcagt gcacccacgg catccgcccc gtggtgagca cccagctgct gctgaacggc 840
agcctggccg aggaggagat cgtgctgcgc tccgagaact tcaccgacaa cgccaagacc 900
atcatcgtgc agctgaacga gtccgtggag atcaactgca tccgccccaa caacaacacg 960
cgtaagagca tccacatcgg ccccggccgc gccttctacg ccaccggcga catcatcggc 1020
gacatccgcc aggcccactg caacatcagc aaggccaact ggaccaacac cctcgagcag 1080
atcgtggaga agctgcgcga gcagttcggc aacaacaaga ccatcatctt caacagcagc 1140
agcggcggcg accccgagat cgtgttccac agcttcaact gcggcggcga gttcttctac 1200
tgcaacacca gccagctgtt caacagcacc tggaacatca ccgaggaggt gaacaagacc 1260
aaggagaacg acaccatcat cctgccctgc cgcatccgcc agatcatcaa catgtggcag 1320

gaggtgggca aggccatgta cgcccccccc atccgcggcc agatcaagtg cagcagcaat 1380
attaccggcc tgctgctgac ccgcgacggc ggcaccaaca acaaccgcac caacgacacc 1440
gagaccttcc gccccggcgg cggcaacatg aaggacaact ggcgcagcga gctgtacaag 1500
tacaaggtgg tgcgcatcga gcccctgggc gtggccccca cccaggccaa gcgccgcgtg 1560
gtgcagcgcg agaagcgcgc cgtgggcctg ggcgccctgt tcatcggctt cctgggcgcc 1620
gccgggagca ccatgggcgc cgcctccgtg accctgaccg tgcaggcccg ccagctgctg 1680
agcggcatcg tgcagcagca gaacaacctg ctgcgcgcca tcgaggccca gcagcacctg 1740
ctgcagctga ccgtgtgggg catcaagcag ctgcaggccc gcatcctggc cgtggagcgc 1800
tacctgaagg accagcagct gctgggcatc tggggctgca gcggcaagct gatctgcacc 1860
accaccgtgc cctggaacag cagctggagc aacaagagcc tgaccgagat ctgggacaac 1920
atgacctgga tggagtggga gcgcgagatc ggcaactaca ccggcctgat ctacaacctg 1980
atcgagatcg cccagaacca gcaggagaag aacgagcagg agctgctgga gctggacaag 2040
tgggccagcc tgtggaactg gttcgacatc accaactggc tgtggtacat ccgcatcttc 2100
atcatgatcg tgggcggcct gatcggcctg cgcatcgtgt tcgccgtgct gagcatcgtg 2160
aaccgcgtgc gccagggcta cagccccatc agcctgcaga cccgcctgcc cgcccagcgc 2220
ggccccgacc gccccgaggg catcgaggag gagggcggcg agcgcgaccg cgaccgcagc 2280
aaccgcctgg tgcacggcct gctggccctg atctgggacg acctgcgcag cctgtgcctg 2340
ttcagctacc accgcctgcg cgacctgctg ctgatcgtgg cccgcatcgt ggagctgctg 2400
ggccgccgcg gctgggaggc cctgaagtac tggtggaacc tgctgcagta ctggagccag 2460
gagctgaaga gcagcgccgt gagcctgttc aacgccaccg ccatcgccgt ggccgagggc 2520
accgaccgca tcatcgagat cgtgcagcgc atcttccgcg ccgtgatcca catcccccgc 2580
cgcatccgcc agggcctgga gcgcgccctg ctgtaagata tcggatcctc taga 2634

<210> 65
<211> 2538
<212> DNA
<213> Artificial Sequence

<220>
<223> Description of Artificial Sequence:
      gp160.modUS4.delV1

<400> 65
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgaagctg 360
acccccctgt gcgtgaccct gaactgcacc gacaagctgg gcgccggcgg cgagatcaag 420
aactgcagct tcaacatcac caccagcgtg cgcgacaagg tgcagaagga gtacagcctg 480
ttctacaagc tggacgtggt gcccatcgac aacgacaacg ccagctaccg cctgatcaac 540
tgcaacacca gcgtgatcac ccaggcctgc cccaaggtga gcttcgagcc catccccatc 600
cactactgcg cccccgccgg cttcgccatc ctgaagtgca aggacaagaa gttcaacggc 660
accggcccct gcaagaacgt gagcaccgtg cagtgcaccc acggcatccg ccccgtggtg 720
agcacccagc tgctgctgaa cggcagcctg gccgaggagg agatcgtgct gcgctccgag 780
aacttcaccg acaacgccaa gaccatcatc gtgcagctga acgagtccgt ggagatcaac 840
tgcatccgcc ccaacaacaa cacgcgtaag agcatccaca tcggccccgg ccgcgccttc 900
tacgccaccg gcgacatcat cggcgacatc cgccaggccc actgcaacat cagcaaggcc 960
aactggacca acaccctcga gcagatcgtg gagaagctgc gcgagcagtt cggcaacaac 1020
aagaccatca tcttcaacag cagcagcggc ggcgaccccg agatcgtgtt ccacagcttc 1080
aactgcggcg gcgagttctt ctactgcaac accagccagc tgttcaacag cacctggaac 1140
atcaccgagg aggtgaacaa gaccaaggag aacgacacca tcatcctgcc ctgccgcatc 1200
cgccagatca tcaacatgtg gcaggaggtg ggcaaggcca tgtacgcccc ccccatccgc 1260
ggccagatca agtgcagcag caatattacc ggcctgctgc tgacccgcga cggcggcacc 1320
aacaacaacc gcaccaacga caccgagacc ttccgccccg gcggcggcaa catgaaggac 1380
aactggcgca gcgagctgta caagtacaag gtggtgcgca tcgagcccct gggcgtggcc 1440
cccacccagg ccaagcgccg cgtggtgcag cgcgagaagc gcgccgtggg cctgggcgcc 1500
ctgttcatcg gcttcctggg cgccgccggg agcaccatgg gcgccgcctc cgtgaccctg 1560

accgtgcagg cccgccagct gctgagcggc atcgtgcagc agcagaacaa cctgctgcgc 1620
gccatcgagg cccagcagca cctgctgcag ctgaccgtgt ggggcatcaa gcagctgcag 1680
gcccgcatcc tggccgtgga gcgctacctg aaggaccagc agctgctggg catctggggc 1740
tgcagcggca agctgatctg caccaccacc gtgccctgga acagcagctg gagcaacaag 1800
agcctgaccg agatctggga caacatgacc tggatggagt gggagcgcga gatcggcaac 1860
tacaccggcc tgatctacaa cctgatcgag atcgcccaga accagcagga gaagaacgag 1920
caggagctgc tggagctgga caagtgggcc agcctgtgga actggttcga catcaccaac 1980
tggctgtggt acatccgcat cttcatcatg atcgtgggcg gcctgatcgg cctgcgcatc 2040
gtgttcgccg tgctgagcat cgtgaaccgc gtgcgccagg gctacagccc catcagcctg 2100
cagacccgcc tgcccgccca gcgcggcccc gaccgccccg agggcatcga ggaggagggc 2160
ggcgagcgcg accgcgaccg cagcaaccgc ctggtgcacg gcctgctggc cctgatctgg 2220
gacgacctgc gcagcctgtg cctgttcagc taccaccgcc tgcgcgacct gctgctgatc 2280
gtggcccgca tcgtggagct gctgggccgc cgcggctggg aggccctgaa gtactggtgg 2340
aacctgctgc agtactggag ccaggagctg aagagcagcg ccgtgagcct gttcaacgcc 2400
accgccatcg ccgtggccga gggcaccgac cgcatcatcg agatcgtgca gcgcatcttc 2460
cgcgccgtga tccacatccc ccgccgcatc cgccagggcc tggagcgcgc cctgctgtaa 2520
gatatcggat cctctaga 2538

<210> 66
<211> 2553
<212> DNA
<213> Artificial Sequence

<220>
<223> Description of Artificial Sequence:
      gp160.modUS4.delV2

<400> 66
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgaagctg 360
acccccctgt gcgtgaccct gaactgcacc gacaagctga ccggcagcac caacggcacc 420
aacagcacca gcggcaccaa cagcaccagc ggcaccaaca gcaccagcac caacagcacc 480
gacagctggg agaagatgcc cgagggcgag atcaagaact gcagcttcaa catcggcgcc 540
ggccgcctga tcaactgcaa caccagcgtg atcacccagg cctgccccaa ggtgagcttc 600
gagcccatcc ccatccacta ctgcgccccc gccggcttcg ccatcctgaa gtgcaaggac 660
aagaagttca acggcaccgg cccctgcaag aacgtgagca ccgtgcagtg cacccacggc 720
atccgccccg tggtgagcac ccagctgctg ctgaacggca gcctggccga ggaggagatc 780
gtgctgcgct ccgagaactt caccgacaac gccaagacca tcatcgtgca gctgaacgag 840
tccgtggaga tcaactgcat ccgccccaac aacaacacgc gtaagagcat ccacatcggc 900
cccggccgcg ccttctacgc caccggcgac atcatcggcg acatccgcca ggcccactgc 960
aacatcagca aggccaactg gaccaacacc ctcgagcaga tcgtggagaa gctgcgcgag 1020
cagttcggca acaacaagac catcatcttc aacagcagca gcggcggcga ccccgagatc 1080
gtgttccaca gcttcaactg cggcggcgag ttcttctact gcaacaccag ccagctgttc 1140
aacagcacct ggaacatcac cgaggaggtg aacaagacca aggagaacga caccatcatc 1200
ctgccctgcc gcatccgcca gatcatcaac atgtggcagg aggtgggcaa ggccatgtac 1260
gcccccccca tccgcggcca gatcaagtgc agcagcaata ttaccggcct gctgctgacc 1320
cgcgacggcg gcaccaacaa caaccgcacc aacgacaccg agaccttccg ccccggcggc 1380
ggcaacatga aggacaactg gcgcagcgag ctgtacaagt acaaggtggt gcgcatcgag 1440
cccctgggcg tggcccccac ccaggccaag cgccgcgtgg tgcagcgcga gaagcgcgcc 1500
gtgggcctgg gcgccctgtt catcggcttc ctgggcgccg ccgggagcac catgggcgcc 1560
gcctccgtga ccctgaccgt gcaggcccgc cagctgctga gcggcatcgt gcagcagcag 1620
aacaacctgc tgcgcgccat cgaggcccag cagcacctgc tgcagctgac cgtgtggggc 1680
atcaagcagc tgcaggcccg catcctggcc gtggagcgct acctgaagga ccagcagctg 1740
ctgggcatct ggggctgcag cggcaagctg atctgcacca ccaccgtgcc ctggaacagc 1800
agctggagca acaagagcct gaccgagatc tgggacaaca tgacctggat ggagtgggag 1860

cgcgagatcg gcaactacac cggcctgatc tacaacctga tcgagatcgc ccagaaccag 1920
caggagaaga acgagcagga gctgctggag ctggacaagt gggccagcct gtggaactgg 1980
ttcgacatca ccaactggct gtggtacatc cgcatcttca tcatgatcgt gggcggcctg 2040
atcggcctgc gcatcgtgtt cgccgtgctg agcatcgtga accgcgtgcg ccagggctac 2100
agccccatca gcctgcagac ccgcctgccc gcccagcgcg gccccgaccg ccccgagggc 2160
atcgaggagg agggcggcga gcgcgaccgc gaccgcagca accgcctggt gcacggcctg 2220
ctggccctga tctgggacga cctgcgcagc ctgtgcctgt tcagctacca ccgcctgcgc 2280
gacctgctgc tgatcgtggc ccgcatcgtg gagctgctgg gccgccgcgg ctgggaggcc 2340
ctgaagtact ggtggaacct gctgcagtac tggagccagg agctgaagag cagcgccgtg 2400
agcctgttca acgccaccgc catcgccgtg gccgagggca ccgaccgcat catcgagatc 2460
gtgcagcgca tcttccgcgc cgtgatccac atcccccgcc gcatccgcca gggcctggag 2520
cgcgccctgc tgtaagatat cggatcctct aga 2553

<210> 67
<211> 2340
<212> DNA
<213> Artificial Sequence

<220>
<223> Description of Artificial Sequence:
      gp160.modUS4.delV1/V2

<400> 67
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgggcgcc 360
ggccaggcct gccccaaggt gagcttcgag cccatcccca tccactactg cgcccccgcc 420
ggcttcgcca tcctgaagtg caaggacaag aagttcaacg gcaccggccc ctgcaagaac 480
gtgagcaccg tgcagtgcac ccacggcatc cgccccgtgg tgagcaccca gctgctgctg 540
aacggcagcc tggccgagga ggagatcgtg ctgcgctccg agaacttcac cgacaacgcc 600
aagaccatca tcgtgcagct gaacgagtcc gtggagatca actgcatccg ccccaacaac 660
aacacgcgta agagcatcca catcggcccc ggccgcgcct tctacgccac cggcgacatc 720
atcggcgaca tccgccaggc ccactgcaac atcagcaagg ccaactggac caacaccctc 780
gagcagatcg tggagaagct gcgcgagcag ttcggcaaca acaagaccat catcttcaac 840
agcagcagcg gcggcgaccc cgagatcgtg ttccacagct tcaactgcgg cggcgagttc 900
ttctactgca acaccagcca gctgttcaac agcacctgga acatcaccga ggaggtgaac 960
aagaccaagg agaacgacac catcatcctg ccctgccgca tccgccagat catcaacatg 1020
tggcaggagg tgggcaaggc catgtacgcc ccccccatcc gcggccagat caagtgcagc 1080
agcaatatta ccggcctgct gctgacccgc gacggcggca ccaacaacaa ccgcaccaac 1140
gacaccgaga ccttccgccc cggcggcggc aacatgaagg acaactggcg cagcgagctg 1200
tacaagtaca aggtggtgcg catcgagccc ctgggcgtgg cccccaccca ggccaagcgc 1260
cgcgtggtgc agcgcgagaa gcgcgccgtg ggcctgggcg ccctgttcat cggcttcctg 1320
ggcgccgccg ggagcaccat gggcgccgcc tccgtgaccc tgaccgtgca ggcccgccag 1380
ctgctgagcg gcatcgtgca gcagcagaac aacctgctgc gcgccatcga ggcccagcag 1440
cacctgctgc agctgaccgt gtggggcatc aagcagctgc aggcccgcat cctggccgtg 1500
gagcgctacc tgaaggacca gcagctgctg ggcatctggg gctgcagcgg caagctgatc 1560
tgcaccacca ccgtgccctg gaacagcagc tggagcaaca agagcctgac cgagatctgg 1620
gacaacatga cctggatgga gtgggagcgc gagatcggca actacaccgg cctgatctac 1680
aacctgatcg agatcgccca gaaccagcag gagaagaacg agcaggagct gctggagctg 1740
gacaagtggg ccagcctgtg gaactggttc gacatcacca actggctgtg gtacatccgc 1800
atcttcatca tgatcgtggg cggcctgatc ggcctgcgca tcgtgttcgc cgtgctgagc 1860
atcgtgaacc gcgtgcgcca gggctacagc cccatcagcc tgcagacccg cctgcccgcc 1920
cagcgcggcc ccgaccgccc cgagggcatc gaggaggagg gcggcgagcg cgaccgcgac 1980
cgcagcaacc gcctggtgca cggcctgctg gccctgatct gggacgacct gcgcagcctg 2040
tgcctgttca gctaccaccg cctgcgcgac ctgctgctga tcgtggcccg catcgtggag 2100
ctgctgggcc gccgcggctg ggaggccctg aagtactggt ggaacctgct gcagtactgg 2160

agccaggagc tgaagagcag cgccgtgagc ctgttcaacg ccaccgccat cgccgtggcc 2220
gagggcaccg accgcatcat cgagatcgtg cagcgcatct tccgcgccgt gatccacatc 2280
ccccgccgca tccgccaggg cctggagcgc gccctgctgt aagatatcgg atcctctaga 2340

<210> 68
<211> 2385
<212> DNA
<213> Artificial Sequence

<220>
<223> Description of Artificial Sequence:
      gp160.modUS4del 128-194

<400> 68
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgaagctg 360
acccccctgt gcgtgggggc agggaactgc gagaccagcg tgatcaccca ggcctgcccc 420
aaggtgagct tcgagcccat ccccatccac tactgcgccc ccgccggctt cgccatcctg 480
aagtgcaagg acaagaagtt caacggcacc ggcccctgca agaacgtgag caccgtgcag 540
tgcacccacg gcatccgccc cgtggtgagc acccagctgc tgctgaacgg cagcctggcc 600
gaggaggaga tcgtgctgcg ctccgagaac ttcaccgaca acgccaagac catcatcgtg 660
cagctgaacg agtccgtgga gatcaactgc atccgcccca acaacaacac gcgtaagagc 720
atccacatcg gccccggccg cgccttctac gccaccggcg acatcatcgg cgacatccgc 780
caggcccact gcaacatcag caaggccaac tggaccaaca ccctcgagca gatcgtggag 840
aagctgcgcg agcagttcgg caacaacaag accatcatct tcaacagcag cagcggcggc 900
gaccccgaga tcgtgttcca cagcttcaac tgcggcggcg agttcttcta ctgcaacacc 960
agccagctgt tcaacagcac ctggaacatc accgaggagg tgaacaagac caaggagaac 1020
gacaccatca tcctgccctg ccgcatccgc cagatcatca acatgtggca ggaggtgggc 1080
aaggccatgt acgccccccc catccgcggc cagatcaagt gcagcagcaa tattaccggc 1140
ctgctgctga cccgcgacgg cggcaccaac aacaaccgca ccaacgacac cgagaccttc 1200
cgccccggcg gcggcaacat gaaggacaac tggcgcagcg agctgtacaa gtacaaggtg 1260
gtgcgcatcg agcccctggg cgtggccccc acccaggcca agcgccgcgt ggtgcagcgc 1320
gagaagcgcg ccgtgggcct gggcgccctg ttcatcggct tcctgggcgc cgccgggagc 1380
accatgggcg ccgcctccgt gaccctgacc gtgcaggccc gccagctgct gagcggcatc 1440
gtgcagcagc agaacaacct gctgcgcgcc atcgaggccc agcagcacct gctgcagctg 1500
accgtgtggg gcatcaagca gctgcaggcc cgcatcctgg ccgtggagcg ctacctgaag 1560
gaccagcagc tgctgggcat ctggggctgc agcggcaagc tgatctgcac caccaccgtg 1620
ccctggaaca gcagctggag caacaagagc ctgaccgaga tctgggacaa catgacctgg 1680
atggagtggg agcgcgagat cggcaactac accggcctga tctacaacct gatcgagatc 1740
gcccagaacc agcaggagaa gaacgagcag gagctgctgg agctggacaa gtgggccagc 1800
ctgtggaact ggttcgacat caccaactgg ctgtggtaca tccgcatctt catcatgatc 1860
gtgggcggcc tgatcggcct gcgcatcgtg ttcgccgtgc tgagcatcgt gaaccgcgtg 1920
cgccagggct acagccccat cagcctgcag acccgcctgc ccgcccagcg cggccccgac 1980
cgccccgagg gcatcgagga ggagggcggc gagcgcgacc gcgaccgcag caaccgcctg 2040
gtgcacggcc tgctggccct gatctgggac gacctgcgca gcctgtgcct gttcagctac 2100
caccgcctgc gcgacctgct gctgatcgtg gcccgcatcg tggagctgct gggccgccgc 2160
ggctgggagg ccctgaagta ctggtggaac ctgctgcagt actggagcca ggagctgaag 2220
agcagcgccg tgagcctgtt caacgccacc gccatcgccg tggccgaggg caccgaccgc 2280
atcatcgaga tcgtgcagcg catcttccgc gccgtgatcc acatcccccg ccgcatccgc 2340
cagggcctgg agcgcgccct gctgtaagat atcggatcct ctaga 2385

<210> 69
<211> 144
<212> DNA
<213> Human immunodeficiency virus

<400> 69
gacaccatca tcctgccctg ccgcatccgc cagatcatca acatgtggca ggaggtgggc 60
aaggccatgt acgccccccc catccgcggc cagatcaagt gcagcagcaa catcaccggc 120
ctgctgctga cccgcgacgg cggc 144

<210> 70
<211> 144
<212> DNA
<213> Human immunodeficiency virus

<400> 70
ggaactatca cactcccatg cagaataaaa caaattataa acaggtggca ggaagtagga 60
aaagcaatgt atgcccctcc catcagagga caaattagat gctcatcaaa tattacagga 120
ctgctattaa caagagatgg tggt 144

<210> 71
<211> 144
<212> DNA
<213> Artificial Sequence

<220>
<223> Description of Artificial Sequence: synthetic Env
      US4 common region

<400> 71
gacaccatca tcctgccctg ccgcatccgc cagatcatca acatgtggca ggaggtgggc 60
aaggccatgt acgccccccc catccgcggc cagatcaagt gcagcagcaa catcaccggc 120
ctgctgctga cccgcgacgg cggc 144

<210> 72
<211> 144
<212> DNA
<213> Artificial Sequence

<220>
<223> Description of Artificial Sequence: synthetic Env
      SF162 common region

<400> 72
ggcaccatca ccctgccctg ccgcatcaag cagatcatca accgctggca ggaggtgggc 60
aaggccatgt acgccccccc catccgcggc cagatccgct gcagcagcaa catcaccggc 120
ctgctgctga cccgcgacgg cggc 144

<210> 73
<211> 4766
<212> DNA
<213> Artificial Sequence

<220>
<223> Description of Artificial Sequence:
      gp160.modUS4.gag.modSF2

<400> 73
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120
cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgaagctg 360

acccccctgt gcgtgaccct gaactgcacc gacaagctga ccggcagcac caacggcacc 420
aacagcacca gcggcaccaa cagcaccagc ggcaccaaca gcaccagcac caacagcacc 480
gacagctggg agaagatgcc cgagggcgag atcaagaact gcagcttcaa catcaccacc 540
agcgtgcgcg acaaggtgca gaaggagtac agcctgttct acaagctgga cgtggtgccc 600
atcgacaacg acaacgccag ctaccgcctg atcaactgca acaccagcgt gatcacccag 660
gcctgcccca aggtgagctt cgagcccatc cccatccact actgcgcccc cgccggcttc 720
gccatcctga agtgcaagga caagaagttc aacggcaccg gcccctgcaa gaacgtgagc 780
accgtgcagt gcacccacgg catccgcccc gtggtgagca cccagctgct gctgaacggc 840
agcctggccg aggaggagat cgtgctgcgc tccgagaact tcaccgacaa cgccaagacc 900
atcatcgtgc agctgaacga gtccgtggag atcaactgca tccgccccaa caacaacacg 960
cgtaagagca tccacatcgg ccccggccgc gccttctacg ccaccggcga catcatcggc 1020
gacatccgcc aggcccactg caacatcagc aaggccaact ggaccaacac cctcgagcag 1080
atcgtggaga agctgcgcga gcagttcggc aacaacaaga ccatcatctt caacagcagc 1140
agcggcggcg accccgagat cgtgttccac agcttcaact gcggcggcga gttcttctac 1200
tgcaacacca gccagctgtt caacagcacc tggaacatca ccgaggaggt gaacaagacc 1260
aaggagaacg acaccatcat cctgccctgc cgcatccgcc agatcatcaa catgtggcag 1320
gaggtgggca aggccatgta cgcccccccc atccgcggcc agatcaagtg cagcagcaat 1380
attaccggcc tgctgctgac ccgcgacggc ggcaccaaca acaaccgcac caacgacacc 1440
gagaccttcc gccccggcgg cggcaacatg aaggacaact ggcgcagcga gctgtacaag 1500
tacaaggtgg tgcgcatcga gcccctgggc gtggccccca cccaggccaa gcgccgcgtg 1560
gtgcagcgcg agaagcgcgc cgtgggcctg ggcgccctgt tcatcggctt cctgggcgcc 1620
gccgggagca ccatgggcgc cgcctccgtg accctgaccg tgcaggcccg ccagctgctg 1680
agcggcatcg tgcagcagca gaacaacctg ctgcgcgcca tcgaggccca gcagcacctg 1740
ctgcagctga ccgtgtgggg catcaagcag ctgcaggccc gcatcctggc cgtggagcgc 1800
tacctgaagg accagcagct gctgggcatc tggggctgca gcggcaagct gatctgcacc 1860
accaccgtgc cctggaacag cagctggagc aacaagagcc tgaccgagat ctgggacaac 1920
atgacctgga tggagtggga gcgcgagatc ggcaactaca ccggcctgat ctacaacctg 1980
atcgagatcg cccagaacca gcaggagaag aacgagcagg agctgctgga gctggacaag 2040
tgggccagcc tgtggaactg gttcgacatc accaactggc tgtggtacat ccgcatcttc 2100
atcatgatcg tgggcggcct gatcggcctg cgcatcgtgt tcgccgtgct gagcatcgtg 2160
aaccgcgtgc gccagggcta cagccccatc agcctgcaga cccgcctgcc cgcccagcgc 2220
ggccccgacc gccccgaggg catcgaggag gagggcggcg agcgcgaccg cgaccgcagc 2280
aaccgcctgg tgcacggcct gctggccctg atctgggacg acctgcgcag cctgtgcctg 2340
ttcagctacc accgcctgcg cgacctgctg ctgatcgtgg cccgcatcgt ggagctgctg 2400
ggccgccgcg gctgggaggc cctgaagtac tggtggaacc tgctgcagta ctggagccag 2460
gagctgaaga gcagcgccgt gagcctgttc aacgccaccg ccatcgccgt ggccgagggc 2520
accgaccgca tcatcgagat cgtgcagcgc atcttccgcg ccgtgatcca catcccccgc 2580
cgcatccgcc agggcctgga gcgcgccctg ctgtaagata tcggatcctc tagagaattc 2640
cgcccccccc cccccccccc ctctccctcc ccccccccta acgttactgg ccgaagccgc 2700
ttggaataag gccggtgtgc gtttgtctat atgttatttt ccaccatatt gccgtctttt 2760
ggcaatgtga gggcccggaa acctggccct gtcttcttga cgagcattcc taggggtctt 2820
tcccctctcg ccaaaggaat gcaaggtctg ttgaatgtcg tgaaggaagc agttcctctg 2880
gaagcttctt gaagacaaac aacgtctgta gcgacccttt gcaggcagcg gaacccccca 2940
cctggcgaca ggtgcctctg cggccaaaag ccacgtgtat aagatacacc tgcaaaggcg 3000
gcacaacccc agtgccacgt tgtgagttgg atagttgtgg aaagagtcaa atggctctcc 3060
tcaagcgtat tcaacaaggg gctgaaggat gcccagaagg taccccattg tatgggatct 3120
gatctggggc ctcggtgcac atgctttaca tgtgtttagt cgaggttaaa aaaacgtcta 3180
ggccccccga accacgggga cgtggttttc ctttgaaaaa cacgataata ccatgggcgc 3240
ccgcgccagc gtgctgagcg gcggcgagct ggacaagtgg gagaagatcc gcctgcgccc 3300
cggcggcaag aagaagtaca agctgaagca catcgtgtgg gccagccgcg agctggagcg 3360
cttcgccgtg aaccccggcc tgctggagac cagcgagggc tgccgccaga tcctgggcca 3420
gctgcagccc agcctgcaga ccggcagcga ggagctgcgc agcctgtaca acaccgtggc 3480
caccctgtac tgcgtgcacc agcgcatcga cgtcaaggac accaaggagg ccctggagaa 3540
gatcgaggag gagcagaaca agtccaagaa gaaggcccag caggccgccg ccgccgccgg 3600
caccggcaac agcagccagg tgagccagaa ctaccccatc gtgcagaacc tgcagggcca 3660
gatggtgcac caggccatca gcccccgcac cctgaacgcc tgggtgaagg tggtggagga 3720
gaaggccttc agccccgagg tgatccccat gttcagcgcc ctgagcgagg gcgccacccc 3780
ccaggacctg aacacgatgt tgaacaccgt gggcggccac caggccgcca tgcagatgct 3840
gaaggagacc atcaacgagg aggccgccga gtgggaccgc gtgcaccccg tgcacgccgg 3900

ccccatcgcc cccggccaga tgcgcgagcc ccgcggcagc gacatcgccg gcaccaccag 3960
caccctgcag gagcagatcg gctggatgac caacaacccc cccatccccg tgggcgagat 4020
ctacaagcgg tggatcatcc tgggcctgaa caagatcgtg cggatgtaca gccccaccag 4080
catcctggac atccgccagg gccccaagga gcccttccgc gactacgtgg accgcttcta 4140
caagaccctg cgcgctgagc aggccagcca ggacgtgaag aactggatga ccgagaccct 4200
gctggtgcag aacgccaacc ccgactgcaa gaccatcctg aaggctctcg gccccgcggc 4260
caccctggag gagatgatga ccgcctgcca gggcgtgggc ggccccggcc acaaggcccg 4320
cgtgctggcc gaggcgatga gccaggtgac gaacccggcg accatcatga tgcagcgcgg 4380
caacttccgc aaccagcgga agaccgtcaa gtgcttcaac tgcggcaagg agggccacac 4440
cgccaggaac tgccgcgccc cccgcaagaa gggctgctgg cgctgcggcc gcgagggcca 4500
ccagatgaag gactgcaccg agcgccaggc caacttcctg ggcaagatct ggcccagcta 4560
caagggccgc cccggcaact tcctgcagag ccgccccgag cccaccgccc cccccgagga 4620
gagcttccgc ttcggcgagg agaagaccac ccccagccag aagcaggagc ccatcgacaa 4680
ggagctgtac cccctgacca gcctgcgcag cctgttcggc aacgacccca gcagccagta 4740
agaattcaga ctcgagcaag tctaga 4766

<210> 74
<211> 4689
<212> DNA
<213> Artificial Sequence

<220>
<223> Description of Artificial Sequence:
      gp160.modSF162.gag.modSF2

<400> 74
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgaccacc 480
agcatccgca acaagatgca gaaggagtac gccctgttct acaagctgga cgtggtgccc 540
atcgacaacg acaacaccag ctacaagctg atcaactgca acaccagcgt gatcacccag 600
gcctgcccca aggtgagctt cgagcccatc cccatccact actgcgcccc cgccggcttc 660
gccatcctga agtgcaacga caagaagttc aacggcagcg gcccctgcac caacgtgagc 720
accgtgcagt gcacccacgg catccgcccc gtggtgagca cccagctgct gctgaacggc 780
agcctggccg aggagggcgt ggtgatccgc agcgagaact tcaccgacaa cgccaagacc 840
atcatcgtgc agctgaagga gagcgtggag atcaactgca cccgccccaa caacaacacc 900
cgcaagagca tcaccatcgg ccccggccgc gccttctacg ccaccggcga catcatcggc 960
gacatccgcc aggcccactg caacatcagc ggcgagaagt ggaacaacac cctgaagcag 1020
atcgtgacca agctgcaggc ccagttcggc aacaagacca tcgtgttcaa gcagagcagc 1080
ggcggcgacc ccgagatcgt gatgcacagc ttcaactgcg gcggcgagtt cttctactgc 1140
aacagcaccc agctgttcaa cagcacctgg aacaacacca tcggccccaa caacaccaac 1200
ggcaccatca ccctgccctg ccgcatcaag cagatcatca accgctggca ggaggtgggc 1260
aaggccatgt acgccccccc catccgcggc cagatccgct gcagcagcaa catcaccggc 1320
ctgctgctga cccgcgacgg cggcaaggag atcagcaaca ccaccgagat cttccgcccc 1380
ggcggcggcg acatgcgcga caactggcgc agcgagctgt acaagtacaa ggtggtgaag 1440
atcgagcccc tgggcgtggc ccccaccaag gccaagcgcc gcgtggtgca gcgcgagaag 1500
cgcgccgtga ccctgggcgc catgttcctg ggcttcctgg gcgccgccgg cagcaccatg 1560
ggcgcccgca gcctgaccct gaccgtgcag gcccgccagc tgctgagcgg catcgtgcag 1620
cagcagaaca acctgctgcg cgccatcgag gcccagcagc acctgctgca gctgaccgtg 1680
tggggcatca agcagctgca ggcccgcgtg ctggccgtgg agcgctacct gaaggaccag 1740
cagctgctgg gcatctgggg ctgcagcggc aagctgatct gcaccaccgc cgtgccctgg 1800
aacgccagct ggagcaacaa gagcctggac cagatctgga acaacatgac ctggatggag 1860
tgggagcgcg agatcgacaa ctacaccaac ctgatctaca ccctgatcga ggagagccag 1920
aaccagcagg agaagaacga gcaggagctg ctggagctgg acaagtgggc cagcctgtgg 1980

aactggttcg acatcagcaa gtggctgtgg tacatcaaga tcttcatcat gatcgtgggc 2040
ggcctggtgg gcctgcgcat cgtgttcacc gtgctgagca tcgtgaaccg cgtgcgccag 2100
ggctacagcc ccctgagctt ccagacccgc ttccccgccc cccgcggccc cgaccgcccc 2160
gagggcatcg aggaggaggg cggcgagcgc gaccgcgacc gcagcagccc cctggtgcac 2220
ggcctgctgg ccctgatctg ggacgacctg cgcagcctgt gcctgttcag ctaccaccgc 2280
ctgcgcgacc tgatcctgat cgccgcccgc atcgtggagc tgctgggccg ccgcggctgg 2340
gaggccctga agtactgggg caacctgctg cagtactgga tccaggagct gaagaacagc 2400
gccgtgagcc tgttcgacgc catcgccatc gccgtggccg agggcaccga ccgcatcatc 2460
gaggtggccc agcgcatcgg ccgcgccttc ctgcacatcc cccgccgcat ccgccagggc 2520
ttcgagcgcg ccctgctgta actcgagcaa gtctagagaa ttccgccccc cccccccccc 2580
cccctctccc tccccccccc ctaacgttac tggccgaagc cgcttggaat aaggccggtg 2640
tgcgtttgtc tatatgttat tttccaccat attgccgtct tttggcaatg tgagggcccg 2700
gaaacctggc cctgtcttct tgacgagcat tcctaggggt ctttcccctc tcgccaaagg 2760
aatgcaaggt ctgttgaatg tcgtgaagga agcagttcct ctggaagctt cttgaagaca 2820
aacaacgtct gtagcgaccc tttgcaggca gcggaacccc ccacctggcg acaggtgcct 2880
ctgcggccaa aagccacgtg tataagatac acctgcaaag gcggcacaac cccagtgcca 2940
cgttgtgagt tggatagttg tggaaagagt caaatggctc tcctcaagcg tattcaacaa 3000
ggggctgaag gatgcccaga aggtacccca ttgtatggga tctgatctgg ggcctcggtg 3060
cacatgcttt acatgtgttt agtcgaggtt aaaaaaacgt ctaggccccc cgaaccacgg 3120
ggacgtggtt ttcctttgaa aaacacgata ataccatggg cgcccgcgcc agcgtgctga 3180
gcggcggcga gctggacaag tgggagaaga tccgcctgcg ccccggcggc aagaagaagt 3240
acaagctgaa gcacatcgtg tgggccagcc gcgagctgga gcgcttcgcc gtgaaccccg 3300
gcctgctgga gaccagcgag ggctgccgcc agatcctggg ccagctgcag cccagcctgc 3360
agaccggcag cgaggagctg cgcagcctgt acaacaccgt ggccaccctg tactgcgtgc 3420
accagcgcat cgacgtcaag gacaccaagg aggccctgga gaagatcgag gaggagcaga 3480
acaagtccaa gaagaaggcc cagcaggccg ccgccgccgc cggcaccggc aacagcagcc 3540
aggtgagcca gaactacccc atcgtgcaga acctgcaggg ccagatggtg caccaggcca 3600
tcagcccccg caccctgaac gcctgggtga aggtggtgga ggagaaggcc ttcagccccg 3660
aggtgatccc catgttcagc gccctgagcg agggcgccac cccccaggac ctgaacacga 3720
tgttgaacac cgtgggcggc caccaggccg ccatgcagat gctgaaggag accatcaacg 3780
aggaggccgc cgagtgggac cgcgtgcacc ccgtgcacgc cggccccatc gcccccggcc 3840
agatgcgcga gccccgcggc agcgacatcg ccggcaccac cagcaccctg caggagcaga 3900
tcggctggat gaccaacaac ccccccatcc ccgtgggcga gatctacaag cggtggatca 3960
tcctgggcct gaacaagatc gtgcggatgt acagccccac cagcatcctg gacatccgcc 4020
agggccccaa ggagcccttc cgcgactacg tggaccgctt ctacaagacc ctgcgcgctg 4080
agcaggccag ccaggacgtg aagaactgga tgaccgagac cctgctggtg cagaacgcca 4140
accccgactg caagaccatc ctgaaggctc tcggccccgc ggccaccctg gaggagatga 4200
tgaccgcctg ccagggcgtg ggcggccccg gccacaaggc ccgcgtgctg gccgaggcga 4260
tgagccaggt gacgaacccg gcgaccatca tgatgcagcg cggcaacttc cgcaaccagc 4320
ggaagaccgt caagtgcttc aactgcggca aggagggcca caccgccagg aactgccgcg 4380
ccccccgcaa gaagggctgc tggcgctgcg gccgcgaggg ccaccagatg aaggactgca 4440
ccgagcgcca ggccaacttc ctgggcaaga tctggcccag ctacaagggc cgccccggca 4500
acttcctgca gagccgcccc gagcccaccg ccccccccga ggagagcttc cgcttcggcg 4560
aggagaagac cacccccagc cagaagcagg agcccatcga caaggagctg taccccctga 4620
ccagcctgcg cagcctgttc ggcaacgacc ccagcagcca gtaagaattc agactcgagc 4680
aagtctaga 4689

<210> 75
<211> 4472
<212> DNA
<213> Artificial Sequence

<220>
<223> Description of Artificial Sequence:
      gp160.modUS4.delV1/V2.gag.modSF2

<400> 75
gaattcgcca ccatggatgc aatgaagaga gggctctgct gtgtgctgct gctgtgtgga 60
gcagtcttcg tttcgcccag cgccaccacc gtgctgtggg tgaccgtgta ctacggcgtg 120

cccgtgtgga aggaggccac caccaccctg ttctgcgcca gcgacgccaa ggcttacaag 180
gccgaggccc acaacgtgtg ggccacccac gcctgcgtgc ccaccgaccc caacccccag 240
gaggtgaacc tgaccaacgt gaccgagaac ttcaacatgt ggaagaacaa catggtggag 300
cagatgcatg aggacatcat cagcctgtgg gaccagagcc tgaagccctg cgtgggcgcc 360
ggccaggcct gccccaaggt gagcttcgag cccatcccca tccactactg cgcccccgcc 420
ggcttcgcca tcctgaagtg caaggacaag aagttcaacg gcaccggccc ctgcaagaac 480
gtgagcaccg tgcagtgcac ccacggcatc cgccccgtgg tgagcaccca gctgctgctg 540
aacggcagcc tggccgagga ggagatcgtg ctgcgctccg agaacttcac cgacaacgcc 600
aagaccatca tcgtgcagct gaacgagtcc gtggagatca actgcatccg ccccaacaac 660
aacacgcgta agagcatcca catcggcccc ggccgcgcct tctacgccac cggcgacatc 720
atcggcgaca tccgccaggc ccactgcaac atcagcaagg ccaactggac caacaccctc 780
gagcagatcg tggagaagct gcgcgagcag ttcggcaaca acaagaccat catcttcaac 840
agcagcagcg gcggcgaccc cgagatcgtg ttccacagct tcaactgcgg cggcgagttc 900
ttctactgca acaccagcca gctgttcaac agcacctgga acatcaccga ggaggtgaac 960
aagaccaagg agaacgacac catcatcctg ccctgccgca tccgccagat catcaacatg 1020
tggcaggagg tgggcaaggc catgtacgcc ccccccatcc gcggccagat caagtgcagc 1080
agcaatatta ccggcctgct gctgacccgc gacggcggca ccaacaacaa ccgcaccaac 1140
gacaccgaga ccttccgccc cggcggcggc aacatgaagg acaactggcg cagcgagctg 1200
tacaagtaca aggtggtgcg catcgagccc ctgggcgtgg cccccaccca ggccaagcgc 1260
cgcgtggtgc agcgcgagaa gcgcgccgtg ggcctgggcg ccctgttcat cggcttcctg 1320
ggcgccgccg ggagcaccat gggcgccgcc tccgtgaccc tgaccgtgca ggcccgccag 1380
ctgctgagcg gcatcgtgca gcagcagaac aacctgctgc gcgccatcga ggcccagcag 1440
cacctgctgc agctgaccgt gtggggcatc aagcagctgc aggcccgcat cctggccgtg 1500
gagcgctacc tgaaggacca gcagctgctg ggcatctggg gctgcagcgg caagctgatc 1560
tgcaccacca ccgtgccctg gaacagcagc tggagcaaca agagcctgac cgagatctgg 1620
gacaacatga cctggatgga gtgggagcgc gagatcggca actacaccgg cctgatctac 1680
aacctgatcg agatcgccca gaaccagcag gagaagaacg agcaggagct gctggagctg 1740
gacaagtggg ccagcctgtg gaactggttc gacatcacca actggctgtg gtacatccgc 1800
atcttcatca tgatcgtggg cggcctgatc ggcctgcgca tcgtgttcgc cgtgctgagc 1860
atcgtgaacc gcgtgcgcca gggctacagc cccatcagcc tgcagacccg cctgcccgcc 1920
cagcgcggcc ccgaccgccc cgagggcatc gaggaggagg gcggcgagcg cgaccgcgac 1980
cgcagcaacc gcctggtgca cggcctgctg gccctgatct gggacgacct gcgcagcctg 2040
tgcctgttca gctaccaccg cctgcgcgac ctgctgctga tcgtggcccg catcgtggag 2100
ctgctgggcc gccgcggctg ggaggccctg aagtactggt ggaacctgct gcagtactgg 2160
agccaggagc tgaagagcag cgccgtgagc ctgttcaacg ccaccgccat cgccgtggcc 2220
gagggcaccg accgcatcat cgagatcgtg cagcgcatct tccgcgccgt gatccacatc 2280
ccccgccgca tccgccaggg cctggagcgc gccctgctgt aagatatcgg atcctctaga 2340
gaattccgcc cccccccccc cccccctctc cctccccccc cccctaacgt tactggccga 2400
agccgcttgg aataaggccg gtgtgcgttt gtctatatgt tattttccac catattgccg 2460
tcttttggca atgtgagggc ccggaaacct ggccctgtct tcttgacgag cattcctagg 2520
ggtctttccc ctctcgccaa aggaatgcaa ggtctgttga atgtcgtgaa ggaagcagtt 2580
cctctggaag cttcttgaag acaaacaacg tctgtagcga ccctttgcag gcagcggaac 2640
cccccacctg gcgacaggtg cctctgcggc caaaagccac gtgtataaga tacacctgca 2700
aaggcggcac aaccccagtg ccacgttgtg agttggatag ttgtggaaag agtcaaatgg 2760
ctctcctcaa gcgtattcaa caaggggctg aaggatgccc agaaggtacc ccattgtatg 2820
ggatctgatc tggggcctcg gtgcacatgc tttacatgtg tttagtcgag gttaaaaaaa 2880
cgtctaggcc ccccgaacca cggggacgtg gttttccttt gaaaaacacg ataataccat 2940
gggcgcccgc gccagcgtgc tgagcggcgg cgagctggac aagtgggaga agatccgcct 3000
gcgccccggc ggcaagaaga agtacaagct gaagcacatc gtgtgggcca gccgcgagct 3060
ggagcgcttc gccgtgaacc ccggcctgct ggagaccagc gagggctgcc gccagatcct 3120
gggccagctg cagcccagcc tgcagaccgg cagcgaggag ctgcgcagcc tgtacaacac 3180
cgtggccacc ctgtactgcg tgcaccagcg catcgacgtc aaggacacca aggaggccct 3240
ggagaagatc gaggaggagc agaacaagtc caagaagaag gcccagcagg ccgccgccgc 3300
cgccggcacc ggcaacagca gccaggtgag ccagaactac cccatcgtgc agaacctgca 3360
gggccagatg gtgcaccagg ccatcagccc ccgcaccctg aacgcctggg tgaaggtggt 3420
ggaggagaag gccttcagcc ccgaggtgat ccccatgttc agcgccctga gcgagggcgc 3480
caccccccag gacctgaaca cgatgttgaa caccgtgggc ggccaccagg ccgccatgca 3540
gatgctgaag gagaccatca acgaggaggc cgccgagtgg gaccgcgtgc accccgtgca 3600
cgccggcccc atcgcccccg gccagatgcg cgagccccgc ggcagcgaca tcgccggcac 3660

caccagcacc ctgcaggagc agatcggctg gatgaccaac aaccccccca tccccgtggg 3720
cgagatctac aagcggtgga tcatcctggg cctgaacaag atcgtgcgga tgtacagccc 3780
caccagcatc ctggacatcc gccagggccc caaggagccc ttccgcgact acgtggaccg 3840
cttctacaag accctgcgcg ctgagcaggc cagccaggac gtgaagaact ggatgaccga 3900
gaccctgctg gtgcagaacg ccaaccccga ctgcaagacc atcctgaagg ctctcggccc 3960
cgcggccacc ctggaggaga tgatgaccgc ctgccagggc gtgggcggcc ccggccacaa 4020
ggcccgcgtg ctggccgagg cgatgagcca ggtgacgaac ccggcgacca tcatgatgca 4080
gcgcggcaac ttccgcaacc agcggaagac cgtcaagtgc ttcaactgcg gcaaggaggg 4140
ccacaccgcc aggaactgcc gcgccccccg caagaagggc tgctggcgct gcggccgcga 4200
gggccaccag atgaaggact gcaccgagcg ccaggccaac ttcctgggca agatctggcc 4260
cagctacaag ggccgccccg gcaacttcct gcagagccgc cccgagccca ccgccccccc 4320
cgaggagagc ttccgcttcg gcgaggagaa gaccaccccc agccagaagc aggagcccat 4380
cgacaaggag ctgtaccccc tgaccagcct gcgcagcctg ttcggcaacg accccagcag 4440
ccagtaagaa ttcagactcg agcaagtcta ga 4472

<210> 76
<211> 4608
<212> DNA
<213> Artificial Sequence

<220>
<223> Description of Artificial Sequence:
gp160.modSF162.delV2.gag.modSF2

<400> 76
gaattcgcca ccatggatgc aatgaagaga gggctctgct gtgtgctgct gctgtgtgga 60
gcagtcttcg tttcgcccag cgccgtggag aagctgtggg tgaccgtgta ctacggcgtg 120
cccgtgtgga aggaggccac caccaccctg ttctgcgcca gcgacgccaa ggcctacgac 180
accgaggtgc acaacgtgtg ggccacccac gcctgcgtgc ccaccgaccc caacccccag 240
gagatcgtgc tggagaacgt gaccgagaac ttcaacatgt ggaagaacaa catggtggag 300
cagatgcacg aggacatcat cagcctgtgg gaccagagcc tgaagccctg cgtgaagctg 360
acccccctgt gcgtgaccct gcactgcacc aacctgaaga acgccaccaa caccaagagc 420
agcaactgga aggagatgga ccgcggcgag atcaagaact gcagcttcaa ggtgggcgcc 480
ggcaagctga tcaactgcaa caccagcgtg atcacccagg cctgccccaa ggtgagcttc 540
gagcccatcc ccatccacta ctgcgccccc gccggcttcg ccatcctgaa gtgcaacgac 600
aagaagttca acggcagcgg cccctgcacc aacgtgagca ccgtgcagtg cacccacggc 660
atccgccccg tggtgagcac ccagctgctg ctgaacggca gcctggccga ggagggcgtg 720
gtgatccgca gcgagaactt caccgacaac gccaagacca tcatcgtgca gctgaaggag 780
agcgtggaga tcaactgcac ccgccccaac aacaacaccc gcaagagcat caccatcggc 840
cccggccgcg ccttctacgc caccggcgac atcatcggcg acatccgcca ggcccactgc 900
aacatcagcg gcgagaagtg gaacaacacc ctgaagcaga tcgtgaccaa gctgcaggcc 960
cagttcggca acaagaccat cgtgttcaag cagagcagcg gcggcgaccc cgagatcgtg 1020
atgcacagct tcaactgcgg cggcgagttc ttctactgca acagcaccca gctgttcaac 1080
agcacctgga acaacaccat cggccccaac aacaccaacg gcaccatcac cctgccctgc 1140
cgcatcaagc agatcatcaa ccgctggcag gaggtgggca aggccatgta cgcccccccc 1200
atccgcggcc agatccgctg cagcagcaac atcaccggcc tgctgctgac ccgcgacggc 1260
ggcaaggaga tcagcaacac caccgagatc ttccgccccg gcggcggcga catgcgcgac 1320
aactggcgca gcgagctgta caagtacaag gtggtgaaga tcgagcccct gggcgtggcc 1380
cccaccaagg ccaagcgccg cgtggtgcag cgcgagaagc gcgccgtgac cctgggcgcc 1440
atgttcctgg gcttcctggg cgccgccggc agcaccatgg gcgcccgcag cctgaccctg 1500
accgtgcagg cccgccagct gctgagcggc atcgtgcagc agcagaacaa cctgctgcgc 1560
gccatcgagg cccagcagca cctgctgcag ctgaccgtgt ggggcatcaa gcagctgcag 1620
gcccgcgtgc tggccgtgga gcgctacctg aaggaccagc agctgctggg catctggggc 1680
tgcagcggca agctgatctg caccaccgcc gtgccctgga acgccagctg gagcaacaag 1740
agcctggacc agatctggaa caacatgacc tggatggagt gggagcgcga gatcgacaac 1800
tacaccaacc tgatctacac cctgatcgag gagagccaga accagcagga gaagaacgag 1860
caggagctgc tggagctgga caagtgggcc agcctgtgga actggttcga catcagcaag 1920
tggctgtggt acatcaagat cttcatcatg atcgtgggcg gcctggtggg cctgcgcatc 1980
gtgttcaccg tgctgagcat cgtgaaccgc gtgcgccagg gctacagccc cctgagcttc 2040

cagacccgct tccccgcccc ccgcggcccc gaccgccccg agggcatcga ggaggagggc 2100
ggcgagcgcg accgcgaccg cagcagcccc ctggtgcacg gcctgctggc cctgatctgg 2160
gacgacctgc gcagcctgtg cctgttcagc taccaccgcc tgcgcgacct gatcctgatc 2220
gccgcccgca tcgtggagct gctgggccgc cgcggctggg aggccctgaa gtactggggc 2280
aacctgctgc agtactggat ccaggagctg aagaacagcg ccgtgagcct gttcgacgcc 2340
atcgccatcg ccgtggccga gggcaccgac cgcatcatcg aggtggccca gcgcatcggc 2400
cgcgccttcc tgcacatccc ccgccgcatc cgccagggct tcgagcgcgc cctgctgtaa 2460
ctcgagcaag tctagagaat tccgcccccc cccccccccc ccctctccct cccccccccc 2520
taacgttact ggccgaagcc gcttggaata aggccggtgt gcgtttgtct atatgttatt 2580
ttccaccata ttgccgtctt ttggcaatgt gagggcccgg aaacctggcc ctgtcttctt 2640
gacgagcatt cctaggggtc tttcccctct cgccaaagga atgcaaggtc tgttgaatgt 2700
cgtgaaggaa gcagttcctc tggaagcttc ttgaagacaa acaacgtctg tagcgaccct 2760
ttgcaggcag cggaaccccc cacctggcga caggtgcctc tgcggccaaa agccacgtgt 2820
ataagataca cctgcaaagg cggcacaacc ccagtgccac gttgtgagtt ggatagttgt 2880
ggaaagagtc aaatggctct cctcaagcgt attcaacaag gggctgaagg atgcccagaa 2940
ggtaccccat tgtatgggat ctgatctggg gcctcggtgc acatgcttta catgtgttta 3000
gtcgaggtta aaaaaacgtc taggcccccc gaaccacggg gacgtggttt tcctttgaaa 3060
aacacgataa taccatgggc gcccgcgcca gcgtgctgag cggcggcgag ctggacaagt 3120
gggagaagat ccgcctgcgc cccggcggca agaagaagta caagctgaag cacatcgtgt 3180
gggccagccg cgagctggag cgcttcgccg tgaaccccgg cctgctggag accagcgagg 3240
gctgccgcca gatcctgggc cagctgcagc ccagcctgca gaccggcagc gaggagctgc 3300
gcagcctgta caacaccgtg gccaccctgt actgcgtgca ccagcgcatc gacgtcaagg 3360
acaccaagga ggccctggag aagatcgagg aggagcagaa caagtccaag aagaaggccc 3420
agcaggccgc cgccgccgcc ggcaccggca acagcagcca ggtgagccag aactacccca 3480
tcgtgcagaa cctgcagggc cagatggtgc accaggccat cagcccccgc accctgaacg 3540
cctgggtgaa ggtggtggag gagaaggcct tcagccccga ggtgatcccc atgttcagcg 3600
ccctgagcga gggcgccacc ccccaggacc tgaacacgat gttgaacacc gtgggcggcc 3660
accaggccgc catgcagatg ctgaaggaga ccatcaacga ggaggccgcc gagtgggacc 3720
gcgtgcaccc cgtgcacgcc ggccccatcg cccccggcca gatgcgcgag ccccgcggca 3780
gcgacatcgc cggcaccacc agcaccctgc aggagcagat cggctggatg accaacaacc 3840
cccccatccc cgtgggcgag atctacaagc ggtggatcat cctgggcctg aacaagatcg 3900
tgcggatgta cagccccacc agcatcctgg acatccgcca gggccccaag gagcccttcc 3960
gcgactacgt ggaccgcttc tacaagaccc tgcgcgctga gcaggccagc caggacgtga 4020
agaactggat gaccgagacc ctgctggtgc agaacgccaa ccccgactgc aagaccatcc 4080
tgaaggctct cggccccgcg gccaccctgg aggagatgat gaccgcctgc cagggcgtgg 4140
gcggccccgg ccacaaggcc cgcgtgctgg ccgaggcgat gagccaggtg acgaacccgg 4200
cgaccatcat gatgcagcgc ggcaacttcc gcaaccagcg gaagaccgtc aagtgcttca 4260
actgcggcaa ggagggccac accgccagga actgccgcgc cccccgcaag aagggctgct 4320
ggcgctgcgg ccgcgagggc caccagatga aggactgcac cgagcgccag gccaacttcc 4380
tgggcaagat ctggcccagc tacaagggcc gccccggcaa cttcctgcag agccgccccg 4440
agcccaccgc cccccccgag gagagcttcc gcttcggcga ggagaagacc acccccagcc 4500
agaagcagga gcccatcgac aaggagctgt accccctgac cagcctgcgc agcctgttcg 4560
gcaacgaccc cagcagccag taagaattca gactcgagca agtctaga 4608

<210> 77
<211> 1680
<212> DNA
<213> Human immunodeficiency virus

<400> 77
cccattagtc ctattgaaac tgtaccagta aaattaaagc caggaatgga tggcccaaaa 60
gttaagcaat ggccattgac agaagaaaaa ataaaagcat tagtagagat atgtacagaa 120
atggaaaagg aagggaaaat ttcaaaaatt gggcctgaaa atccatacaa tactccagta 180
tttgctataa agaaaaaaga cagtactaaa tggagaaaac tagtagattt cagagaactt 240
aataaaagaa ctcaagactt ctgggaagtt cagttaggaa taccacaccc cgcagggtta 300
aaaaagaaaa aatcagtaac agtattggat gtgggtgatg catacttttc agttccctta 360
gataaagact ttagaaagta tactgcattt accataccta gtataaacaa tgagacacca 420
gggattagat atcagtacaa tgtgctgcca cagggatgga aaggatcacc agcaatattc 480
caaagtagca tgacaaaaat cttagagcct tttagaaaac agaatccaga catagttatc 540

tatcaataca tggatgattt gtatgtagga tctgacttag aaatagggca gcatagaaca 600
aaaatagagg aactgagaca gcatctgttg aggtggggat ttaccacacc agacaaaaaa 660
catcagaaag aacctccatt cctttggatg ggttatgaac tccatcctga taaatggaca 720
gtacagccta taatgctgcc agaaaaagac agctggactg tcaatgacat acagaagtta 780
gtgggaaaat tgaattgggc aagtcagatt tatgcaggga ttaaagtaaa gcagttatgt 840
aaactcctta gaggaaccaa agcactaaca gaagtaatac cactaacaga agaagcagag 900
ctagaactgg cagaaaacag ggagattcta aaagaaccag tacatgaagt atattatgac 960
ccatcaaaag acttagtagc agaaatacag aagcaggggc aaggccaatg gacatatcaa 1020
atttatcaag agccatttaa aaatctgaaa acaggaaagt atgcaaggat gaggggtgcc 1080
cacactaatg atgtaaaaca gttaacagag gcagtgcaaa aagtatccac agaaagcata 1140
gtaatatggg gaaagattcc taaatttaaa ctacccatac aaaaggaaac atgggaagca 1200
tggtggatgg agtattggca agctacctgg attcctgagt gggagtttgt caatacccct 1260
cccttagtga aattatggta ccagttagag aaagaaccca tagtaggagc agaaactttc 1320
tatgtagatg gggcagctaa tagggagact aaattaggaa aagcaggata tgttactgac 1380
agaggaagac aaaaagttgt ctccatagct gacacaacaa atcagaagac tgaattacaa 1440
gcaattcatc tagctttgca ggattcggga ttagaagtaa acatagtaac agactcacaa 1500
tatgcattag gaatcattca agcacaacca gataagagtg aatcagagtt agtcagtcaa 1560
ataatagagc agttaataaa aaaggaaaag gtctacctgg catgggtacc agcacacaaa 1620
ggaattggag gaaatgaaca agtagataaa ttagtcagtg ctggaatcag gaaagtacta 1680

<210> 78
<211> 1865
<212> DNA
<213> Artificial Sequence

<220>
<223> Description of Artificial Sequence: GP1

<400> 78
gtcgacgcca ccatgggcgc ccgcgccagc gtgctgagcg gcggcgagct ggacaagtgg 60
gagaagatcc gcctgcgccc cggcggcaag aagaagtaca agctgaagca catcgtgtgg 120
gccagccgcg agctggagcg cttcgccgtg aaccccggcc tgctggagac cagcgagggc 180
tgccgccaga tcctgggcca gctgcagccc agcctgcaga ccggcagcga ggagctgcgc 240
agcctgtaca acaccgtggc caccctgtac tgcgtgcacc agcgcatcga cgtcaaggac 300
accaaggagg ccctggagaa gatcgaggag gagcagaaca agtccaagaa gaaggcccag 360
caggccgccg ccgccgccgg caccggcaac agcagccagg tgagccagaa ctaccccatc 420
gtgcagaacc tgcagggcca gatggtgcac caggccatca gcccccgcac cctgaacgcc 480
tgggtgaagg tggtggagga gaaggccttc agccccgagg tgatccccat gttcagcgcc 540
ctgagcgagg gcgccacccc ccaggacctg aacacgatgt tgaacaccgt gggcggccac 600
caggccgcca tgcagatgct gaaggagacc atcaacgagg aggccgccga gtgggaccgc 660
gtgcaccccg tgcacgccgg ccccatcgcc cccggccaga tgcgcgagcc ccgcggcagc 720
gacatcgccg gcaccaccag caccctgcag gagcagatcg gctggatgac caacaacccc 780
cccatccccg tgggcgagat ctacaagcgg tggatcatcc tgggcctgaa caagatcgtg 840
cggatgtaca gccccaccag catcctggac atccgccagg gccccaagga gcccttccgc 900
gactacgtgg accgcttcta caagaccctg cgcgctgagc aggccagcca ggacgtgaag 960
aactggatga ccgagaccct gctggtgcag aacgccaacc ccgactgcaa gaccatcctg 1020
aaggctctcg gccccgcggc caccctggag gagatgatga ccgcctgcca gggcgtgggc 1080
ggccccggcc acaaggcccg cgtgctggcc gaggcgatga gccaggtgac gaacccggcg 1140
accatcatga tgcagcgcgg caacttccgc aaccagcgga agaccgtcaa gtgcttcaac 1200
tgcggcaagg agggccacac cgccaggaac tgccgcgccc cccgcaagaa gggctgctgg 1260
cgctgcggcc gcgaaggaca ccaaatgaaa gattgcactg agagacaggc taatttttta 1320
gggaagatct ggccttccta caagggaagg ccagggaatt ttcttcagag cagaccagag 1380
ccaacagccc caccagaaga gagcttcagg tttggggagg agaaaacaac tccctctcag 1440
aagcaggagc cgatagacaa ggaactgtat cctttaactt ccctcagatc actctttggc 1500
aacgacccct cgtcacagta aggatcggcg gccagctcaa ggaggcgctg ctcgacaccg 1560
gcgccgacga caccgtgctg gaggagatga acctgcccgg caagtggaag cccaagatga 1620
tcggcgggat cgggggcttc atcaaggtgc ggcagtacga ccagatcccc gtggagatct 1680
gcggccacaa ggccatcggc accgtgctgg tgggccccac ccccgtgaac atcatcggcc 1740
gcaacctgct gacccagatc ggctgcaccc tgaacttccc catcagcccc atcgagacgg 1800

tgcccgtgaa gctgaagccg gggatggacg gccccaaggt caagcagtgg cccctgtaag 1860
aattc 1865

<210> 79
<211> 1865
<212> DNA
<213> Artificial Sequence

<220>
<223> Description of Artificial Sequence: GP2

<400> 79
gtcgacgcca ccatgggcgc ccgcgccagc gtgctgagcg gcggcgagct ggacaagtgg 60
gagaagatcc gcctgcgccc cggcggcaag aagaagtaca agctgaagca catcgtgtgg 120
gccagccgcg agctggagcg cttcgccgtg aaccccggcc tgctggagac cagcgagggc 180
tgccgccaga tcctgggcca gctgcagccc agcctgcaga ccggcagcga ggagctgcgc 240
agcctgtaca acaccgtggc caccctgtac tgcgtgcacc agcgcatcga cgtcaaggac 300
accaaggagg ccctggagaa gatcgaggag gagcagaaca agtccaagaa gaaggcccag 360
caggccgccg ccgccgccgg caccggcaac agcagccagg tgagccagaa ctaccccatc 420
gtgcagaacc tgcagggcca gatggtgcac caggccatca gcccccgcac cctgaacgcc 480
tgggtgaagg tggtggagga gaaggccttc agccccgagg tgatccccat gttcagcgcc 540
ctgagcgagg gcgccacccc ccaggacctg aacacgatgt tgaacaccgt gggcggccac 600
caggccgcca tgcagatgct gaaggagacc atcaacgagg aggccgccga gtgggaccgc 660
gtgcaccccg tgcacgccgg ccccatcgcc cccggccaga tgcgcgagcc ccgcggcagc 720
gacatcgccg gcaccaccag caccctgcag gagcagatcg gctggatgac caacaacccc 780
cccatccccg tgggcgagat ctacaagcgg tggatcatcc tgggcctgaa caagatcgtg 840
cggatgtaca gccccaccag catcctggac atccgccagg gccccaagga gcccttccgc 900
gactacgtgg accgcttcta caagaccctg cgcgctgagc aggccagcca ggacgtgaag 960
aactggatga ccgagaccct gctggtgcag aacgccaacc ccgactgcaa gaccatcctg 1020
aaggctctcg gccccgcggc caccctggag gagatgatga ccgcctgcca gggcgtgggc 1080
ggccccggcc acaaggcccg cgtgctggcc gaggcgatga gccaggtgac gaacccggcg 1140
accatcatga tgcagcgcgg caacttccgc aaccagcgga agaccgtcaa gtgcttcaac 1200
tgcggcaagg agggccacac cgccaggaac tgccgcgccc cccgcaagaa gggctgctgg 1260
cgctgcggcc gcgaaggaca ccaaatgaaa gattgcactg agagacaggc taatttttta 1320
gggaagatct ggccttccta caagggaagg ccagggaatt ttcttcagag cagaccagag 1380
ccaacagccc caccagaaga gagcttcagg tttggggagg agaaaacaac tccctctcag 1440
aagcaggagc cgatagacaa ggaactgtat cctttaactt ccctcagatc actctttggc 1500
aacgacccct cgtcacagta aggatcgggg ggcaactcaa ggaagcgctg ctcgatacag 1560
gagcagatga tacagtatta gaagaaatga atttgccagg aaaatggaaa ccaaaaatga 1620
taggggggat cgggggcttc atcaaggtga ggcagtacga ccagatacct gtagaaatct 1680
gtggacataa agctataggt acagtattag taggacctac acctgtcaac ataattggaa 1740
gaaatctgtt gacccagatc ggctgcacct tgaacttccc catcagccct attgagacgg 1800
tgcccgtgaa gttgaagccg gggatggacg gccccaaggt caagcaatgg ccattgtaag 1860
aattc 1865

<210> 80
<211> 2305
<212> DNA
<213> Artificial Sequence

<220>
<223> Description of Artificial Sequence:
      FS(+).proinact.RTopt.YM

<400> 80
gcggccgcga aggacaccaa atgaaagatt gcactgagag acaggctaat tttttaggga 60
agatctggcc ttcctacaag ggaaggccag ggaattttct tcagagcaga ccagagccaa 120
cagccccacc agaagagagc ttcaggtttg gggaggagaa aacaactccc tctcagaagc 180
aggagccgat agacaaggaa ctgtatcctt taacttccct cagatcactc tttggcaacg 240

acccctcgtc acaataagga tcggggggca actcaaggaa gcgctgctcg atacaggagc 300
agatgataca gtattagaag aaatgaattt gccaggaaaa tggaaaccaa aaatgatagg 360
ggggatcggg ggcttcatca aggtgaggca gtacgaccag atacctgtag aaatctgtgg 420
acataaagct ataggtacag tattagtagg acctacacct gtcaacataa ttggaagaaa 480
tctgttgacc cagatcggct gcaccttgaa cttccccatc agccctattg agacggtgcc 540
cgtgaagttg aagccgggga tggacggccc caaggtcaag caatggccat tgaccgagga 600
gaagatcaag gccctggtgg agatctgcac cgagatggag aaggagggca agatcagcaa 660
gatcggcccc gagaacccct acaacacccc cgtgttcgcc atcaagaaga aggacagcac 720
caagtggcgc aagctggtgg acttccgcga gctgaacaag cgcacccagg acttctggga 780
ggtgcagctg ggcatccccc accccgccgg cctgaagaag aagaagagcg tgaccgtgct 840
ggacgtgggc gacgcctact tcagcgtgcc cctggacaag gacttccgca agtacaccgc 900
cttcaccatc cccagcatca acaacgagac ccccggcatc cgctaccagt acaacgtgct 960
gccccagggc tggaagggca gccccgccat cttccagagc agcatgacca agatcctgga 1020
gcccttccgc aagcagaacc ccgacatcgt gatctaccag gcccccctgt acgtgggcag 1080
cgacctggag atcggccagc accgcaccaa gatcgaggag ctgcgccagc acctgctgcg 1140
ctggggcttc accacccccg acaagaagca ccagaaggag ccccccttcc tgtggatggg 1200
ctacgagctg caccccgaca agtggaccgt gcagcccatc atgctgcccg agaaggacag 1260
ctggaccgtg aacgacatcc agaagctggt gggcaagctg aactgggcca gccagatcta 1320
cgccggcatc aaggtgaagc agctgtgcaa gctgctgcgc ggcaccaagg ccctgaccga 1380
ggtgatcccc ctgaccgagg aggccgagct ggagctggcc gagaaccgcg agatcctgaa 1440
ggagcccgtg cacgaggtgt actacgaccc cagcaaggac ctggtggccg agatccagaa 1500
gcagggccag ggccagtgga cctaccagat ctaccaggag cccttcaaga acctgaagac 1560
cggcaagtac gcccgcatgc gcggcgccca caccaacgac gtgaagcagc tgaccgaggc 1620
cgtgcagaag gtgagcaccg agagcatcgt gatctggggc aagatcccca agttcaagct 1680
gcccatccag aaggagacct gggaggcctg gtggatggag tactggcagg ccacctggat 1740
ccccgagtgg gagttcgtga acaccccccc cctggtgaag ctgtggtacc agctggagaa 1800
ggagcccatc gtgggcgccg agaccttcta cgtggacggc gccgccaacc gcgagaccaa 1860
gctgggcaag gccggctacg tgaccgaccg gggccggcag aaggtggtga gcatcgccga 1920
caccaccaac cagaagaccg agctgcaggc catccacctg gccctgcagg acagcggcct 1980
ggaggtgaac atcgtgaccg acagccagta cgccctgggc atcatccagg cccagcccga 2040
caagagcgag agcgagctgg tgagccagat catcgagcag ctgatcaaga aggagaaggt 2100
gtacctggcc tgggtgcccg cccacaaggg catcggcggc aacgagcagg tggacaagct 2160
ggtgagcgcc ggcatccgca aggtgctgtt cctgaacggc atcgatggcg gcatcgtgat 2220
ctaccagtac atggacgacc tgtacgtggg cagcggcggc cctaggatcg attaaaagct 2280
tcccggggct agcaccggtg aattc 2305

<210> 81
<211> 2299
<212> DNA
<213> Artificial Sequence

<220>
<223> Description of Artificial Sequence:
      FS(+).proinact.RTopt.YMWM

<400> 81
gcggccgcga aggacaccaa atgaaagatt gcactgagag acaggctaat tttttaggga 60
agatctggcc ttcctacaag ggaaggccag ggaattttct tcagagcaga ccagagccaa 120
cagccccacc agaagagagc ttcaggtttg gggaggagaa aacaactccc tctcagaagc 180
aggagccgat agacaaggaa ctgtatcctt taacttccct cagatcactc tttggcaacg 240
acccctcgtc acaataagga tcggggggca actcaaggaa gcgctgctcg atacaggagc 300
agatgataca gtattagaag aaatgaattt gccaggaaaa tggaaaccaa aaatgatagg 360
ggggatcggg ggcttcatca aggtgaggca gtacgaccag atacctgtag aaatctgtgg 420
acataaagct ataggtacag tattagtagg acctacacct gtcaacataa ttggaagaaa 480
tctgttgacc cagatcggct gcaccttgaa cttccccatc agccctattg agacggtgcc 540
cgtgaagttg aagccgggga tggacggccc caaggtcaag caatggccat tgaccgagga 600
gaagatcaag gccctggtgg agatctgcac cgagatggag aaggagggca agatcagcaa 660
gatcggcccc gagaacccct acaacacccc cgtgttcgcc atcaagaaga aggacagcac 720
caagtggcgc aagctggtgg acttccgcga gctgaacaag cgcacccagg acttctggga 780

ggtgcagctg ggcatccccc accccgccgg cctgaagaag aagaagagcg tgaccgtgct 840
ggacgtgggc gacgcctact tcagcgtgcc cctggacaag gacttccgca agtacaccgc 900
cttcaccatc cccagcatca acaacgagac ccccggcatc cgctaccagt acaacgtgct 960
gccccagggc tggaagggca gccccgccat cttccagagc agcatgacca agatcctgga 1020
gcccttccgc aagcagaacc ccgacatcgt gatctaccag gcccccctgt acgtgggcag 1080
cgacctggag atcggccagc accgcaccaa gatcgaggag ctgcgccagc acctgctgcg 1140
ctggggcttc accacccccg acaagaagca ccagaaggag ccccccttcc tgcccatcga 1200
gctgcacccc gacaagtgga ccgtgcagcc catcatgctg cccgagaagg acagctggac 1260
cgtgaacgac atccagaagc tggtgggcaa gctgaactgg gccagccaga tctacgccgg 1320
catcaaggtg aagcagctgt gcaagctgct gcgcggcacc aaggccctga ccgaggtgat 1380
ccccctgacc gaggaggccg agctggagct ggccgagaac cgcgagatcc tgaaggagcc 1440
cgtgcacgag gtgtactacg accccagcaa ggacctggtg gccgagatcc agaagcaggg 1500
ccagggccag tggacctacc agatctacca ggagcccttc aagaacctga agaccggcaa 1560
gtacgcccgc atgcgcggcg cccacaccaa cgacgtgaag cagctgaccg aggccgtgca 1620
gaaggtgagc accgagagca tcgtgatctg gggcaagatc cccaagttca agctgcccat 1680
ccagaaggag acctgggagg cctggtggat ggagtactgg caggccacct ggatccccga 1740
gtgggagttc gtgaacaccc cccccctggt gaagctgtgg taccagctgg agaaggagcc 1800
catcgtgggc gccgagacct tctacgtgga cggcgccgcc aaccgcgaga ccaagctggg 1860
caaggccggc tacgtgaccg accggggccg gcagaaggtg gtgagcatcg ccgacaccac 1920
caaccagaag accgagctgc aggccatcca cctggccctg caggacagcg gcctggaggt 1980
gaacatcgtg accgacagcc agtacgccct gggcatcatc caggcccagc ccgacaagag 2040
cgagagcgag ctggtgagcc agatcatcga gcagctgatc aagaaggaga aggtgtacct 2100
ggcctgggtg cccgcccaca agggcatcgg cggcaacgag caggtggaca agctggtgag 2160
cgccggcatc cgcaaggtgc tgttcctgaa cggcatcgat ggcggcatcg tgatctacca 2220
gtacatggac gacctgtacg tgggcagcgg cggccctagg atcgattaaa agcttcccgg 2280
ggctagcacc ggtgaattc 2299

<210> 82
<211> 2306
<212> DNA
<213> Artificial Sequence

<220>
<223> Description of Artificial Sequence:
      FS(-).protmod.RTopt.YM

<400> 82
gcggccgcga aggacaccaa atgaaagatt gcactgagag acaggctaat ttcttccgcg 60
aggacctggc cttcctgcag ggcaaggccc gcgagttcag cagcgagcag acccgcgcca 120
acagccccac ccgccgcgag ctgcaggtgt ggggcggcga gaacaacagc ctgagcgagg 180
ccggcgccga ccgccagggc accgtgagct tcaacttccc ccagatcacc ctgtggcagc 240
gccccctggt gaccatcagg atcggcggcc agctcaagga ggcgctgctc gacaccggcg 300
ccgacgacac cgtgctggag gagatgaacc tgcccggcaa gtggaagccc aagatgatcg 360
gcgggatcgg gggcttcatc aaggtgcggc agtacgacca gatccccgtg gagatctgcg 420
gccacaaggc catcggcacc gtgctggtgg gccccacccc cgtgaacatc atcggccgca 480
acctgctgac ccagatcggc tgcaccctga acttccccat cagccccatc gagacggtgc 540
ccgtgaagct gaagccgggg atggacggcc ccaaggtcaa gcagtggccc ctgaccgagg 600
agaagatcaa ggccctggtg gagatctgca ccgagatgga gaaggagggc aagatcagca 660
agatcggccc cgagaacccc tacaacaccc ccgtgttcgc catcaagaag aaggacagca 720
ccaagtggcg caagctggtg gacttccgcg agctgaacaa gcgcacccag gacttctggg 780
aggtgcagct gggcatcccc caccccgccg gcctgaagaa gaagaagagc gtgaccgtgc 840
tggacgtggg cgacgcctac ttcagcgtgc ccctggacaa ggacttccgc aagtacaccg 900
ccttcaccat ccccagcatc aacaacgaga cccccggcat ccgctaccag tacaacgtgc 960
tgccccaggg ctggaagggc agccccgcca tcttccagag cagcatgacc aagatcctgg 1020
agcccttccg caagcagaac cccgacatcg tgatctacca ggcccccctg tacgtgggca 1080
gcgacctgga gatcggccag caccgcacca agatcgagga gctgcgccag cacctgctgc 1140
gctggggctt caccaccccc gacaagaagc accagaagga gccccccttc ctgtggatgg 1200
gctacgagct gcaccccgac aagtggaccg tgcagcccat catgctgccc gagaaggaca 1260
gctggaccgt gaacgacatc cagaagctgg tgggcaagct gaactgggcc agccagatct 1320

acgccggcat caaggtgaag cagctgtgca agctgctgcg cggcaccaag gccctgaccg 1380
aggtgatccc cctgaccgag gaggccgagc tggagctggc cgagaaccgc gagatcctga 1440
aggagcccgt gcacgaggtg tactacgacc ccagcaagga cctggtggcc gagatccaga 1500
agcagggcca gggccagtgg acctaccaga tctaccagga gcccttcaag aacctgaaga 1560
ccggcaagta cgcccgcatg cgcggcgccc acaccaacga cgtgaagcag ctgaccgagg 1620
ccgtgcagaa ggtgagcacc gagagcatcg tgatctgggg caagatcccc aagttcaagc 1680
tgcccatcca gaaggagacc tgggaggcct ggtggatgga gtactggcag gccacctgga 1740
tccccgagtg ggagttcgtg aacacccccc ccctggtgaa gctgtggtac cagctggaga 1800
aggagcccat cgtgggcgcc gagaccttct acgtggacgg cgccgccaac cgcgagacca 1860
agctgggcaa ggccggctac gtgaccgacc ggggccggca gaaggtggtg agcatcgccg 1920
acaccaccaa ccagaagacc gagctgcagg ccatccacct ggccctgcag gacagcggcc 1980
tggaggtgaa catcgtgacc gacagccagt acgccctggg catcatccag gcccagcccg 2040
acaagagcga gagcgagctg gtgagccaga tcatcgagca gctgatcaag aaggagaagg 2100
tgtacctggc ctgggtgccc gcccacaagg gcatcggcgg caacgagcag gtggacaagc 2160
tggtgagcgc cggcatccgc aaggtgctgt tcctgaacgg catcgatggc ggcatcgtga 2220
tctaccagta catggacgac ctgtacgtgg gcagcggcgg ccctaggatc gattaaaagc 2280
ttcccggggc tagcaccggt gaattc 2306

<210> 83
<211> 2300
<212> DNA
<213> Artificial Sequence

<220>
<223> Description of Artificial Sequence:
      FS(-).protmod.RTopt.YMWM

<400> 83
gcggccgcga aggacaccaa atgaaagatt gcactgagag acaggctaat ttcttccgcg 60
aggacctggc cttcctgcag ggcaaggccc gcgagttcag cagcgagcag acccgcgcca 120
acagccccac ccgccgcgag ctgcaggtgt ggggcggcga gaacaacagc ctgagcgagg 180
ccggcgccga ccgccagggc accgtgagct tcaacttccc ccagatcacc ctgtggcagc 240
gccccctggt gaccatcagg atcggcggcc agctcaagga ggcgctgctc gacaccggcg 300
ccgacgacac cgtgctggag gagatgaacc tgcccggcaa gtggaagccc aagatgatcg 360
gcgggatcgg gggcttcatc aaggtgcggc agtacgacca gatccccgtg gagatctgcg 420
gccacaaggc catcggcacc gtgctggtgg gccccacccc cgtgaacatc atcggccgca 480
acctgctgac ccagatcggc tgcaccctga acttccccat cagccccatc gagacggtgc 540
ccgtgaagct gaagccgggg atggacggcc ccaaggtcaa gcagtggccc ctgaccgagg 600
agaagatcaa ggccctggtg gagatctgca ccgagatgga gaaggagggc aagatcagca 660
agatcggccc cgagaacccc tacaacaccc ccgtgttcgc catcaagaag aaggacagca 720
ccaagtggcg caagctggtg gacttccgcg agctgaacaa gcgcacccag gacttctggg 780
aggtgcagct gggcatcccc caccccgccg gcctgaagaa gaagaagagc gtgaccgtgc 840
tggacgtggg cgacgcctac ttcagcgtgc ccctggacaa ggacttccgc aagtacaccg 900
ccttcaccat ccccagcatc aacaacgaga cccccggcat ccgctaccag tacaacgtgc 960
tgccccaggg ctggaagggc agccccgcca tcttccagag cagcatgacc aagatcctgg 1020
agcccttccg caagcagaac cccgacatcg tgatctacca ggcccccctg tacgtgggca 1080
gcgacctgga gatcggccag caccgcacca agatcgagga gctgcgccag cacctgctgc 1140
gctggggctt caccaccccc gacaagaagc accagaagga gccccccttc ctgcccatcg 1200
agctgcaccc cgacaagtgg accgtgcagc ccatcatgct gcccgagaag gacagctgga 1260
ccgtgaacga catccagaag ctggtgggca agctgaactg ggccagccag atctacgccg 1320
gcatcaaggt gaagcagctg tgcaagctgc tgcgcggcac caaggccctg accgaggtga 1380
tccccctgac cgaggaggcc gagctggagc tggccgagaa ccgcgagatc ctgaaggagc 1440
ccgtgcacga ggtgtactac gaccccagca aggacctggt ggccgagatc cagaagcagg 1500
gccagggcca gtggacctac cagatctacc aggagccctt caagaacctg aagaccggca 1560
agtacgcccg catgcgcggc gcccacacca acgacgtgaa gcagctgacc gaggccgtgc 1620
agaaggtgag caccgagagc atcgtgatct ggggcaagat ccccaagttc aagctgccca 1680
tccagaagga gacctgggag gcctggtgga tggagtactg gcaggccacc tggatccccg 1740
agtgggagtt cgtgaacacc ccccccctgg tgaagctgtg gtaccagctg gagaaggagc 1800
ccatcgtggg cgccgagacc ttctacgtgg acggcgccgc caaccgcgag accaagctgg 1860

gcaaggccgg ctacgtgacc gaccggggcc ggcagaaggt ggtgagcatc gccgacacca 1920
ccaaccagaa gaccgagctg caggccatcc acctggccct gcaggacagc ggcctggagg 1980
tgaacatcgt gaccgacagc cagtacgccc tgggcatcat ccaggcccag cccgacaaga 2040
gcgagagcga gctggtgagc cagatcatcg agcagctgat caagaaggag aaggtgtacc 2100
tggcctgggt gcccgcccac aagggcatcg gcggcaacga gcaggtggac aagctggtga 2160
gcgccggcat ccgcaaggtg ctgttcctga acggcatcga tggcggcatc gtgatctacc 2220
agtacatgga cgacctgtac gtgggcagcg gcggccctag gatcgattaa aagcttcccg 2280
gggctagcac cggtgaattc 2300

<210> 84
<211> 2312
<212> DNA
<213> Artificial Sequence

<220>
<223> Description of Artificial Sequence:
      FS(-).protmod.RTopt(+)

<400> 84
gcggccgcga aggacaccaa atgaaagatt gcactgagag acaggctaat ttcttccgcg 60
aggacctggc cttcctgcag ggcaaggccc gcgagttcag cagcgagcag acccgcgcca 120
acagccccac ccgccgcgag ctgcaggtgt ggggcggcga gaacaacagc ctgagcgagg 180
ccggcgccga ccgccagggc accgtgagct tcaacttccc ccagatcacc ctgtggcagc 240
gccccctggt gaccatcagg atcggcggcc agctcaagga ggcgctgctc gacaccggcg 300
ccgacgacac cgtgctggag gagatgaacc tgcccggcaa gtggaagccc aagatgatcg 360
gcgggatcgg gggcttcatc aaggtgcggc agtacgacca gatccccgtg gagatctgcg 420
gccacaaggc catcggcacc gtgctggtgg gccccacccc cgtgaacatc atcggccgca 480
acctgctgac ccagatcggc tgcaccctga acttccccat cagccccatc gagacggtgc 540
ccgtgaagct gaagccgggg atggacggcc ccaaggtcaa gcagtggccc ctgaccgagg 600
agaagatcaa ggccctggtg gagatctgca ccgagatgga gaaggagggc aagatcagca 660
agatcggccc cgagaacccc tacaacaccc ccgtgttcgc catcaagaag aaggacagca 720
ccaagtggcg caagctggtg gacttccgcg agctgaacaa gcgcacccag gacttctggg 780
aggtgcagct gggcatcccc caccccgccg gcctgaagaa gaagaagagc gtgaccgtgc 840
tggacgtggg cgacgcctac ttcagcgtgc ccctggacaa ggacttccgc aagtacaccg 900
ccttcaccat ccccagcatc aacaacgaga cccccggcat ccgctaccag tacaacgtgc 960
tgccccaggg ctggaagggc agccccgcca tcttccagag cagcatgacc aagatcctgg 1020
agcccttccg caagcagaac cccgacatcg tgatctacca gtacatggac gacctgtacg 1080
tgggcagcga cctggagatc ggccagcacc gcaccaagat cgaggagctg cgccagcacc 1140
tgctgcgctg gggcttcacc acccccgaca agaagcacca gaaggagccc cccttcctgt 1200
ggatgggcta cgagctgcac cccgacaagt ggaccgtgca gcccatcatg ctgcccgaga 1260
aggacagctg gaccgtgaac gacatccaga agctggtggg caagctgaac tgggccagcc 1320
agatctacgc cggcatcaag gtgaagcagc tgtgcaagct gctgcgcggc accaaggccc 1380
tgaccgaggt gatccccctg accgaggagg ccgagctgga gctggccgag aaccgcgaga 1440
tcctgaagga gcccgtgcac gaggtgtact acgaccccag caaggacctg gtggccgaga 1500
tccagaagca gggccagggc cagtggacct accagatcta ccaggagccc ttcaagaacc 1560
tgaagaccgg caagtacgcc cgcatgcgcg gcgcccacac caacgacgtg aagcagctga 1620
ccgaggccgt gcagaaggtg agcaccgaga gcatcgtgat ctggggcaag atccccaagt 1680
tcaagctgcc catccagaag gagacctggg aggcctggtg gatggagtac tggcaggcca 1740
cctggatccc cgagtgggag ttcgtgaaca ccccccccct ggtgaagctg tggtaccagc 1800
tggagaagga gcccatcgtg ggcgccgaga ccttctacgt ggacggcgcc gccaaccgcg 1860
agaccaagct gggcaaggcc ggctacgtga ccgaccgggg ccggcagaag gtggtgagca 1920
tcgccgacac caccaaccag aagaccgagc tgcaggccat ccacctggcc ctgcaggaca 1980
gcggcctgga ggtgaacatc gtgaccgaca gccagtacgc cctgggcatc atccaggccc 2040
agcccgacaa gagcgagagc gagctggtga gccagatcat cgagcagctg atcaagaagg 2100
agaaggtgta cctggcctgg gtgcccgccc acaagggcat cggcggcaac gagcaggtgg 2160
acaagctggt gagcgccggc atccgcaagg tgctgttcct gaacggcatc gatggcggca 2220
tcgtgatcta ccagtacatg gacgacctgt acgtgggcag cggcggccct aggatcgatt 2280
aaaagcttcc cggggctagc accggtgaat tc 2312

<210> 85
<211> 306
<212> DNA
<213> Human immunodeficiency virus

<400> 85
atggagccag tagatcctag attagagccc tggaagcatc caggaagtca gcctaagact 60
gcttgtacaa attgctattg taaaaagtgt tgctttcatt gccaagtttg tttcataaca 120
aaaggcttag gcatctccta tggcaggaag aagcggagac agcgacgaag agctcctcca 180
gacagtgagg ttcatcaagt ttctctacca aagcaacccg cttcccagcc ccaaggggac 240
ccgacaggcc cgaaggaatc gaagaagaag gtggagagag agacagagac agatccagtc 300
cattag 306

<210> 86
<211> 101
<212> PRT
<213> Human immunodeficiency virus

<400> 86
Met Glu Pro Val Asp Pro Arg Leu Glu Pro Trp Lys His Pro Gly Ser
1               5                   10                  15

Gln Pro Lys Thr Ala Cys Thr Asn Cys Tyr Cys Lys Lys Cys Cys Phe
            20                  25                  30

His Cys Gln Val Cys Phe Ile Thr Lys Gly Leu Gly Ile Ser Tyr Gly
        35                  40                  45

Arg Lys Lys Arg Arg Gln Arg Arg Arg Ala Pro Pro Asp Ser Glu Val
    50                  55                  60

His Gln Val Ser Leu Pro Lys Gln Pro Ala Ser Gln Pro Gln Gly Asp
65                  70                  75                  80

Pro Thr Gly Pro Lys Glu Ser Lys Lys Lys Val Glu Arg Glu Thr Glu
                85                  90                  95

Thr Asp Pro Val His
            100


<210> 87
<211> 306
<212> DNA
<213> Artificial Sequence

<220>
<223> Description of Artificial Sequence: tat.SF162.opt

<400> 87
atggagcccg tggacccccg cctggagccc tggaagcacc ccggcagcca gcccaagacc 60
gcctgcacca actgctactg caagaagtgc tgcttccact gccaggtgtg cttcatcacc 120
aagggcctgg gcatcagcta cggccgcaag aagcgccgcc agcgccgccg cgcccccccc 180
gacagcgagg tgcaccaggt gagcctgccc aagcagcccg ccagccagcc ccagggcgac 240
cccaccggcc ccaaggagag caagaagaag gtggagcgcg agaccgagac cgaccccgtg 300
cactag 306

<210> 88
<211> 306

<212> DNA
<213> Artificial Sequence

<220>
<223> Description of Artificial Sequence:
      tat.cys22.SF162.opt

<400> 88
atggagcccg tggacccccg cctggagccc tggaagcacc ccggcagcca gcccaagacc 60
gccggcacca actgctactg caagaagtgc tgcttccact gccaggtgtg cttcatcacc 120
aagggcctgg gcatcagcta cggccgcaag aagcgccgcc agcgccgccg cgcccccccc 180
gacagcgagg tgcaccaggt gagcctgccc aagcagcccg ccagccagcc ccagggcgac 240
cccaccggcc ccaaggagag caagaagaag gtggagcgcg agaccgagac cgaccccgtg 300
cactag                                                            306

<210> 89
<211> 168
<212> DNA
<213> Artificial Sequence

<220>
<223> Description of Artificial Sequence:
      tatamino.SF162.opt

<400> 89
atggagcccg tggacccccg cctggagccc tggaagcacc ccggcagcca gcccaagacc 60
gcctgcacca actgctactg caagaagtgc tgcttccact gccaggtgtg cttcatcacc 120
aagggcctgg gcatcagcta cggccgcaag aagcgccgcc agcgccgc              168

<210> 90
<211> 102
<212> PRT
<213> Artificial Sequence

<220>
<223> Description of Artificial Sequence: tat cys22
      SF162 protein

<400> 90
Met Glu Pro Val Asp Pro Arg Leu Glu Pro Trp Lys His Pro Gly Ser
  1               5                  10                  15

Gln Pro Lys Thr Ala Gly Thr Asn Cys Tyr Cys Lys Lys Cys Cys Phe
             20                  25                  30

His Cys Gln Val Cys Phe Ile Thr Lys Gly Leu Gly Ile Ser Tyr Gly
         35                  40                  45

Arg Lys Lys Arg Arg Gln Arg Arg Arg Ala Pro Pro Asp Ser Glu Val
     50                  55                  60

His Gln Val Ser Leu Pro Lys Gln Pro Ala Ser Gln Pro Gln Gly Asp
 65                  70                  75                  80

Pro Thr Gly Pro Lys Glu Ser Lys Lys Lys Val Glu Arg Glu Thr Glu
                 85                  90                  95

Thr Asp Pro Val His Glx
            100

## Description

### TECHNICAL FIELD

Synthetic expression cassettes encoding the HIV polypeptides (e.g., Gag-, pol-, prot-, reverse transcriptase, Env- or tat-containing polypeptides) are described, as are uses of the expression cassettes. The present invention relates to the efficient expression of HIV polypeptides in a variety of cell types. Further, the invention provides methods of producing Virus-Like Particles (VLPs), as well as, uses of the VLPs and high level expression of oligomeric envelope proteins.

### BACKGROUND OF THE INVENTION

Acquired immune deficiency syndrome (AIDS) is recognized as one of the greatest health threats facing modern medicine. There is, as yet, no cure for this disease.

In 1983-1984, three groups independently identified the suspected etiological agent of AIDS. See, e.g., Barre-Sinoussi et al. (1983) Science 220:868-871; Montagnier et al., in Human T-Cell Leukemia Viruses (Gallo, Essex & Gross, eds., 1984); Vilmer et al. (1984) The Lancet 1:753; Popovic et al. (1984) Science 224:497-500; Levy et al. (1984) Science 225:840-842. These isolates were variously called lymphadenopathy-associated virus (LAV), human T-cell lymphotropic virus type III (HTLV-III), or AIDS-associated retrovirus (ARV). All of these isolates are strains of the same virus, and were later collectively named Human Immunodeficiency Virus (HIV). With the isolation of a related AIDS-causing virus, the strains originally called HIV are now termed HIV-1 and the related virus is called HIV-2 See, e.g., Guyader et al. (1987) Nature 326:662-669; Brun-Vezinet et al. (1986) Science 233:343-346; Clavel et al. (1986) Nature 324:691-695.

A great deal of information has been gathered about the HIV virus, however, to date an effective vaccine has not been identified. Several targets for vaccine development have been examined including the *env, Gag, pol* and tat gene products encoded by HIV.

Haas, et al., *(Current Biology* 6(3):315-324, 1996) suggested that selective codon usage by HIV-1 appeared to account for a substantial fraction of the inefficiency of viral protein synthesis. Andre, et al., *(J. Virol.* 72(2):1497-1503, 1998) described an increased immune response elicited by DNA vaccination employing a synthetic gp120 sequence with optimized codon usage. Schneider, et al., *(J Virol.* 71(7):4892-4903, 1997) discuss inactivation of inhibitory (or instability) elements (INS) located within the coding sequences of the Gag and Gag-protease coding sequences.

The Gag proteins of HIV-1 are necessary for the assembly of virus-like particles. HIV-1 Gag proteins are involved in many stages of the life cycle of the virus including, assembly, virion maturation after particle release, and early post-entry steps in virus replication. The roles of HIV-1 Gag proteins are numerous and complex (Freed, E.O., *Virology* 251:1-15, 1998).

Wolf, et al., (PCT International Application, WO 96/30523, published 3 October 1996; European Patent Application, Publication No. 0 449 116 A1, published 2 October 1991) have described the use of altered pr55 *Gag* of HIV-1 to act as a non-infectious retroviral-like particulate carrier, in particular, for the presentation of immunologically important epitopes. Wang, et al., *(Virology* 200:524-534, 1994) describe a system to study assembly of HIV Gag-β-galactosidase fusion proteins into virions. They describe the construction of sequences encoding HIV Gag-β-galactosidase fusion proteins, the expression of such sequences in the presence of HIV Gag proteins, and assembly of these proteins into virus particles.

Recently, Shiver, et al., (PCT International Application, WO 98/34640, published 13 August 1998) described altering HIV-1 (CAM1) *Gag* coding sequences to produce synthetic DNA molecules encoding HIV *Gag* and modifications of HIV *Gag.* The codons of the synthetic molecules were codons preferred by a projected host cell.

The envelope protein of HIV-1 is a glycoprotein of about 160 kD (gp160). During virus infection of the host cell, gp160 is cleaved by host cell proteases to form gp120 and the integral membrane protein, gp41. The gp41 portion is anchored in (and spans) the membrane bilayer of virion, while the gp120 segment protrudes into the surrounding environment. As there is no covalent attachment between gp120 and gp41, free gp120 is released from the surface of virions and infected cells.

Haas, et al., *(Current Biology* 6(3):315-324, 1996) suggested that selective codon usage by HIV-1 appeared to account for a substantial fraction of the inefficiency of viral protein synthesis. Andre, et al., *(J. Virol.* 72(2):1497-1503, 1998) described an increased immune response elicited by DNA vaccination employing a synthetic gp120 sequence with optimized codon usage.

### SUMMARY OF THE INVENTION

The present invention relates to improved expression of HIV *Env-, tat-, pol-, prot-, reverse transcriptase,* or Gag-containing polypeptides and production of virus-like particles.

In one embodiment the present invention includes an expression cassette, comprising a polynucleotide encoding an HIV Gag polypeptide comprising a sequence having at least 90% sequence identity to the sequence presented as SEQ ID NO:20. In certain embodiments, the polynucleotide sequence encoding said Gag polypeptide comprises a sequence having at least 90% sequence identity to the sequence presented as SEQ ID NO:9 or SEQ ID NO:4. The expression cassettes may further include a polynucleotide sequence encoding an HIV protease polypeptide, for example a nucleotide sequence having at least 90% sequence identity to a sequence selected from the group consisting of: SEQ ID NO:5, SEQ ID NO:78, and SEQ ID NO:79. The expression cassettes may further include a polynucleotide sequence encoding an HIV reverse transcriptase polypeptide, for example a sequence having at least 90% sequence identity to a sequence selected from the group consisting of: SEQ ID NO:80, SEQ ID NO:81, SEQ ID NO:82, SEQ ID NO:83, and SEQ ID NO:84. The expression cassettes may further include a polynucleotide sequence encoding an HIV tat polypeptide, for example a sequence selected from the group consisting of: SEQ ID NO:87, SEQ ID NO:88, and SEQ ID NO:89. The expression cassettes may further include a polynucleotide sequence encoding an HIV *polymerase* polypeptide, for example a sequence having at least 90% sequence identity to the sequence presented as SEQ ID NO:6. The expression cassettes may include a polynucleotide sequence encoding an HIV *polymerase* polypeptide, wherein (i) the nucleotide sequence encoding said polypeptide comprises a sequence having at least 90% sequence identity to the sequence presented as SEQ ID NO:4, and (ii) wherein the sequence is modified by deletions of coding regions corresponding to reverse transcriptase and integrase. The expression cassettes described above may preserves T-helper cell and CTL epitopes. The expression cassettes may further include a polynucleotide sequence encoding an HCV core polypeptide, for example a sequence having at least 90% sequence identity to the sequence presented as SEQ ID NO: 7.

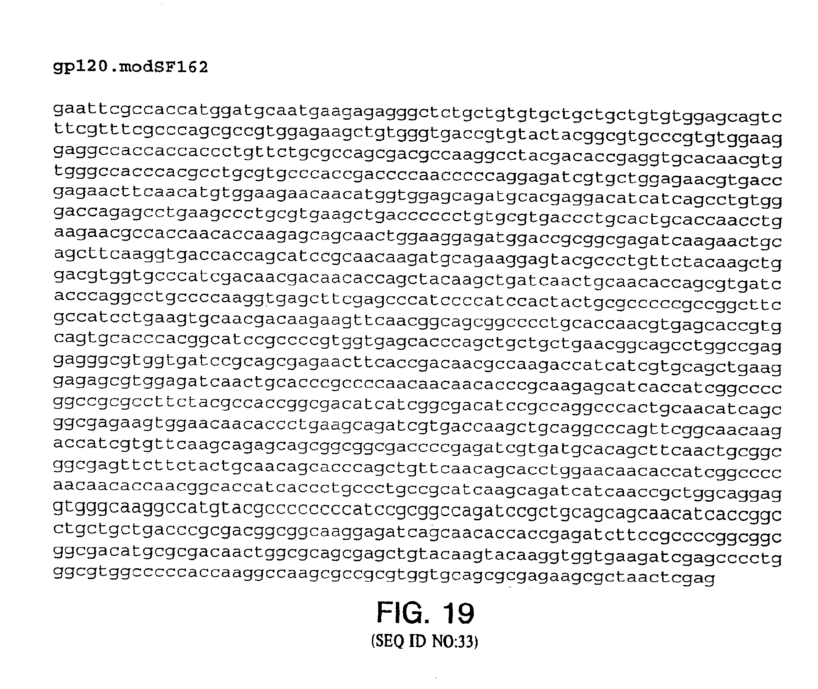

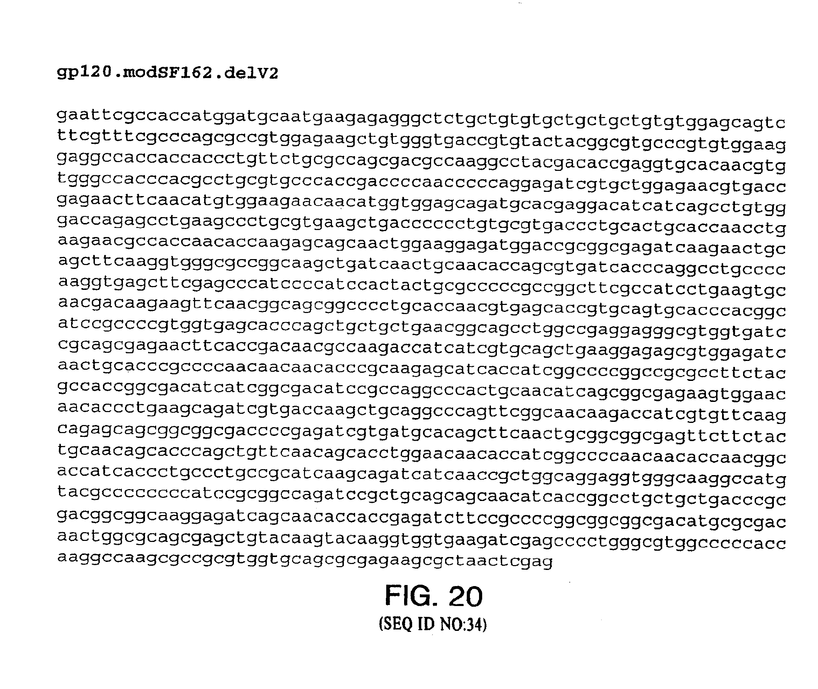

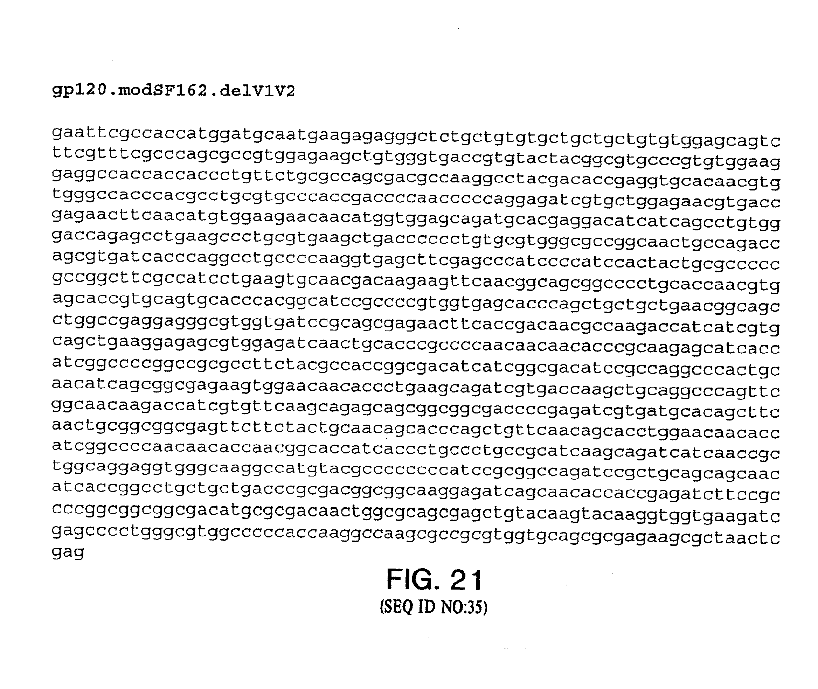

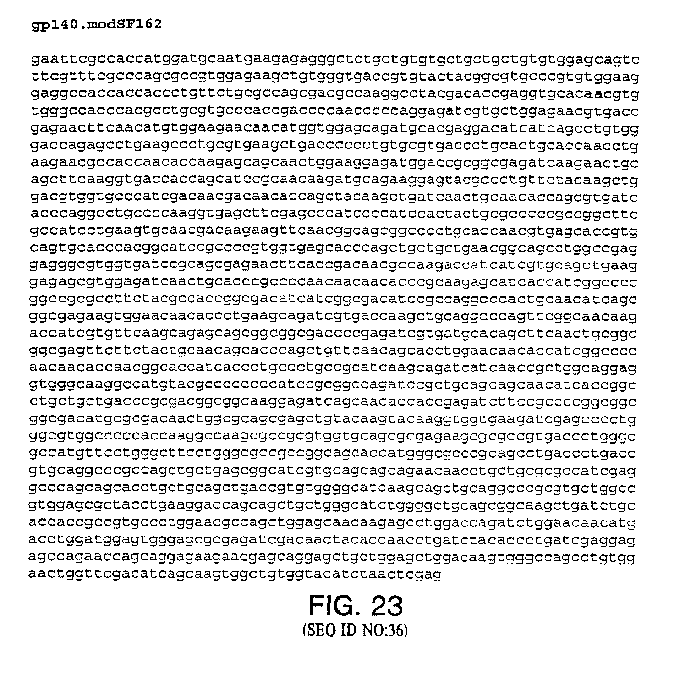

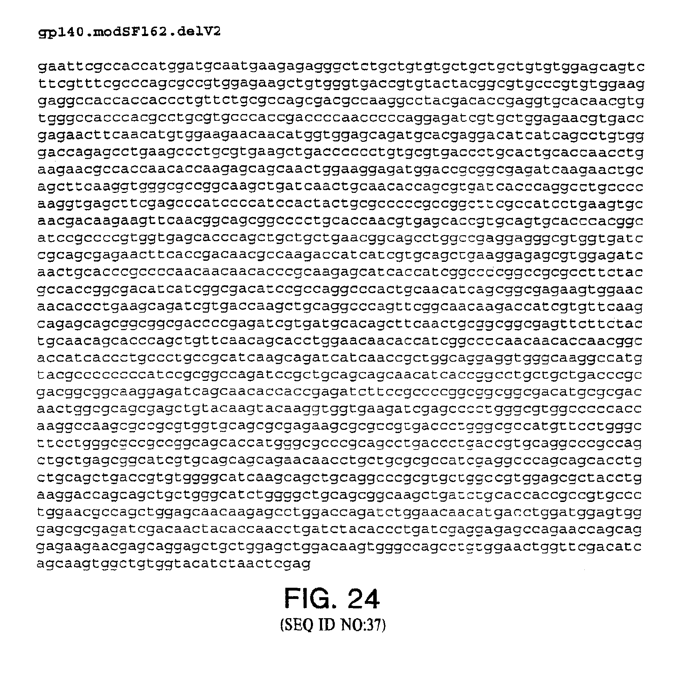

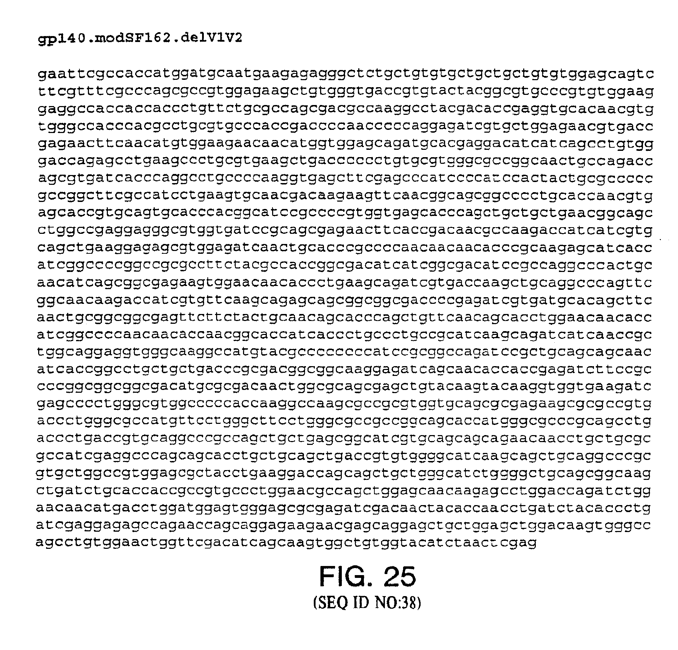

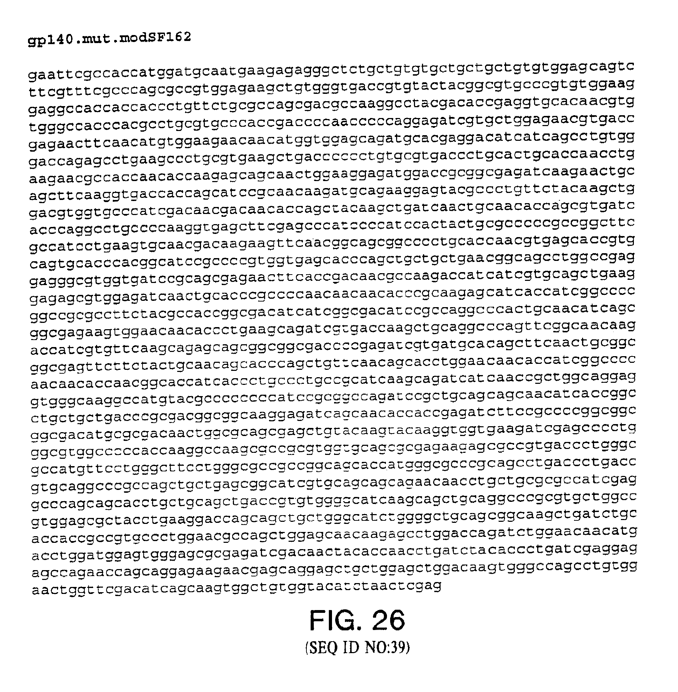

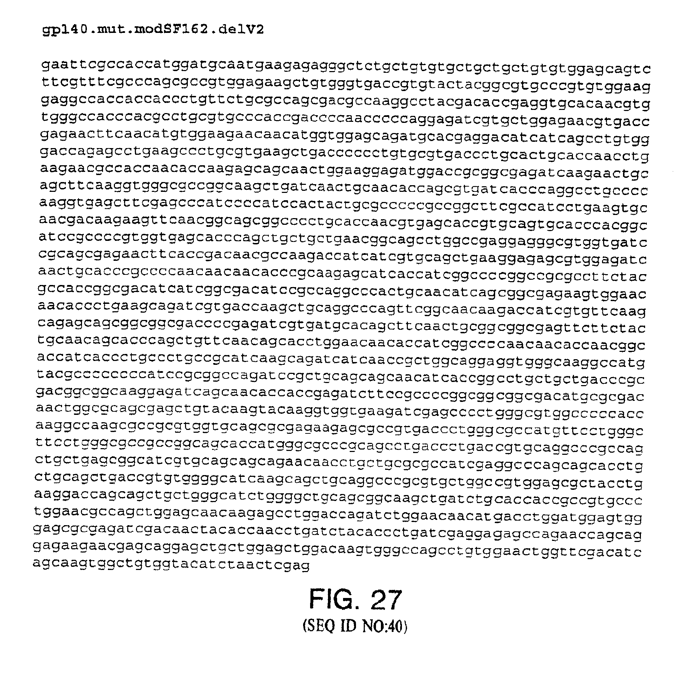

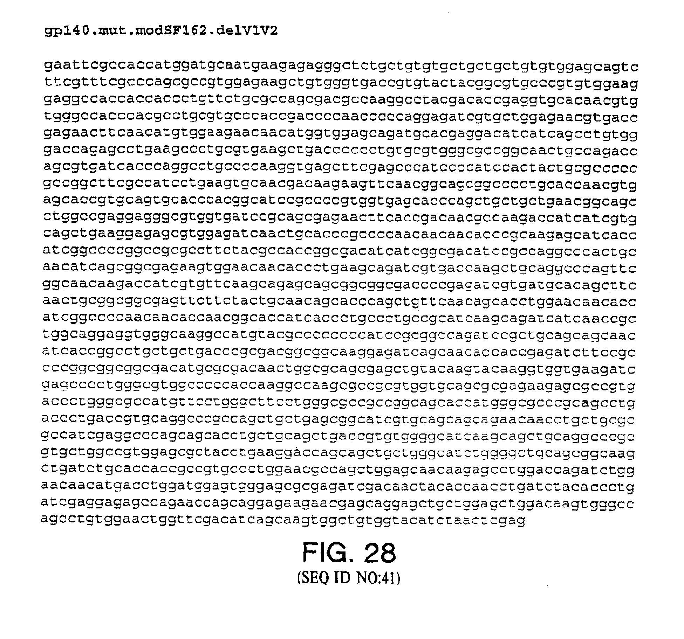

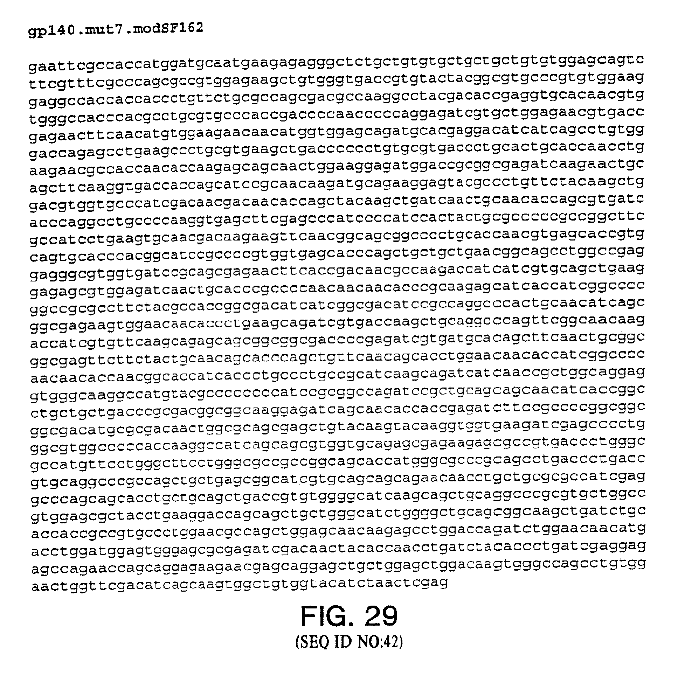

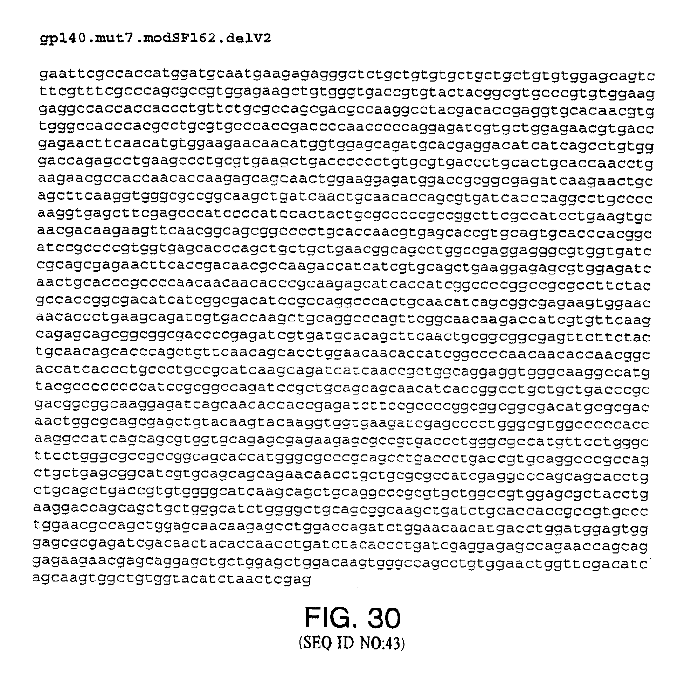

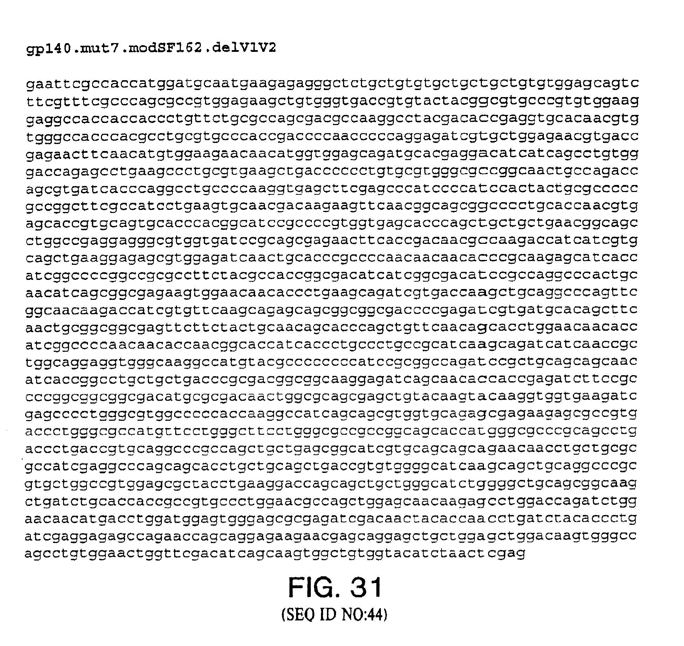

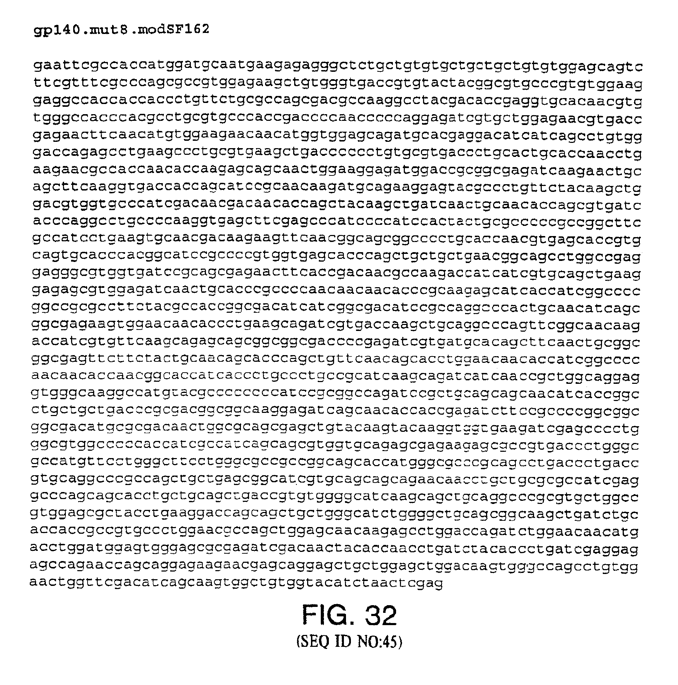

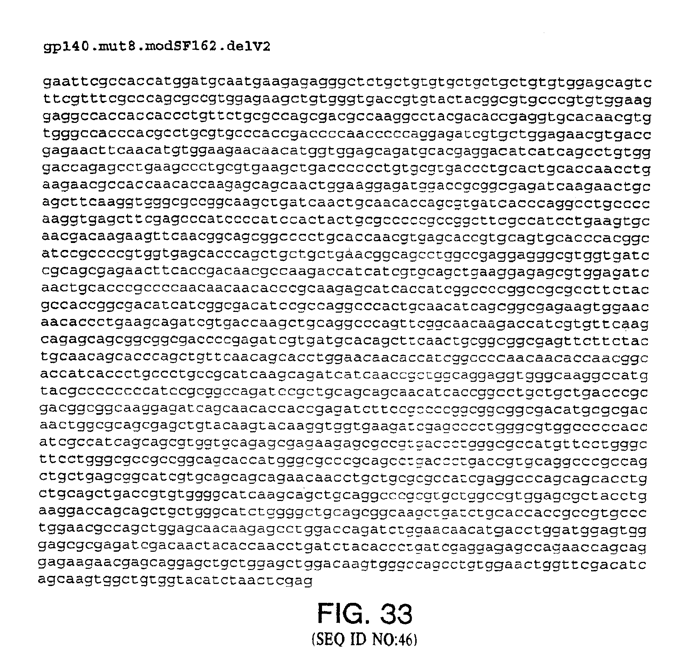

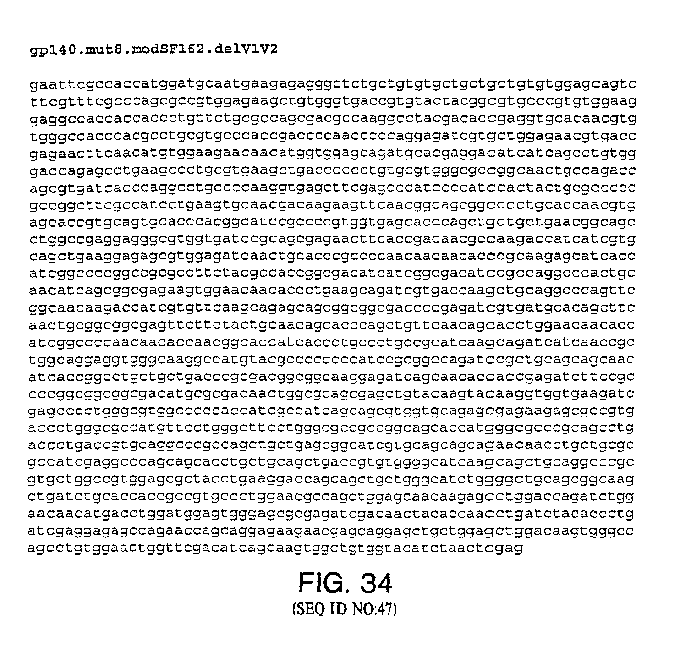

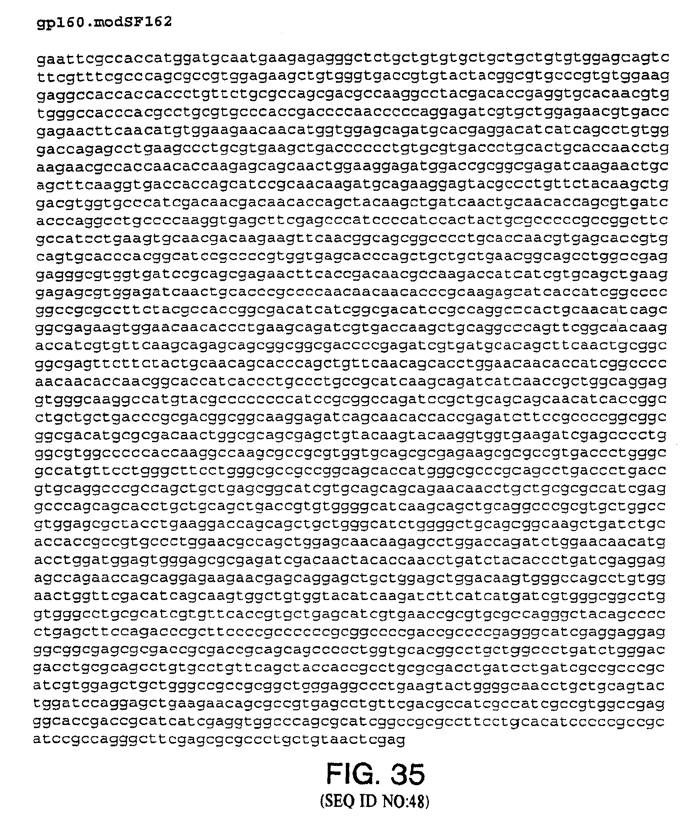

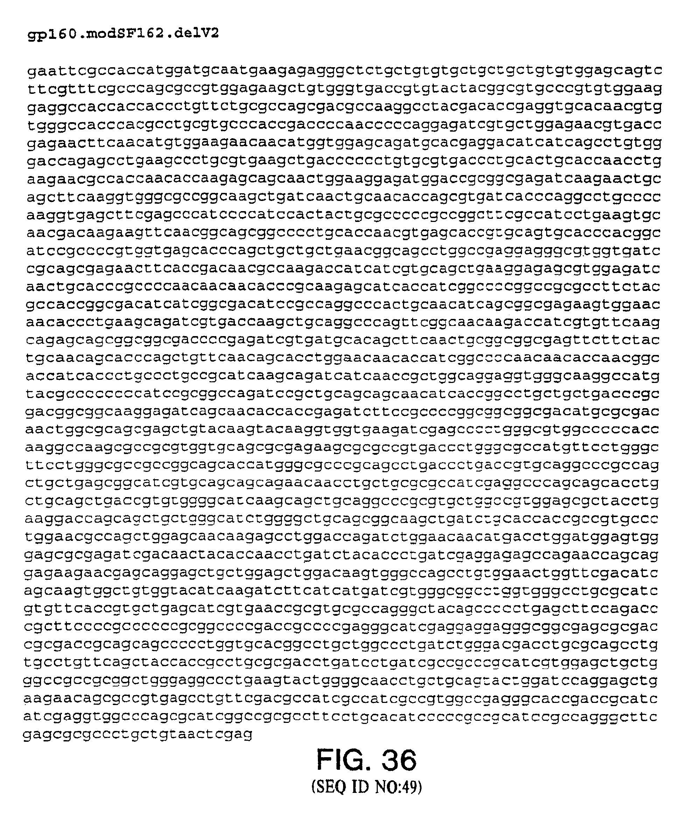

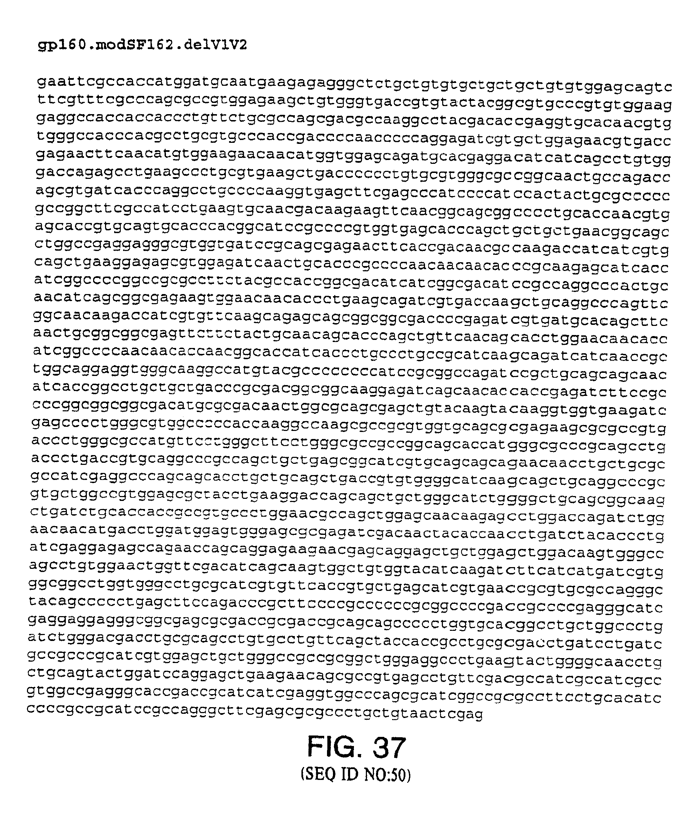

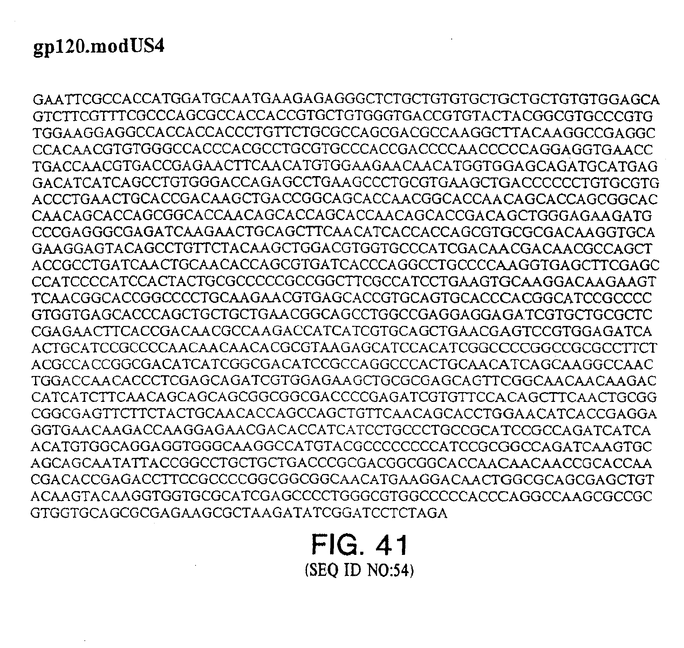

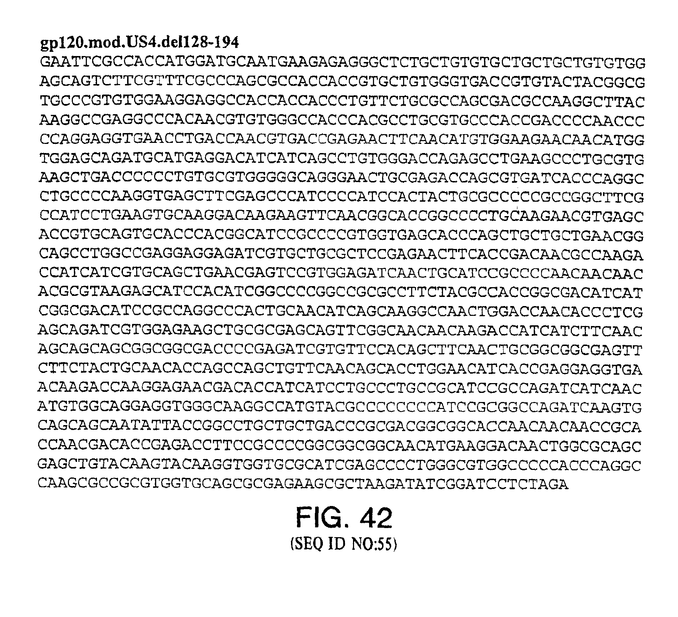

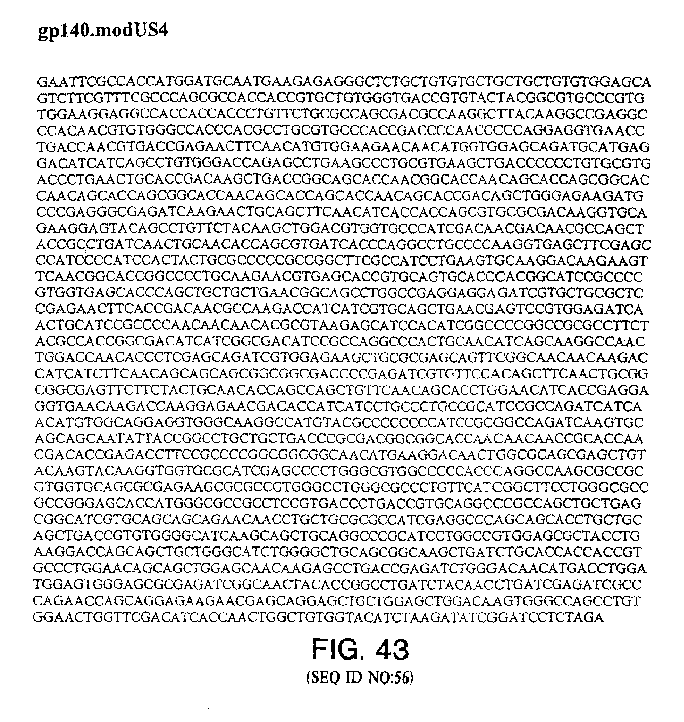

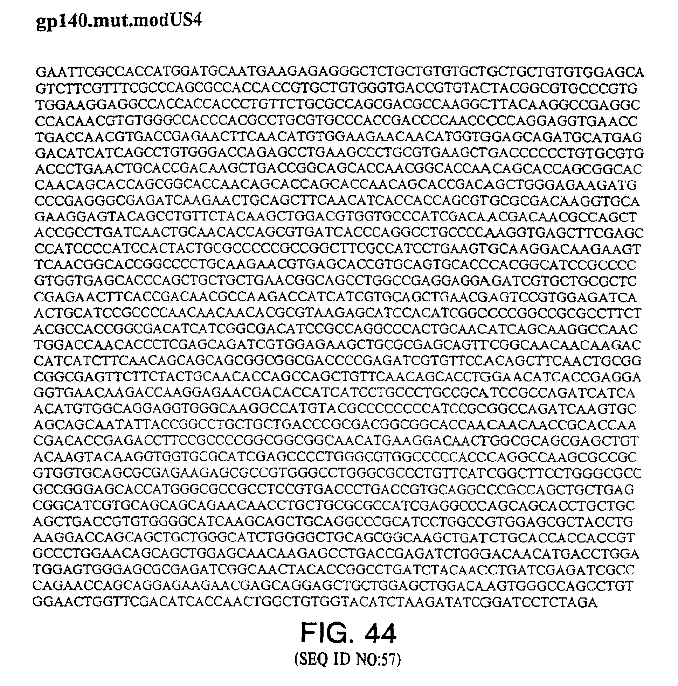

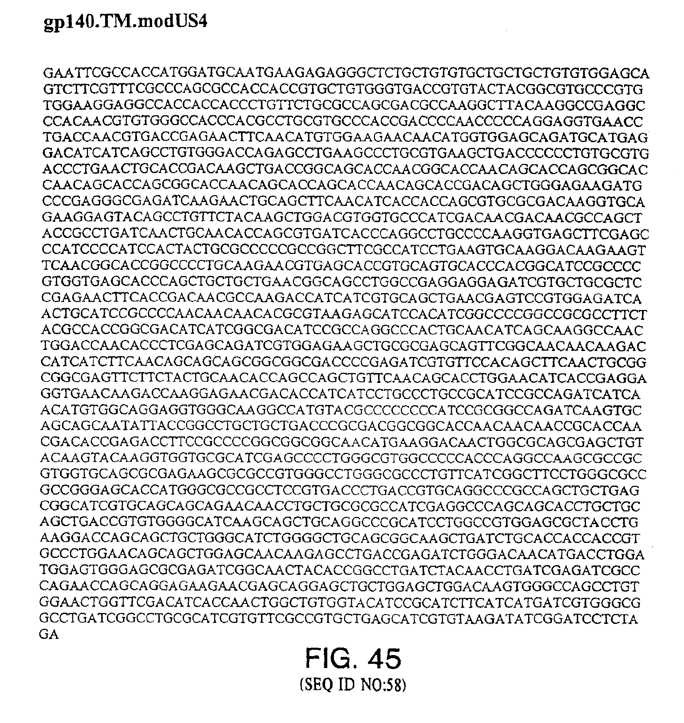

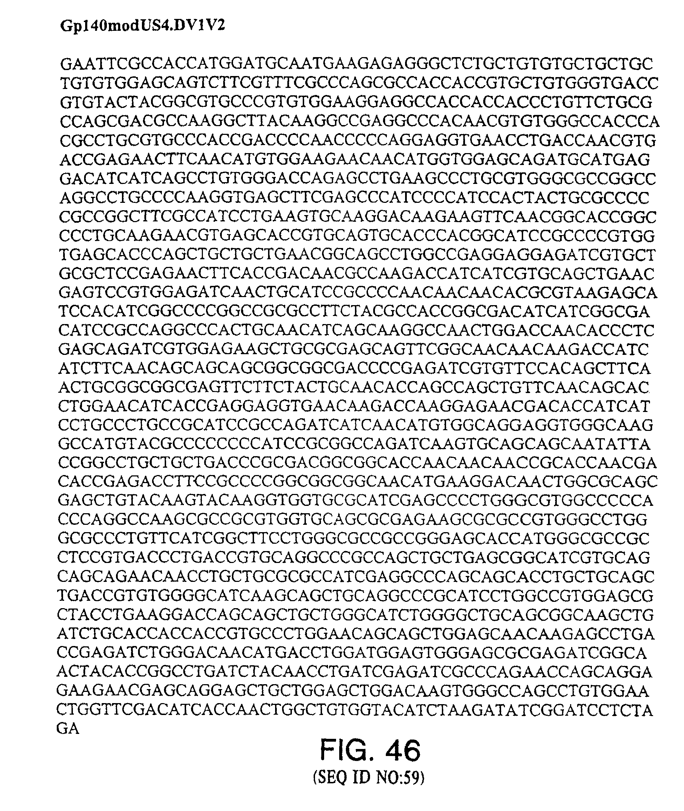

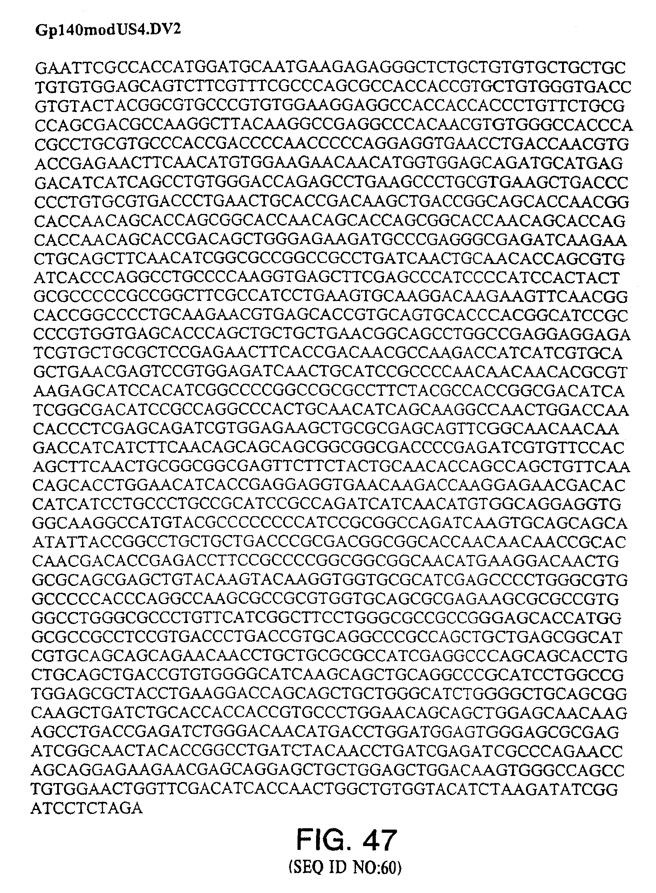

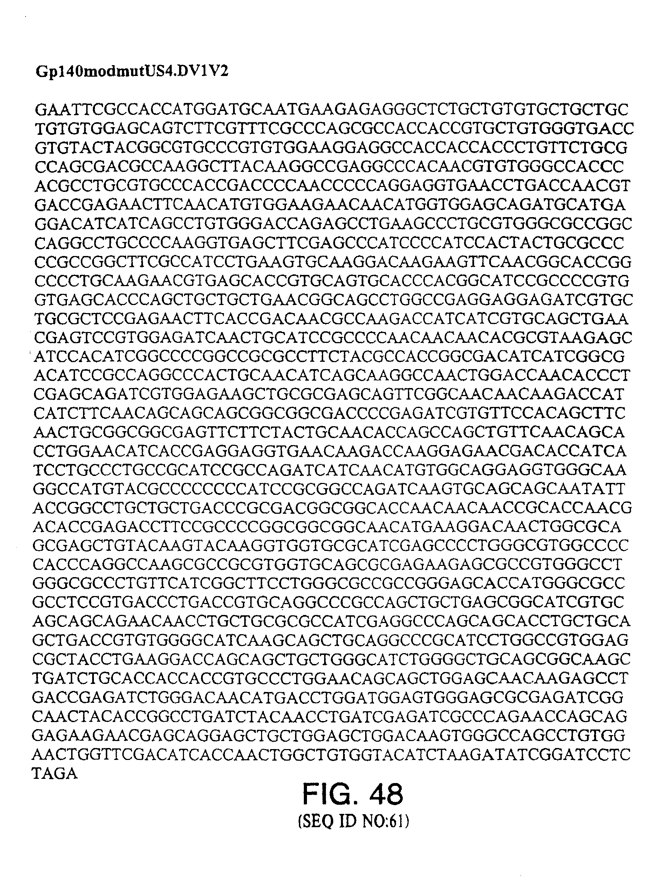

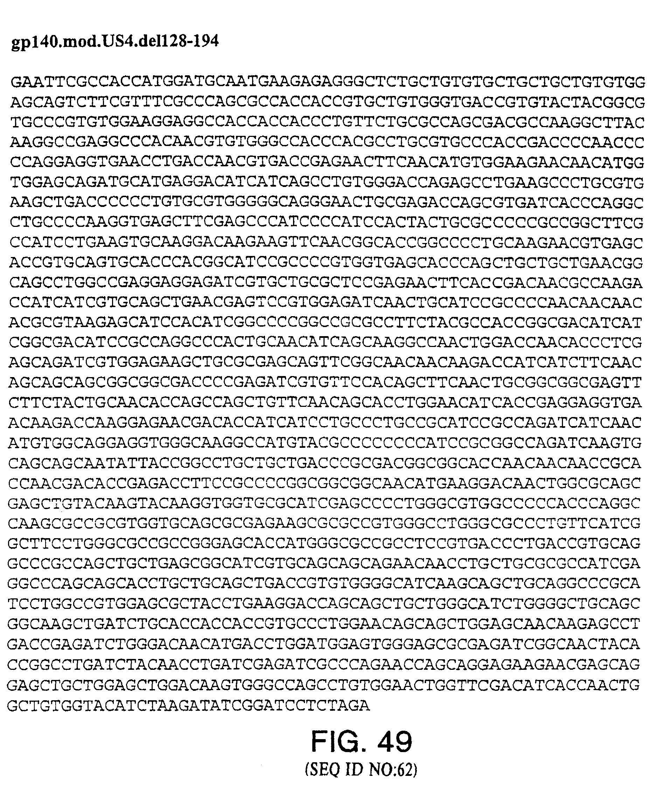

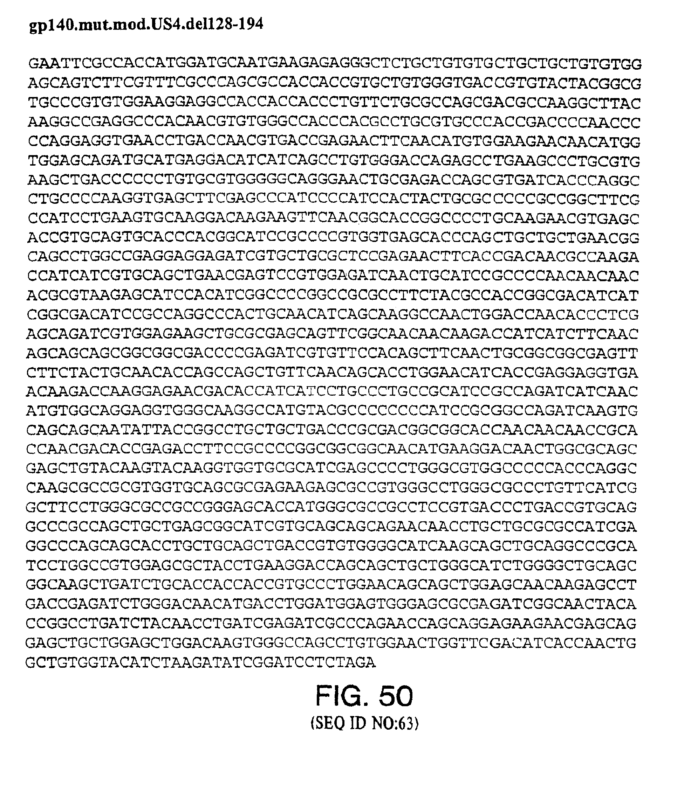

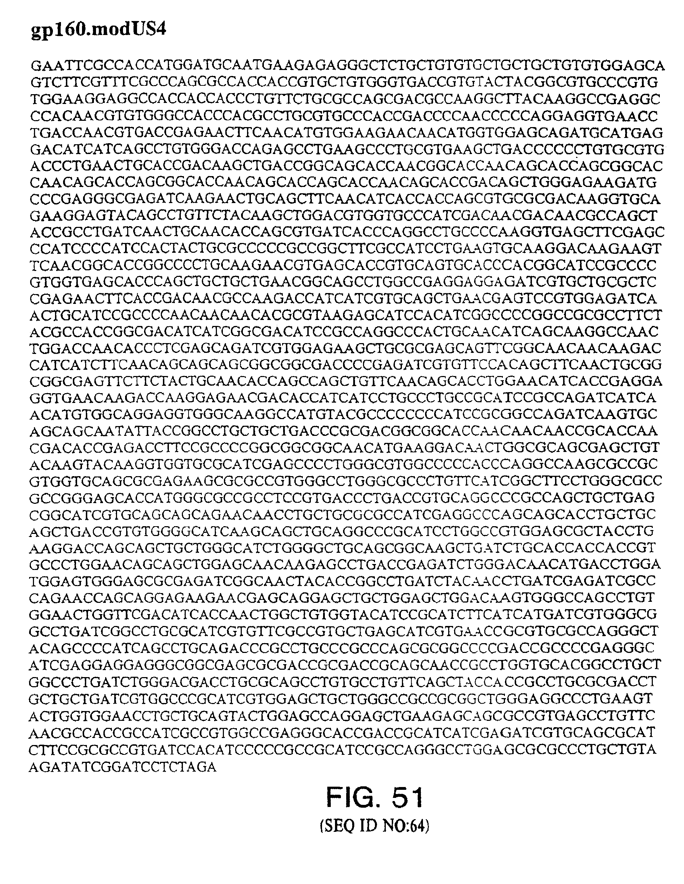

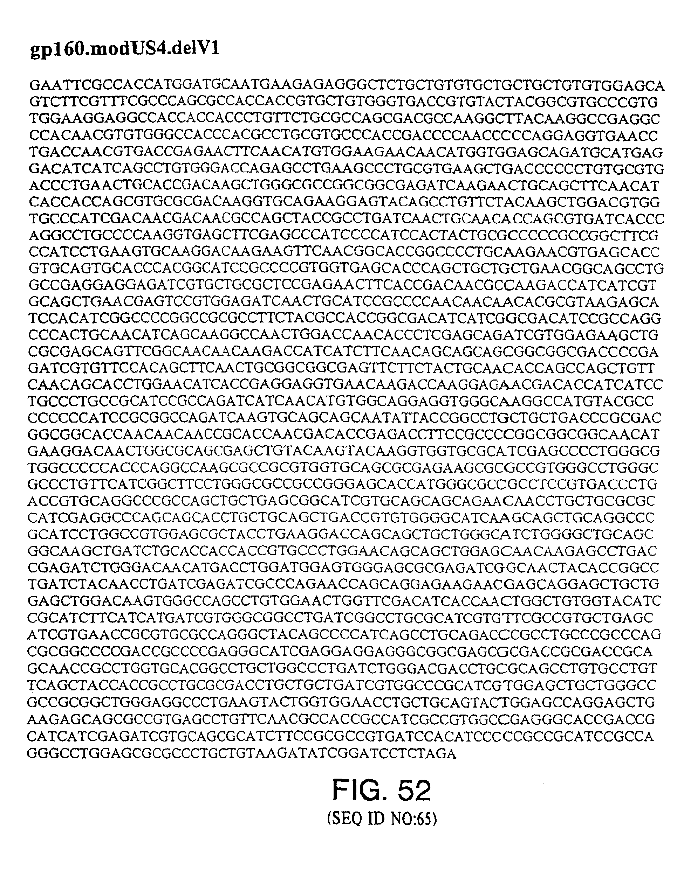

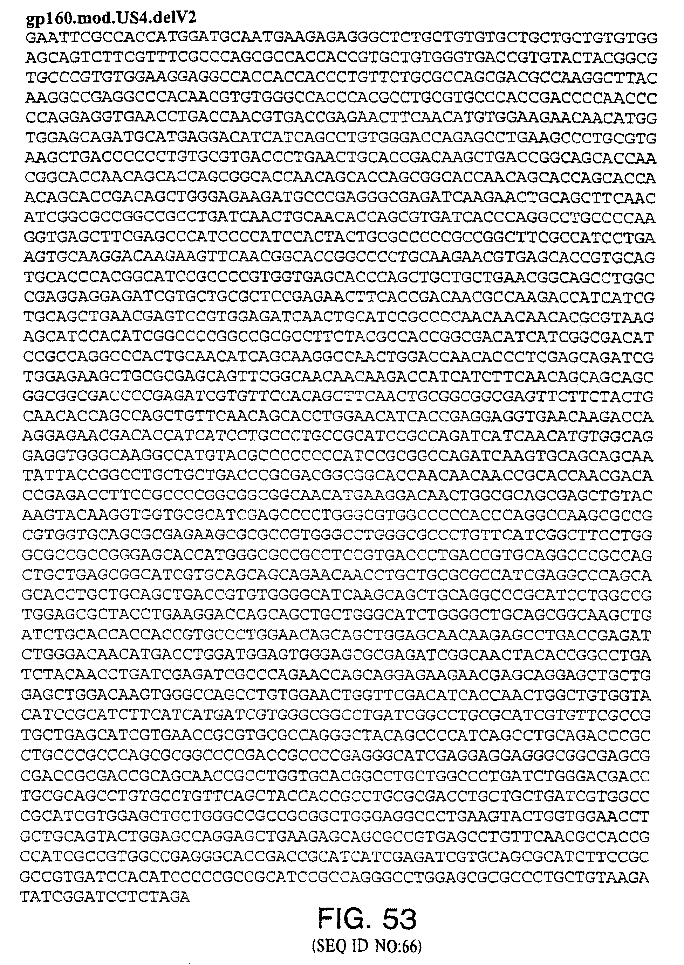

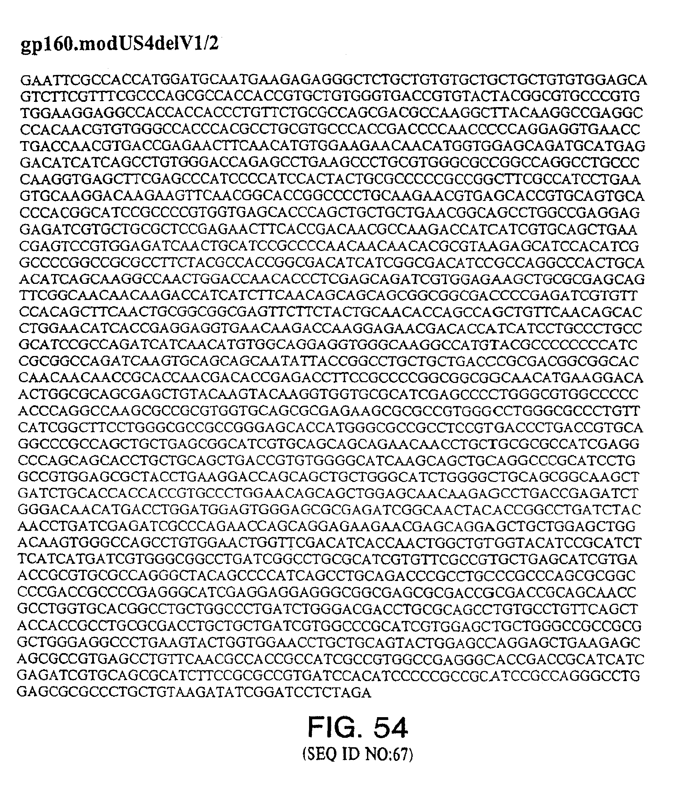

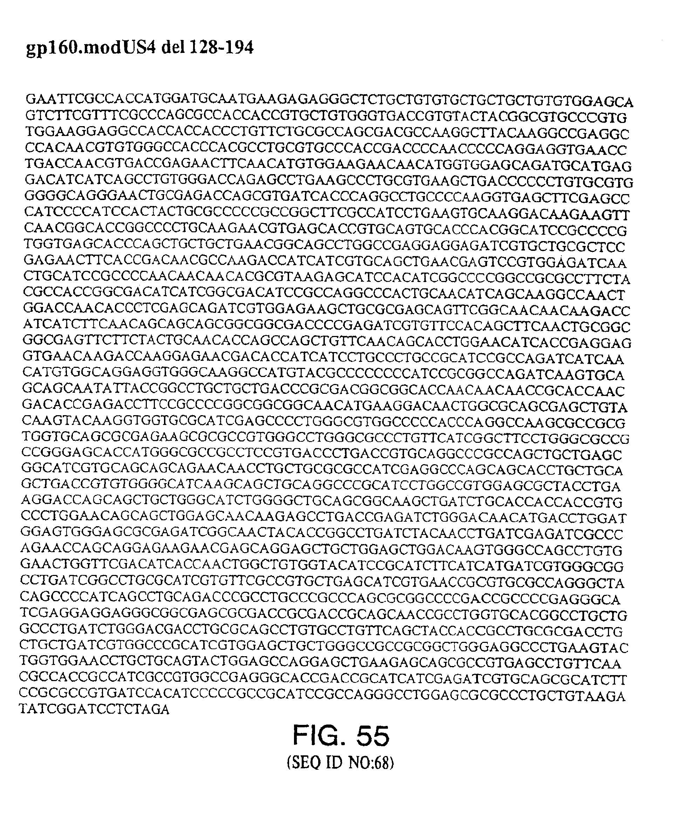

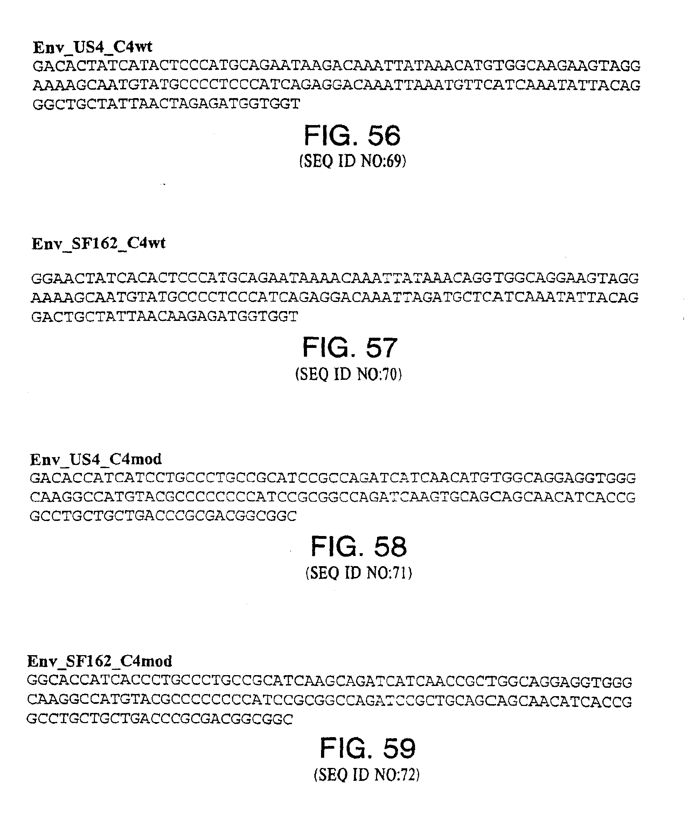

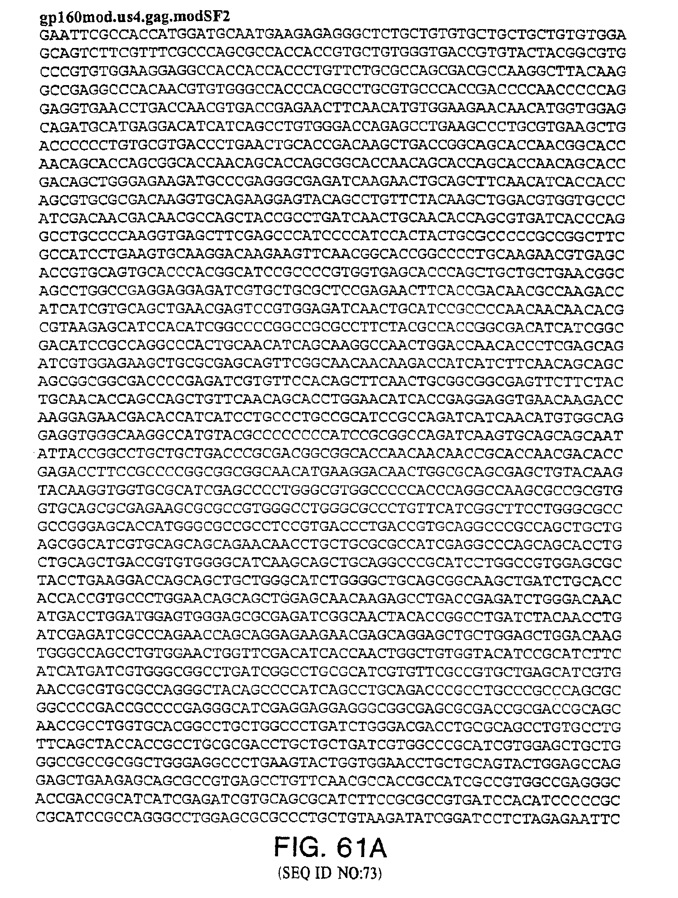

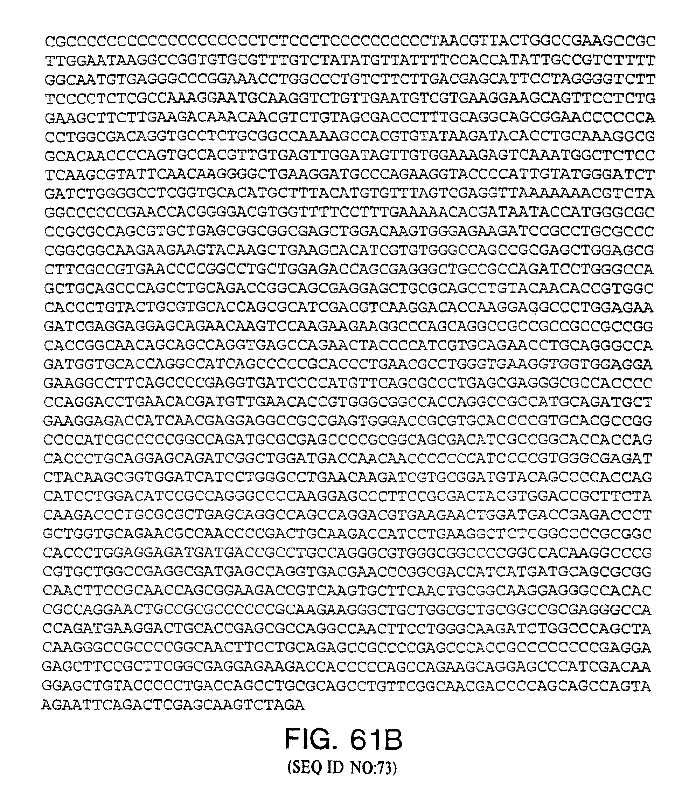

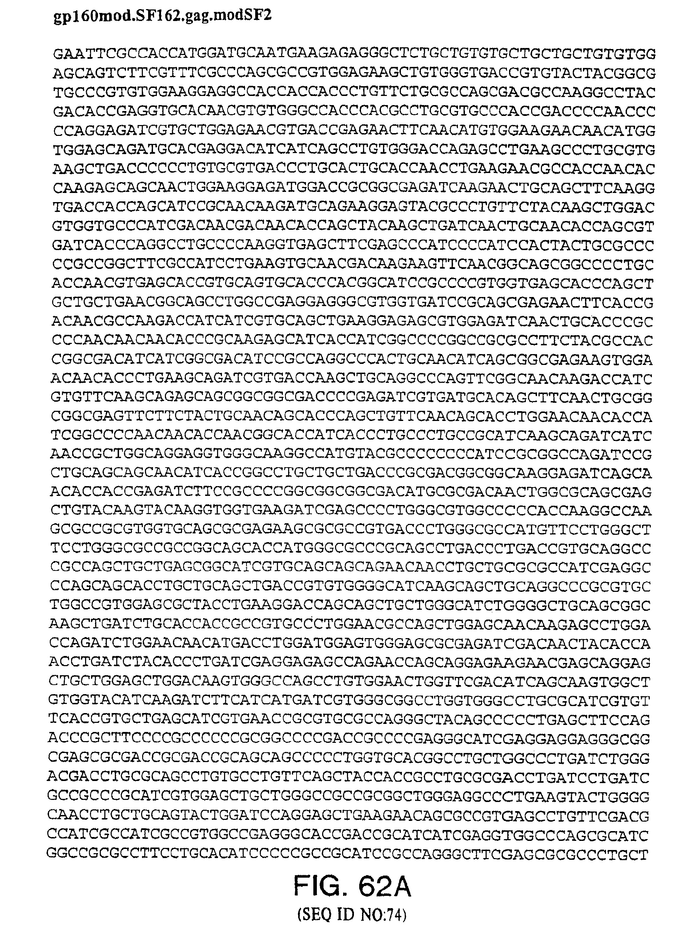

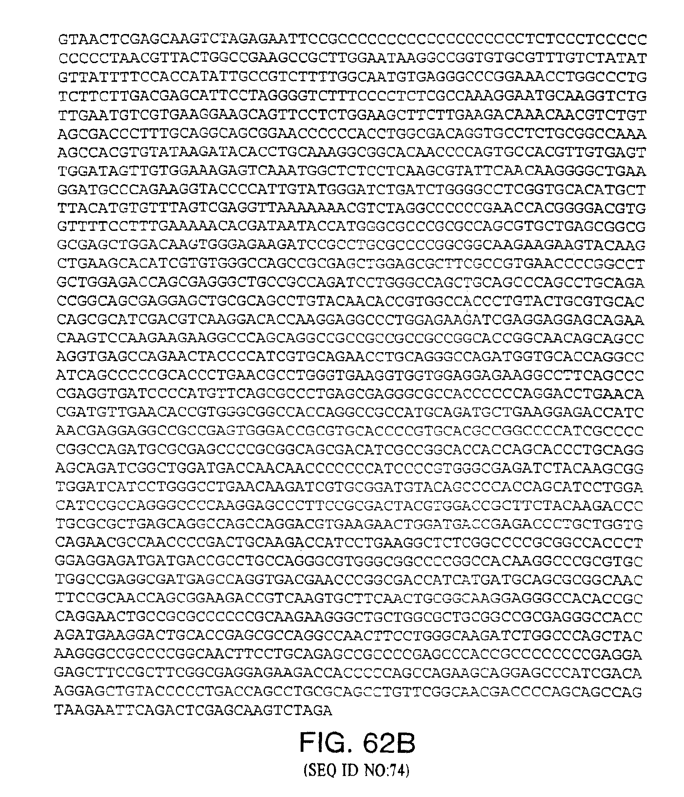

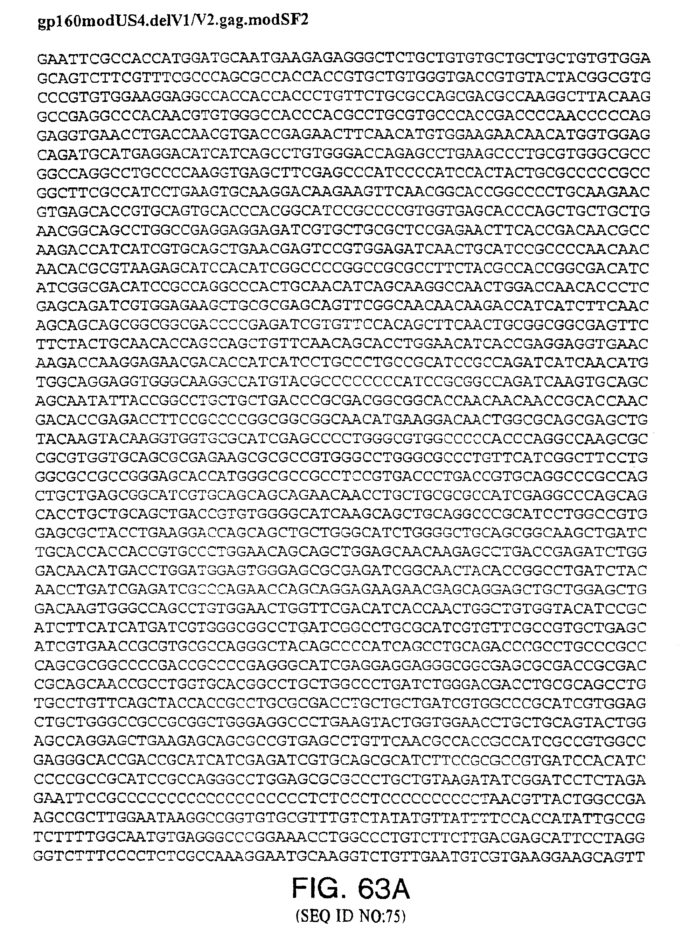

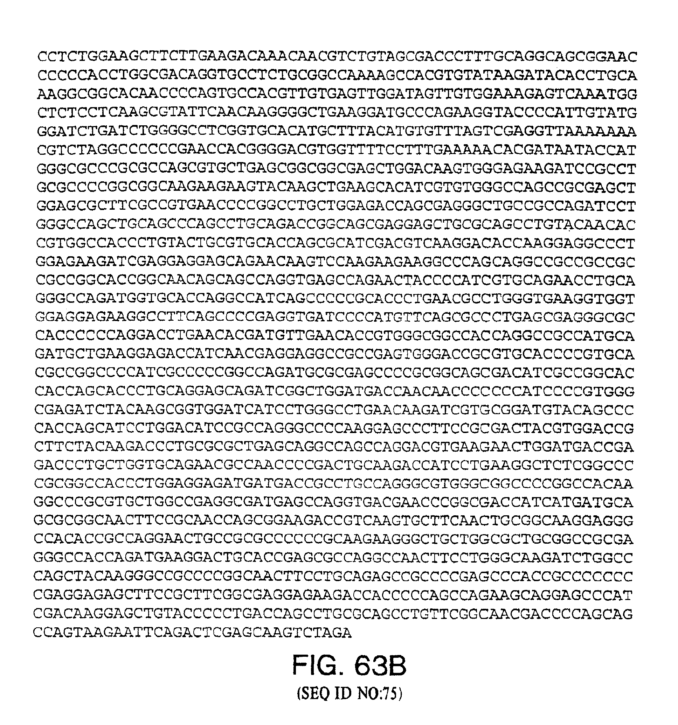

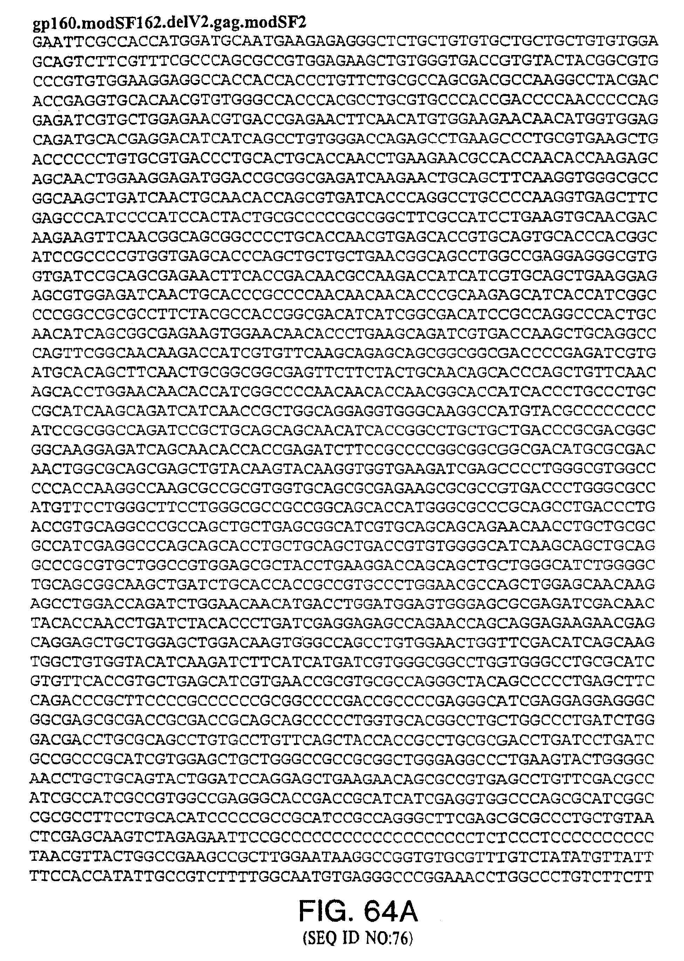

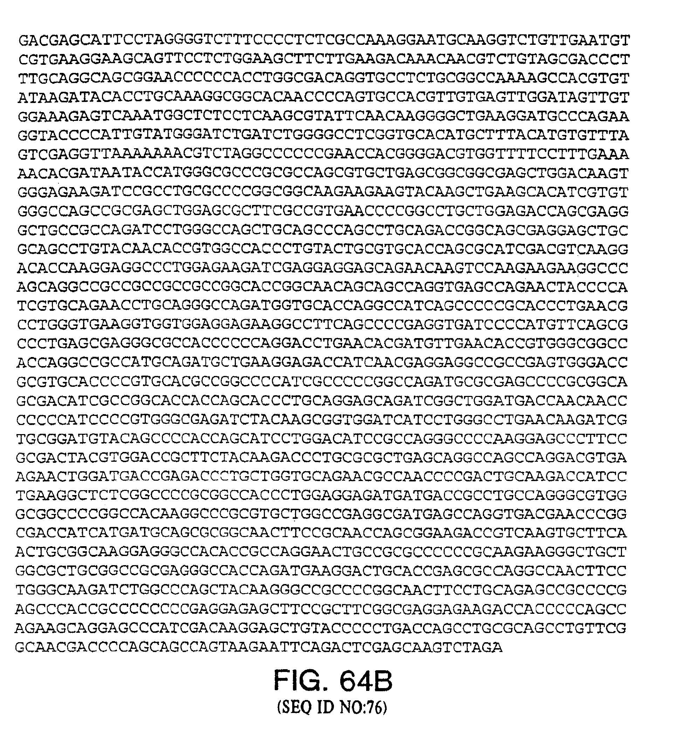

In another aspect, the invention includes an expression cassette, comprising a polynucleotide sequence encoding a polypeptide including an HIV Env polypeptide, wherein the polynucleotide sequence encoding said Env polypeptide comprises a sequence having at least 90% sequence identity to SEQ ID NO:71 (Figure 58) or SEQ ID NO:72 (Figure 59). In certain embodiments, the Env expression cassettes includes sequences flanking a V1 region but have a deletion in the V1 region itself, for example the sequence presented as SEQ ID NO:65 (Figure 52, gp160.modUS4.delV1). In certain embodiments, the Env expression cassettes, include sequences flanking a V2 region but have a deletion in the V2 region itself, for example the sequences shown in SEQ ID NO:60 (Figure 47); SEQ ID NO:66 (Figure 53); SEQ ID NO:34 (Figure 20); SEQ ID NO:37 (Figure 24); SEQ ID NO:40 (Figure 27); SEQ ID NO:43 (Figure 30); SEQ ID NO:46 (Figure 33); SEQ ID NO:76 (Figure 64) and SEQ ID NO:49 (Figure 36). In certain embodiments, the Env expression cassettes include sequences flanking a V1/V2 region but have a deletion in the V1/V2 region itself, for example, SEQ ID NO:59 (Figure 46); SEQ ID NO:61 (Figure 48); SEQ ID NO:67 (Figure 54); SEQ ID NO:75 (Figure 63); SEQ ID NO:35 (Figure 21); SEQ ID NO:38 (Figure 25); SEQ ID NO:41 (Figure 28); SEQ ID NO:44 (Figure 31); SEQ ID NO:47 (Figure 34) and SEQ ID NO:50 (Figure 37). The Env-encoding expression cassettes may also include a mutated cleavage site that prevents the cleavage of a gp140 polypeptide into a gp120 polypeptide and a gp41 polypeptide, for example, SEQ ID NO:57 (Figure 44); SEQ ID NO:61 (Figure 48); SEQ ID NO:63 (Figure 50); SEQ ID NO:39 (Figure 26); SEQ ID NO:40 (Figure 27); SEQ ID NO:41 (Figure 28); SEQ ID NO:42 (Figure 29); SEQ ID NO:43 (Figure 30); SEQ ID NO:44 (Figure 31); SEQ ID NO:45 (Figure 32); SEQ ID NO:46 (Figure 33); and SEQ ID NO:47 (Figure 34). The Env expression cassettes may include a gp160 *Env* polypeptide or a polypeptide derived from a gp160 *Env* polypeptide, for example SEQ ID NO:64 (Figure 51); SEQ ID NO:65 (Figure 52); SEQ ID NO:66 (Figure 53); SEQ ID NO:67 (Figure 54); SEQ ID NO:68 (Figure 55); SEQ ID NO:75 (Figure 63); SEQ ID NO:73 (Figure 61); SEQ ID NO:48 (Figure 35); SEQ ID NO:49 (Figure 36); SEQ ID NO:50 (Figure 37); SEQ ID NO:76 (Figure 64); and SEQ ID NO:74 (Figure 62). The Env expression cassettes may include a gp140 Env polypeptide or a polypeptide derived from a gp140 Env polypeptide, for example SEQ ID NO:56 (Figure 43); SEQ ID NO:57 (Figure 44); SEQ ID NO:58 (Figure 45); SEQ ID NO:59 (Figure 46); SEQ ID NO:60 (Figure 47); SEQ ID NO:61 (Figure 48); SEQ ID NO:62 (Figure 49); SEQ ID NO:63 (Figure 50); SEQ ID NO:36 (Figure 23); SEQ ID NO:37 (Figure 24); SEQ ID NO:38 (Figure 25); SEQ ID NO:39 (Figure 26); SEQ ID NO:40 (Figure 27); SEQ ID NO:41 (Figure 28); SEQ ID NO:42 (Figure 29); SEQ ID NO:43 (Figure 30); SEQ ID NO:44 (Figure 31); SEQ ID NO:45 (Figure 32); SEQ ID NO:46 (Figure 33); and SEQ ID NO:47 (Figure 34). The Env expression cassettes may also include a gp120 Env polypeptide or a polypeptide derived from a gp120 Env polypeptide, for example SEQ ID NO:54 (Figure 41); and SEQ ID NO:55 (Figure 42); SEQ ID NO:33 (Figure 19); SEQ ID NO:34 (Figure 20); and SEQ ID NO:35 (Figure 21). The Env expression cassettes may include an Env polypeptide lacking the amino acids corresponding to residues 128 to about 194, relative to strains SF162 or US4, for example, SEQ ID NO:55 (Figure 42); SEQ ID NO:62 (Figure 49); SEQ ID NO:63 (Figure 50); and SEQ ID NO:68 (Figure 55).

In another aspect, the invention includes a recombinant expression system for use in a selected host cell, comprising, one or more of the expression cassettes described herein operably linked to control elements compatible with expression in the selected host cell. The expression cassettes may be included on one or on multiple vectors and may use the same or different promoters. Exemplary control elements include a transcription promoter (e.g., CMV, CMV+intron A, SV40, RSV, HIV-Ltr, MMLV-ltr, and metallothionein), a transcription enhancer element, a transcription termination signal, polyadenylation sequences, sequences for optimization of initiation of translation, and translation termination sequences.

In another aspect, the invention includes a recombinant expression system for use in a selected host cell, comprising, any one of the expression cassettes described herein operably linked to control elements compatible with expression in the selected host cell. Exemplary control elements include, but are not limited to, a transcription promoter (*e.g.*, CMV, CMV+intron A, SV40, RSV, HIV-LTR, MMLV-LTR, and metallothionein), a transcription enhancer element, a transcription termination signal, polyadenylation sequences, sequences for optimization of initiation of translation, and translation termination sequences.

In yet another aspect, the invention includes a cell comprising one or more of the expression cassettes described herein operably linked to control elements compatible with expression in the cell. The cell can be, for example, a mammalian cell (e.g., BHK, VERO, HT1080, 293, RD, COS-7, or CHO cells), an insect cell (*e.g.*, *Trichoplusia ni* (Tn5) or Sf9), a bacterial cell, a plant cell, a yeast cell, an antigen presenting cell (*e.g.*, primary, immortalized or tumor-derived lymphoid cells such as macrophages, monocytes, dendritic cells, B-cells, T-cells, stem cells, and progenitor cells thereof).

In another aspect, the invention includes methods for producing a polypeptide including HIV *Gag-, prot-, pol-, reverse transcriptase, Env-* or *Tat*-containing polypeptide sequences, said method comprising, incubating the cells comprising one or more the expression cassettes describe herein, under conditions for producing said polypeptide.

In yet another aspect, the invention includes compositions for generating an immunological response, comprising one or more of the expression cassettes described herein. In certain embodiments, the compositions also include an adjuvant.

In a still further aspect, the invention includes methods of generating an immune response in a subject, comprising introducing a composition comprising one or more of the expression cassettes described herein into the subject under conditions that are compatible with expression of said expression cassette in the subject. In certain embodiments, the expression cassette is introduced using a gene delivery vector. More than one expression cassette may be introduced using one or more gene delivery vectors.

In yet another aspect, the invention includes a purified polynucleotide comprising a polynucleotide sequence encoding a polypeptide including an HIV Env polypeptide, wherein the polynucleotide sequence encoding said Env polypeptide comprises a sequence having at least 90% sequence identity to SEQ ID NO:71 (Figure 58) or SEQ ID NO:72 (Figure 59). Further exemplary purified polynucleotide sequences were presented above.

The polynucleotides of the present invention can be produced by recombinant techniques, synthetic techniques, or combinations thereof.

In another embodiment, the invention includes a method for producing a polypeptide including HIV Gag polypeptide sequences, where the method comprises incubating any of the above cells containing an expression cassette of interest under conditions for producing the polypeptide.

The invention further includes, a method for producing virus-like particles (VLPs) where the method comprises incubating any of the above-described cells containing an expression cassette of interest under conditions for producing VLPs.

In another aspect the invention includes a method for producing a composition of virus-like particles (VLPs) where, any of the above-described cells containing an expression cassette of interest are incubated under conditions for producing VLPs, and the VLPs are substantially purified to produce a composition of VLPs.

In a further embodiment of the present invention, packaging cell lines are produced using the expression cassettes of the present invention. For example, a cell line useful for packaging lentivirus vectors comprises suitable host cells that have an expression vector containing an expression cassette of the present invention wherein said polynucleotide sequence is operably linked to control elements compatible with expression in the host cell. In a preferred embodiment, such host cells may be transfected with one or more expression cassettes having a polynucleotide sequence that encodes an HIV *polymerase* polypeptide or polypeptides derived therefrom, for example, where the nucleotide sequence encoding said polypeptide comprises a sequence having at least 90% sequence identity to the sequence presented as SEQ ID NO:6. Further, the HIV polymerase polypeptide may be modified by deletions of coding regions corresponding to reverse transcriptase and integrase. Such a polynucleotide sequence may preserve T-helper cell and CTL epitopes, for example when used in a vaccine application. In addition, the polynucleotide sequence may also include other polypeptides. Further, polynucleotide sequences encoding additional polypeptides whose expression are useful for packaging cell line function may also be utilized.

In another aspect, the present invention includes a gene delivery or vaccine vector for use in a subject, where the vector is a suitable gene delivery vector for use in the subject, and the vector comprises one or more of any of the expression cassettes of the present invention where the polynucleotide sequences of interest are operably linked to control elements compatible with expression in the subject. Such gene delivery vectors can be used in a method of DNA immunization of a subject, for example, by introducing a gene delivery vector into the subject under conditions that are compatible with expression of the expression cassette in the subject. Gene delivery vectors useful in the practice of the present invention include, but are not limited to, nonviral vectors, bacterial plasmid vectors, viral vectors, particulate carriers (where the vector is coated on a polylactide co-glycolide particles, gold or tungsten particle, for example, the coated particle can be delivered to a subject cell using a gene gun), liposome preparations, and viral vectors (e.g., vectors derived from alphaviruses, pox viruses, and vaccinia viruses, as well as, retroviral vectors, including, but not limited to, lentiviral vectors). Alphavirus-derived vectors include, for example, an alphavirus cDNA construct, a recombinant alphavirus particle preparation and a eukaryotic layered vector initiation system. In one embodiment, the subject is a vertebrate, preferably a mammal, and in a further embodiment the subject is a human.

The invention further includes a method of generating an immune response in a subject, where cells of a subject are transfected with any of the above-described gene delivery vectors (e.g., alphavirus constructs; alphavirus cDNA constructs; eukaryotic layered vector initiation systems (see, e.g., U.S. Patent Number 5,814,482 for description of suitable eukaryotic layered vector initiation systems); alphavirus particle preparations; etc.) under conditions that permit the expression of a selected polynucleotide and production of a polypeptide of interest (i.e., encoded by any expression cassette of the present invention), thereby eliciting an immunological response to the polypeptide. Transfection of the cells may be performed *ex vivo* and the transfected cells are reintroduced into the subject. Alternately, or in addition, the cells may be transfected in vivo in the subject. The immune response may be humoral and/or cell-mediated (cellular).

Further embodiments of the present invention include purified polynucleotides. In one embodiment, the purified polynucleotide comprises a polynucleotide sequence having at least 90% sequence identity to the sequence presented as SEQ ID NO:20, and complements thereof. In another embodiment, the purified polynucleotide comprises a polynucleotide sequence encoding an HIV Gag polypeptide, wherein the polynucleotide sequence comprises a sequence having at least 90% sequence identity to the sequence presented as SEQ ID NO:20, and complements thereof. In still another embodiment, the purified polynucleotide comprises a polynucleotide sequence encoding an HIV Gag polypeptide, wherein the polynucleotide sequence comprises a sequence having at least 90% sequence identity to the sequence presented as SEQ ID NO:9, and complements thereof. In further embodiments the polynucleotide sequence comprises a sequence having at least 90% sequence identity to one of the following sequences: SEQ ID NO:4, SEQ ID NO:5, SEQ ID NO:6, SEQ ID NO:7, and complements thereof.

The polynucleotides of the present invention can be produced by recombinant techniques, synthetic techniques, or combinations thereof.

These and other embodiments of the present invention will readily occur to those of ordinary skill in the art in view of the disclosure herein.

### BRIEF DESCRIPTION OF THE FIGURES

Figure 1 shows the locations of the inactivation sites for the native HIV-1SF2 Gag protein coding sequence.
Figure 2 shows the locations of the inactivation sites for the native HIV-1SF2 Gag-protease protein coding sequence.
Figures 3A and 3B show electron micrographs of virus-like particles. Figure 3A shows immature p55Gag virus-like particles in COS-7 cells transfected with a synthetic HIV-1_{SF2} *gag* construct while Figure 3B shows mature (arrows) and immature VLP in cells transfected with a modified HIV-1_{SF2} *gag*protease construct (GP2, SEQ ID NO:70). Transfected cells were fixed at 24 h (gag) or 48 h (gagprotease) post-transfection and subsequently analyzed by electron microscopy (magnification at 100,000X). Cells transfected with vector alone (pCMVKm2) served as negative control (data not shown).
Figure 4 presents an image of samples from a series of fractions which were electrophoresed on an 8-16% SDS polyacrylamide gel and the resulting bands visualized by commassie blue staining. The results show that the native p55 Gag virus-like particles (VLPs) banded at a sucrose density of range of 1.15 - 1.19 g/ml with the peak at approximately 1.17 g/ml.
Figure 5 presents an image similar to Figure 4 where the analysis was performed using Gag VLPs produced by a synthetic Gag expression cassette.
Figure 6 presents a comparison of the total amount of purified HIV p55 Gag from several preparations obtained from two baculovirus expression cassettes encoding native and modified Gag.
Figure 7 presents an alignment of modified coding sequences of the present invention including a synthetic Gag expression cassette (SEQ ID NO:4), a synthetic Gag-protease expression cassette (SEQ ID NO:5), and a synthetic Gag-polymerase expression cassette (SEQ ID NO:6). A common region (Gag-common; SEQ ID NO:9) extends from position 1 to position 1262.
Figure 8 presents an image of wild-type Gag-HCV core expression samples from a series of fractions which were electrophoresed on an 8-16% SDS polyacrylamide gel and the resulting bands visualized by commassie staining.
Figure 9 shows the results of Western blot analysis of the gel shown presented in Figure 8.
Figure 10 presents results similar to those shown in Figure 9. The results in Figure 10 indicate that the main HCV Core-specific reactivity migrates at an approximate molecular weight of 72,000 kD, which is in accordance with the predicted molecular weight of the Gag-HCV core chimeric protein.
Figures 11A to 11D present a comparison of AT content, in percent, of cDNAs corresponding to an unstable human mRNA (human IFN_{γ} mRNA; 11A), wild-type HIV Gag native RNA (11B), a stable human mRNA (human GAPDH mRNA; 11C), and synthetic HIV Gag RNA (11D).
Figure 12 shows the location of the inactivation sites for the native HIV-1SF2 Gag-polymerase sequence.
Figure 13A presents a vector map of pESN2dhfr.
Figure 13B presents a map of the pCMVIII vector.
Figure 14 presents a vector map of pCMV-LINK.
Figure 15 presents a schematic diagram showing the relationships between the following forms of the HIV Env polypeptide: gp160, gp140, gp120, and gp41.
Figure 16 depicts the nucleotide sequence of wild-type gp120 from SF162 (SEQ ID NO:30).
Figure 17 depicts the nucleotide sequence of the wild-type gp140 from SF162 (SEQ ID NO:31).
Figure 18 depicts the nucleotide sequence of the wild-type gp160 from SF162 (SEQ ID NO:32).
Figure 19 depicts the nucleotide sequence of the construct designated gp120.modSF162 (SEQ ID NO:33).
Figure 20 depicts the nucleotide sequence of the construct designated gp120.modSF162.delV2 (SEQ ID NO:34).
Figure 21 depicts the nucleotide sequence of the construct designated gp120.modSF162.delV1/V2 (SEQ ID NO:35).
Figures 22A-H show the percent A-T content over the length of the sequences for IFNγ (Figures 2C and 2G); native gp160 Env US4 and SF162 (Figures 2A and 2E, respectively); GAPDH (Figures 2D and 2H); and the synthetic gp160 Env for US4 and SF162 (Figures 2B and 2F, respectively).
Figure 23 depicts the nucleotide sequence of the construct designated gp140.modSF162 (SEQ ID NO:36).
Figure 24 depicts the nucleotide sequence of the construct designated gp140.modSF162.delV2 (SEQ ID NO:37).
Figure 25 depicts the nucleotide sequence of the construct designated gp140.modSF162.delV1/V2 (SEQ ID NO:38).
Figure 26 depicts the nucleotide sequence of the construct designated gp140.mut.modSF162 (SEQ ID NO:39).
Figure 27 depicts the nucleotide sequence of the construct designated gp140.mut.modSF162.delV2 (SEQ ID NO:40).
Figure 28 depicts the nucleotide sequence of the construct designated gp140.mut.modSF162.delV1/V2 (SEQ ID NO:41).
Figure 29 depicts the nucleotide sequence of the construct designated gp140.mut7.modSF162 (SEQ ID NO:42).
Figure 30 depicts the nucleotide sequence of the construct designated gp140.mut7.modSF162.delV2 (SEQ ID NO:43).
Figure 31 depicts the nucleotide sequence of the construct designated gp140.mut7.modSF162.delV1/V2 (SEQ ID NO:44).
Figure 32 depicts the nucleotide sequence of the construct designated gp140.mut8.modSF162 (SEQ ID NO:45).
Figure 33 depicts the nucleotide sequence of the construct designated gp140.mut8.modSF162.delV2 (SEQ ID NO:46).
Figure 34 depicts the nucleotide sequence of the construct designated gp140.mut8.modSF162.delV1/V2 (SEQ ID NO:47).
Figure 35 depicts the nucleotide sequence of the construct designated gp160.modSF162 (SEQ ID NO:48).
Figure 36 depicts the nucleotide sequence of the construct designated gp160.modSF162.delV2 (SEQ ID NO:49).
Figure 37 depicts the nucleotide sequence of the construct designated gp160.modSF162.delV1/V2 (SEQ ID NO:50).
Figure 38 depicts the nucleotide sequence of the wild-type gp120 from US4 (SEQ ID NO:51).
Figure 39 depicts the nucleotide sequence of the wild-type gp140 from US4 (SEQ ID NO:52).
Figure 40 depicts the nucleotide sequence of the wild-type gp160 from US4 (SEQ ID NO:53).
Figure 41 depicts the nucleotide sequence of the construct designated gp120.modUS4 (SEQ ID NO:54).
Figure 42 depicts the nucleotide sequence of the construct designated gp120.modUS4.del 128-194 (SEQ ID NO:55).
Figure 43 depicts the nucleotide sequence of the construct designated gp140.modUS4 (SEQ ID NO:56).
Figure 44 depicts the nucleotide sequence of the construct designated gp140.mut.modUS4 (SEQ ID NO:57).
Figure 45 depicts the nucleotide sequence of the construct designated gp140.TM.modUS4 (SEQ ID NO:58).
Figure 46 depicts the nucleotide sequence of the construct designated gp140.modUS4.delV1/V2 (SEQ ID NO:59).
Figure 47 depicts the nucleotide sequence of the construct designated gp140.modUS4.delV2 (SEQ ID NO:60).
Figure 48 depicts the nucleotide sequence of the construct designated gp140.mut.modUS4.delV1/V2 (SEQ ID NO:61).
Figure 49 depicts the nucleotide sequence of the construct designated gp140.modUS4.del 128-194 (SEQ ID NO:62).
Figure 50 depicts the nucleotide sequence of the construct designated gp140.mut.modUS4.del 128-194 (SEQ ID NO:63).
Figure 51 depicts the nucleotide sequence of the construct designated gp160.modUS4 (SEQ ID NO:64).
Figure 52 depicts the nucleotide sequence of the construct designated gp160.modUS4.delV1 (SEQ ID NO:65).
Figure 53 depicts the nucleotide sequence of the construct designated gp160.modUS4.delV2 (SEQ ID NO:66).
Figure 54 depicts the nucleotide sequence of the construct designated gp160.modUS4.delV1/V2 (SEQ ID NO:67).
Figure 55 depicts the nucleotide sequence of the construct designated gp160.modUS4.del 128-194 (SEQ ID NO:68).
Figure 56 depicts the nucleotide sequence of the common region of Env from wild-type US4 (SEQ ID NO:69).
Figure 57 depicts the nucleotide sequence of the common region of Env from wild-type SF162 (SEQ ID NO:70).
Figure 58 depicts the nucleotide sequence of synthetic sequences corresponding to the common region of Env from US4 (SEQ ID NO:71).
Figure 59 depicts the nucleotide sequence of synthetic sequences corresponding to the common region of Env from SF162 (SEQ ID NO:72).
Figure 60 presents a schematic representation of an Env polypeptide purification strategy.
Figure 61 depicts the nucleotide sequence of the bicistronic construct designated gp160.modUS4.Gag.modSF2 (SEQ ID NO:73).
Figure 62 depicts the nucleotide sequence of the bicistronic construct designated gp160.modSF162.Gag.modSF2 (SEQ ID NO:74).
Figure 63 depicts the nucleotide sequence of the bicistronic construct designated gp160.modUS4.-delV1/V2.Gag.modSF2 (SEQ ID NO:75).
Figure 64 depicts the nucleotide sequence of the bicistronic construct designated gp160.modSF162.delV2.Gag.modSF2 (SEQ ID NO:76).
Figures 65A-65F show micrographs of 293T cells transfected with the following polypeptide encoding sequences: Figure 65A, gag.modSF2; Figure 65B, gp160.modUS4; Figure 65C, gp160.modUS4.delV1/V2.gag.modSF2 (bicistronic Env and Gag); Figures 65D and 65E, gp160.modUS4.delV1/V2 and gag.modSF2; and Figure 65F, gp120.modSF162.delV2 and gag.modSF2.
Figures 66A and 66B present alignments of selected modified coding sequences of the present invention including a common region defined for each group of synthetic Env expression cassettes. Figure 66A presents alignments of modified SF162 sequences. Figure 66B presents alignments of modified US4 sequences. The SEQ ID NOs for these sequences are presented in Tables 1A and 1B.

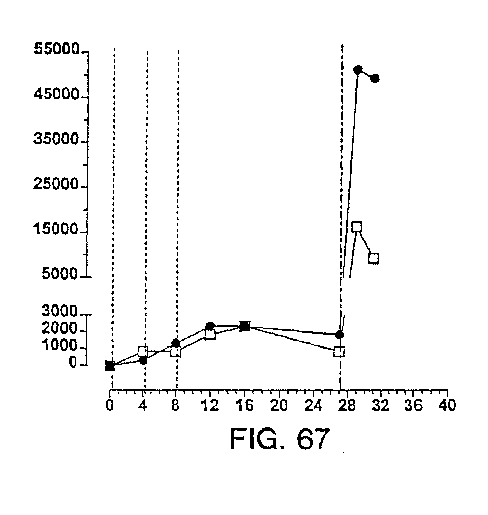

Figure 67 shows the ELISA titers (binding antibodies) obtained in two rhesus macaques (H445, lines with solid black dots; and J408, lines with open squares). The y-axis is the end-point gp140 ELISA titers and the x-axis shows weeks post-immunization. The dashed lines at 0, 4, and 8 weeks represent DNA immunizations. The alternating dash/dotted line at 27 weeks indicates a DNA plus protein boost immunization.

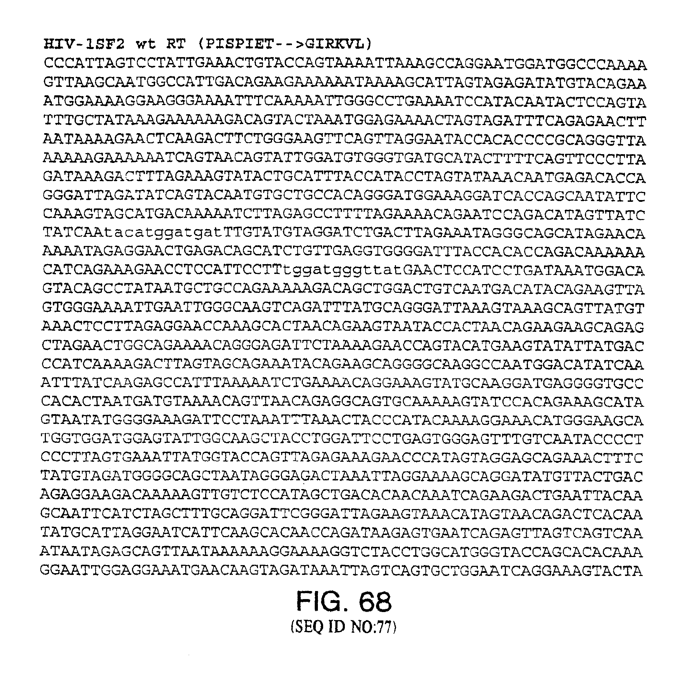

Figure 68 (SEQ ID NO:77) depicts the wild-type nucleotide sequence of Gag reverse transcriptase from SF2.
Figure 69 (SEQ ID NO:78) depicts the nucleotide sequence of the construct designated GP1.
Figure 70 (SEQ ID NO:79) depicts the nucleotide sequence of the construct designated GP2.
Figure 71 (SEQ ID NO:80) depicts the nucleotide sequence of the construct designated FS(+).protinact.RTopt.YM. FS(+) indicates that there is a frameshift in the GagPol coding sequence.
Figure 72 (SEQ ID NO:81) depicts the nucleotide sequence of the construct designated FS(+).protinact.RTopt.YMWM.
Figure 73 (SEQ ID NO:82) depicts the nucleotide sequence of the construct designated FS(-).protmod.RTopt.YM. FS(-) indicates that there is no frameshift in the GagPol coding sequence.
Figure 74 (SEQ ID NO:83) depicts the nucleotide sequence of the construct designated FS(-).protmod.RTopt.YMWM.
Figure 75 (SEQ ID NO:84) depicts the nucleotide sequence of the construct designated FS(-).protmod.RTopt(+).
Figure 76 (SEQ ID NO:85) depicts the nucleotide sequence of wild type Tat from isolate SF162.
Figure 77 (SEQ ID NO:86) depicts the amino acid sequence of the tat polypeptide.
Figure 78 (SEQ ID NO:87) depicts the nucleotide sequence of a synthetic Tat construct designated Tat.SF162.opt.
Figure 79 (SEQ ID NO:88) depicts the nucleotide sequence of a synthetic Tat construct designated tat.cys22.sf162.opt. The construct encodes a tat polypeptide in which the cystein residue at position 22 of the wild type Tat polypeptide is replaced by a glycine residue.

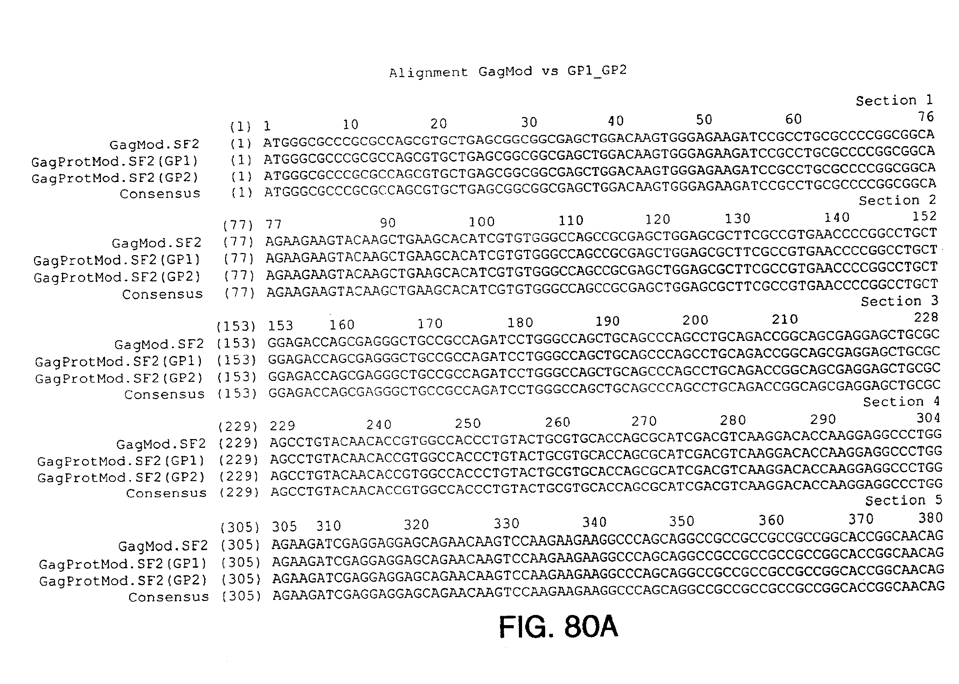

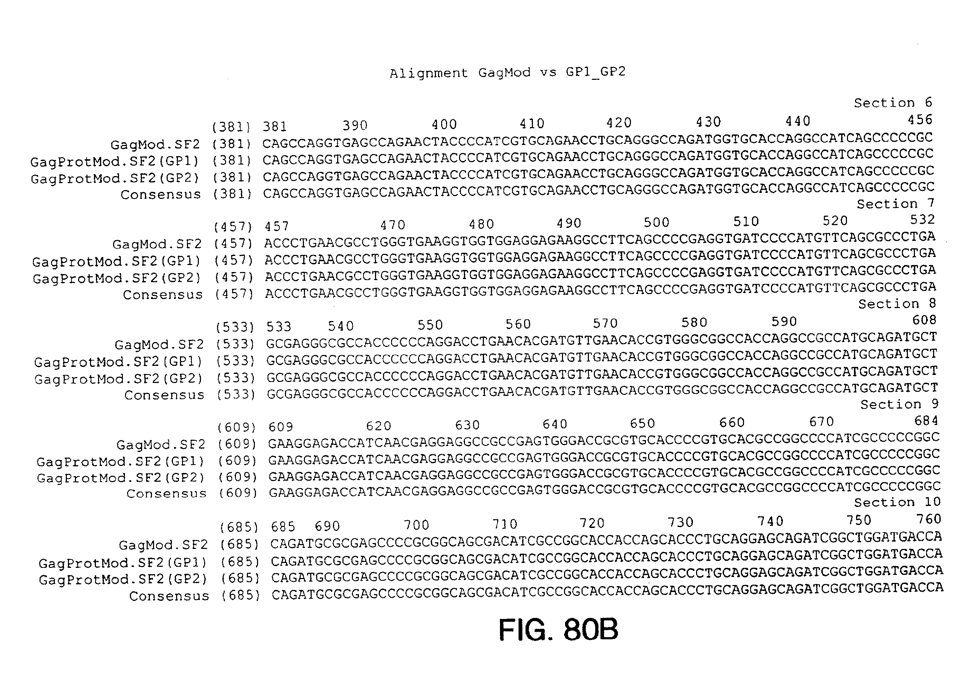

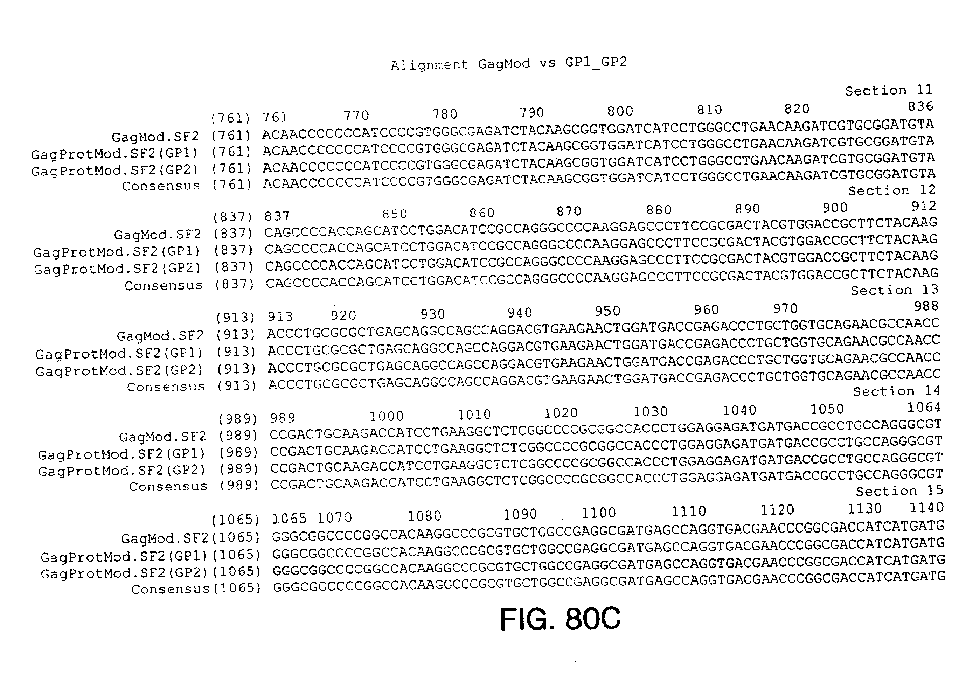

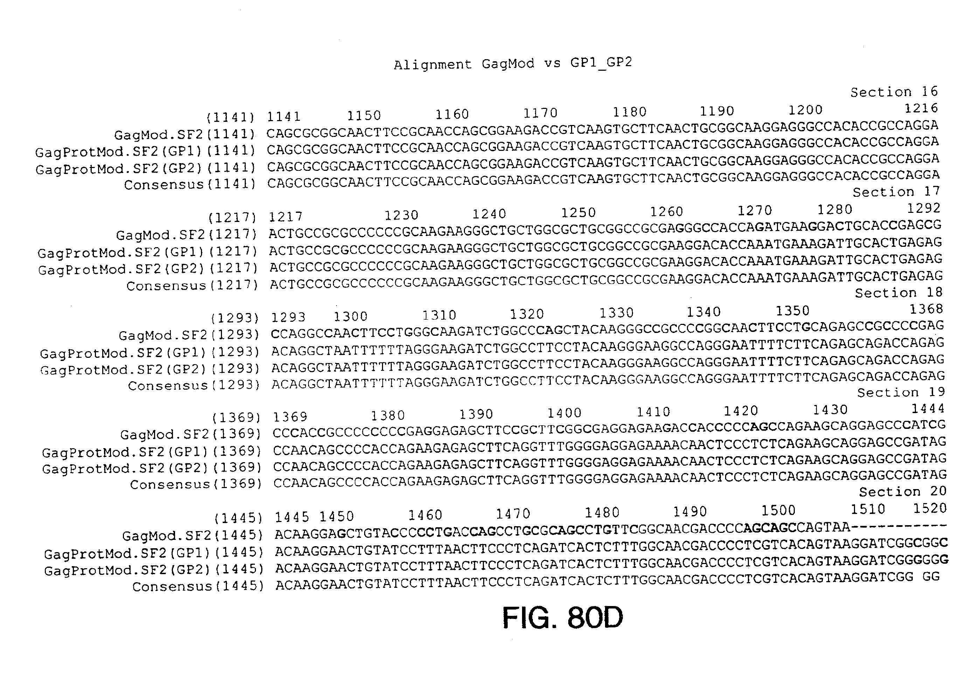

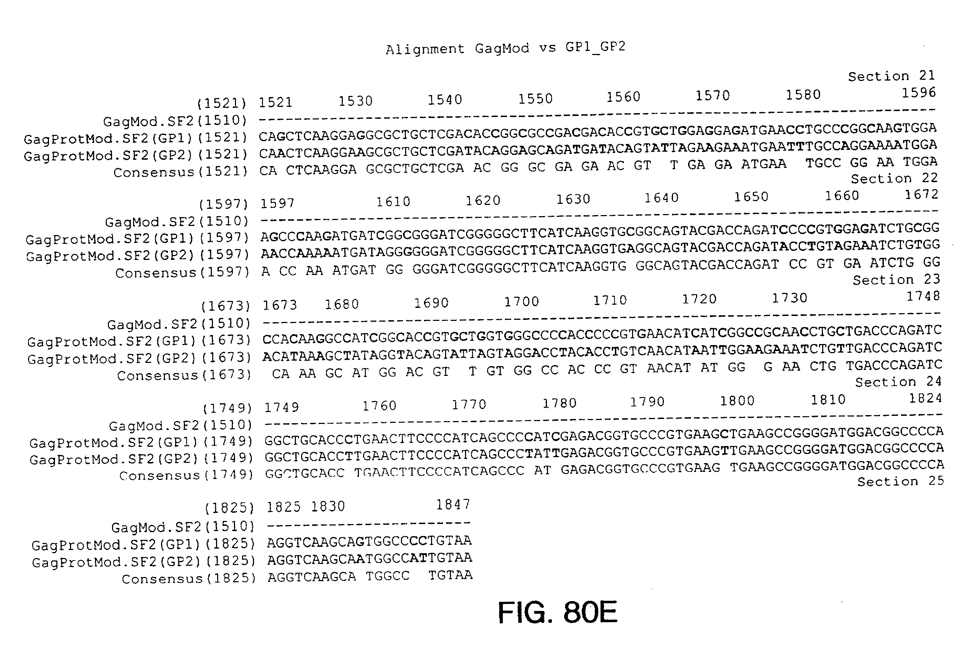

Figures 80A to 80E are an alignment of the nucleotide sequences of the constructs designated Gag.mod.SF2, GP1 (SEQ ID NO:78), and GP2 (SEQ ID NO:79).
Figure 81 (SEQ ID NO:89) depicts the nucleotide sequence of the construct designated tataminoSF162.opt, which encodes the amino terminus of that tat protein. The codon encoding the cystein-22 residue is underlined.
Figure 82 (SEQ ID NO:90) depicts the amino acid sequence of the polypeptide encoded by the construct designated tat.cys22.SF162.opt (SEQ ID NO:88).

### DETAILED DESCRIPTION OF THE INVENTION

The practice of the present invention will employ, unless otherwise indicated, conventional methods of chemistry, biochemistry, molecular biology, immunology and pharmacology, within the skill of the art. Such techniques are explained fully in the literature. See, e.g., *Remington's Pharmaceutical Sciences,* 18th Edition (Easton, Pennsylvania: Mack Publishing Company, 1990); Methods *In Enzymology* (S. Colowick and N. Kaplan, eds., Academic Press, Inc.); and *Handbook of Experimental Immunology,* Vols. I-IV (D.M. Weir and C.C. Blackwell, eds., 1986, Blackwell Scientific Publications); Sambrook, et al., *Molecular Cloning: A Laboratory Manual* (2nd Edition, 1989); *Short Protocols in Molecular Biology,* 4th ed. (Ausubel et al. eds., 1999, John Wiley & Sons); *Molecular Biology Techniques: An Intensive Laboratory Course,* (Ream et al., eds., 1998, Academic Press); *PCR (Introduction* to *Biotechniques Series),* 2nd ed. (Newton & Graham eds., 1997, Springer Verlag).

As used in this specification and the appended claims, the singular forms "a," "an" and "the" include plural references unless the content clearly dictates otherwise. Thus, for example, reference to "an antigen" includes a mixture of two or more such agents.

### 1. DEFINITIONS

In describing the present invention, the following terms will be employed, and are intended to be defined as indicated below.

"Synthetic" sequences, as used herein, refers to Env-, tat- or Gag-encoding polynucleotides whose expression has been optimized as described herein, for example, by codon substitution, deletions, replacements and/or inactivation of inhibitory sequences. "Wild-type" or "native" sequences, as used herein, refers to polypeptide encoding sequences that are essentially as they are found in nature, e.g., Gag encoding sequences as found in the isolate HIV-1SF2 or Env encoding sequences as found in the isolates HIV-1SF162 or HIV1US4.

As used herein, the term "virus-like particle" or "VLP" refers to a nonreplicating, viral shell, derived from any of several viruses discussed further below. VLPs are generally composed of one or more viral proteins, such as, but not limited to those proteins referred to as capsid, coat, shell, surface and/or envelope proteins, or particle-forming polypeptides derived from these proteins. VLPs can form spontaneously upon recombinant expression of the protein in an appropriate expression system. Methods for producing particular VLPs are known in the art and discussed more fully below. The presence of VLPs following recombinant expression of viral proteins can be detected using conventional techniques known in the art, such as by electron microscopy, biophysical characterization, and the like. See, e.g., Baker et al., *Biophys. J.* (1991) 60:1445-1456; Hagensee et al., *J. Virol.* (1994) 68:4503-4505. For example, VLPs can be isolated by density gradient centrifugation and/or identified by characteristic density banding (e.g., Example 7). Alternatively, cryoelectron microscopy can be performed on vitrified aqueous samples of the VLP preparation in question, and images recorded under appropriate exposure conditions.

By "particle-forming polypeptide" derived from a particular viral protein is meant a full-length or near full-length viral protein, as well as a fragment thereof, or a viral protein with internal deletions, which has the ability to form VLPs under conditions that favor VLP formation. Accordingly, the polypeptide may comprise the full-length sequence, fragments, truncated and partial sequences, as well as analogs and precursor forms of the reference molecule. The term therefore intends deletions, additions and substitutions to the sequence, so long as the polypeptide retains the ability to form a VLP. Thus, the term includes natural variations of the specified polypeptide since variations in coat proteins often occur between viral isolates. The term also includes deletions, additions and substitutions that do not naturally occur in the reference protein, so long as the protein retains the ability to form a VLP. Preferred substitutions are those which are conservative in nature, i.e., those substitutions that take place within a family of amino acids that are related in their side chains. Specifically, amino acids are generally divided into four families: (1) acidic -- aspartate and glutamate; (2) basic -- lysine, arginine, histidine; (3) non-polar -- alanine, valine, leucine, isoleucine, proline, phenylalanine, methionine, tryptophan; and (4) uncharged polar -- glycine, asparagine, glutamine, cystine, serine threonine, tyrosine. Phenylalanine, tryptophan, and tyrosine are sometimes classified as aromatic amino acids.

An "antigen" refers to a molecule containing one or more epitopes (either linear, conformational or both) that will stimulate a host's immune system to make a humoral and/or cellular antigen-specific response. The term is used interchangeably with the term "immunogen." Normally, a B-cell epitope will include at least about 5 amino acids but can be as small as 3-4 amino acids. A T-cell epitope, such as a CTL epitope, will include at least about 7-9 amino acids, and a helper T-cell epitope at least about 12-20 amino acids. Normally, an epitope will include between about 7 and 15 amino acids, such as, 9, 10, 12 or 15 amino acids. The term "antigen" denotes both subunit antigens, (i.e., antigens which are separate and discrete from a whole organism with which the antigen is associated in nature), as well as, killed, attenuated or inactivated bacteria, viruses, fungi, parasites or other microbes. Antibodies such as anti-idiotype antibodies, or fragments thereof, and synthetic peptide mimotopes, which can mimic an antigen or antigenic determinant, are also captured under the definition of antigen as used herein. Similarly, an oligonucleotide or polynucleotide which expresses an antigen or antigenic determinant in vivo, such as in gene therapy and DNA immunization applications, is also included in the definition of antigen herein.

For purposes of the present invention, antigens can be derived from any of several known viruses, bacteria, parasites and fungi, as described more fully below. The term also intends any of the various tumor antigens. Furthermore, for purposes of the present invention, an "antigen" refers to a protein which includes modifications, such as deletions, additions and substitutions (generally conservative in nature), to the native sequence, so long as the protein maintains the ability to elicit an immunological response, as defined herein. These modifications may be deliberate, as through site-directed mutagenesis, or may be accidental, such as through mutations of hosts which produce the antigens.

An "immunological response" to an antigen or composition is the development in a subject of a humoral and/or a cellular immune response to an antigen present in the composition of interest. For purposes of the present invention, a "humoral immune response" refers to an immune response mediated by antibody molecules, while a "cellular immune response" is one mediated by T-lymphocytes and/or other white blood cells. One important aspect of cellular immunity involves an antigen-specific response by cytolytic T-cells ("CTL"s). CTLs have specificity for peptide antigens that are presented in association with proteins encoded by the major histocompatibility complex (MHC) and expressed on the surfaces of cells. CTLs help induce and promote the destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemokines and other such molecules produced by activated T-cells and/or other white blood cells, including those derived from CD4+ and CD8+ T-cells.

A composition or vaccine that elicits a cellular immune response may serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular antigen to stimulate a cell-mediated immunological response may be determined by a number of assays, such as by lymphoproliferation (lymphocyte activation) assays, CTL cytotoxic cell assays, or by assaying for T-lymphocytes specific for the antigen in a sensitized subject. Such assays are well known in the art. See, e.g., Erickson et al., *J. Immunol.* (1993) 151:4189-4199; Doe et al., *Eur. J. Immunol.* (1994) 24:2369-2376. Recent methods of measuring cell-mediated immune response include measurement of intracellular cytokines or cytokine secretion by T-cell populations, or by measurement of epitope specific T-cells (e.g., by the tetramer technique)(reviewed by McMichael, A.J., and O'Callaghan, C.A., *J. Exp. Med.* 187(9)1367-1371, 1998; Mcheyzer-Williams, M.G., et al, *Immunol. Rev.* 150:5-21, 1996; Lalvani, A., et al, *J. Exp. Med.* 186:859-865, 1997).

Thus, an immunological response as used herein may be one which stimulates the production of CTLs, and/or the production or activation of helper T- cells. The antigen of interest may also elicit an antibody-mediated immune response. Hence, an immunological response may include one or more of the following effects: the production of antibodies by B-cells; and/or the activation of suppressor T-cells and/or γδ T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art.

An "immunogenic composition" is a composition that comprises an antigenic molecule where administration of the composition to a subject results in the development in the subject of a humoral and/or a cellular immune response to the antigenic molecule of interest.

By "subunit vaccine" is meant a vaccine composition which includes one or more selected antigens but not all antigens, derived from or homologous to, an antigen from a pathogen of interest such as from a virus, bacterium, parasite or fungus. Such a composition is substantially free of intact pathogen cells or pathogenic particles, or the lysate of such cells or particles. Thus, a "subunit vaccine" can be prepared from at least partially purified (preferably substantially purified) immunogenic polypeptides from the pathogen, or analogs thereof. The method of obtaining an antigen included in the subunit vaccine can thus include standard purification techniques, recombinant production, or synthetic production.

"Substantially purified" general refers to isolation of a substance (compound, polynucleotide, protein, polypeptide, polypeptide composition) such that the substance comprises the majority percent of the sample in which it resides. Typically in a sample a substantially purified component comprises 50%, preferably 80%-85%, more preferably 90-95% of the sample. Techniques for purifying polynucleotides and polypeptides of interest are well-known in the art and include, for example, ion-exchange chromatography, affinity chromatography and sedimentation according to density.

A "coding sequence" or a sequence which "encodes" a selected polypeptide, is a nucleic acid molecule which is transcribed (in the case of DNA) and translated (in the case of mRNA) into a polypeptide in vivo when placed under the control of appropriate regulatory sequences (or "control elements"). The boundaries of the coding sequence are determined by a start codon at the 5' (amino) terminus and a translation stop codon at the 3' (carboxy) terminus. A coding sequence can include, but is not limited to, cDNA from viral, procaryotic or eucaryotic mRNA, genomic DNA sequences from viral or procaryotic DNA, and even synthetic DNA sequences. A transcription termination sequence may be located 3' to the coding sequence.

Typical "control elements", include, but are not limited to, transcription promoters, transcription enhancer elements, transcription termination signals, polyadenylation sequences (located 3' to the translation stop codon), sequences for optimization of initiation of translation (located 5' to the coding sequence), and translation termination sequences, see *e.g.,* McCaughan et al. (1995) *PNAS USA* 92:5431-5435; Kochetov et al (1998) *FEBS Letts.* 440:351-355.

A "nucleic acid" molecule can include, but is not limited to, procaryotic sequences, eucaryotic mRNA, cDNA from eucaryotic mRNA, genomic DNA sequences from eucaryotic (e.g., mammalian) DNA, and even synthetic DNA sequences. The term also captures sequences that include any of the known base analogs of DNA and RNA.

"Operably linked" refers to an arrangement of elements wherein the components so described are configured so as to perform their usual function. Thus, a given promoter operably linked to a coding sequence is capable of effecting the expression of the coding sequence when the proper enzymes are present. The promoter need not be contiguous with the coding sequence, so long as it functions to direct the expression thereof. Thus, for example, intervening untranslated yet transcribed sequences can be present between the promoter sequence and the coding sequence and the promoter sequence can still be considered "operably linked" to the coding sequence.

"Recombinant" as used herein to describe a nucleic acid molecule means a polynucleotide of genomic, cDNA, semisynthetic, or synthetic origin which, by virtue of its origin or manipulation: (1) is not associated with all or a portion of the polynucleotide with which it is associated in nature; and/or (2) is linked to a polynucleotide other than that to which it is linked in nature. The term "recombinant" as used with respect to a protein or polypeptide means a polypeptide produced by expression of a recombinant polynucleotide. "Recombinant host cells," "host cells," "cells," "cell lines," "cell cultures," and other such terms denoting procaryotic microorganisms or eucaryotic cell lines cultured as unicellular entities, are used interchangeably, and refer to cells which can be, or have been, used as recipients for recombinant vectors or other transfer DNA, and include the progeny of the original cell which has been transfected. It is understood that the progeny of a single parental cell may not necessarily be completely identical in morphology or in genomic or total DNA complement to the original parent, due to accidental or deliberate mutation. Progeny of the parental cell which are sufficiently similar to the parent to be characterized by the relevant property, such as the presence of a nucleotide sequence encoding a desired peptide, are included in the progeny intended by this definition, and are covered by the above terms.

Techniques for determining amino acid sequence "similarity" are well known in the art. In general, "similarity" means the exact amino acid to amino acid comparison of two or more polypeptides at the appropriate place, where amino acids are identical or possess similar chemical and/or physical properties such as charge or hydrophobicity. A so-termed "percent similarity" then can be determined between the compared polypeptide sequences. Techniques for determining nucleic acid and amino acid sequence identity also are well known in the art and include determining the nucleotide sequence of the mRNA for that gene (usually via a cDNA intermediate) and determining the amino acid sequence encoded thereby, and comparing this to a second amino acid sequence. In general, "identity" refers to an exact nucleotide to nucleotide or amino acid to amino acid correspondence of two polynucleotides or polypeptide sequences, respectively.

Two or more polynucleotide sequences can be compared by determining their "percent identity." Two or more amino acid sequences likewise can be compared by determining their "percent identity." The percent identity of two sequences, whether nucleic acid or peptide sequences, is generally described as the number of exact matches between two aligned sequences divided by the length of the shorter sequence and multiplied by 100. An approximate alignment for nucleic acid sequences is provided by the local homology algorithm of Smith and Waterman, Advances in Applied Mathematics 2:482-489 (1981). This algorithm can be extended to use with peptide sequences using the scoring matrix developed by Dayhoff, Atlas of Protein Sequences and Structure, M.O. Dayhoff ed., 5 suppl. 3:353-358, National Biomedical Research Foundation, Washington, D.C., USA, and normalized by Gribskov, Nucl. Acids Res. 14(6):6745-6763 (1986). An implementation of this algorithm for nucleic acid and peptide sequences is provided by the Genetics Computer Group (Madison, WI) in their BestFit utility application. The default parameters for this method are described in the Wisconsin Sequence Analysis Package Program Manual, Version 8 (1995) (available from Genetics Computer Group, Madison, WI). Other equally suitable programs for calculating the percent identity or similarity between sequences are generally known in the art.

For example, percent identity of a particular nucleotide sequence to a reference sequence can be determined using the homology algorithm of Smith and Waterman with a default scoring table and a gap penalty of six nucleotide positions. Another method of establishing percent identity in the context of the present invention is to use the MPSRCH package of programs copyrighted by the University of Edinburgh, developed by John F. Collins and Shane S. Sturrok, and distributed by IntelliGenetics, Inc. (Mountain View, CA). From this suite of packages, the Smith-Waterman algorithm can be employed where default parameters are used for the scoring table (for example, gap open penalty of 12, gap extension penalty of one, and a gap of six). From the data generated, the "Match" value reflects "sequence identity." Other suitable programs for calculating the percent identity or similarity between sequences are generally known in the art, such as the alignment program BLAST, which can also be used with default parameters. For example, BLASTN and BLASTP can be used with the following default parameters: genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR. Details of these programs can be found at the following internet address:
http://www.ncbi.nlm.gov/cgi-bin/BLAST.

One of skill in the art can readily determine the proper search parameters to use for a given sequence in the above programs. For example, the search parameters may vary based on the size of the sequence in question. Thus, for example, a representative embodiment of the present invention would include an isolated polynucleotide having X contiguous nucleotides, wherein (i) the X contiguous nucleotides have at least about 50% identity to Y contiguous nucleotides derived from any of the sequences described herein, (ii) X equals Y, and (iii) X is greater than or equal to 6 nucleotides and up to 5000 nucleotides, preferably greater than or equal to 8 nucleotides and up to 5000 nucleotides, more preferably 10-12 nucleotides and up to 5000 nucleotides, and even more preferably 15-20 nucleotides, up to the number of nucleotides present in the full-length sequences described herein (e.g., see the Sequence Listing and claims), including all integer values falling within the above-described ranges.

The synthetic expression cassettes (and purified polynucleotides) of the present invention include related polynucleotide sequences having about 80% to 100%, greater than 80-85%, preferably greater than 90-92%, more preferably greater than 95%, and most preferably greater than 98% sequence (including all integer values falling within these described ranges) identity to the synthetic expression cassette sequences disclosed herein (for example, to the sequences presented in Tables 1A and 1B) when the sequences of the present invention are used as the query sequence.

Two nucleic acid fragments are considered to "selectively hybridize" as described herein. The degree of sequence identity between two nucleic acid molecules affects the efficiency and strength of hybridization events between such molecules. A partially identical nucleic acid sequence will at least partially inhibit a completely identical sequence from hybridizing to a target molecule. Inhibition of hybridization of the completely identical sequence can be assessed using hybridization assays that are well known in the art (e.g., Southern blot, Northern blot, solution hybridization, or the like, see Sambrook, et al., *Molecular Cloning: A Laboratory Manual,* Second Edition, (1989) Cold Spring Harbor, N.Y.). Such assays can be conducted using varying degrees of selectivity, for example, using conditions varying from low to high stringency. If conditions of low stringency are employed, the absence of non-specific binding can be assessed using a secondary probe that lacks even a partial degree of sequence identity (for example, a probe having less than about 30% sequence identity with the target molecule), such that, in the absence of non-specific binding events, the secondary probe will not hybridize to the target.

When utilizing a hybridization-based detection system, a nucleic acid probe is chosen that is complementary to a target nucleic acid sequence, and then by selection of appropriate conditions the probe and the target sequence "selectively hybridize," or bind, to each other to form a hybrid molecule. A nucleic acid molecule that is capable of hybridizing selectively to a target sequence under "moderately stringent" typically hybridizes under conditions that allow detection of a target nucleic acid sequence of at least about 10-14 nucleotides in length having at least approximately 70% sequence identity with the sequence of the selected nucleic acid probe. Stringent hybridization conditions typically allow detection of target nucleic acid sequences of at least about 10-14 nucleotides in length having a sequence identity of greater than about 90-95% with the sequence of the selected nucleic acid probe. Hybridization conditions useful for probe/target hybridization where the probe and target have a specific degree of sequence identity, can be determined as is known in the art (see, for example, Nucleic Acid Hybridization: A Practical Approach, editors B.D. Hames and S.J. Higgins, (1985) Oxford; Washington, DC; IRL Press).

With respect to stringency conditions for hybridization, it is well known in the art that numerous equivalent conditions can be employed to establish a particular stringency by varying, for example, the following factors: the length and nature of probe and target sequences, base composition of the various sequences, concentrations of salts and other hybridization solution components, the presence or absence of blocking agents in the hybridization solutions (e.g., formamide, dextran sulfate, and polyethylene glycol), hybridization reaction temperature and time parameters, as well as, varying wash conditions. The selection of a particular set of hybridization conditions is selected following standard methods in the art (see, for example, Sambrook, et al., Molecular Cloning: A Laboratory Manual, Second Edition, (1989) Cold Spring Harbor, N.Y.).

A first polynucleotide is "derived from" second polynucleotide if it has the same or substantially the same basepair sequence as a region of the second polynucleotide, its cDNA, complements thereof, or if it displays sequence identity as described above.

A first polypeptide is "derived from" a second polypeptide if it is (i) encoded by a first polynucleotide derived from a second polynucleotide, or (ii) displays sequence identity to the second polypeptides as described above.

Generally, a viral polypeptide is "derived from" a particular polypeptide of a virus (viral polypeptide) if it is (i) encoded by an open reading frame of a polynucleotide of that virus (viral polynucleotide), or (ii) displays sequence identity to polypeptides of that virus as described above.

"Encoded by" refers to a nucleic acid sequence which codes for a polypeptide sequence, wherein the polypeptide sequence or a portion thereof contains an amino acid sequence of at least 3 to 5 amino acids, more preferably at least 8 to 10 amino acids, and even more preferably at least 15 to 20 amino acids from a polypeptide encoded by the nucleic acid sequence. Also encompassed are polypeptide sequences which are immunologically identifiable with a polypeptide encoded by the sequence.

"Purified polynucleotide" refers to a polynucleotide of interest or fragment thereof which is essentially free, e.g., contains less than about 50%, preferably less than about 70%, and more preferably less than about 90%, of the protein with which the polynucleotide is naturally associated. Techniques for purifying polynucleotides of interest are well-known in the art and include, for example, disruption of the cell containing the polynucleotide with a chaotropic agent and separation of the polynucleotide(s) and proteins by ion-exchange chromatography, affinity chromatography and sedimentation according to density.

By "nucleic acid immunization" is meant the introduction of a nucleic acid molecule encoding one or more selected antigens into a host cell, for the *in vivo* expression of an antigen, antigens, an epitope, or epitopes. The nucleic acid molecule can be introduced directly into a recipient subject, such as by injection, inhalation, oral, intranasal and mucosal administration, or the like, or can be introduced *ex vivo,* into cells which have been removed from the host. In the latter case, the transformed cells are reintroduced into the subject where an immune response can be mounted against the antigen encoded by the nucleic acid molecule.

"Gene transfer" or "gene delivery" refers to methods or systems for reliably inserting DNA or RNA of interest into a host cell. Such methods can result in transient expression of non-integrated transferred DNA, extrachromosomal replication and expression of transferred replicons (e.g., episomes), or integration of transferred genetic material into the genomic DNA of host cells. Gene delivery expression vectors include, but are not limited to, vectors derived from bacterial plasmid vectors, viral vectors, non-viral vectors, alphaviruses, pox viruses and vaccinia viruses. When used for immunization, such gene delivery expression vectors may be referred to as vaccines or vaccine vectors.

"T lymphocytes" or "T cells" are non-antibody producing lymphocytes that constitute a part of the cell-mediated arm of the immune system. T cells arise from immature lymphocytes that migrate from the bone marrow to the thymus, where they undergo a maturation process under the direction of thymic hormones. Here, the mature lymphocytes rapidly divide increasing to very large numbers. The maturing T cells become immunocompetent based on their ability to recognize and bind a specific antigen. Activation of immunocompetent T cells is triggered when an antigen binds to the lymphocyte's surface receptors.

The term "transfection" is used to refer to the uptake of foreign DNA by a cell. A cell has been "transfected" when exogenous DNA has been introduced inside the cell membrane. A number of transfection techniques are generally known in the art. *See, e.g.,* Graham et al. (1973) *Virology,* 52:456, Sambrook et al. (1989) *Molecular Cloning, a laboratory manual,* Cold Spring Harbor Laboratories, New York, Davis et al. (1986) *Basic Methods in Molecular Biology,* Elsevier, and Chu et al. (1981) *Gene* 13:197. Such techniques can be used to introduce one or more exogenous DNA moieties into suitable host cells. The term refers to both stable and transient uptake of the genetic material, and includes uptake of peptide- or antibody-linked DNAs.

A "vector" is capable of transferring gene sequences to target cells (e.g., bacterial plasmid vectors, viral vectors, non-viral vectors, particulate carriers, and liposomes). Typically, "vector construct," "expression vector," and "gene transfer vector," mean any nucleic acid construct capable of directing the expression of a gene of interest and which can transfer gene sequences to target cells. Thus, the term includes cloning and expression vehicles, as well as viral vectors.

Transfer of a "suicide gene" (e.g., a drug-susceptibility gene) to a target cell renders the cell sensitive to compounds or compositions that are relatively nontoxic to normal cells. Moolten, F.L. (1994) *Cancer Gene Ther.* 1:279-287. Examples of suicide genes are thymidine kinase of herpes simplex virus (HSV-tk), cytochrome P450 (Manome et al. (1996) *Gene Therapy* 3:513-520), human deoxycytidine kinase (Manome et al. (1996) *Nature Medicine* 2(5):567-573) and the bacterial enzyme cytosine deaminase (Dong et al. (1996) *Human Gene Therapy* 7:713-720). Cells which express these genes are rendered sensitive to the effects of the relatively nontoxic prodrugs ganciclovir (HSV-tk), cyclophosphamide (cytochrome P450 2B1), cytosine arabinoside (human deoxycytidine kinase) or 5-fluorocytosine (bacterial cytosine deaminase). Culver et al. (1992) *Science* 256:1550-1552, Huber et al. (1994) *Proc. Natl. Acad. Sci. USA* 91:8302-8306.

A "selectable marker" or "reporter marker" refers to a nucleotide sequence included in a gene transfer vector that has no therapeutic activity, but rather is included to allow for simpler preparation, manufacturing, characterization or testing of the gene transfer vector.

A "specific binding agent" refers to a member of a specific binding pair of molecules wherein one of the molecules specifically binds to the second molecule through chemical and/or physical means. One example of a specific binding agent is an antibody directed against a selected antigen.

By "subject" is meant any member of the subphylum chordata, including, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The term does not denote a particular age. Thus, both adult and newborn individuals are intended to be covered. The system described above is intended for use in any of the above vertebrate species, since the immune systems of all of these vertebrates operate similarly.

By "pharmaceutically acceptable" or "pharmacologically acceptable" is meant a material which is not biologically or otherwise undesirable, i.e., the material may be administered to an individual in a formulation or composition without causing any undesirable biological effects or interacting in a deleterious manner with any of the components of the composition in which it is contained.

By "physiological pH" or a "pH in the physiological range" is meant a pH in the range of approximately 7.2 to 8.0 inclusive, more typically in the range of approximately 7.2 to 7.6 inclusive.

As used herein, "treatment" refers to any of (I) the prevention of infection or reinfection, as in a traditional vaccine, (ii) the reduction or elimination of symptoms, and (iii) the substantial or complete elimination of the pathogen in question. Treatment may be effected prophylactically (prior to infection) or therapeutically (following infection).

"Lentiviral vector", and "recombinant lentiviral vector" are derived from the subset of retroviral vectors known as lentiviruses. Lentiviral vectors refer to a nucleic acid construct which carries, and within certain embodiments, is capable of directing the expression of a nucleic acid molecule of interest. The lentiviral vector includes at least one transcriptional promoter/enhancer or locus defining element(s), or other elements which control gene expression by other means such as alternate splicing, nuclear RNA export, post-translational modification of messenger, or post-transcriptional modification of protein. Such vector constructs must also include a packaging signal, long terminal repeats (LTRS) or portion thereof, and positive and negative strand primer binding sites appropriate to the lentiviral vector used (if these are not already present in the retroviral vector). Optionally, the recombinant lentiviral vector may also include a signal which directs polyadenylation, selectable markers such as Neo, TK, hygromycin, phleomycin, histidinol, or DHFR, as well as one or more restriction sites and a translation termination sequence. By way of example, such vectors typically include a 5' LTR, a tRNA binding site, a packaging signal, an origin of second strand DNA synthesis, and a 3'LTR or a portion thereof.

"Lentiviral vector particle" as utilized within the present invention refers to a lentivirus which carries at least one gene of interest. The retrovirus may also contain a selectable marker. The recombinant lentivirus is capable of reverse transcribing its genetic material (RNA) into DNA and incorporating this genetic material into a host cell's DNA upon infection. Lentiviral vector particles may have a lentiviral envelope, a non-lentiviral envelope (e.g., an ampho or VSV-G envelope), or a chimeric envelope.

"Nucleic acid expression vector" or "Expression cassette" refers to an assembly which is capable of directing the expression of a sequence or gene of interest. The nucleic acid expression vector includes a promoter which is operably linked to the sequences or gene(s) of interest. Other control elements may be present as well. Expression cassettes described herein may be contained within a plasmid construct. In addition to the components of the expression cassette, the plasmid construct may also include a bacterial origin of replication, one or more selectable markers, a signal which allows the plasmid construct to exist as single-stranded DNA *(e.g.,* a M13 origin of replication), a multiple cloning site, and a "mammalian" origin of replication (e.g., a SV40 or adenovirus origin of replication).

"Packaging cell" refers to a cell which contains those elements necessary for production of infectious recombinant retrovirus (e.g., lentivirus) which are lacking in a recombinant retroviral vector. Typically, such packaging cells contain one or more expression cassettes which are capable of expressing proteins which encode *Gag, pol and* env proteins.

"Producer cell" or "vector producing cell" refers to a cell which contains all elements necessary for production of recombinant retroviral vector particles.

### 2. MODES OF CARRYING OUT THE INVENTION

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

Although a number of methods and materials similar or equivalent to those described herein can be used in the practice of the present invention, the preferred materials and methods are described herein.

### 2.1 SYNTHETIC EXPRESSION CASSETTES

### 2.1.1 MODIFICATION OF HIV-1 GAG NUCLEIC ACID CODING SEQUENCES

One aspect of the present invention is the generation of HIV-1 Gag protein coding sequences, and related sequences, having improved expression relative to the corresponding wild-type sequence. An exemplary embodiment of the present invention is illustrated herein modifying the Gag protein wild-type sequences obtained from the HIV-1SF2 strain (SEQ ID NO:1; Sanchez-Pescador, R., et al., *Science* 227(4686): 484-492, 1985; Luciw, P.A., et al. U.S. Patent No. 5,156,949, issued October 20, 1992; Luciw, P.A., et al., U.S. Patent No. 5,688,688, November 18, 1997). Gag sequence obtained from other HIV variants may be manipulated in similar fashion following the teachings of the present specification. Such other variants include, but are not limited to, Gag protein encoding sequences obtained from the isolates HIV_{IIIb}, HIV_{SF2}, HIV-1_{SF262}, HIV-1_{SF170}, HIV_{LAV}, HIV_{LAI}, HIV_{MN}, HIV-1_{CM235}, , HIV-1_{US4}, other HIV-1 strains from diverse subtypes (e.g., subtypes, A through G, and O) HIV-2 strains and diverse subtypes (e.g., HIV-2_{UC1} and HIV-2_{UC2}), and simian immunodeficiency virus (SIV). (See, e.g., Virology, 3rd Edition (W.K. Joklik ed. 1988); *Fundamental Virology,* 2nd Edition (B.N. Fields and D.M. Knipe, eds. 1991); *Virology,* 3rd Edition (Fields, BN, DM Knipe, PM Howley, Editors, 1996, Lippincott-Raven, Philadelphia, PA; for a description of these and other related viruses).

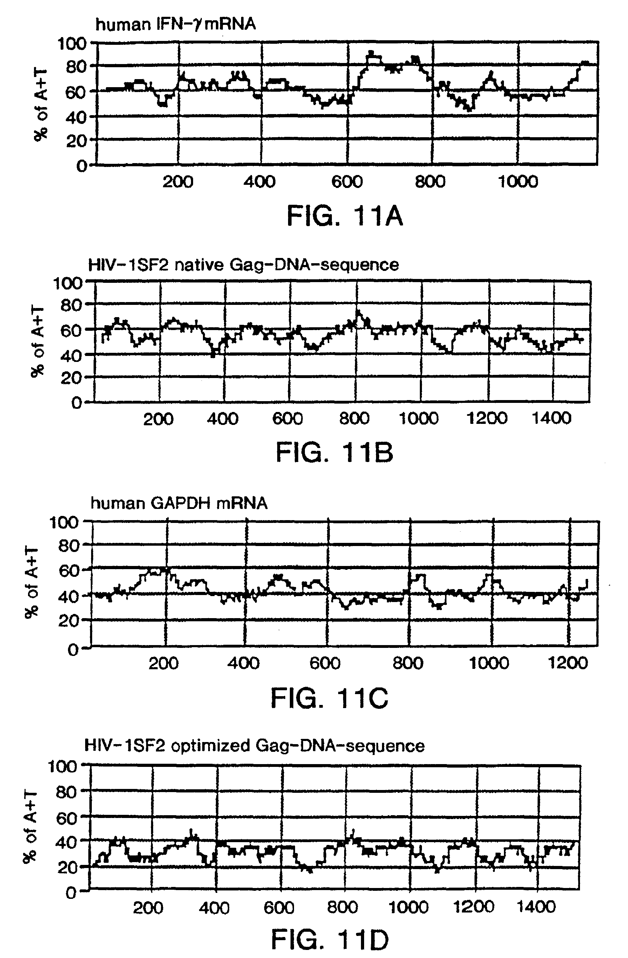

First, the HIV-1 codon usage pattern was modified so that the resulting nucleic acid coding sequence was comparable to codon usage found in highly expressed human genes (Example 1). The HIV codon usage reflects a high content of the nucleotides A or T of the codon-triplet. The effect of the HIV-1 codon usage is a high AT content in the DNA sequence that results in a decreased translation ability and instability of the mRNA. In comparison, highly expressed human codons prefer the nucleotides G or C. The Gag coding sequences were modified to be comparable to codon usage found in highly expressed human genes. In Figure 11 (Example 1), the percent A-T content of cDNA sequences corresponding to the mRNA for a known unstable mRNA and a known stable mRNA are compared to the percent A-T content of native HIV-1SF2 Gag cDNA and to the synthetic Gag cDNA sequence of the present invention. Experiments performed in support of the present invention showed that the synthetic Gag sequences were capable of higher level of protein production (see the Examples) relative to the native Gag sequences. The data in Figure 11 suggest that one reason for this increased production is increased stability of the mRNA corresponding to the synthetic Gag coding sequences versus the mRNA corresponding to the native Gag coding sequences.

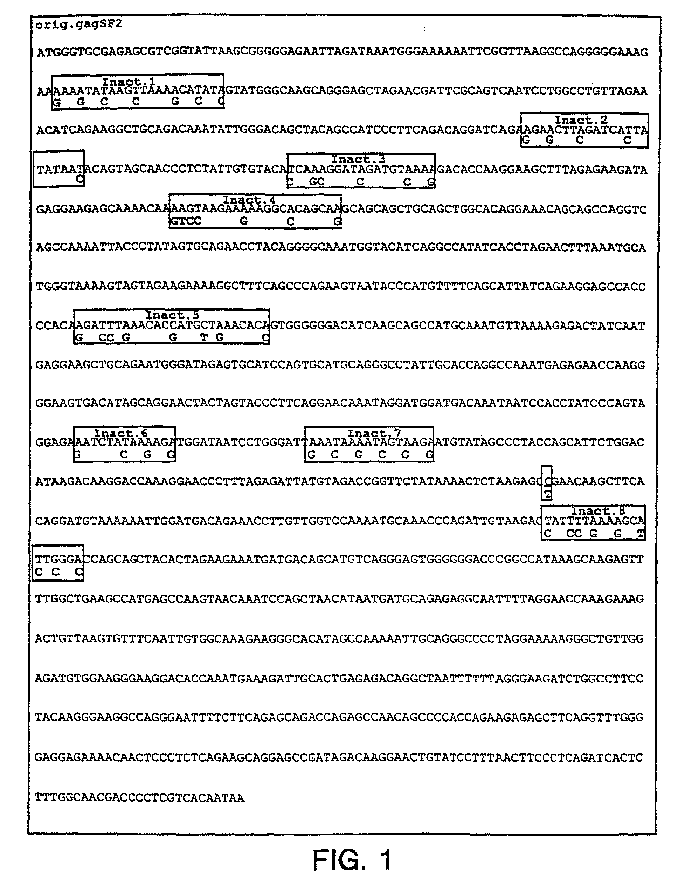

Second, there are inhibitory (or instability) elements (INS) located within the coding sequences of the Gag coding sequences (Example 1). The RRE is a secondary RNA structure that interacts with the HIV encoded Rev-protein to overcome the expression down-regulating effects of the INS. To overcome the post-transcriptional activating mechanisms of RRE and Rev, the instability elements were inactivated by introducing multiple point mutations that did not alter the reading frame of the encoded proteins. Figure 1 shows the original SF2 Gag sequence, the location of the INS sequences, and the modifications made to the INS sequences to reduce their effects. The resulting modified coding sequences are presented as a synthetic Gag expression cassette (SEQ ID NO:4).

Modification of the Gag polypeptide coding sequences resulted in improved expression relative to the wild-type coding sequences in a number of mammalian cell lines (as well as other types of cell lines, including, but not limited to, insect cells). Further, expression of the sequences resulted in production of virus-like particles (VLPs) by these cell lines (see below). Similar Gag polypeptide coding sequences can be obtained from a variety of isolates (families, sub-types, strains, etc.) including, but not limited to such other variants include, but are not limited to, Gag polypeptide encoding sequences obtained from the isolates HIV_{IIIb}, HIV_{SF2}, HIV-1_{SF162}, HIV-1_{SF170}, HIV_{LAV}, HIV_{LAI}, HIV_{MN}, HIV-1_{CM235}, HIV-1_{US4}, other HIV-1 strains from diverse subtypes(e.g., subtypes, A through G, and O), HIV-2 strains and diverse subtypes (e.g., HIV-2_{UC1} and HIV-2_{UC2}), and simian immunodeficiency virus (SIV). (See, e.g., Virology, 3rd Edition (W.K. Joklik ed. 1988); *Fundamental Virology,* 2nd Edition (B.N. Fields and D.M. Knipe, eds. 1991; *Virology,* 3rd Edition (Fields, BN, DM Knipe, PM Howley, Editors, 1996, Lippincott-Raven, Philadelphia, PA). Gag polypeptide encoding sequences derived from these variants can be optimized and tested for improved expression in mammals by following the teachings of the present specification (see the Examples, in particular Example 1).

### 2.1.2 FURTHER MODIFICATION OF SEQUENCES INCLUDING HIV-1 GAG NUCLEIC ACID CODING SEQUENCES

Experiments performed in support of the present invention have shown that similar modifications of HIV-1 Gag-protease, Gag-reverse transcriptase and Gag-polymerase sequences also result in improved expression of the polyproteins, as well as, the production of VLPs formed by polypeptides produced from such modified coding sequences.

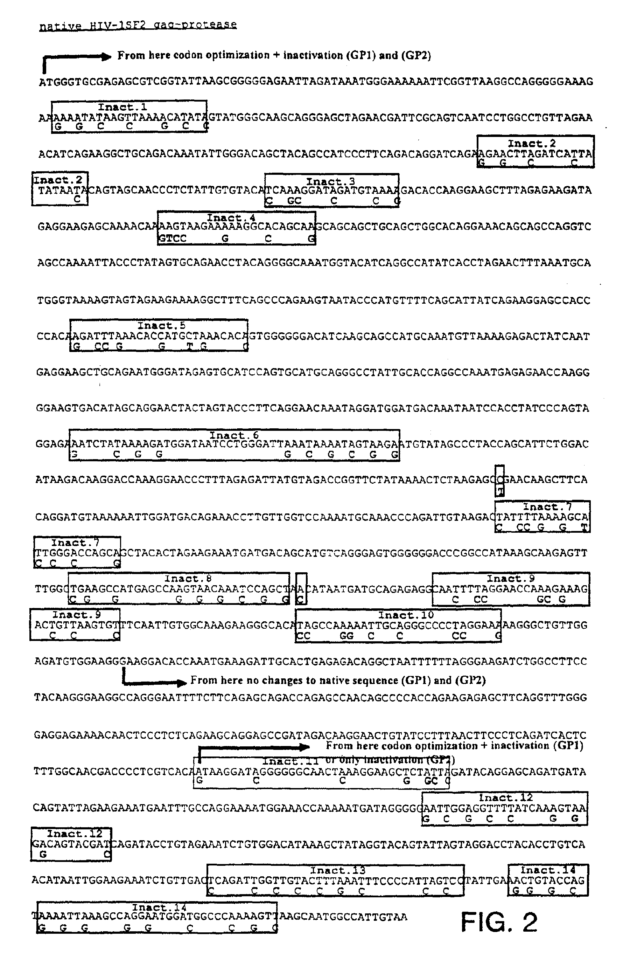

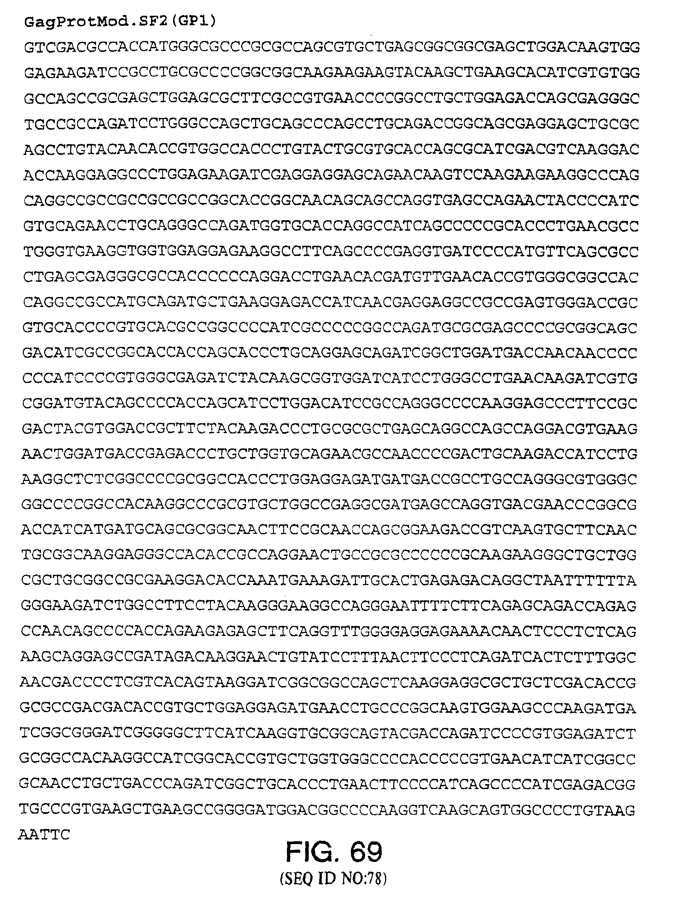

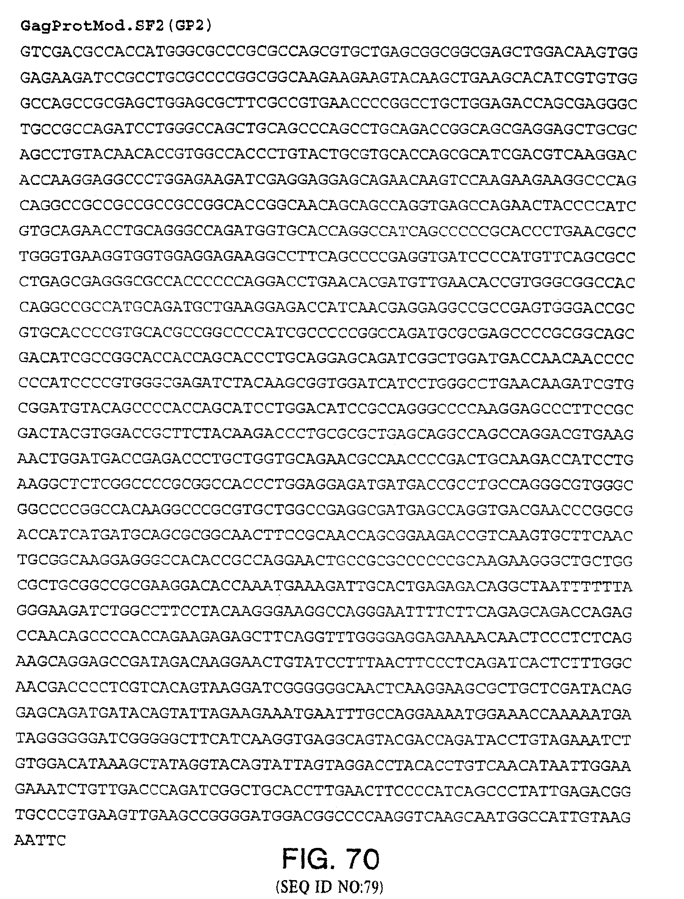

For the Gag-protease sequence (wild type, SEQ ID NO:2; modified, SEQ ID NOs:5, 78, 79), the changes in codon usage were restricted to the regions upstream of the -1 frameshift (Figure 2). Further, inhibitory (or instability) elements (INS) located within the coding sequences of the Gag-protease polypeptide coding sequence were altered as well (indicated in Figure 2). Exemplary constructs (which include the -1 frameshift) encoding modified Gag-protease sequences include those shown in SEQ ID NOs:78 and 79 (Figures 69 and 70). These are: GP1 (SEQ ID No:78) in which the protease region was also codon optimized and INS inactivated and GP2 (SEQ ID NO:79), in which the protease region was only subjected to INS inactivation.

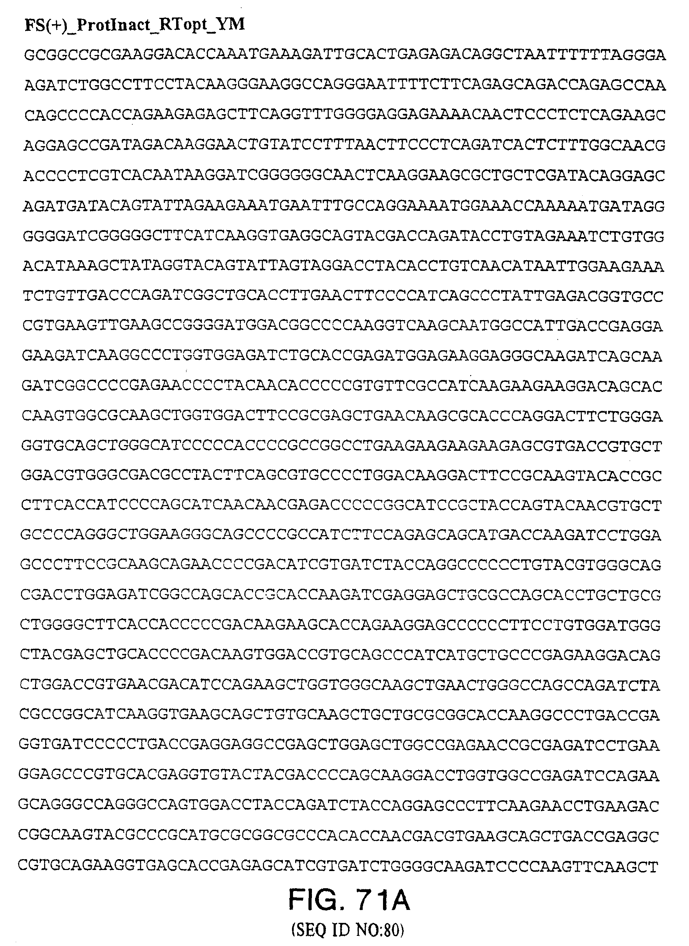

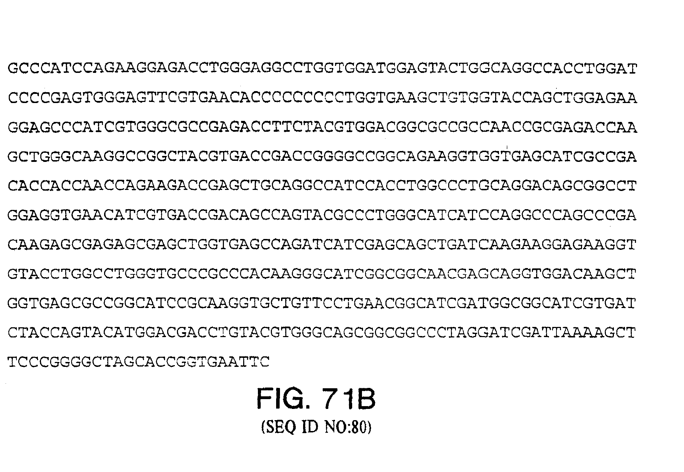

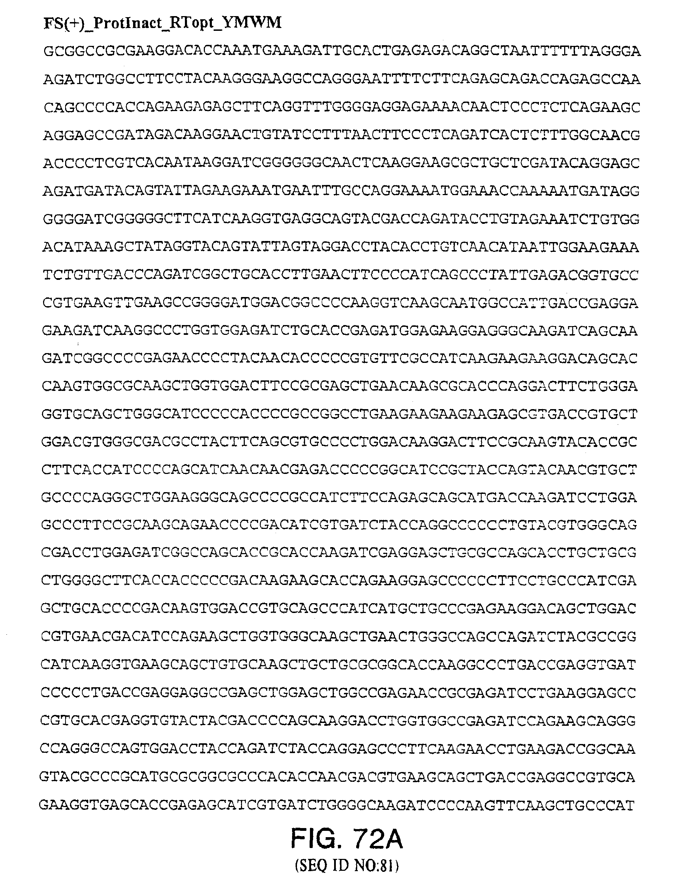

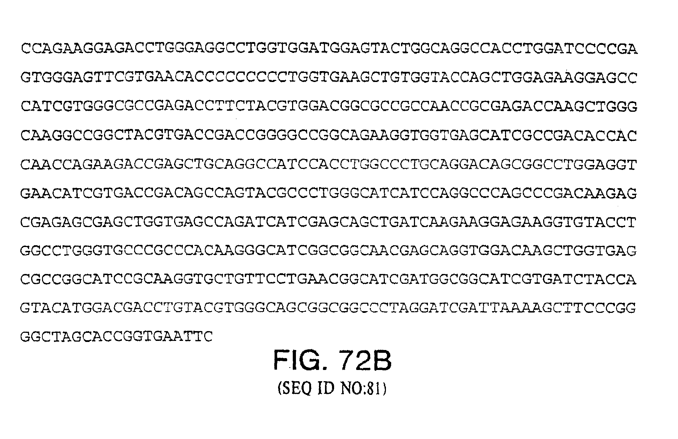

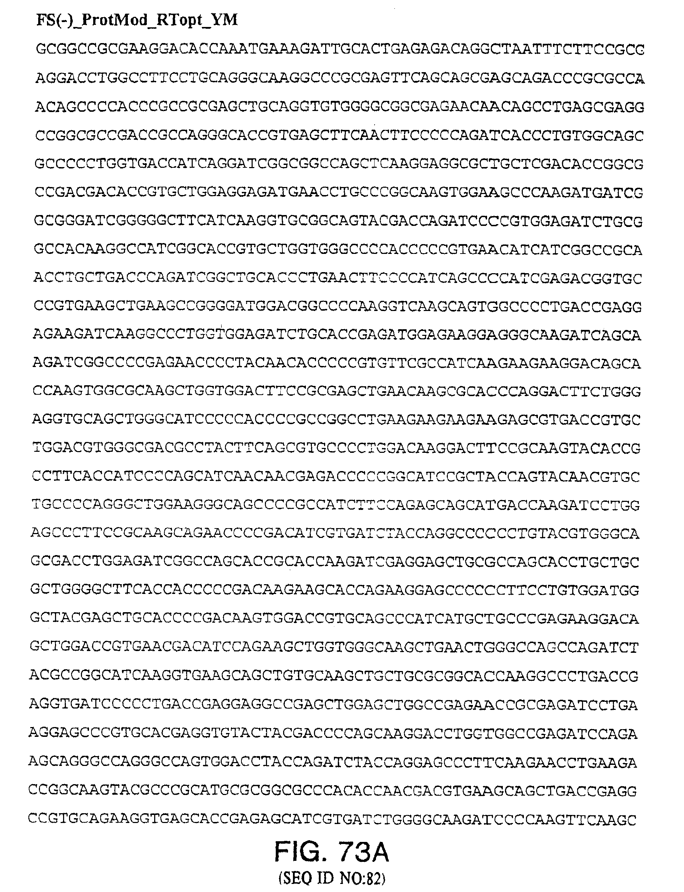

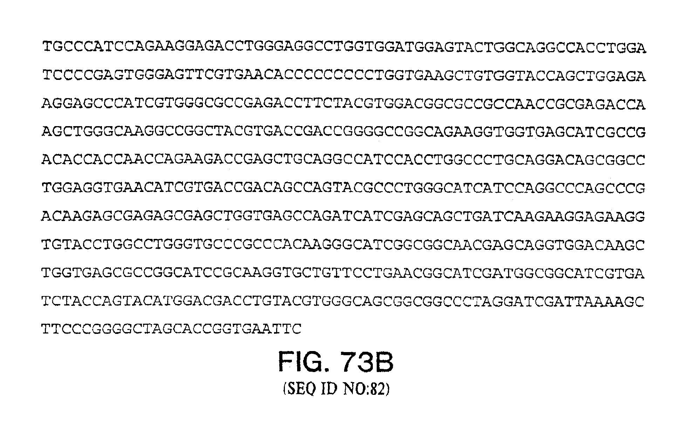

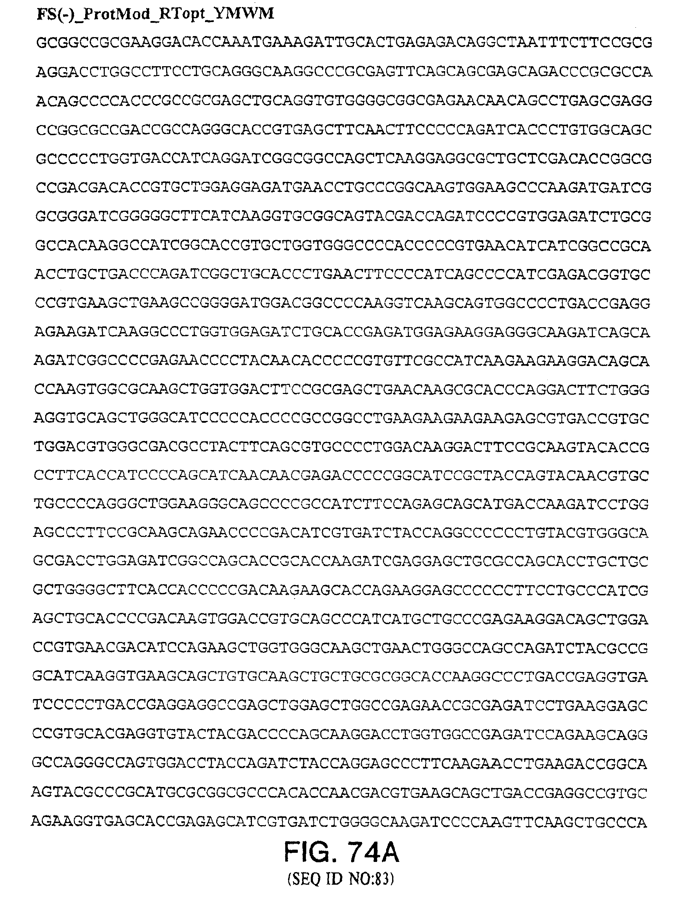

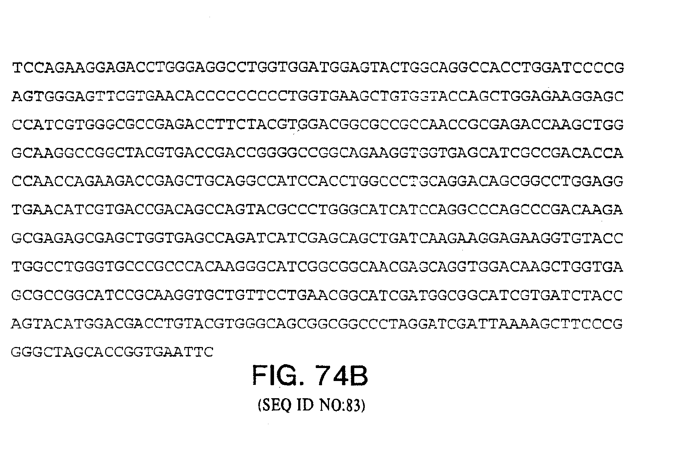

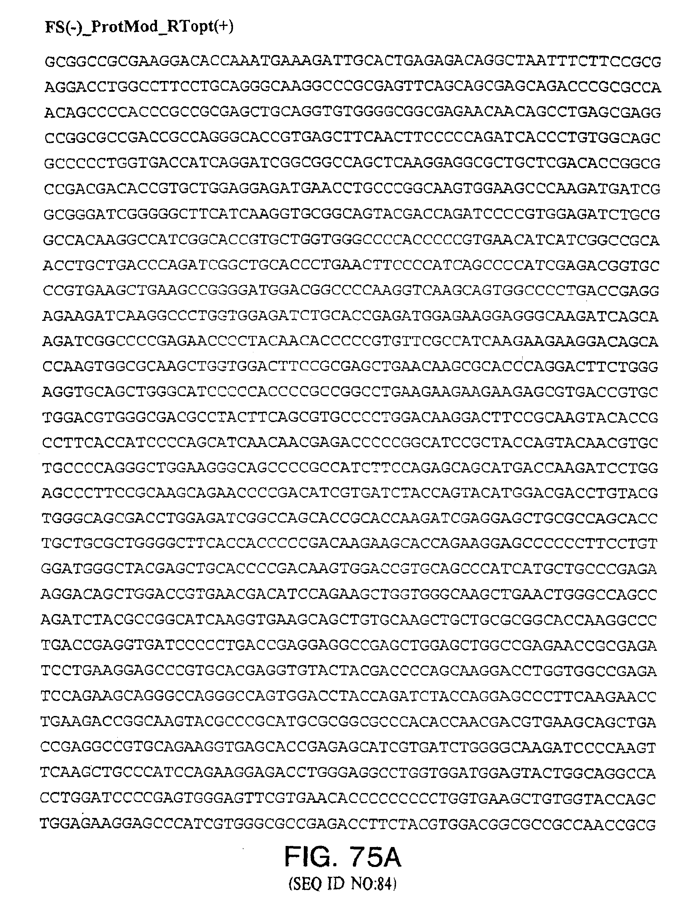

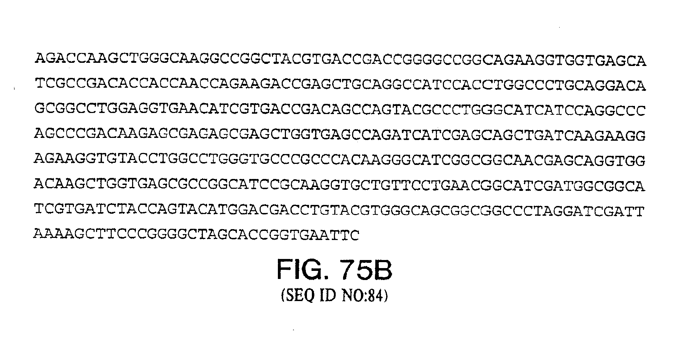

For other Gag-containing sequences, for example the Gag-polymerase sequence (wild type, SEQ ID NO:3; modified, SEQ ID NO:6) or Gag-reverse transcriptase (wild type, SEQ ID NO:77; modified SEQ ID NOs:80-84), the changes in codon usage are similar to those for the Gag-protease sequence. Those expression cassettes which contain a frameshift in the GagPol coding sequence are designated "FS(+)" (SEQ ID NOs:80 and 81, Figures 71 and 72) while the designation "FS(-)" (SEQ ID Nos: 82, 83 and 84, Figures 73, 74 and 75) indicates that there is no frameshift utilized in this coding sequence.

In addition to polyproteins containing HIV-related sequences, the various Gag-, Gag-prot, Gag-pol, Gag-reverse transcriptase encoding sequences of the present invention can be fused to other polypeptides (creating chimeric polypeptides) for which an immunogenic response is desired. An example of such a chimeric protein is the joining of the improved expression Gag encoding sequences to the Hepatitis C Virus (HCV) core protein. In this case, the HCV-core encoding sequences were placed in-frame with the HIV-Gag encoding sequences, resulting in the Gag/HCV-core encoding sequence presented as SEQ ID NO:7 (wild type sequence presented as SEQ ID NO:8).

Further sequences useful in the practice of the present invention include, but are not limited to, sequences encoding viral epitopes/antigens {including but not limited to, HCV antigens (e.g., E1, E2; Houghton, M.., et al., U.S. Patent No. 5,714,596, issued February 3, 1998; Houghton, M.., et al., U.S. Patent No. 5,712,088, issued January 27, 1998; Houghton, M.., et al., U.S. Patent No. 5,683,864, issued November 4, 1997; Weiner, A.J., et al., U.S. Patent No. 5,728,520, issued March 17, 1998; Weiner, A.J., et al., U.S. Patent No. 5,766,845, issued June 16, 1998; Weiner, A.J., et al., U.S. Patent No. 5,670,152, issued September 23, 1997), HIV antigens (e.g., derived from *nef, tat, rev, vpu, vif, vpr* and/or *env*); and sequences encoding tumor antigens/epitopes. Additional sequences are described below. Also, variations on the orientation of the Gag and other coding sequences, relative to each other, are also described below.

Gag, Gag-protease, Gag-reverse transcriptase and/or Gag-polymerase polypeptide coding sequences can be obtained from any HIV isolates (different families, subtypes, and strains) including but not limited to the isolates HIV_{IIIb}, HIV_{SF2}, HIV_{SF162}, HIVus4, HIV_{cm235}, HIV_{LAV}, HIV_{LAI}, HIV_{MN}) (see, e.g., Myers et al. Los Alamos Database, Los Alamos National Laboratory, Los Alamos, New Mexico (1992); Myers et al., *Human Retroviruses and Aids, 1997,* Los Alamos, New Mexico: Los Alamos National Laboratory). Synthetic expression cassettes can be generated using such coding sequences as starting material by following the teachings of the present specification (e.g., see Example 1). Further, the synthetic expression cassettes of the present invention include related Gag polypeptide coding sequences having greater than 75%, preferably greater than 80-85%, more preferably greater than 90-95%, and most preferably greater than 98% sequence identity (or any integer value within these ranges) to the synthetic expression cassette sequences disclosed herein (for example, SEQ ID NO:4; SEQ ID NO:5; SEQ ID NO:6; and SEQ ID NO:20, the Gag Major Homology Region).

### 2.1.3 EXPRESSION OF SYNTHETIC SEQUENCES ENCODING HIV-1 GAG AND RELATED POLYPEPTIDES

Several synthetic Gag-encoding sequences (expression cassettes) of the present invention were cloned into a number of different expression vectors (Example 1) to evaluate levels of expression and production of VLPs. Two modified synthetic coding sequences are presented as a synthetic Gag expression cassette (SEQ ID No:4) and a synthetic Gag-protease expression cassette (SEQ ID NOs:78 and 79). Other synthetic Gag-encoding proteins are presented, for example, as SEQ ID NOs:80 through 84. The synthetic DNA fragments for Gag-encoding polypeptides (e.g., Gag, Gag-protease, Gag-polymerase, Gag-reverse transcriptase) were cloned into expression vectors described in Example 1, including, a transient expression vector, CMV-promoter-based mammalian vectors, and a shuttle vector for use in baculovirus expression systems. Corresponding wild-type sequences were cloned into the same vectors.

These vectors were then transfected into a several different cell types, including a variety of mammalian cell lines,(293, RD, COS-7, and CHO, cell lines available, for example, from the A.T.C.C.). The cell lines were cultured under appropriate conditions and the levels of p24 (Gag) expression in supernatants were evaluated (Example 2). The results of these assays demonstrated that expression of synthetic Gag-encoding sequences were significantly higher than corresponding wild-type sequences (Example 2; Table 2).

Further, Western Blot analysis showed that cells containing the synthetic Gag expression cassette produced the expected 55 kD (p55) protein at higher per-cell concentrations than cells containing the native expression cassette. The Gag p55 protein was seen in both cell lysates and supernatants. The levels of production were significantly higher in cell supernatants for cells transfected with the synthetic Gag expression cassette of the present invention. Experiments performed in support of the present invention suggest that cells containing the synthetic Gag-prot expression cassettes produced the expected Gag-prot protein at comparably higher per-cell concentrations than cells containing the wild-type expression cassette.

Fractionation of the supernatants from mammalian cells transfected with the synthetic Gag expression cassette showed that it provides superior production of both p55 protein and VLPs, relative to the wild-type Gag sequences (Examples 6 and 7).

Efficient expression of these Gag-containing polypeptides in mammalian cell lines provides the following benefits: the Gag polypeptides are free of baculovirus contaminants; production by established methods approved by the FDA; increased purity; greater yields (relative to native coding sequences); and a novel method of producing the Gag-containing polypeptides in CHO or other mammalian cells which is not feasible in the absence of the increased expression obtained using the constructs of the present invention. Exemplary Mammalian cell lines include, but are not limited to, BHK, VERO, HT1080, 293, 293T, RD, COS-7, CHO, Jurkat, HUT, SUPT, C8166, MOLT4/clone8, MT-2, MT-4, H9, PM1, CEM, myeloma cells (e.g., SB20 cells) and CEMX174, such cell lines are available, for example, from the A.T.C.C.).

A synthetic Gag expression cassette of the present invention also demonstrated high levels of expression and VLP production when transfected into insect cells (Example 7). Further, in addition to a higher total protein yield, the final product from the synthetic p55-expressed Gag consistently contained lower amounts of contaminating baculovirus proteins than the final purified product from the native p55-expressed Gag.

Further, synthetic Gag expression cassettes of the present invention have also been introduced into yeast vectors which were transformed into and efficiently expressed by yeast cells *(Saccharomyces cerevisea;* using vectors as described in Rosenberg, S. and Tekamp-Olson, P., U.S. Patent No. RE35,749, issued, March 17, 1998).

In addition to the mammalian and insect vectors described in the Examples, the synthetic expression cassettes of the present invention can be incorporated into a variety of expression vectors using selected expression control elements. Appropriate vectors and control elements for any given cell type can be selected by one having ordinary skill in the art in view of the teachings of the present specification and information known in the art about expression vectors.

For example, a synthetic Gag expression cassette can be inserted into a vector which includes control elements operably linked to the desired coding sequence, which allow for the expression of the gene in a selected cell-type. For example, typical promoters for mammalian cell expression include the SV40 early promoter, a CMV promoter such as the CMV immediate early promoter (a CMV promoter can include intron A), RSV, HIV-LTR, the mouse mammary tumor virus LTR promoter (MMLV-LTR), FIV-LTR, the adenovirus major late promoter (Ad MLP), and the herpes simplex virus promoter, among others. Other nonviral promoters, such as a promoter derived from the murine metallothionein gene, will also find use for mammalian expression. Typically, transcription termination and polyadenylation sequences will also be present, located 3' to the translation stop codon. Preferably, a sequence for optimization of initiation of translation, located 5' to the coding sequence, is also present. Examples of transcription terminator/polyadenylation signals include those derived from SV40, as described in Sambrook, et al., supra, as well as a bovine growth hormone terminator sequence. Introns, containing splice donor and acceptor sites, may also be designed into the constructs for use with the present invention (Chapman et al., *Nuc. Acids Res.* (1991) 19:3979-3986).

Enhancer elements may also be used herein to increase expression levels of the mammalian constructs. Examples include the SV40 early gene enhancer, as described in Dijkema et al., *EMBO J.* (1985) 4:761, the enhancer/promoter derived from the long terminal repeat (LTR) of the Rous Sarcoma Virus, as described in Gorman et al., *Proc. Natl. Acad. Sci. USA* (1982b) 79:6777 and elements derived from human CMV, as described in Boshart et al., *Cell* (1985) 41:521, such as elements included in the CMV intron A sequence (Chapman et al., *Nuc. Acids Res.* (1991) 19:3979-3986).

The desired synthetic Gag polypeptide encoding sequences can be cloned into any number of commercially available vectors to generate expression of the polypeptide in an appropriate host system. These systems include, but are not limited to, the following: baculovirus expression {Reilly, P.R., *et al.,* BACULOVIRUS EXPRESSION VECTORS: A LABORATORY MANUAL (1992); Beames, *et al., Biotechniques* 11:378 (1991); Pharmingen; Clontech, Palo Alto, CA)}, vaccinia expression {Earl, P. L., *et al.*, "Expression of proteins in mammalian cells using vaccinia" In *Current Protocols in Molecular Biology* (F. M. Ausubel, *et al.* Eds.), Greene Publishing Associates & Wiley Interscience, New York (1991); Moss, B., *et al.,* U.S. Patent Number 5,135,855, issued 4 August 1992}, expression in bacteria {Ausubel, F.M., et *al.,* CURRENT PROTOCOLS IN MOLECULAR BIOLOGY? John Wiley and Sons, Inc., Media PA; Clontech}, expression in yeast {Rosenberg, S. and Tekamp-Olson, P., U.S. Patent No. RE35,749, issued, March 17, 1998; Shuster, J.R., U.S. Patent No. 5,629,203, issued May 13, 1997; Gellissen, G., et *al., Antonie Van Leeuwenhoek,* 62(1-2):79-93 (1992); Romanos, M.A., *et al.*, Yeast 8(6):423-488 (1992); Goeddel, D.V., *Methods in Enzymology* 185 (1990); Guthrie, C., and G.R. Fink, *Methods in Enzymology* 194 (1991)}, expression in mammalian cells {Clontech; Gibco-BRL, Ground Island, NY; e.g., Chinese hamster ovary (CHO) cell lines (Haynes, J., *et al., Nuc. Acid. Res.* 11:687-706 (1983); 1983, Lau, Y.F., *et al., Mol. Cell. Biol.* 4:1469-1475 (1984); Kaufman, R. J., "Selection and coamplification of heterologous genes in mammalian cells," in *Methods in Enzymology,* vol. 185, pp537-566. Academic Press, Inc., San Diego CA (1991)}, and expression in plant cells {plant cloning vectors, Clontech Laboratories, Inc., Palo Alto, CA, and Pharmacia LKB Biotechnology, Inc., Pistcataway, NJ; Hood, E., et al., *J. Bacteriol.* 168:1291-1301 (1986); Nagel, R., et al., *FEMS Microbiol. Lett.* 67:325 (1990); An, et al., "Binary Vectors", and others in Plant Molecular Biology Manual A3:1-19 (1988); Miki, B.L.A., et *al.,* pp.249-265, and others in Plant DNA Infectious Agents (Hohn, T., *et al.*, eds.) Springer-Verlag, Wien, Austria, (1987); *Plant Molecular Biology: Essential Techniques,* P.G. Jones and J.M. Sutton, New York, J. Wiley, 1997; Miglani, Gurbachan *Dictionary of Plant Genetics and Molecular Biology,* New York, Food Products Press, 1998; Henry, R. J., *Practical Applications of Plant Molecular Biology,* New York, Chapman & Hall, 1997}.

Also included in the invention is an expression vector, such as the CMV promoter-containing vectors described in Example 1, containing coding sequences and expression control elements which allow expression of the coding regions in a suitable host. The control elements generally include a promoter, translation initiation codon, and translation and transcription termination sequences, and an insertion site for introducing the insert into the vector. Translational control elements have been reviewed by M. Kozak (e.g., Kozak, M., *Mamm. Genome* 7(8):563-574, 1996; Kozak, M., *Biochimie* 76(9):815-821, 1994; Kozak, M., *J Cell Biol* 108(2):229-241, 1989; Kozak, M., and Shatkin, A.J., *Methods Enzymol* 60:360-375, 1979).

Expression in yeast systems has the advantage of commercial production. Recombinant protein production by vaccinia and CHO cell line have the advantage of being mammalian expression systems. Further, vaccinia virus expression has several advantages including the following: (i) its wide host range; (ii) faithful post-transcriptional modification, processing, folding, transport, secretion, and assembly of recombinant proteins; (iii) high level expression of relatively soluble recombinant proteins; and (iv) a large capacity to accommodate foreign DNA.

The recombinantly expressed polypeptides from synthetic Gag-encoding expression cassettes are typically isolated from lysed cells or culture media. Purification can be carried out by methods known in the art including salt fractionation, ion exchange chromatography, gel filtration, size-exclusion chromatography, size-fractionation, and affinity chromatography. Immunoaffinity chromatography can be employed using antibodies generated based on, for example, Gag antigens.

Advantages of expressing the Gag-containing proteins of the present invention using mammalian cells include, but are not limited to, the following: well-established protocols for scale-up production; the ability to produce VLPs; cell lines are suitable to meet good manufacturing process (GMP) standards; culture conditions for mammalian cells are known in the art.

### 2.1.4 MODIFICATION OF HIV-1 ENV NUCLEIC ACID CODING SEQUENCES

One aspect of the present invention is the generation of HIV-1 Env protein coding sequences, and related sequences, having improved expression relative to the corresponding wild-type sequence. Exemplary embodiments of the present invention are illustrated herein modifying the Env protein wild-type sequences obtained from the HIV-1 subtype B strains HIV-1US4 and HIV-1SF162 (Myers et al., Los Alamos Database, Los Alamos National Laboratory, Los Alamos, New Mexico (1992); Myers et al., *Human Retroviruses and Aids, 1997,* Los Alamos, New Mexico: Los Alamos National Laboratory). Env sequence obtained from other HIV variants may be manipulated in similar fashion following the teachings of the present specification. Such other variants include those described above in Section 2.1.1 and on the World Wide Web (Internet), for example at http://hiv-web.lan1.Gov/cgi-bin/hivDB3/public/wdb/ssampublic and http://hiv-web.lan1.gov.

First, the HIV-1 codon usage pattern was modified so that the resulting nucleic acid coding sequence was comparable to codon usage found in highly expressed human genes (Example 1). The HIV codon usage reflects a high content of the nucleotides A or T of the codon-triplet. The effect of the HIV-1 codon usage is a high AT content in the DNA sequence that results in a decreased translation ability and instability of the mRNA. In comparison, highly expressed human codons prefer the nucleotides G or C. The Env coding sequences were modified to be comparable to codon usage found in highly expressed human genes. Experiments performed in support of the present invention showed that the synthetic Env sequences were capable of higher level of protein production (see the Examples) relative to the native Env sequences. One reason for this increased production may be increased stability of the mRNA corresponding to the synthetic Env coding sequences versus the mRNA corresponding to the native Env coding sequences.

Modification of the Env polypeptide coding sequences resulted in improved expression relative to the wild-type coding sequences in a number of mammalian cell lines. Similar Env polypeptide coding sequences can be obtained from a variety of isolates (families, sub-types, etc.). Env polypeptide encoding sequences derived from these variants can be optimized and tested for improved expression in mammals by following the teachings of the present specification (see the Examples, in particular Example 2).

### 2.1.5 FURTHER MODIFICATION OF HIV-1 ENV NUCLEIC ACID CODING SEQUENCES

In addition to proteins containing HIV-related sequences, the Env encoding sequences of the present invention can be fused to other polypeptides (creating chimeric polypeptides). Also, variations on the orientation of the Env and other coding sequences, relative to each other, are contemplated. Further, the HIV protein encoding cassettes of the present invention can be co-expressed using one vector or multiple vectors. In addition, the polyproteins can be operably linked to the same or different promoters.

Env polypeptide coding sequences can be obtained from any HIV isolates (different families, subtypes, and strains) including but not limited to the isolates HIV_{IIIb}, HIV_{SF2}, HIVᵤₛ₄, HIV_{CM235}, HIV_{SF162}, HIV_{LAV}, HIV_{LAI}, HIV_{MN}) (see, e.g., Myers et al., Los Alamos Database, Los Alamos National Laboratory, Los Alamos, New Mexico (1992); Myers et al., *Human Retroviruses and Aids,* 1997, Los Alamos, New Mexico: Los Alamos National Laboratory). Synthetic expression cassettes can be generated using such coding sequences as starting material by following the teachings of the present specification (e.g., see Example 1). Further, the synthetic expression cassettes (and purified polynucleotides) of the present invention include related Env polypeptide coding sequences having greater than 90%, preferably greater than 92%, more preferably greater than 95%, and most preferably greater than 98% sequence identity to the synthetic expression cassette sequences disclosed herein (for example, SEQ ID NOs:71-72; and/or the sequences presented in Tables 1A and 1B) when the sequences of the present invention are used as the query sequence.

### 2.1.6 EXPRESSION OF SYNTHETIC SEQUENCES ENCODING HIV-1 ENV AND RELATED POLYPEPTIDES

Several synthetic Env-encoding sequences (expression cassettes) of the present invention were cloned into a number of different expression vectors (Example 1) to evaluate levels of expression and production of Env polypeptide. A modified synthetic coding sequence is presented as synthetic Env expression cassettes (Example 1, e.g., Tables 1A and 1B). The synthetic DNA fragments for Env were cloned into eucaryotic expression vectors described in Example 1 and in Section 2.1.3 above, including, a transient expression vector and CMV-promoter-based mammalian vectors. Corresponding wild-type sequences were cloned into the same vectors.

These vectors were then transfected into a several different cell types, including a variety of mammalian cell lines,(293, RD, COS-7, and CHO, cell lines available, for example, from the A.T.C.C.). The cell lines were cultured under appropriate conditions and the levels of gp120, gp140 and gp160 Env expression in supernatants were evaluated (Example 2). Env polypeptides include, but are not limited to, for example, native gp160, oligomeric gp140, monomeric gp120 as well as modified sequences of these polypeptides. The results of these assays demonstrated that expression of synthetic Env encoding sequences were significantly higher than corresponding wild-type sequences (Example 2; Tables 3 and 4).

Further, Western Blot analysis showed that cells containing the synthetic Env expression cassette produced the expected protein (gp120, gp140 or gp160) at higher per-cell concentrations than cells containing the native expression cassette. The Env proteins were seen in both cell lysates and supernatants. The levels of production were significantly higher in cell supernatants for cells transfected with the synthetic Env expression cassettes of the present invention as compared to wild type.

Fractionation of the supernatants from mammalian cells transfected with the synthetic Env expression cassettes showed that it provides superior production of Env proteins, relative to the wild-type Env sequences (Examples 2 and 3).

Efficient expression of these Env-containing polypeptides in mammalian cell lines provides the following benefits: the Env polypeptides are free of baculovirus or other viral contaminants; production by established methods approved by the FDA; increased purity; greater yields (relative to native coding sequences); and a novel method of producing the Env-containing polypeptides in CHO cells which is less feasible in the absence of the increased expression obtained using the constructs of the present invention.

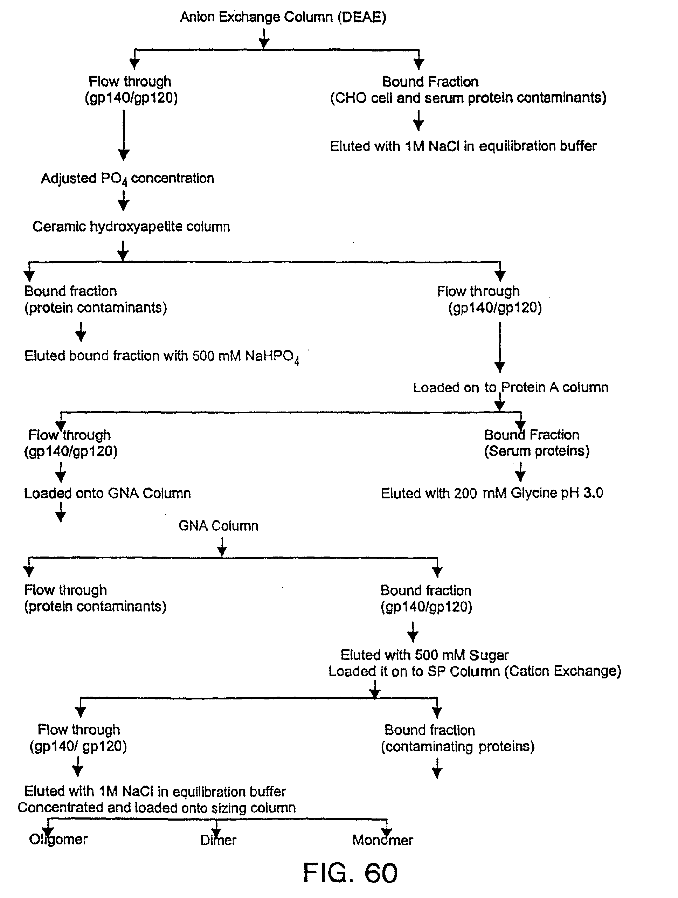

Exemplary cell lines (e.g., mammalian, yeast, insect, etc.) include those described above in Section 2.1.3 for Gag-containing constructs. Further, appropriate vectors and control elements (e.g., promoters, enhancers, polyadenylation sequences, etc.) for any given cell type can be selected, as described above in Section 2.1.3, by one having ordinary skill in the art in view of the teachings of the present specification and information known in the art about expression vectors. In addition, the recombinantly expressed polypeptides from synthetic Env-encoding expression cassettes are typically isolated and purified from lysed cells or culture media, as described above for Gag-encoding expression cassettes. An exemplary purification is described in Example 4 and shown in Figure 60.

### 2.1.7 MODIFICATION OF HIV-1 TAT NUCLEIC ACID CODING SEQUENCES

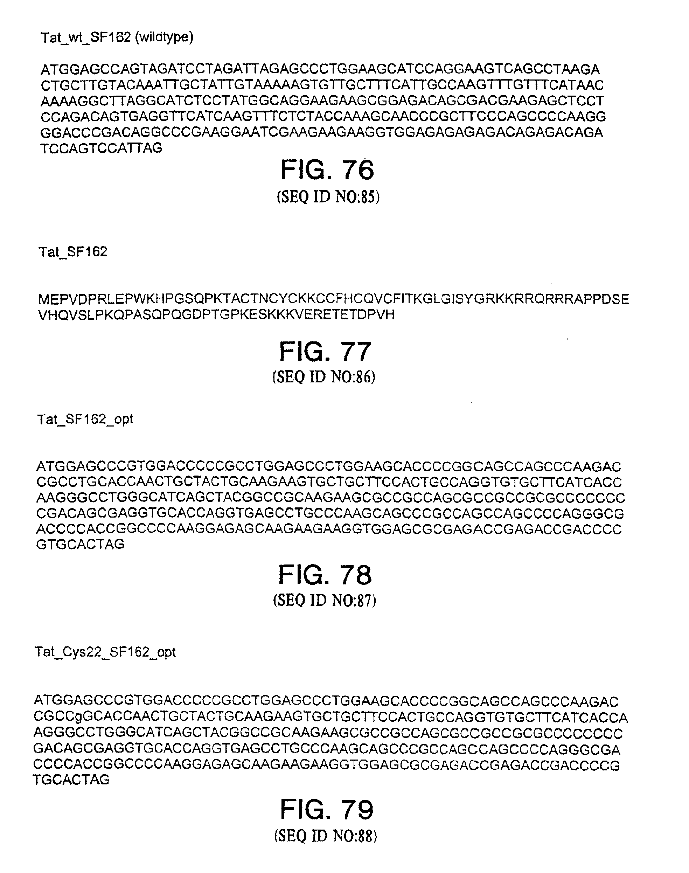

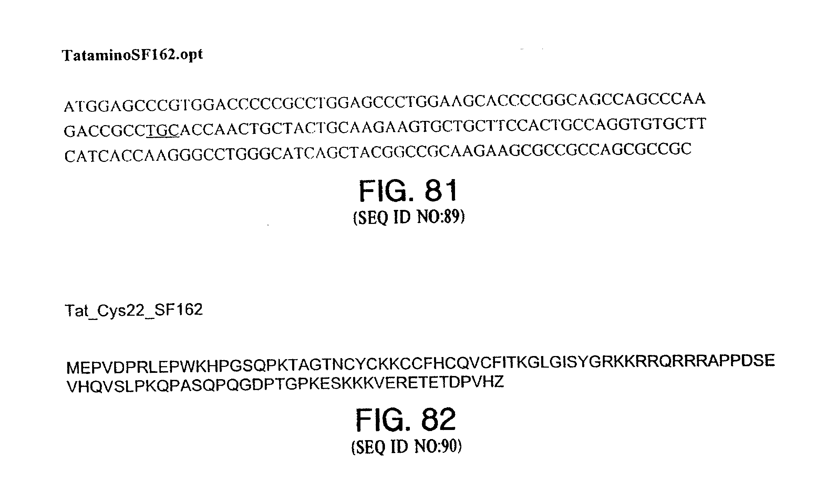

Another aspect of the present invention is the generation of HIV-1 tat protein coding sequences, and related sequences, having improved expression relative to the corresponding wild-type sequence. Exemplary embodiments of the present invention are illustrated herein modifying the tat wild-type nucleotide sequence (SEQ ID NO:85, Figure 76) obtained from SF162 as described above. Exemplary synthetic tat constructs are shown in SEQ ID NO:87, which depicts a tat construct encoding a full-length tat polypeptide from strain SF162; SEQ ID NO:88, which depicts a tat construct encoding a tat polypeptide having the cystein residue at position 22 changed; and SEQ ID NO:89, which depicts a tat construct encoding the amino terminal portion of a tat polypeptide from strain SF162. The amino portion of the tat protein appears to contain many of the epitopes that induce an immune response. In addition, further modifications include replacement or deletion of the cystein residue at position 22, for example with a valine residue, an alanine residue or a glycine residue (SEQ ID Nos: 88 and 89, Figures 79 and 81), see, e.g., Caputo et al. (1996) *Gene Ther.* 3:235. In Figure 81, which depicts a tat construct encoding the amino terminal portion of a tat polypeptide, the nucleotides (nucleotides 64-66) encoding the cystein residues are underlined. The design and construction of suitable construct can be readily done using the teachings of the present specification. As with Gag, pol, prot and Env, tat polypeptide coding sequences can be obtained from a variety of isolates (families, sub-types, etc.).

Modification of the tat polypeptide coding sequences result in improved expression relative to the wild-type coding sequences in a number of cell lines (*e.g.*, mammalian, yeast, bacterial and insect cells). Tat polypeptide encoding sequences derived from these variants can be optimized and tested for improved expression in mammals by following the teachings of the present specification (see the Examples, in particular Example 2).

Various forms of the different embodiments of the invention, described herein, may be combined. For example, polynucleotides may be derived from the polynucleotide sequences of the present invention, including, but not limited to, coding sequences for Gag polypeptides, Env polypeptides, polymerase polypeptides, protease polypeptides, tat polypeptides, and reverse transcriptase polypeptides. Further, the polynucleotide coding sequences of the present invention may be combined into multi-cistronic expression cassettes where typically each coding sequence for each polypeptide is preceded by IRES sequences.

### 2.2 PRODUCTION OF VIRUS-LIKE PARTICLES AND USE OF THE CONSTRUCTS OF THE PRESENT INVENTION TO CREATE PACKAGING CELL LINES

The group-specific antigens (Gag) of human immunodeficiency virus type-1 (HIV-1) self-assemble into noninfectious virus-like particles (VLP) that are released from various eucaryotic cells by budding (reviewed by Freed, E.O., *Virology* 251:1-15, 1998). The synthetic expression cassettes of the present invention provide efficient means for the production of HIV-Gag virus-like particles (VLPs) using a variety of different cell types, including, but not limited to, mammalian cells.

Viral particles can be used as a matrix for the proper presentation of an antigen entrapped or associated therewith to the immune system of the host. For example, U.S. Patent No. 4,722,840 describes hybrid particles comprised of a particle-forming fragment of a structural protein from a virus, such as a particle-forming fragment of hepatitis B virus (HBV) surface antigen (HBsAg), fused to a heterologous polypeptide. Tindle et al., *Virology* (1994) 200:547-557, describes the production and use of chimeric HBV core antigen particles containing epitopes of human papillomavirus (HPV) type 16 E7 transforming protein.

Adams et al., *Nature* (1987) 329:68-70, describes the recombinant production of hybrid HIVgp120:Ty VLPs in yeast and Brown et al., *Virology* (1994) 198:477-488, the production of chimeric proteins consisting of the VP2 protein of human parvovirus B19 and epitopes from human herpes simplex virus type 1, as well as mouse hepatitis virus A59. Wagner et al., *(Virology* (1994) 200:162-175, Brand et al., *J. Virol. Meth.* (1995) 51:153-168; *Virology* (1996) 220:128-140) and Wolf, et al., (EP 0 449 116 A1, published 2 October 1991; WO 96/30523, published 3 October 1996) describe the assembly of chimeric HIV-1 p55Gag particles. U.S. Patent No. 5,503,833 describes the use of rotavirus VP6 spheres for encapsulating and delivering therapeutic agents.

### 2.2.1 VLP PRODUCTION USING THE SYNTHETIC EXPRESSION CASSETTES OF THE PRESENT INVENTION

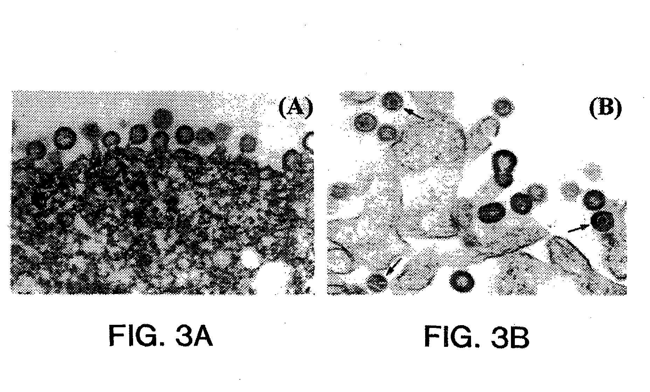

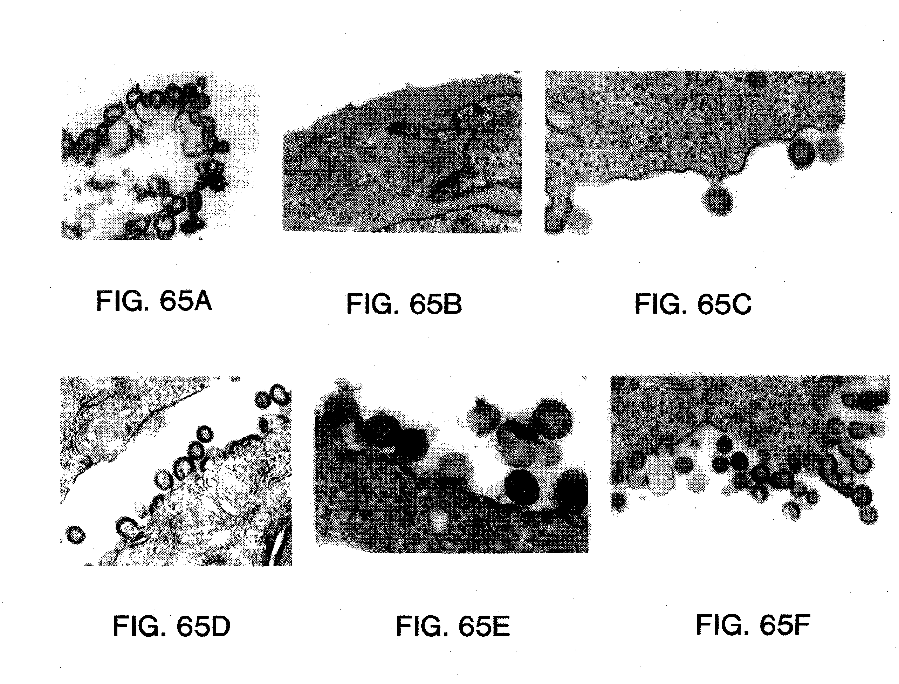

Experiments performed in support of the present invention have demonstrated that the synthetic expression cassettes of the present invention provide superior production of both protein and VLPs, relative to native coding sequences (Examples 7 and 15). Further, electron microscopic evaluation of VLP production (Examples 6 and 15, Figures 3A-B and 65A-F) showed that free and budding immature virus particles of the expected size were produced by cells containing the synthetic expression cassettes.

Using the synthetic expression cassettes of the present invention, rather than native coding sequences, for the production of virus-like particles provide several advantages. First, VLPs can be produced in enhanced quantity making isolation and purification of the VLPs easier. Second, VLPs can be produced in a variety of cell types using the synthetic expression cassettes, in particular, mammalian cell lines can be used for VLP production, for example, CHO cells. Production using CHO cells provides (i) VLP formation; (ii) correct myristylation and budding; (iii) absence of non-mammalian cell contaminants (e.g., insect viruses and/or cells); and (iv) ease of purification. The synthetic expression cassettes of the present invention are also useful for enhanced expression in cell-types other than mammalian cell lines. For example, infection of insect cells with baculovirus vectors encoding the synthetic expression cassettes resulted in higher levels of total protein yield and higher levels of VLP production (relative to wild-type coding sequences). Further, the final product from insect cells infected with the baculovirus-Gag synthetic expression cassettes consistently contained lower amounts of contaminating insect proteins than the final product when wild-type coding sequences were used (Examples).

VLPs can spontaneously form when the particle-forming polypeptide of interest is recombinantly expressed in an appropriate host cell. Thus, the VLPs produced using the synthetic expression cassettes of the present invention are conveniently prepared using recombinant techniques. As discussed below, the Gag polypeptide encoding synthetic expression cassettes of the present invention can include other polypeptide coding sequences of interest (for example, Env, tat, rev, HIV protease, HIV polymerase, HCV core; see, Example 1). Expression of such synthetic expression cassettes yields VLPs comprising the product of the synthetic expression cassette, as well as, the polypeptide of interest.

Once coding sequences for the desired particle-forming polypeptides have been isolated or synthesized, they can be cloned into any suitable vector or replicon for expression. Numerous cloning vectors are known to those of skill in the art, and the selection of an appropriate cloning vector is a matter of choice. See, generally, Ausubel et al, *supra* or Sambrook et al, *supra.* The vector is then used to transform an appropriate host cell. Suitable recombinant expression systems include, but are not limited to, bacterial, mammalian, baculovirus/insect, vaccinia, Semliki Forest virus (SFV), Alphaviruses (such as, Sindbis, Venezuelan Equine Encephalitis (VEE)), mammalian, yeast and Xenopus expression systems, well known in the art. Particularly preferred expression systems are mammalian cell lines, vaccinia, Sindbis, insect and yeast systems.

For example, a number of mammalian cell lines are known in the art and include immortalized cell lines available from the American Type Culture Collection (A.T.C.C.), such as, but not limited to, Chinese hamster ovary (CHO) cells, 293 cells, HeLa cells, baby hamster kidney (BHK) cells, mouse myeloma (SB20), monkey kidney cells (COS), as well as others. Similarly, bacterial hosts such as *E. coli, Bacillus subtilis,* and *Streptococcus spp.,* will find use with the present expression constructs. Yeast hosts useful in the present invention include *inter alia, Saccharomyces cerevisiae, Candida albicans, Candida maltosa, Hansenula polymorpha, Kluyveromyces fragilis, Kluyveromyces lactis, Pichia guillerimondii, Pichia pastoris, Schizosaccharomyces pombe* and *Yarrowia lipolytica.* Insect cells for use with baculovirus expression vectors include, *inter alia*, *Aedes aegypti, Autographa californica, Bombyx mori, Drosophila melanogaster, Spodoptera frugiperda,* and *Trichoplusia ni.* See, e.g., Summers and Smith, *Texas Agricultural Experiment Station Bulletin No. 1555* (1987). Fungal hosts include, for example, *Aspergillus.*

Viral vectors can be used for the production of particles in eucaryotic cells, such as those derived from the pox family of viruses, including vaccinia virus and avian poxvirus. Additionally, a vaccinia based infection/transfection system, as described in Tomei et al., *J. Virol.* (1993) 67:4017-4026 and Selby et al., *J. Gen. Virol.* (1993) 74:1103-1113, will also find use with the present invention. In this system, cells are first infected *in vitro* with a vaccinia virus recombinant that encodes the bacteriophage T7 RNA polymerase. This polymerase displays exquisite specificity in that it only transcribes templates bearing T7 promoters. Following infection, cells are transfected with the DNA of interest, driven by a T7 promoter. The polymerase expressed in the cytoplasm from the vaccinia virus recombinant transcribes the transfected DNA into RNA which is then translated into protein by the host translational machinery. Alternately, T7 can be added as a purified protein or enzyme as in the "Progenitor" system (Studier and Moffatt, *J. Mol. Biol.* (1986) 189:113-130). The method provides for high level, transient, cytoplasmic production of large quantities of RNA and its translation product(s).

Depending on the expression system and host selected, the VLPS are produced by growing host cells transformed by an expression vector under conditions whereby the particle-forming polypeptide is expressed and VLPs can be formed. The selection of the appropriate growth conditions is within the skill of the art. If the VLPs are formed intracellularly, the cells are then disrupted, using chemical, physical or mechanical means, which lyse the cells yet keep the VLPs substantially intact. Such methods are known to those of skill in the art and are described in, e.g., *Protein Purification Applications: A Practical Approach,* (E.L.V. Harris and S. Angal, Eds., 1990).

The particles are then isolated (or substantially purified) using methods that preserve the integrity thereof, such as, by density gradient centrifugation, e.g., sucrose gradients, PEG-precipitation, pelleting, and the like (see, e.g., Kirnbauer et al. *J. Virol.* (1993) 67:6929-6936), as well as standard purification techniques including, e.g., ion exchange and gel filtration chromatography.

VLPs produced by cells containing the synthetic expression cassettes of the present invention can be used to elicit an immune response when administered to a subject. One advantage of the present invention is that VLPs can be produced by mammalian cells carrying the synthetic expression cassettes at levels previously not possible. As discussed above, the VLPs can comprise a variety of antigens in addition to the Gag polypeptides (e.g., Env, tat, Gag-protease, Gag-polymerase, Gag-HCV-core). Purified VLPs, produced using the synthetic expression cassettes of the present invention, can be administered to a vertebrate subject, usually in the form of vaccine compositions. Combination vaccines may also be used, where such vaccines contain, for example, other subunit proteins derived from HIV or other organisms (e.g., env) or gene delivery vaccines encoding such antigens. Administration can take place using the VLPs formulated alone or formulated with other antigens. Further, the VLPs can be administered prior to, concurrent with, or subsequent to, delivery of the synthetic expression cassettes for DNA immunization (see below) and/or delivery of other vaccines. Also, the site of VLP administration may be the same or different as other vaccine compositions that are being administered. Gene delivery can be accomplished by a number of methods including, but are not limited to, immunization with DNA, alphavirus vectors, pox virus vectors, and vaccinia virus vectors.

VLP immune-stimulating (or vaccine) compositions can include various excipients, adjuvants, carriers, auxiliary substances, modulating agents, and the like. The immune stimulating compositions will include an amount of the VLP/antigen sufficient to mount an immunological response. An appropriate effective amount can be determined by one of skill in the art. Such an amount will fall in a relatively broad range that can be determined through routine trials and will generally be an amount on the order of about 0.1 µg to about 1000 µg, more preferably about 1 µg to about 300 µg, of VLP/antigen.

A carrier is optionally present which is a molecule that does not itself induce the production of antibodies harmful to the individual receiving the composition. Suitable carriers are typically large, slowly metabolized macromolecules such as proteins, polysaccharides, polylactic acids, polyglycollic acids, polymeric amino acids, amino acid copolymers, lipid aggregates (such as oil droplets or liposomes), and inactive virus particles. Examples of particulate carriers include those derived from polymethyl methacrylate polymers, as well as microparticles derived from poly(lactides) and poly(lactide-co-glycolides), known as PLG. See, e.g., Jeffery et al., *Pharm. Res.* (1993) 10:362-368; McGee JP, et al., *J Microencapsul.* 14(2):197-210, 1997; O'Hagan DT, et al., *Vaccine* 11(2):149-54, 1993. Such carriers are well known to those of ordinary skill in the art. Additionally, these carriers may function as immunostimulating agents ("adjuvants"). Furthermore, the antigen may be conjugated to a bacterial toxoid, such as toxoid from diphtheria, tetanus, cholera, etc., as well as toxins derived from *E. coli.*

Such adjuvants include, but are not limited to: (1) aluminum salts (alum), such as aluminum hydroxide, aluminum phosphate, aluminum sulfate, etc.; (2) oil-in-water emulsion formulations (with or without other specific immunostimulating agents such as muramyl peptides (see below) or bacterial cell wall components), such as for example (a) MF59 (International Publication No. WO 90/14837), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particle generated therefrom such as ISCOMs (immunostimulating complexes); (4) Complete Freunds Adjuvant (CFA) and Incomplete Freunds Adjuvant (IFA); (5) cytokines, such as interleukins (IL-1, IL-2, etc.), macrophage colony stimulating factor (M-CSF), tumor necrosis factor (TNF), beta chemokines (MIP, 1-alpha, 1-beta Rantes, etc.); (6) detoxified mutants of a bacterial ADP-ribosylating toxin such as a cholera toxin (CT), a pertussis toxin (PT), or an E. coli heat-labile toxin (LT), particularly LT-K63 (where lysine is substituted for the wild-type amino acid at position 63) LT-R72 (where arginine is substituted for the wild-type amino acid at position 72), CT-S109 (where serine is substituted for the wild-type amino acid at position 109), and PT-K9/G129 (where lysine is substituted for the wild-type amino acid at position 9 and glycine substituted at position 129) (see, e.g., International Publication Nos. W093/13202 and W092/19265); and (7) other substances that act as immunostimulating agents to enhance the effectiveness of the composition.

Muramyl peptides include, but are not limited to, N-acetyl-muramyl-L-threonyl-D-isoglutamine (thr-MDP), N-acteyl-normuramyl-L-alanyl-D-isogluatme (nor-MDP), N-acetylmuramyl-L-alanyl-D-isogluatminyl-L-alanine-2-(1'-2'-dipalmitoyl-*sn*-glycero-3-huydroxyphosphoryloxy)-ethylamine (MTP-PE), etc.

Dosage treatment with the VLP composition may be a single dose schedule or a multiple dose schedule. A multiple dose schedule is one in which a primary course of vaccination may be with 1-10 separate doses, followed by other doses given at subsequent time intervals, chosen to maintain and/or reinforce the immune response, for example at 1-4 months for a second dose, and if needed, a subsequent dose(s) after several months. The dosage regimen will also, at least in part, be determined by the potency of the modality, the vaccine delivery employed, the need of the subject and be dependent on the judgment of the practitioner.

If prevention of disease is desired (e.g., reduction of symptoms, recurrences or of disease progression), the antigen carrying VLPs are generally administered prior to primary infection with the pathogen of interest. If treatment is desired, e.g., the reduction of symptoms or recurrences, the VLP compositions are generally administered subsequent to primary infection.

### 2.2.2 USING THE SYNTHETIC EXPRESSION CASSETTES OF THE PRESENT INVENTION TO CREATE PACKAGING CELL LINES

A number of viral based systems have been developed for use as gene transfer vectors for mammalian host cells. For example, retroviruses (in particular, lentiviral vectors) provide a convenient platform for gene delivery systems. A coding sequence of interest (for example, a sequence useful for gene therapy applications) can be inserted into a gene delivery vector and packaged in retroviral particles using techniques known in the art. Recombinant virus can then be isolated and delivered to cells of the subject either *in vivo* or *ex vivo.* A number of retroviral systems have been described, including, for example, the following: (U.S. Patent No. 5,219,740; Miller et al. (1989) *Biotechniques* 7:980; Miller, A.D. (1990) *Human Gene Therapy* 1:5; Scarpa et al. (1991) *Virology* 180:849; Burns et al. (1993) *Proc. Natl. Acad. Sci. USA* 90:8033; Boris-Lawrie et al. (1993) *Cur. Opin. Genet. Develop.* 3:102; GB 2200651; EP 0415731; EP 0345242; WO 89/02468; WO 89/05349; WO 89/09271; WO 90/02806; WO 90/07936; WO 90/07936; WO 94/03622; WO 93/25698; WO 93/25234; WO 93/11230; WO 93/10218; WO 91/02805; in U.S. 5,219,740; U.S. 4,405,712; U.S. 4,861,719; U.S. 4,980,289 and U.S. 4,777,127; in U.S. Serial No. 07/800,921; and in Vile (1993) *Cancer Res* 53:3860-3864; Vile (1993) *Cancer Res* 53:962-967; Ram (1993) *Cancer Res* 53:83-88; Takamiya (1992) *J Neurosci* Res 33:493-503; Baba (1993) *J Neurosurg* 79:729-735; Mann (1983) *Cell* 33:153; Cane (1984) *Proc Natl Acad Sci USA* 81;6349; and Miller (1990) *Human Gene Therapy* 1.

Sequences useful for gene therapy applications include, but are not limited to, the following. Factor VIII cDNA, including derivatives and deletions thereof (International Publication Nos. WO 96/21035, WO 97/03193, WO 97/03194, WO 97/03195, and WO 97/03191). Factor IX cDNA (Kurachi et al. (1982) *Proc. Natl. Acad. Sci. USA* 79:6461-6464). Factor V cDNA can be obtained from pMT2-V (Jenny (1987) *Proc. Natl. Acad. Sci. USA* 84:4846, A.T.C.C. Deposit No. 40515). A full-length factor V cDNA, or a B domain deletion or B domain substitution thereof, can be used. B domain deletions of factor V, include those reported by Marquette (1995) *Blood* 86:3026 and Kane (1990) *Biochemistry* 29:6762. Antithrombin III cDNA (Prochownik (1983) *J. Biol. Chem.* 258:8389, A.T.C.C. Deposit No. 57224/57225). Protein C encoding cDNA (Foster (1984) *Proc. Natl. Acad. Sci. USA* 81:4766; Beckmann (1985) *Nucleic Acids Res.* 13:5233). Prothrombin cDNA can be obtained by restriction enzyme digestion of a published vector (Degen (1983) *Biochemistry* 22:2087). The endothelial cell surface protein, thrombomodulin, is a necessary cofactor for the normal activation of protein C by thrombin. A soluble recombinant form has been described (Parkinson (1990) *J. Biol. Chem.* 265:12602; Jackman (1987) *Proc. Natl. Acad. Sci. USA* 84:6425; Shirai (1988) *J. Biochem.* 103:281; Wen (1987) *Biochemistry* 26:4350; Suzuki (1987) *EMBO* J. 6:1891, A.T.C.C. Deposit No. 61348, 61349).

Many genetic diseases caused by inheritance of defective genes result in the failure to produce normal gene products, for example, thalassemia, phenylketonuria, Lesch-Nyhan syndrome, severe combined immunodeficiency (SCID), hemophilia A and B, cystic fibrosis, Duchenne's Muscular Dystrophy, inherited emphysema and familial hypercholesterolemia (Mulligan et al. (1993) *Science* 260:926; Anderson et al. (1992) *Science* 256:808; Friedman et al. (1989) *Science* 244:1275). Although genetic diseases may result in the absence of a gene product, endocrine disorders, such as diabetes and hypopituitarism, are caused by the inability of the gene to produce adequate levels of the appropriate hormone insulin and human growth hormone respectively.

In one aspect, gene therapy employing the constructs and methods of the present invention involves the introduction of normal recombinant genes into T cells so that new or missing proteins are produced by the T cells after introduction or reintroduction thereof into a patient. A number of genetic diseases have been selected for treatment with gene therapy, including adenine deaminase deficiency, cystic fibrosis, α₁-antitrypsin deficiency, Gaucher's syndrome, as well as non-genetic diseases.

In particular, Gaucher's syndrome is a genetic disorder characterized by a deficiency of the enzyme glucocerebrosidase. This enzyme deficiency leads to the accumulation of glucocerebroside in the lysosomes of all cells in the body. For a review see Science 256:794 (1992) and Scriver et al., *The Metabolic Basis of Inherited Disease,* 6th ed., vol. 2, page 1677). Thus, gene transfer vectors that express glucocerebrosidase can be constructed for use in the treatment of this disorder. Likewise, gene transfer vectors encoding lactase can be used in the treatment of hereditary lactose intolerance, those expressing AD can be used for treatment of ADA deficiency, and gene transfer vectors encoding α₁-antitrypsin can be used to treat α₁-antitrypsin deficiency. See Ledley, F.D. (1987) *J. Pediatrics* 110:157-174, Verma, I. (Nov. 1987) *Scientific American* pp. 68-84, and International Publication No. WO 95/27512 entitled "Gene Therapy Treatment for a Variety of Diseases and Disorders," for a description of gene therapy treatment of genetic diseases.

In still further embodiments of the invention, nucleotide sequences which can be incorporated into a gene transfer vector include, but are not limited to, proteins associated with enzyme-deficiency disorders, such as the cystic fibrosis transmembrane regulator (see, for example, U.S. Patent No. 5,240,846 and Larrick et al. (1991) *Gene Therapy Applications of Molecular Biology,* Elsevier, New York and adenosine deaminase (ADA) (see U.S. Patent No. 5,399,346); growth factors, or an agonist or antagonist of a growth factor (Bandara et al. (1992) *DNA and Cell Biology,* 11:227); one or more tumor suppressor genes such as p53, Rb, or C-CAMI (Kleinerman et al. (1995) *Cancer Research* 55:2831); a molecule that modulates the immune system of an organism, such as a HLA molecule (Nabel et al. (1993) *Proc. Natl. Acad. Sci. USA* 90:11307); a ribozyme (Larsson et al. (1996) *Virology* 219:161); a peptide nucleic acid (Hirshman et al. (1996) *J. Invest. Med.* 44:347); an antisense molecule (Bordier et al. (1995) *Proc. Natl. Acad. Sci. USA* 92:9383) which can be used to down-regulate the expression or synthesis of aberrant or foreign proteins, such as HIV proteins or a wide variety of oncogenes such as p53 (Hesketh, *The Oncogene Facts Book,* Academic Press, New York, (1995); a biopharmaceutical agent or antisense molecule used to treat HIV-infection, such as an inhibitor of p24 (Nakashima et al. (1994) *Nucleic Acids Res.* 22:5004); or reverse-transcriptase (see, Bordier, *supra).*

Other proteins of therapeutic interest can be expressed in vivo by gene transfer vectors using the methods of the invention. For instance sustained *in vivo* expression of tissue factor inhibitory protein (TFPI) is useful for treatment of conditions including sepsis and DIC and in preventing reperfusion injury. (See International Publications Nos. WO 93/24143, WO 93/25230 and WO 96/06637). Nucleic acid sequences encoding various forms of TFPI can be obtained, for example, as described in US Patent Nos. 4,966,852; 5,106,833; and 5,466,783, and incorporated into the gene transfer vectors described herein.

Erythropoietin (EPO) and leptin can also be expressed *in vivo* from genetically modified T cells according to the methods of the invention. For instance EPO is useful in gene therapy treatment of a variety of disorders including anemia (see International Publication No. WO 95/13376 entitled "Gene Therapy for Treatment of Anemia"). Sustained delivery of leptin by the methods of the invention is useful in treatment of obesity. See International Publication No. WO 96/05309 for a description of the leptin gene and the use thereof in the treatment of obesity.

A variety of other disorders can also be treated by the methods of the invention. For example, sustained in vivo systemic production of apolipoprotein E or apolipoprotein A from genetically modified T cells can be used for treatment of hyperlipidemia (see Breslow et al. (1994) *Biotechnology* 12:365). Sustained production of angiotensin receptor inhibitor (Goodfriend et al. (1996) *N. Engl. J. Med.* 334:1469) can be provided by the methods described herein. As yet an additional example, the long term in vivo systemic production of angiostatin is useful in the treatment of a variety of tumors. (See O'Reilly et al. (1996) *Nature Med.* 2:689).

In other embodiments, gene transfer vectors can be constructed to encode a cytokine or other immunomodulatory molecule. For example, nucleic acid sequences encoding native IL-2 and gamma-interferon can be obtained as described in US Patent Nos. 4,738,927 and 5,326,859, respectively, while useful muteins of these proteins can be obtained as described in U.S. Patent No. 4,853,332. Nucleic acid sequences encoding the short and long forms of mCSF can be obtained as described in US Patent Nos. 4,847,201 and 4,879,227, respectively. In particular aspects of the invention, retroviral vectors expressing cytokine or immunomodulatory genes can be produced as described herein (for example, employing the packaging cell lines of the present invention) and in International Application No. PCT US 94/02951, entitled "Compositions and Methods for Cancer Immunotherapy."

Examples of suitable immunomodulatory molecules for use herein include the following: IL-1 and IL-2 (Karupiah et al. (1990) *J. Immunology* 144:290-298, Weber et al. (1987) *J. Exp. Med.* 166:1716-1733, Gansbacher et al. (1990) *J. Exp. Med.* 172:1217-1224, and U.S. Patent No. 4,738,927); IL-3 and IL-4 (Tepper et al. (1989) *Cell* 57:503-512, Golumbek et al. (1991) *Science* 254:713-716, and U.S. Patent No. 5,017,691); IL-5 and IL-6 (Brakenhof et al. (1987) *J. Immunol.* 139:4116-4121, and International Publication No. WO 90/06370); IL-7 (U.S. Patent No. 4,965,195); IL-8, IL-9, IL-10, IL-11, IL-12, and IL-13 *(Cytokine* Bulletin, Summer 1994); IL-14 and IL-15; alpha interferon (Finter et al. (1991) *Drugs* 42:749-765, U.S. Patent Nos. 4,892,743 and 4,966,843, International Publication No. WO 85/02862, Nagata et al. (1980) Nature 284:316-320, Familletti et al. (1981) *Methods in Enz.* 78:387-394, Twu et al. (1989) *Proc.* Natl. *Acad. Sci. USA* 86:2046-2050, and Faktor et al. (1990) Oncogene 5:867-872); beta-interferon (Seif et al. (1991) *J. Virol.* 65:664-671); gamma-interferons (Radford et al. (1991) *The American Society of Hepatology* 20082015, Watanabe et al. (1989) *Proc. Natl. Acad. Sci. USA* 86:9456-9460, Gansbacher et al. (1990) *Cancer Research* 50:7820-7825, Maio et al. (1989) *Can. Immunol. Immunother.* 30:34-42, and U.S. Patent Nos. 4,762,791 and 4,727,138); G-CSF (U.S. Patent Nos. 4,999,291 and 4,810,643); GM-CSF (International Publication No. WO 85/04188); tumor necrosis factors (TNFs) (Jayaraman et al. (1990) *J. Immunology* 144:942-951); CD3 (Krissanen et al. (1987) *Immunogenetics* 26:258-266); ICAM-1 (Altman et al. (1989) *Nature* 338:512-514, Simmons et al. (1988) Nature 331:624-627); ICAM-2, LFA-1, LFA-3 (Wallner et al. (1987) *J. Exp. Med.* 166:923-932); MHC class I molecules, MHC class II molecules, B7.1-.3, β₂-microglobulin (Parnes et al. (1981) *Proc. Natl. Acad. Sci.* USA 78:2253-2257); chaperones such as calnexin; and MHC-linked transporter proteins or analogs thereof (Powis et al. (1991) *Nature* 354:528-531). Immunomodulatory factors may also be agonists, antagonists, or ligands for these molecules. For example, soluble forms of receptors can often behave as antagonists for these types of factors, as can mutated forms of the factors themselves.

Nucleic acid molecules that encode the above-described substances, as well as other nucleic acid molecules that are advantageous for use within the present invention, may be readily obtained from a variety of sources, including, for example, depositories such as the American Type Culture Collection, or from commercial sources such as British Bio-Technology Limited (Cowley, Oxford England). Representative examples include BBG 12 (containing the GM-CSF gene coding for the mature protein of 127 amino acids), BBG 6 (which contains sequences encoding gamma interferon), A.T.C.C. Deposit No. 39656 (which contains sequences encoding TNF), A.T.C.C. Deposit No. 20663 (which contains sequences encoding alpha-interferon), A.T.C.C. Deposit Nos. 31902, 31902 and 39517 (which contain sequences encoding beta-interferon), A.T.C.C. Deposit No. 67024 (which contains a sequence which encodes Interleukin-1b), A.T.C.C. Deposit Nos. 39405, 39452, 39516, 39626 and 39673 (which contain sequences encoding Interleukin-2), A.T.C.C. Deposit Nos. 59399, 59398, and 67326 (which contain sequences encoding Interleukin-3), A.T.C.C. Deposit No. 57592 (which contains sequences encoding Interleukin-4), A.T.C.C. Deposit Nos. 59394 and 59395 (which contain sequences encoding Interleukin-5), and A.T.C.C. Deposit No. 67153 (which contains sequences encoding Interleukin-6).

Plasmids containing cytokine genes or immunomodulatory genes (International Publication Nos. WO 94/02951 and WO 96/21015)can be digested with appropriate restriction enzymes, and DNA fragments containing the particular gene of interest can be inserted into a gene transfer vector using standard molecular biology techniques. *(See, e.g.*, Sambrook et al., *supra.,* or Ausubel et al. (eds) *Current Protocols in Molecular Biology,* Greene Publishing and Wiley-Interscience).

Exemplary hormones, growth factors and other proteins which are useful for long term expression are described, for example, in European Publication No. 0437478B1, entitled "Cyclodextrin-Peptide Complexes." Nucleic acid sequences encoding a variety of hormones can be used, including those encoding human growth hormone, insulin, calcitonin, prolactin, follicle stimulating hormone (FSH), luteinizing hormone (LH), human chorionic gonadotropin (HCG), and thyroid stimulating hormone (TSH). A variety of different forms of IGF-1 and IGF-2 growth factor polypeptides are also well known the art and can be incorporated into gene transfer vectors for long term expression *in vivo. See, e.g.*, European Patent No. 0123228B1, published for grant September 19, 1993, entitled "Hybrid DNA Synthesis of Mature Insulin-like Growth Factors." As an additional example, the long term *in vivo* expression of different forms of fibroblast growth factor can also be effected employing the compositions and methods of invention. See, e.g., U.S. Patent Nos. 5,464,774, 5,155,214, and 4,994,559 for a description of different fibroblast growth factors.

Polynucleotide sequences coding for the above-described molecules can be obtained using recombinant methods, such as by screening cDNA and genomic libraries from cells expressing the gene, or by deriving the gene from a vector known to include the same. For example, plasmids which contain sequences that encode altered cellular products may be obtained from a depository such as the A.T.C.C., or from commercial sources. Plasmids containing the nucleotide sequences of interest can be digested with appropriate restriction enzymes, and DNA fragments containing the nucleotide sequences can be inserted into a gene transfer vector using standard molecular biology techniques.

Alternatively, cDNA sequences for use with the present invention may be obtained from cells which express or contain the sequences, using standard techniques, such as phenol extraction and PCR of cDNA or genomic DNA. See, e.g., Sambrook et al., *supra*, for a description of techniques used to obtain and isolate DNA. Briefly, mRNA from a cell which expresses the gene of interest can be reverse transcribed with reverse transcriptase using oligo-dT or random primers. The single stranded cDNA may then be amplified by PCR (see U.S. Patent Nos. 4,683,202, 4,683,195 and 4,800,159, see also *PCR Technology: Principles and Applications for DNA Amplification*, Erlich (ed.), Stockton Press, 1989)) using oligonucleotide primers complementary to sequences on either side of desired sequences.

The nucleotide sequence of interest can also be produced synthetically, rather than cloned, using a DNA synthesizer (*e.g.*, an Applied Biosystems Model 392 DNA Synthesizer, available from ABI, Foster City, California). The nucleotide sequence can be designed with the appropriate codons for the expression product desired. The complete sequence is assembled from overlapping oligonucleotides prepared by standard methods and assembled into a complete coding sequence. See, e.g., Edge (1981) *Nature* 292:756; Nambair et al. (1984) *Science* 223:1299; Jay et al. (1984) *J. Biol. Chem.* 259:6311.

The synthetic expression cassettes of the present invention can be employed in the construction of packaging cell lines for use with retroviral vectors.

One type of retrovirus, the murine leukemia virus, or "MLV", has been widely utilized for gene therapy applications (see generally Mann et al. *(Cell 33:153,* 1993), Cane and Mulligan *(Proc, Nat'l. Acad. Sci. USA* 81:6349, 1984), and Miller et al., *Human Gene 21erapy* 1:5-14,1990.

Lentiviral vectors typically, comprise a 5' lentiviral LTR, a tRNA binding site, a packaging signal, a promoter operably linked to one or more genes of interest, an origin of second strand DNA synthesis and a 3' lentiviral LTR, wherein the lentiviral vector contains a nuclear transport element. The nuclear transport element may be located either upstream (5') or downstream (3') of a coding sequence of interest. Within certain embodiments, the nuclear transport element is not RRE. Within one embodiment the packaging signal is an extended packaging signal. Within other embodiments the promoter is a tissue specific promoter, or, alternatively, a promoter such as CMV. Within other embodiments, the lentiviral vector further comprises an internal ribosome entry site.

A wide variety of lentiviruses may be utilized within the context of the present invention, including for example, lentiviruses selected from the group consisting of HIV, HIV-1, HIV-2, FIV and SIV.

In one embodiment of the present invention synthetic Env and/or Gag-polymerase expression cassettes are provided comprising a promoter and a sequence encoding synthetic Gag-polymerase (SEQ ID NO:6) and at least one of vpr, vpu, nef or vif, wherein the promoter is operably linked to Gag-polymerase and vpr, vpu, nef or vif.

Within yet another aspect of the invention, host cells (e.g., packaging cell lines) are provided which contain any of the expression cassettes described herein. For example, within one aspect packaging cell line are provided comprising an expression cassette that comprises a sequence encoding synthetic Env and/or Gag-polymerase, and a nuclear transport element, wherein the promoter is operably linked to the sequence encoding Env and/or Gag-polymerase. Packaging cell lines may further comprise a promoter and a sequence encoding tat, rev, or an envelope, wherein the promoter is operably linked to the sequence encoding tat, rev, or, the envelope. The packaging cell line may further comprise a sequence encoding any one or more of nef, vif, vpu or vpr.

In one embodiment, the expression cassette (carrying, for example, the synthetic Env, synthetic tat and/or synthetic Gag-polymerase) is stably integrated. The packaging cell line, upon introduction of a lentiviral vector, typically produces viral particles. The promoter regulating expression of the synthetic expression cassette may be inducible. Typically, the packaging cell line, upon introduction of a lentiviral vector, produces viral particles that are essentially free of replication competent virus.

Packaging cell lines are provided comprising an expression cassette which directs the expression of a synthetic *Env* (or *Gag-polymerase)* gene, an expression cassette which directs the expression of a Gag (or Env) gene optimized for expression (e.g., Andre, S., et al., *Journal of Virology* 72(2):1497-1503, 1998; Haas, J., et al., *Current Biology* 6(3):315-324, 1996). A lentiviral vector is introduced into the packaging cell line to produce a vector particle producing cell line.

As noted above, lentiviral vectors can be designed to carry or express a selected gene(s) or sequences of interest. Lentiviral vectors may be readily constructed from a wide variety of lentiviruses (*see* RNA Tumor Viruses, Second Edition, Cold Spring Harbor Laboratory, 1985). Representative examples of lentiviruses included HIV, HIV-1, HIV-2, FIV and SIV. Such lentiviruses may either be obtained from patient isolates, or, more preferably, from depositories or collections such as the American Type Culture Collection, or isolated from known sources using available techniques.

Portions of the lentiviral gene delivery vectors (or vehicles) may be derived from different viruses. For example, in a given recombinant lentiviral vector, LTRs may be derived from an HIV, a packaging signal from SIV, and an origin of second strand synthesis from HrV-2. Lentiviral vector constructs may comprise a 5' lentiviral LTR, a tRNA binding site, a packaging signal, one or more heterologous sequences, an origin of second strand DNA synthesis and a 3' LTR, wherein said lentiviral vector contains a nuclear transport element that is not RRE.

Briefly, Long Terminal Repeats ("LTRs") are subdivided into three elements, designated U5, R and U3. These elements contain a variety of signals which are responsible for the biological activity of a retrovirus, including for example, promoter and enhancer elements which are located within U3. LTRs may be readily identified in the provirus (integrated DNA form) due to their precise duplication at either end of the genome. As utilized herein, a 5' LTR should be understood to include a 5' promoter element and sufficient LTR sequence to allow reverse transcription and integration of the DNA form of the vector. The 3' LTR should be understood to include a polyadenylation signal, and sufficient LTR sequence to allow reverse transcription and integration of the DNA form of the vector.

The tRNA binding site and origin of second strand DNA synthesis are also important for a retrovirus to be biologically active, and may be readily identified by one of skill in the art. For example, retroviral tRNA binds to a tRNA binding site by Watson-Crick base pairing, and is carried with the retrovirus genome into a viral particle. The tRNA is then utilized as a primer for DNA synthesis by reverse transcriptase. The tRNA binding site may be readily identified based upon its location just downstream from the 5'LTR. Similarly, the origin of second strand DNA synthesis is, as its name implies, important for the second strand DNA synthesis of a retrovirus. This region, which is also referred to as the poly-purine tract, is located just upstream of the 3'LTR.

In addition to a 5' and 3' LTR, tRNA binding site, and origin of second strand DNA synthesis, recombinant retroviral vector constructs may also comprise a packaging signal, as well as one or more genes or coding sequences of interest. In addition, the lentiviral vectors have a nuclear transport element which, in preferred embodiments is not RRE. Representative examples of suitable nuclear transport elements include the element in Rous sarcoma virus (Ogert, et al., J *ViroL* 70, 3834-3843, 1996), the element in Rous sarcoma virus (Liu & *Mertz, Genes & Dev., 9*, 1766-1789, 1995) and the element in the genome of simian retrovirus type I (Zolotukhin, et al., *J Virol. 68,* 7944-7952, 1994). Other potential elements include the elements in the histone gene (Kedes, Annu. *Rev. Biochem. 48,* 837-870, 1970), the α-interferon gene (Nagata et al., *Nature 287,* 401-408, 1980), the β-adrenergic receptor gene (Koilka, et al., *Nature 329,* 75-79, 1987), and the c-Jun gene (Hattorie, et al., *Proc. Natl. Acad. Sci. USA 85,* 9148-9152, 1988).

Recombinant lentiviral vector constructs typically lack both *Gag-polymerase* and *env* coding sequences. Recombinant lentiviral vector typically contain less than 20, preferably 15, more preferably 10, and most preferably 8 consecutive nucleotides found in *Gag-polymerase* or *env* genes. One advantage of the present invention is that the synthetic *Gag-polymerase* expression cassettes, which can be used to construct packaging cell lines for the recombinant retroviral vector constructs, have little homology to wild-type Gag-polymerase sequences and thus considerably reduce or eliminate the possibility of homologous recombination between the synthetic and wild-type sequences.

Lentiviral vectors may also include tissue-specific promoters to drive expression of one or more genes or sequences of interest. For example, lentiviral vector particles of the invention can contain a liver specific promoter to maximize the potential for liver specific expression of the exogenous DNA sequence contained in the vectors. Preferred liver specific promoters include the hepatitis B X-gene promoter and the hepatitis B core protein promoter. These liver specific promoters are preferably employed with their respective enhancers. The enhancer element can be linked at either the 5' or the 3' end of the nucleic acid encoding the sequences of interest. The hepatitis B X gene promoter and its enhancer can be obtained from the viral genome as a 332 base pair *EcoRV-NcoI* DNA fragment employing the methods described in Twu, et al., *J Virol. 61:3448-3453, 1987.* The hepatitis B core protein promoter can be obtained from the viral genome as a 584 base pair *BamHI-BglII* DNA fragment employing the methods described in Gerlach,et *al., Virol* 189:59-66, 1992. It may be necessary to remove the negative regulatory sequence in the *BamHI-BglII* fragment prior to inserting it. Other liver specific promoters include the AFP (alpha fetal protein) gene promoter and the albumin gene promoter, as disclosed in EP Patent Publication 0 415 731, the -1 antitrypsin gene promoter, as disclosed in Rettenger, et al., Proc. *Natl. Acad. Sci.* 91:1460-1464, 1994, the fibrinogen gene promoter, the APO-A1 (Apolipoprotein A1) gene promoter, and the promoter genes for liver transference enzymes such as, for example, SGOT, SGPT and glutamyle transferase. See also PCT Patent Publications WO 90/07936 and WO 91/02805 for a description of the use of liver specific promoters in lentiviral vector particles.

Lentiviral vector constructs may be generated such that more than one gene of interest is expressed. This may be accomplished through the use of di- or oligo-cistronic cassettes (e.g., where the coding regions are separated by 80 nucleotides or less, *see generally* Levin et al., *Gene* 108:167-174, 1991), or through the use of Internal Ribosome Entry Sites ("IRES").

Packaging cell lines suitable for use with the above described recombinant retroviral vector constructs may be readily prepared given the disclosure provided herein. Briefly, the parent cell line from which the packaging cell line is derived can be selected from a variety of mammalian cell lines, including for example, 293, RD, COS-7, CHO, BHK, VERO, HT1080, and myeloma cells.

After selection of a suitable host cell for the generation of a packaging cell line, one or more expression cassettes are introduced into the cell line in order to complement or supply in trans components of the vector which have been deleted.

Representative examples of suitable expression cassettes have been described herein and include synthetic Env, tat, Gag, synthetic Gag-protease, synthetic Gag-reverse transcriptase and synthetic Gag-polymerase expression cassettes, which comprise a promoter and a sequence encoding, e.g., Env, tat, or Gag-polymerase and at least one of vpr, vpu, nef or vif, wherein the promoter is operably linked to Env, tat or Gag-polymerase and vpr, vpu, nef or vif. As described above, optimized Env, Gag and/or tat coding sequences may also be utilized in various combinations in the generation of packaging cell lines.

Utilizing the above-described expression cassettes, a wide variety of packaging cell lines can be generated. For example, within one aspect packaging cell line are provided comprising an expression cassette that comprises a sequence encoding synthetic HIV (e.g., Gag, Env, tat, Gag-polymerase, Gag-reverse transcriptase or Gag-protease) polypeptide, and a nuclear transport element, wherein the promoter is operably linked to the sequence encoding the HIV polypeptide. Within other aspects, packaging cell lines are provided comprising a promoter and a sequence encoding Gag, tat, rev, or an envelope (e.g., HIV env), wherein the promoter is operably linked to the sequence encoding Gag, tat, rev, or, the envelope. Within further embodiments, the packaging cell line may comprise a sequence encoding any one or more of nef, vif, vpu or vpr. For example, the packaging cell line may contain only nef, vif, vpu, or vpr alone, nef and vif, nef and vpu, nef and vpr, vif and vpu, vif and vpr, vpu and vpr, nef vif and vpu, nef vif and vpr, nef vpu and vpr, vvir vpu and vpr, or, all four of nef vif vpu and vpr.

In one embodiment, the expression cassette is stably integrated. Within another embodiment, the packaging cell line, upon introduction of a lentiviral vector, produces particles. Within further embodiments the promoter is inducible. Within certain preferred embodiments of the invention, the packaging cell line, upon introduction of a lentiviral vector, produces particles that are free of replication competent virus.

The synthetic cassettes containing optimized coding sequences are transfected into a selected cell line. Transfected cells are selected that (i) carry, typically, integrated, stable copies of the Gag, Pol, and Env coding sequences, and (ii) are expressing acceptable levels of these polypeptides (expression can be evaluated by methods known in the prior art, e.g., see Examples 1-4). The ability of the cell line to produce VLPs may also be verified (Examples 6, 7 and 15).

A sequence of interest is constructed into a suitable viral vector as discussed above. This defective virus is then transfected into the packaging cell line. The packaging cell line provides the viral functions necessary for producing virus-like particles into which the defective viral genome, containing the sequence of interest, are packaged. These VLPs are then isolated and can be used, for example, in gene delivery or gene therapy.

Further, such packaging cell lines can also be used to produce VLPs alone, which can, for example, be used as adjuvants for administration with other antigens or in vaccine compositions. Also, co-expression of a selected sequence of interest encoding a polypeptide (for example, an antigen) in the packaging cell line can also result in the entrapment and/or association of the selected polypeptide in/with the VLPs.

### 2.3 DNA IMMUNIZATION AND GENE DELIVERY

A variety of polypeptide antigens can be used in the practice of the present invention. Polypeptide antigens can be included in DNA immunization constructs containing, for example, any of the synthetic expression cassettes described herein fused in-frame to a coding sequence for the polypeptide antigen, where expression of the construct results in VLPs presenting the antigen of interest. Antigens can be derived from a wide variety of viruses, bacteria, fungi, plants, protozoans and other parasites. For example, the present invention will find use for stimulating an immune response against a wide variety of proteins from the herpesvirus family, including proteins derived from herpes simplex virus (HSV) types 1 and 2, such as HSV-1 and HSV-2 gB, gD, gH, VP16 and VP22; antigens derived from varicella zoster virus (VZV), Epstein-Barr virus (EBV) and cytomegalovirus (CMV) including CMV gB and gH; and antigens derived from other human herpesviruses such as HHV6 and HHV7. (See, e.g. Chee et al., *Cytomegaloviruses* (J.K. McDougall, ed., Springer-Verlag 1990) pp. 125-169, for a review of the protein coding content of cytomegalovirus; McGeoch et al., *J. Gen. Virol.* (1988) 69:1531-1574, for a discussion of the various HSV-1 encoded proteins; U.S. Patent No. 5,171,568 for a discussion of HSV-1 and HSV-2 gB and gD proteins and the genes encoding therefore; Baer et al., Nature (1984) 310:207-211, for the identification of protein coding sequences in an EBV genome; and Davison and Scott, *J. Gen. Virol.* (1986) 67:1759-1816, for a review of VZV.)

Additionally, immune responses to antigens from the hepatitis family of viruses, including hepatitis A virus (HAV), hepatitis B virus (HBV), hepatitis C virus (HCV), the delta hepatitis virus (HDV), hepatitis E virus (HEV), and hepatitis G virus, can also be stimulated using the constructs of the present invention. By way of example, the HCV genome encodes several viral proteins, including E1 (also known as E) and E2 (also known as E2/NSI), which will find use with the present invention (see, Houghton et al. *Hepatology* (1991) 14:381-388, for a discussion of HCV proteins, including E1 and E2). The 5-antigen from HDV can also be used (see, e.g., U.S. Patent No. 5,389,528, for a description of the δ-antigen).

Similarly, influenza virus is another example of a virus for which the present invention will be particularly useful. Specifically, the envelope glycoproteins HA and NA of influenza A are of particular interest for generating an immune response. Numerous HA subtypes of influenza A have been identified (Kawaoka et al., *Virology* (1990) 179:759-767; Webster et al. "Antigenic variation among type A influenza viruses," p. 127-168. In: P. Palese and D.W. Kingsbury (ed.), *Genetics of influenza viruses.* Springer-Verlag, New York).

Other antigens of particular interest to be used in the practice of the present invention include antigens and polypeptides derived therefrom from human papillomavirus (HPV), such as one or more of the various early proteins including E6 and E7; tick-borne encephalitis viruses; and HIV-1 (also known as HTLV-III, LAV, ARV, etc.), including, but not limited to, antigens such as gp120, gp41, gp160, Gag and pol from a variety of isolates including, but not limited to, HIV _{IIIb}, HIV_{SF2}, HIV-1_{SF162}, HIV-1_{SF170}, HIV_{LAV}, HIV_{LAI}, HIV_{MN}, HIV-1_{CM235}, HIV-1_{USA}, other HIV-1 strains from diverse subtypes(e.g., subtypes, A through G, and O), HIV-2 strains and diverse subtypes (e.g., HIV-2_{UC1} and HIV-2_{UC2}). See, e.g., Myers, et al., Los Alamos Database, Los Alamos National Laboratory, Los Alamos, New Mexico; Myers, et al., Human *Retroviruses and Aids, 1990,* Los Alamos, New Mexico: Los Alamos National Laboratory.

Proteins derived from other viruses will also find use in the claimed methods, such as without limitation, proteins from members of the families Picornaviridae (e.g., polioviruses, etc.); Caliciviridae; Togaviridae (e.g., rubella virus, dengue virus, etc.); Flaviviridae; Coronaviridae; Reoviridae; Birnaviridae; Rhabodoviridae (e.g., rabies virus, etc.); Filoviridae; Paramyxoviridae (e.g., mumps virus, measles virus, respiratory syncytial virus, etc.); Orthomyxoviridae (e.g., influenza virus types A, B and C, etc.); Bunyaviridae; Arenaviridae; Retroviradae, e.g., HTLV-I; HTLV-II; HIV-1; HIV-2; simian immunodeficiency virus (SIV) among others. See, e.g. Virology, 3rd Edition (W.K. Joklik ed. 1988); Fundamental Virology, 2nd Edition (B.N. Fields and D.M. Knipe, eds. 1991; *Virology,* 3rd Edition (Fields, BN, DM Knipe, PM Howley, Editors, 1996, Lippincott-Raven, Philadelphia, PA) for a description of these and other viruses.

Particularly preferred bacterial antigens are derived from organisms that cause diphtheria, tetanus, pertussis, meningitis, and other pathogenic states, including, without limitation, antigens derived from *Corynebacterium diphtheriae, Clostridium tetani, Bordetella pertusis, Neisseria meningitidis,* including serotypes *Meningococcus* A, B, C, Y and WI35 (MenA, B, C, Y and WI35), *Haemophilus influenza* type B (Hib), and *Helicobacter pylori.* Examples of parasitic antigens include those derived from organisms causing malaria, tuberculosis, and Lyme disease.

Furthermore, the methods described herein provide means for treating a variety of malignant cancers. For example, the system of the present invention can be used to enhance both humoral and cell-mediated immune responses to particular proteins specific to a cancer in question, such as an activated oncogene, a fetal antigen, or an activation marker. Such tumor antigens include any of the various MAGEs (melanoma associated antigen E), including MAGE 1, 2, 3, 4, etc. (Boon, T. *Scientific American* (March 1993):82-89); any of the various tyrosinases; MART 1 (melanoma antigen recognized by T cells), mutant ras; mutant p53; p97 melanoma antigen; CEA (carcinoembryonic antigen), among others.

DNA immunization using synthetic expression cassettes of the present invention has been demonstrated to be efficacious (Examples 8 and 10-12). Animals were immunized with both the synthetic expression cassette and the wild type expression cassette. The results of the immunizations with plasmid-DNAs showed that the synthetic expression cassettes provide a clear improvement of immunogenicity relative to the native expression cassettes. Also, the second boost immunization induced a secondary immune response, for example after two to eight weeks. Further, the results of CTL assays showed increased potency of synthetic expression cassettes for induction of cytotoxic T-lymphocyte (CTL) responses by DNA immunization.

It is readily apparent that the subject invention can be used to mount an immune response to a wide variety of antigens and hence to treat or prevent a large number of diseases.

### 2.3.1 DELIVERY OF THE SYNTHETIC EXPRESSION CASSETTES OF THE PRESENT INVENTION

Polynucleotide sequences coding for the above-described molecules can be obtained using recombinant methods, such as by screening cDNA and genomic libraries from cells expressing the gene, or by deriving the gene from a vector known to include the same. The sequences can be analyzed by conventional sequencing techniques. Furthermore, the desired gene can be isolated directly from cells and tissues containing the same, using standard techniques, such as phenol extraction and PCR of cDNA or genomic DNA. See, e.g., Sambrook et al., *supra,* for a description of techniques used to obtain, isolate and sequence DNA. Once the sequence is known, the gene of interest can also be produced synthetically, rather than cloned. The nucleotide sequence can be designed with the appropriate codons for the particular amino acid sequence desired. In general, one will select preferred codons for the intended host in which the sequence will be expressed. The complete sequence is assembled from overlapping oligonucleotides prepared by standard methods and assembled into a complete coding sequence. See, e.g., Edge, Nature (1981) 292:756; Nambair et al., *Science* (1984) 223:1299; Jay et al., *J. Biol. Chem.* (1984) 259:6311; Stemmer, W.P.C., (1995) *Gene* 164:49-53.

Next, the gene sequence encoding the desired antigen can be inserted into a vector containing a synthetic expression cassette of the present invention (e.g., see Example 1 for construction of various exemplary synthetic expression cassette). The antigen is inserted into the synthetic coding sequence such that when the combined sequence is expressed it results in the production of VLPs comprising the polypeptide and/or the antigen of interest. Insertions can be made within the Gag coding sequence or at either end of the coding sequence (5', amino terminus of the expressed polypeptide; or 3', carboxy terminus of the expressed polypeptide -- e.g., see Example 1)(Wagner, R., et al., *Arch Virol.* 127:117-137, 1992; Wagner, R., et al., *Virology* 200:162-175, 1994; Wu, X., et al., *J. Virol.* 69(6):3389-3398, 1995; Wang, C-T., et al., Virology 200:524-534, 1994; Chazal, N., et al., *Virology* 68(1):111-122, 1994; Griffiths, J.C., et al., *J. Virol.* 67(6):3191-3198, 1993; Reicin, A.S., et al., *J. Virol.* 69(2):642-650, 1995).

Up to 50% of the coding sequences of p55Gag can be deleted without affecting the assembly to virus-like particles and expression efficiency (Borsetti, A., et al, *J. Virol.* 72(11):9313-9317, 1998; Gamier, L., et al., *J Virol* 72(6):4667-4677, 1998; Zhang, Y., et al., *J Virol* 72(3):1782-1789, 1998; Wang, C., et al., *J Virol* 72(10): 7950-7959, 1998). In one embodiment of the present invention, immunogenicity of the high level expressing synthetic p55GagMod and p55GagProtMod expression cassettes can be increased by the insertion of different structural or non-structural HIV antigens, multiepitope cassettes, or cytokine sequences into deleted, mutated or truncated regions of p55GagMod sequence. In another embodiment of the present invention, immunogenicity of the high level expressing synthetic Env expression cassettes can be increased by the insertion of different structural or non-structural HIV antigens, multiepitope cassettes, or cytokine sequences into deleted regions of gp120Mod, gp140Mod or gp160Mod sequences. Such deletions may be generated following the teachings of the present invention and information available to one of ordinary skill in the art. One possible advantage of this approach, relative to using full-length modified Env sequences fused to heterologous polypeptides, can be higher expression/secretion efficiency and/or higher immunogenicity of the expression product. Such deletions may be generated following the teachings of the present invention and information available to one of ordinary skill in the art. One possible advantage of this approach, relative to using full-length Env, Gag or Tat sequences fused to heterologous polypeptides, can be higher expression/secretion efficiency and/or immunogenicity of the expression product.

When sequences are added to the amino terminal end of Gag (for example, when using the synthetic p55GagMod expression cassette of the present invention), the polynucletide can contain coding sequences at the 5' end that encode a signal for addition of a myristic moiety to the Gag-containing polypeptide (e.g., sequences that encode Met-Gly).

The ability of Gag-containing polypeptide constructs to form VLPs can be empirically determined following the teachings of the present specification.

HIV polypeptide/antigen synthetic expression cassettes include control elements operably linked to the coding sequence, which allow for the expression of the gene in vivo in the subject species. For example, typical promoters for mammalian cell expression include the SV40 early promoter, a CMV promoter such as the CMV immediate early promoter, the mouse mammary tumor virus LTR promoter, the adenovirus major late promoter (Ad MLP), and the herpes simplex virus promoter, among others. Other nonviral promoters, such as a promoter derived from the murine metallothionein gene, will also find use for mammalian expression. Typically, transcription termination and polyadenylation sequences will also be present, located 3' to the translation stop codon. Preferably, a sequence for optimization of initiation of translation, located 5' to the coding sequence, is also present. Examples of transcription terminator/polyadenylation signals include those derived from SV40, as described in Sambrook et al., supra, as well as a bovine growth hormone terminator sequence.

Enhancer elements may also be used herein to increase expression levels of the mammalian constructs. Examples include the SV40 early gene enhancer, as described in Dijkema et al., *EMBO J.* (1985) 4:761, the enhancer/promoter derived from the long terminal repeat (LTR) of the Rous Sarcoma Virus, as described in Gorman et al., *Proc. Natl. Acad. Sci. USA* (1982b) 79:6777 and elements derived from human CMV, as described in Boshart et al., *Cell* (1985) 41:521, such as elements included in the CMV intron A sequence.

Furthermore, plasmids can be constructed which include a chimeric antigen-coding gene sequences, encoding, e.g., multiple antigens/epitopes of interest, for example derived from a single or from more than one viral isolate.

Typically the antigen coding sequences precede or follow the synthetic coding sequences and the chimeric transcription unit will have a single open reading frame encoding both the antigen of interest and the synthetic Gag coding sequences. Alternatively, multi-cistronic cassettes (e.g., bi-cistronic cassettes) can be constructed allowing expression of multiple antigens from a single mRNA using the EMCV IRES, or the like. Lastly, antigens can be encoded on separate transcripts from independent promoters on a single plasmid or other vector.

Once complete, the constructs are used for nucleic acid immunization or the like using standard gene delivery protocols. Methods for gene delivery are known in the art. See, e.g., U.S. Patent Nos. 5,399,346, 5,580,859, 5,589,466. Genes can be delivered either directly to the vertebrate subject or, alternatively, delivered *ex vivo,* to cells derived from the subject and the cells reimplanted in the subject.

A number of viral based systems have been developed for gene transfer into mammalian cells. For example, retroviruses provide a convenient platform for gene delivery systems. Selected sequences can be inserted into a vector and packaged in retroviral particles using techniques known in the art. The recombinant virus can then be isolated and delivered to cells of the subject either in vivo or ex vivo. A number of retroviral systems have been described (U.S. Patent No. 5,219,740; Miller and Rosman, *BioTechniques* (1989) 7.:980-990; Miller, A.D., *Human Gene Therapy* (1990) 1:5-14; Scarpa et al., *Virology* (1991) 180:849-852; Burns et al., *Proc. Natl. Acad. Sci. USA* (1993) 90:8033-8037; and Boris-Lawrie and Temin, *Cur. Opin. Genet. Develop.* (1993) 3:102-109.

A number of adenovirus vectors have also been described. Unlike retroviruses which integrate into the host genome, adenoviruses persist extrachromosomally thus minimizing the risks associated with insertional mutagenesis (Haj-Ahmad and Graham, *J. Virol.* (1986) 57:267-274; Bett et al., J. *Virol.* (1993) 67:5911-5921; Mittereder et al., *Human Gene Therapy* (1994) 5:717-729; Seth et al., *J. Virol.* (1994) 68:933-940; Barr et al., *Gene Therapy* (1994) 1:51-58; Berkner, K.L. *BioTechniques* (1988) 6:616-629; and Rich et al., *Human Gene Therapy* (1993) 4:461-476).

Additionally, various adeno-associated virus (AAV) vector systems have been developed for gene delivery. AAV vectors can be readily constructed using techniques well known in the art. See, e.g., U.S. Patent Nos. 5,173,414 and 5,139,941; International Publication Nos. WO 92/01070 (published 23 January 1992) and WO 93/03769 (published 4 March 1993); Lebkowski et al., *Molec. Cell. Biol.* (1988) 8:3988-3996; Vincent et al., *Vaccines 90* (1990) (Cold Spring Harbor Laboratory Press); Carter, B.J. *Current Opinion in Biotechnology* (1992) 3:533-539; Muzyczka, N. *Current Topics in Microbiol. and Immunol.* (1992) 158:97-129; Kotin, R.M. *Human Gene Therapy* (1994) 5:793-801; Shelling and Smith, *Gene Therapy* (1994) 1:165-169; and Zhou et al., *J. Exp. Med.* (1994) 179:1867-1875.

Another vector system useful for delivering the polynucleotides of the present invention is the enterically administered recombinant poxvirus vaccines described by Small, Jr., P.A., et al. (U.S. Patent No. 5,676,950, issued October 14, 1997).

Additional viral vectors which will find use for delivering the nucleic acid molecules encoding the antigens of interest include those derived from the pox family of viruses, including vaccinia virus and avian poxvirus. By way of example, vaccinia virus recombinants expressing the genes can be constructed as follows. The DNA encoding the particular synthetic Gag/antigen coding sequence is first inserted into an appropriate vector so that it is adjacent to a vaccinia promoter and flanking vaccinia DNA sequences, such as the sequence encoding thymidine kinase (TK). This vector is then used to transfect cells which are simultaneously infected with vaccinia. Homologous recombination serves to insert the vaccinia promoter plus the gene encoding the coding sequences of interest into the viral genome. The resulting TK-recombinant can be selected by culturing the cells in the presence of 5-bromodeoxyuridine and picking viral plaques resistant thereto.

Alternatively, avipoxviruses, such as the fowlpox and canarypox viruses, can also be used to deliver the genes. Recombinant avipox viruses, expressing immunogens from mammalian pathogens, are known to confer protective immunity when administered to non-avian species. The use of an avipox vector is particularly desirable in human and other mammalian species since members of the avipox genus can only productively replicate in susceptible avian species and therefore are not infective in mammalian cells. Methods for producing recombinant avipoxviruses are known in the art and employ genetic recombination, as described above with respect to the production of vaccinia viruses. See, e.g., WO 91/12882; WO 89/03429; and WO 92/03545.

Molecular conjugate vectors, such as the adenovirus chimeric vectors described in Michael et al., *J. Biol. Chem.* (1993) 268:6866-6869 and Wagner et al., *Proc. Natl. Acad. Sci. USA* (1992) 89:6099-6103, can also be used for gene delivery.

Members of the Alphavirus genus, such as, but not limited to, vectors derived from the Sindbis, Semliki Forest, and Venezuelan Equine Encephalitis viruses, will also find use as viral vectors for delivering the polynucleotides of the present invention (for example, a synthetic Gag- or Env-polypeptide encoding expression cassette as described in Example 14 below). For a description of Sindbis-virus derived vectors useful for the practice of the instant methods, see, Dubensky et al., *J. Virol.* (1996) 70:508-519; and International Publication Nos. WO 95/07995 and WO 96/17072; as well as, Dubensky, Jr., T.W., et al., U.S. Patent No. 5,843,723, issued December 1, 1998, and Dubensky, Jr., T.W., U.S. Patent No. 5,789,245, issued August 4, 1998.

A vaccinia based infection/transfection system can be conveniently used to provide for inducible, transient expression of the coding sequences of interest (for example, a synthetic Gag/HCV-core expression cassette) in a host cell. In this system, cells are first infected in vitro with a vaccinia virus recombinant that encodes the bacteriophage T7 RNA polymerase. This polymerase displays exquisite specificity in that it only transcribes templates bearing T7 promoters. Following infection, cells are transfected with the polynucleotide of interest, driven by a T7 promoter. The polymerase expressed in the cytoplasm from the vaccinia virus recombinant transcribes the transfected DNA into RNA which is then translated into protein by the host translational machinery. The method provides for high level, transient, cytoplasmic production of large quantities of RNA and its translation products. See, e.g., Elroy-Stein and Moss, *Proc. Natl. Acad. Sci. USA* (1990) 87:6743-6747; Fuerst et al., *Proc. Natl. Acad. Sci. USA* (1986) 83:8122-8126.

As an alternative approach to infection with vaccinia or avipox virus recombinants, or to the delivery of genes using other viral vectors, an amplification system can be used that will lead to high level expression following introduction into host cells. Specifically, a T7 RNA polymerase promoter preceding the coding region for T7 RNA polymerase can be engineered. Translation of RNA derived from this template will generate T7 RNA polymerase which in turn will transcribe more template. Concomitantly, there will be a cDNA whose expression is under the control of the T7 promoter. Thus, some of the T7 RNA polymerase generated from translation of the amplification template RNA will lead to transcription of the desired gene. Because some T7 RNA polymerase is required to initiate the amplification, T7 RNA polymerase can be introduced into cells along with the template(s) to prime the transcription reaction. The polymerase can be introduced as a protein or on a plasmid encoding the RNA polymerase. For a further discussion of T7 systems and their use for transforming cells, see, e.g., International Publication No. WO 94/26911; Studier and Moffatt, *J. Mol. Biol.* (1986) 189:113-130; Deng and Wolff, *Gene* (1994) 143:245-249; Gao et al., *Biochem. Biophys. Res. Commun.* (1994) 200:1201-1206; Gao and Huang, *Nuc. Acids Res.* (1993) 21:2867-2872; Chen et al., *Nuc. Acids Res.* (1994) 22:2114-2120; and U.S. Patent No. 5,135,855.

The synthetic expression cassette of interest can also be delivered without a viral vector. For example, the synthetic expression cassette can be packaged as DNA or RNA in liposomes prior to delivery to the subject or to cells derived therefrom. Lipid encapsulation is generally accomplished using liposomes which are able to stably bind or entrap and retain nucleic acid. The ratio of condensed DNA to lipid preparation can vary but will generally be around 1:1 (mg DNA:micromoles lipid), or more of lipid. For a review of the use of liposomes as carriers for delivery of nucleic acids, see, Hug and Sleight, *Biochim. Biophys. Acta.* (1991) 1097:1-17; Straubinger et al., in *Methods of Enzymology* (1983), Vol. 101, pp. 512-527.

Liposomal preparations for use in the present invention include cationic (positively charged), anionic (negatively charged) and neutral preparations, with cationic liposomes particularly preferred. Cationic liposomes have been shown to mediate intracellular delivery of plasmid DNA (Felgner et al., *Proc. Natl. Acad. Sci. USA* (1987) 84:7413-7416); mRNA (Malone et al., *Proc. Natl. Acad. Sci. USA* (1989) 86:6077-6081); and purified transcription factors (Debs et al., *J. Biol. Chem.* (1990) 265:10189-10192), in functional form.

Cationic liposomes are readily available. For example, N[1-2,3-dioleyloxy)propyl]-N,N,N-triethyl-ammonium (DOTMA) liposomes are available under the trademark Lipofectin, from GIBCO BRL, Grand Island, NY. (See, also, Felgner et al., *Proc. Natl. Acad. Sci. USA* (1987) 84:7413-7416). Other commercially available lipids include (DDAB/DOPE) and DOTAP/DOPE (Boerhinger). Other cationic liposomes can be prepared from readily available materials using techniques well known in the art. See, e.g., Szoka et al., *Proc. Natl. Acad. Sci. USA* (1978) 75:4194-4198; PCT Publication No. WO 90/11092 for a description of the synthesis of DOTAP (1,2-bis(oleoyloxy)-3-(trimethylammonio)propane) liposomes.

Similarly, anionic and neutral liposomes are readily available, such as, from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See, e.g., Straubinger et al., in METHODS OF IMMUNOLOGY (1983), Vol. 101, pp. 512-527; Szoka et al., *Proc. Natl. Acad. Sci. USA* (1978) 75:4194-4198; Papahadjopoulos et al., *Biochim. Biophys. Acta* (1975) 394:483; Wilson et al., *Cell* (1979) 17:77); Deamer and Bangham, *Biochim. Biophys. Acta* (1976) 443:629; Ostro et al., *Biochem. Biophys. Res. Commun.* (1977) 76:836; Fraley et al., *Proc. Natl. Acad. Sci. USA* (1979) 76:3348); Enoch and Strittmatter, *Proc. Natl. Acad. Sci. USA* (1979) 76:145); Fraley et al., J. *Biol. Chem.* (1980) 255:10431; Szoka and Papahadjopoulos, *Proc. Natl. Acad. Sci. USA* (1978) 75:145; and Schaefer-Ridder et al., *Science* (1982) 215:166.

The DNA and/or protein antigen(s) can also be delivered in cochleate lipid compositions similar to those described by Papahadjopoulos et al., *Biochem. Biophys. Acta.* (1975) 394:483-491. See, also, U.S. Patent Nos. 4,663,161 and 4,871,488.

The synthetic expression cassette of interest (e.g., any of the synthetic expression cassettes described in Example 1) may also be encapsulated, adsorbed to, or associated with, particulate carriers. Such carriers present multiple copies of a selected antigen to the immune system and promote migration, trapping and retention of antigens in local lymph nodes. The particles can be taken up by profession antigen presenting cells such as macrophages and dendritic cells, and/or can enhance antigen presentation through other mechanisms such as stimulation of cytokine release. Examples of particulate carriers include those derived from polymethyl methacrylate polymers, as well as microparticles derived from poly(lactides) and poly(lactide-co-glycolides), known as PLG. See, e.g., Jeffery et al., *Pharm. Res.* (1993) 10:362-368; McGee JP, et al., *J Microencapsul.* 14(2):197-210, 1997; O'Hagan DT, et al., *Vaccine* 11(2):149-54, 1993.

Furthermore, other particulate systems and polymers can be used for the *in vivo* or *ex vivo* delivery of the gene of interest. For example, polymers such as polylysine, polyarginine, polyornithine, spermine, spermidine, as well as conjugates of these molecules, are useful for transferring a nucleic acid of interest. Similarly, DEAE dextran-mediated transfection, calcium phosphate precipitation or precipitation using other insoluble inorganic salts, such as strontium phosphate, aluminum silicates including bentonite and kaolin, chromic oxide, magnesium silicate, talc, and the like, will find use with the present methods. See, e.g., Felgner, P.L., *Advanced Drug Delivery Reviews* (1990) 5:163-187, for a review of delivery systems useful for gene transfer. Peptoids (Zuckerman, R.N., et al., U.S. Patent No. 5,831,005, issued November 3, 1998) may also be used for delivery of a construct of the present invention.

Additionally, biolistic delivery systems employing particulate carriers such as gold and tungsten, are especially useful for delivering synthetic expression cassettes of the present invention. The particles are coated with the synthetic expression cassette(s) to be delivered and accelerated to high velocity, generally under a reduced atmosphere, using a gun powder discharge from a "gene gun." For a description of such techniques, and apparatuses useful therefore, see, e.g., U.S. Patent Nos. 4,945,050; 5,036,006; 5,100,792; 5,179,022; 5,371,015; and 5,478,744. Also, needle-less injection systems can be used (Davis, H.L., et al, *Vaccine* 12:1503-1509, 1994; Bioject, Inc., Portland, OR).

Recombinant vectors carrying a synthetic expression cassette of the present invention are formulated into compositions for delivery to the vertebrate subject. These compositions may either be prophylactic (to prevent infection) or therapeutic (to treat disease after infection). The compositions will comprise a "therapeutically effective amount" of the gene of interest such that an amount of the antigen can be produced *in vivo* so that an immune response is generated in the individual to which it is administered. The exact amount necessary will vary depending on the subject being treated; the age and general condition of the subject to be treated; the capacity of the subject's immune system to synthesize antibodies; the degree of protection desired; the severity of the condition being treated; the particular antigen selected and its mode of administration, among other factors. An appropriate effective amount can be readily determined by one of skill in the art. Thus, a "therapeutically effective amount" will fall in a relatively broad range that can be determined through routine trials.

The compositions will generally include one or more "pharmaceutically acceptable excipients or vehicles" such as water, saline, glycerol, polyethyleneglycol, hyaluronic acid, ethanol, etc. Additionally, auxiliary substances, such as wetting or emulsifying agents, pH buffering substances, surfactants and the like, may be present in such vehicles. Certain facilitators of immunogenicity or of nucleic acid uptake and/or expression can also be included in the compositions or coadministered, such as, but not limited to, bupivacaine, cardiotoxin and sucrose.

Once formulated, the compositions of the invention can be administered directly to the subject (e.g., as described above) or, alternatively, delivered *ex vivo,* to cells derived from the subject, using methods such as those described above. For example, methods for the ex vivo delivery and reimplantation of transformed cells into a subject are known in the art and can include, e.g., dextran-mediated transfection, calcium phosphate precipitation, polybrene mediated transfection, lipofectamine and LT-1 mediated transfection, protoplast fusion, electroporation, encapsulation of the polynucleotide(s) (with or without the corresponding antigen) in liposomes, and direct microinjection of the DNA into nuclei.

Direct delivery of synthetic expression cassette compositions in vivo will generally be accomplished with or without viral vectors, as described above, by injection using either a conventional syringe, needless devices such as Bioject® or a gene gun, such as the Accell® gene delivery system (PowderJect Technologies, Inc., Oxford, England). The constructs can be delivered (e.g., injected) either subcutaneously, epidermally, intradermally, intramuscularly, intravenous, intramucosally (such as nasally, rectally and vaginally), intraperitoneally or orally. Delivery of DNA into cells of the epidermis is particularly preferred as this mode of administration provides access to skin-associated lymphoid cells and provides for a transient presence of DNA in the recipient. Other modes of administration include oral ingestion and pulmonary administration, suppositories, needle-less injection, transcutaneous and transdermal applications. Dosage treatment may be a single dose schedule or a multiple dose schedule.

### 2.3.2 EX VIVO DELIVERY OF THE SYNTHETIC EXPRESSION CASSETTES OF THE PRESENT INVENTION

In one embodiment, T cells, and related cell types (including but not limited to antigen presenting cells, such as, macrophage, monocytes, lymphoid cells, dendritic cells, B-cells, T-cells, stem cells, and progenitor cells thereof), can be used for *ex vivo* delivery of the synthetic expression cassettes of the present invention. T cells can be isolated from peripheral blood lymphocytes (PBLs) by a variety of procedures known to those skilled in the art. For example, T cell populations can be "enriched" from a population of PBLs through the removal of accessory and B cells. In particular, T cell enrichment can be accomplished by the elimination of non-T cells using anti-MHC class II monoclonal antibodies. Similarly, other antibodies can be used to deplete specific populations of non-T cells. For example, anti-Ig antibody molecules can be used to deplete B cells and anti-MacI antibody molecules can be used to deplete macrophages.

T cells can be further fractionated into a number of different subpopulations by techniques known to those skilled in the art. Two major subpopulations can be isolated based on their differential expression of the cell surface markers CD4 and CD8. For example, following the enrichment of T cells as described above, CD4⁺ cells can be enriched using antibodies specific for CD4 (see Coligan et al., *supra).* The antibodies may be coupled to a solid support such as magnetic beads. Conversely, CD8+ cells can be enriched through the use of antibodies specific for CD4 (to remove CD4⁺ cells), or can be isolated by the use of CD8 antibodies coupled to a solid support. CD4 lymphocytes from HIV-1 infected patients can be expanded ex vivo, before or after transduction as described by Wilson et. al. (1995) *J. Infect. Dis.* 172:88.

Following purification of T cells, a variety of methods of genetic modification known to those skilled in the art can be performed using non-viral or viral-based gene transfer vectors constructed as described herein. For example, one such approach involves transduction of the purified T cell population with vector-containing supernatant of cultures derived from vector producing cells. A second approach involves co-cultivation of an irradiated monolayer of vector-producing cells with the purified T cells. A third approach involves a similar co-cultivation approach; however, the purified T cells are pre-stimulated with various cytokines and cultured 48 hours prior to the co-cultivation with the irradiated vector producing cells. Pre-stimulation prior to such transduction increases effective gene transfer (Nolta et al. (1992) *Exp. Hematol.* 20:1065). Stimulation of these cultures to proliferate also provides increased cell populations for re-infusion into the patient. Subsequent to co-cultivation, T cells are collected from the vector producing cell monolayer, expanded, and frozen in liquid nitrogen.

Gene transfer vectors, containing one or more synthetic expression cassette of the present invention (associated with appropriate control elements for delivery to the isolated T cells) can be assembled using known methods.

Selectable markers can also be used in the construction of gene transfer vectors. For example, a marker can be used which imparts to a mammalian cell transduced with the gene transfer vector resistance to a cytotoxic agent. The cytotoxic agent can be, but is not limited to, neomycin, aminoglycoside, tetracycline, chloramphenicol, sulfonamide, actinomycin, netropsin, distamycin A, anthracycline, or pyrazinamide. For example, neomycin phosphotransferase II imparts resistance to the neomycin analogue geneticin (G418).

The T cells can also be maintained in a medium containing at least one type of growth factor prior to being selected. A variety of growth factors are known in the art which sustain the growth of a particular cell type. Examples of such growth factors are cytokine mitogens such as rIL-2, IL-10, IL-12, and IL-15, which promote growth and activation of lymphocytes. Certain types of cells are stimulated by other growth factors such as hormones, including human chorionic gonadotropin (hCG) and human growth hormone. The selection of an appropriate growth factor for a particular cell population is readily accomplished by one of skill in the art.

For example, white blood cells such as differentiated progenitor and stem cells are stimulated by a variety of growth factors. More particularly, IL-3, IL-4, IL-5, IL-6, IL-9, GM-CSF, M-CSF, and G-CSF, produced by activated T_{H} and activated macrophages, stimulate myeloid stem cells, which then differentiate into pluripotent stem cells, granulocyte-monocyte progenitors, eosinophil progenitors, basophil progenitors, megakaryocytes, and erythroid progenitors. Differentiation is modulated by growth factors such as GM-CSF, IL-3, IL-6, IL-11, and EPO.

Pluripotent stem cells then differentiate into lymphoid stem cells, bone marrow stromal cells, T cell progenitors, B cell progenitors, thymocytes, T_{H} Cells, T_{C} cells, and B cells. This differentiation is modulated by growth factors such as IL-3, IL-4, IL-6, IL-7, GM-CSF, M-CSF, G-CSF, IL-2, and IL-5.

Granulocyte-monocyte progenitors differentiate to monocytes, macrophages, and neutrophils. Such differentiation is modulated by the growth factors GM-CSF, M-CSF, and IL-8. Eosinophil progenitors differentiate into eosinophils. This process is modulated by GM-CSF and IL-5.

The differentiation of basophil progenitors into mast cells and basophils is modulated by GM-CSF, IL-4, and IL-9. Megakaryocytes produce platelets in response to GM-CSF, EPO, and IL-6. Erythroid progenitor cells differentiate into red blood cells in response to EPO.

Thus, during activation by the CD3-binding agent, T cells can also be contacted with a mitogen, for example a cytokine such as IL-2. In particularly preferred embodiments, the IL-2 is added to the population of T cells at a concentration of about 50 to 100 µg/ml. Activation with the CD3-binding agent can be carried out for 2 to 4 days.

Once suitably activated, the T cells are genetically modified by contacting the same with a suitable gene transfer vector under conditions that allow for transfection of the vectors into the T cells. Genetic modification is carried out when the cell density of the T cell population is between about 0.1 x 10⁶ and 5 x 10⁶, preferably between about 0.5 x 10⁶ and 2 x 10⁶. A number of suitable viral and nonviral-based gene transfer vectors have been described for use herein.

After transduction, transduced cells are selected away from non-transduced cells using known techniques. For example, if the gene transfer vector used in the transduction includes a selectable marker which confers resistance to a cytotoxic agent, the cells can be contacted with the appropriate cytotoxic agent, whereby non-transduced cells can be negatively selected away from the transduced cells. If the selectable marker is a cell surface marker, the cells can be contacted with a binding agent specific for the particular cell surface marker, whereby the transduced cells can be positively selected away from the population. The selection step can also entail fluorescence-activated cell sorting (FACS) techniques, such as where FACS is used to select cells from the population containing a particular surface marker, or the selection step can entail the use of magnetically responsive particles as retrievable supports for target cell capture and/or background removal.

More particularly, positive selection of the transduced cells can be performed using a FACS cell sorter (e.g. a FACSVantage™ Cell Sorter, Becton Dickinson Immunocytometry Systems, San Jose, CA) to sort and collect transduced cells expressing a selectable cell surface marker. Following transduction, the cells are stained with fluorescent-labeled antibody molecules directed against the particular cell surface marker. The amount of bound antibody on each cell can be measured by passing droplets containing the cells through the cell sorter. By imparting an electromagnetic charge to droplets containing the stained cells, the transduced cells can be separated from other cells. The positively selected cells are then harvested in sterile collection vessels. These cell sorting procedures are described in detail, for example, in the FACSVantage™ Training Manual, with particular reference to sections 3-11 to 3-28 and 10-1 to 10-17.

Positive selection of the transduced cells can also be performed using magnetic separation of cells based on expression or a particular cell surface marker. In such separation techniques, cells to be positively selected are first contacted with specific binding agent (e.g., an antibody or reagent the interacts specifically with the cell surface marker). The cells are then contacted with retrievable particles (e.g., magnetically responsive particles) which are coupled with a reagent that binds the specific binding agent (that has bound to the positive cells). The cell-binding agent-particle complex can then be physically separated from non-labeled cells, for example using a magnetic field. When using magnetically responsive particles, the labeled cells can be retained in a container using a magnetic filed while the negative cells are removed. These and similar separation procedures are known to those of ordinary skill in the art.

Expression of the vector in the selected transduced cells can be assessed by a number of assays known to those skilled in the art. For example, Western blot or Northern analysis can be employed depending on the nature of the inserted nucleotide sequence of interest. Once expression has been established and the transformed T cells have been tested for the presence of the selected synthetic expression cassette, they are ready for infusion into a patient via the peripheral blood stream.

The invention includes a kit for genetic modification of an *ex vivo* population of primary mammalian cells. The kit typically contains a gene transfer vector coding for at least one selectable marker and at least one synthetic expression cassette contained in one or more containers, ancillary reagents or hardware, and instructions for use of the kit.

### EXPERIMENTAL

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

### Example 1

### Generation of Synthetic Gag and Env Expression Cassettes

### A. Modification of HTV-1 Gag, Gag-protease, Gag-reverse transcriptase and Gag-polymerase Nucleic Acid Coding Sequences

The Gag (SEQ ID NO:1), Gag-protease (SEQ ID NO:2), Gag-polymerase (SEQ ID NO:3), and Gag-reverse transcriptase (SEQ ID NO:77) coding sequences were selected from the HIV-1SF2 strain (Sanchez-Pescador, R., et al., *Science* 227(4686): 484-492, 1985; Luciw, P.A., et al. U.S. Patent No. 5,156,949, issued October 20, 1992; Luciw, P.A., et al., U.S. Patent No. 5,688,688, November 18, 1997). These sequences were manipulated to maximize expression of their gene products.

First, the HIV-1 codon usage pattern was modified so that the resulting nucleic acid coding sequence was comparable to codon usage found in highly expressed human genes. The HIV codon usage reflects a high content of the nucleotides A or T of the codon-triplet. The effect of the HIV-1 codon usage is a high AT content in the DNA sequence that results in a high AU content in the RNA and in a decreased translation ability and instability of the mRNA. In comparison, highly expressed human codons prefer the nucleotides G or C. The Gag-encoding sequences were modified to be comparable to codon usage found in highly expressed human genes.

Figure 11 presents a comparison of the percent A-T content for the cDNAs of stable versus unstable RNAs (comparison window size = 50). Human IFNγ mRNA is known to (i) be unstable, (ii) have a short half-life, and (iii) have a high A-U content. Human GAPDH (glyceraldehyde-3-phosphate dehydrogenase) mRNA is known to (i) be a stable RNA, and (i) have a low A-U content. In Figure 11, the percent A-T content of these two sequences are compared to the percent A-T content of native HIV-1SF2 Gag cDNA and to the synthetic Gag cDNA sequence of the present invention. The top two panels of the figure show the percent A-T content over the length of the sequences for IFNγ and native Gag. The bottom two panels of the figure show the percent A-T content over the length of the sequences for GAPDH and the synthetic Gag. Experiments performed in support of the present invention showed that the synthetic Gag sequences were capable of higher level of protein production (see the Examples) than the native Gag sequences. The data in Figure 11 suggest that one reason for this increased production may be increased stability of the mRNA corresponding to the synthetic Gag coding sequences versus the mRNA corresponding to the native Gag coding sequences.

Second, there are inhibitory (or instability) elements (INS) located within the coding sequences of the Gag and Gag-protease coding sequences (Schneider R, et al., J Virol. 71(7):4892-4903, 1997). RRE is a secondary RNA structure that interacts with the HIV encoded Rev-protein to overcome the expression down-regulating effects of the INS. To overcome the requirement for post-transcriptional activating mechanisms of RRE and Rev, and to enhance independent expression of the Gag polypeptide, the INS were inactivated by introducing multiple point mutations that did not alter the reading frame of the encoded proteins. Figure 1 shows the original SF2 Gag sequence, the location of the INS sequences, and the modifications made to the INS sequences to reduce their effects.

For the Gag-protease sequence (wild type, SEQ ID NO:2; synthetic, SEQ ID NOs:5, 78 and 79), the changes in codon usage were restricted to the regions up to the -1 frameshift and starting again at the end of the Gag reading frame (Figure 2; the region indicated in lower case letters in Figure 2 is the unmodified region). Further, inhibitory (or instability) elements (INS) located within the coding sequences of the Gag-protease polypeptide coding sequence were altered as well (indicated in Figure 2). The synthetic coding sequences were assembled by the Midland Certified Reagent Company (Midland, Texas).

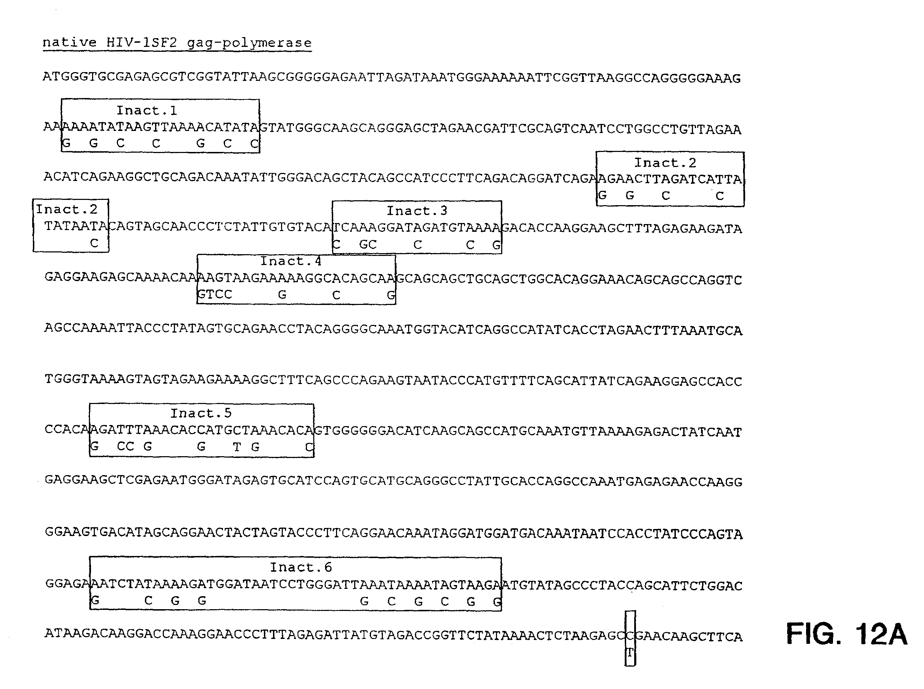

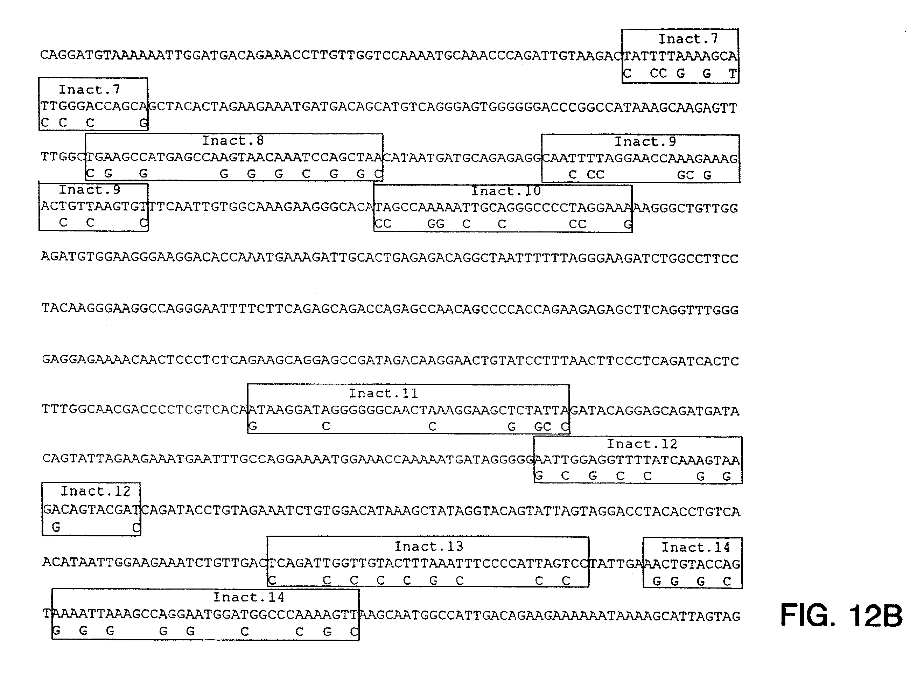

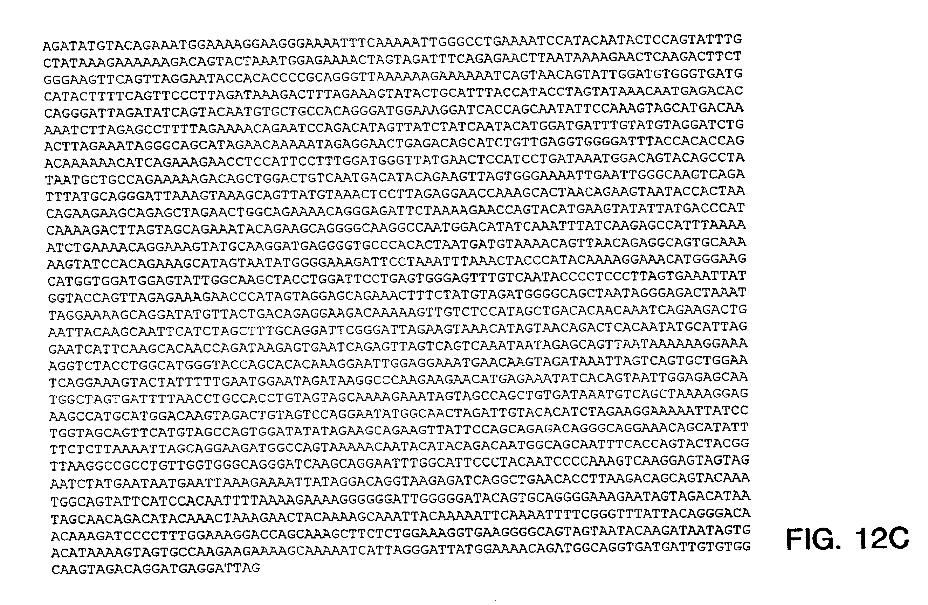

Modification of the Gag-polymerase sequences (wild type, SEQ ID NO:3; synthetic, SEQ ID NO:6) and Gag-reverse transcriptase sequences (SEQ ID NOs:80 through 84) include similar modifications as described for Gag-protease in order to preserve the frameshift region. Locations of the inactivation sites and changes to the sequence to alter the inactivation sites are presented in Figure 12 for the native HTV-1_{SF2} Gag-polymerase sequence.

In one embodiment of the invention, the full length polymerase coding region of the Gag-polymerase sequence is included with the synthetic Gag sequences in order to increase the number of epitopes for virus-like particles expressed by the synthetic, optimized Gag expression cassette. Because synthetic HIV-1 Gag-polymerase expresses the potentially deleterious functional enzymes reverse transcriptase (RT) and integrase (INT) (in addition to the structural proteins and protease), it is important to inactivate RT and INT functions. Several in-frame deletions in the RT and INT reading frame can be made to achieve catalytic nonfunctional enzymes with respect to their RT and INT activity. {Jay. A. Levy (Editor) (1995) *The Retroviridae,* Plenum Press, New York. ISBN 0-306-45033X. Pages 215-20; Grimison, B. and Laurence, J. (1995), *Journal Of Acquired Immune Deficiency Syndromes and Human Retrovirology* 9(1):58-68; Wakefield, J. K.,et al., (1992) *Journal Of Virology* 66(11):6806-6812; Esnouf, R.,et al., (1995) Nature *Structural Biology* 2(4):303-308; Maignan, S., et al., (1998) *Journal Of Molecular Biology* 282(2):359-368; Katz, R. A. and Skalka, A. M. (1994) *Annual Review Of Biochemistry* 73 (1994); Jacobo-Molina, A., et al., (1993) *Proceedings Of the National Academy Of Sciences Of the United States Of America* 90(13):6320-6324; Hickman, A. B., et al., (1994) *Journal Of Biological Chemistry* 269(46):29279-29287; Goldgur, Y., et al., (1998) *Proceedings Of the National Academy Of Sciences Of the United States Of America* 95(16):9150-9154; Goette, M., et al., (1998) *Journal Of Biological Chemistry* 273(17):10139-10146; Gorton, J. L., et al., (1998) *Journal of Virology* 72(6):5046-5055; Engelman, A., et al., (1997) *Journal Of Virology* 71(5):3507-3514; Dyda, F., et al., *Science* 266(5193):1981-1986; Davies, J. F., et al., (1991) *Science* 252(5002):88-95; Bujacz, G., et al., (1996) *Febs Letters* 398 (2-3):175-178; Beard, W. A., et al., (1996) *Journal Of Biological Chemistry* 271(21):12213-12220; Kohlstaedt, L. A., et al., (1992) Science 256(5065):1783-1790; Krug, M. S. and Berger, S. L. (1991) *Biochemistry* 30(44):10614-10623; Mazumder, A., et al., (1996) *Molecular Pharmacology* 49(4):621-628; Palaniappan, C., et al., (1997) *Journal Of Biological Chemistry* 272(17):11157-11164; Rodgers, D. W., et al., (1995) *Proceedings Of the National Academy Of Sciences Of the United States Of America* 92(4):1222-1226; Sheng, N. and Dennis, D. (1993) *Biochemistry* 32(18):4938-4942; Spence, R. A., et al., (1995) Science 267(5200):988-993.}

Furthermore selected B- and/or T-cell epitopes can be added to the Gag-polymerase constructs within the deletions of the RT- and INT-coding sequence to replace and augment any epitopes deleted by the functional modifications of RT and INT. Alternately, selected Band T-cell epitopes (including CTL epitopes) from RT and INT can be included in a minimal VLP formed by expression of the synthetic Gag or synthetic GagProt cassette, described above. (For descriptions of known HIV B- and T-cell epitopes see, HIV Molecular Immunology Database CTL Search Interface; Los Alamos Sequence Compendia, 1987-1997;Internet address: http://hiv-web.lanl.gov/immunology/index.html.)

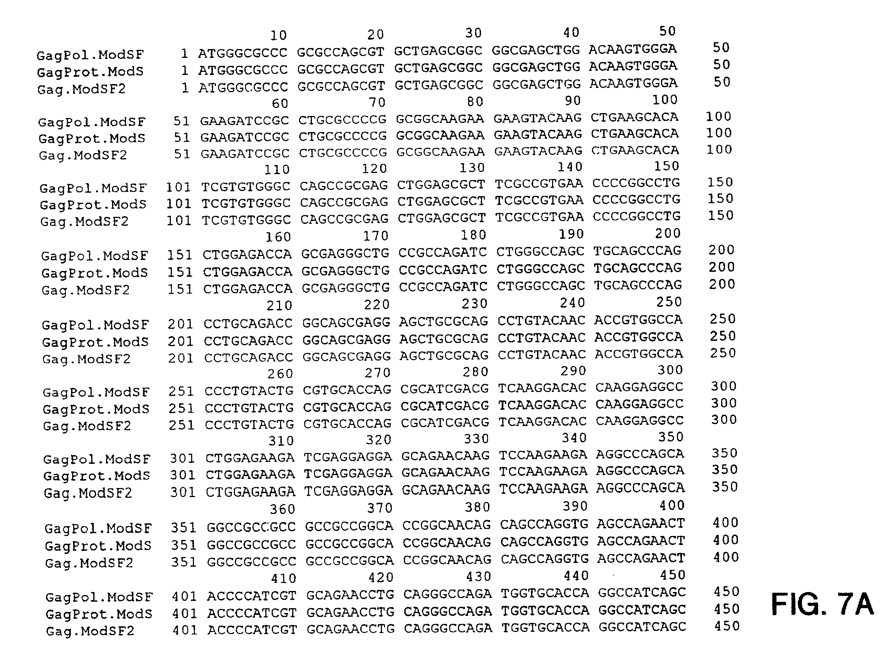

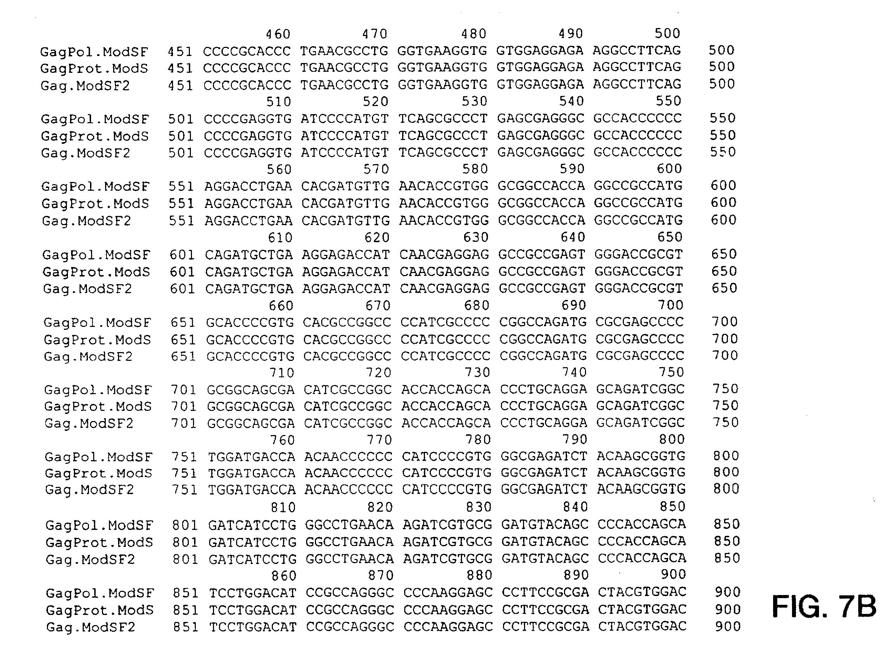

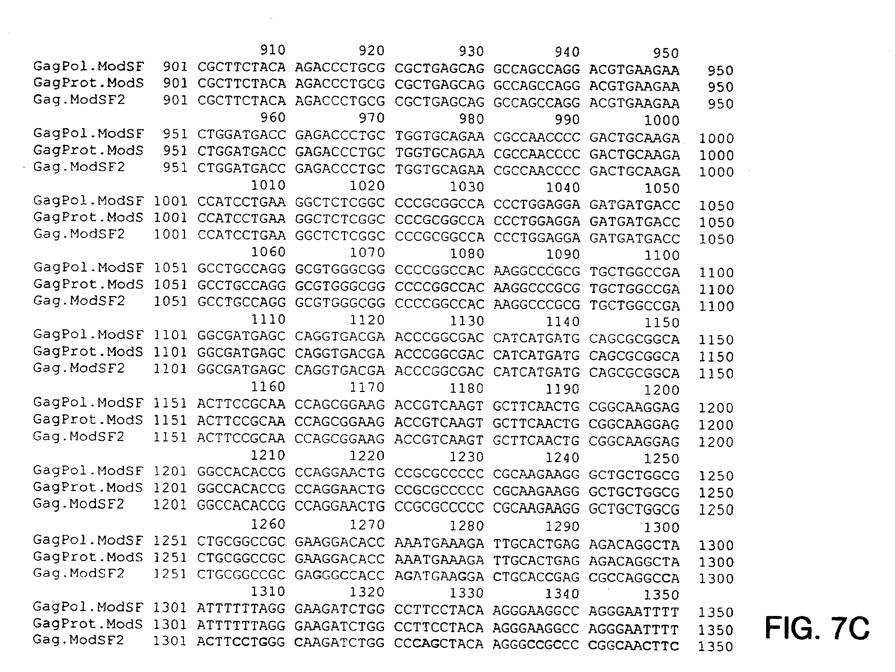

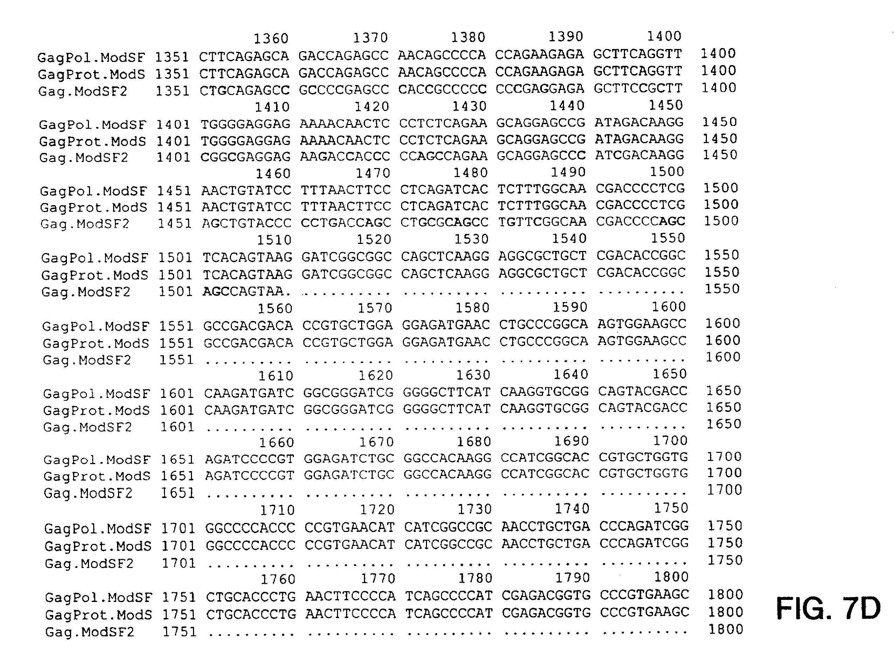

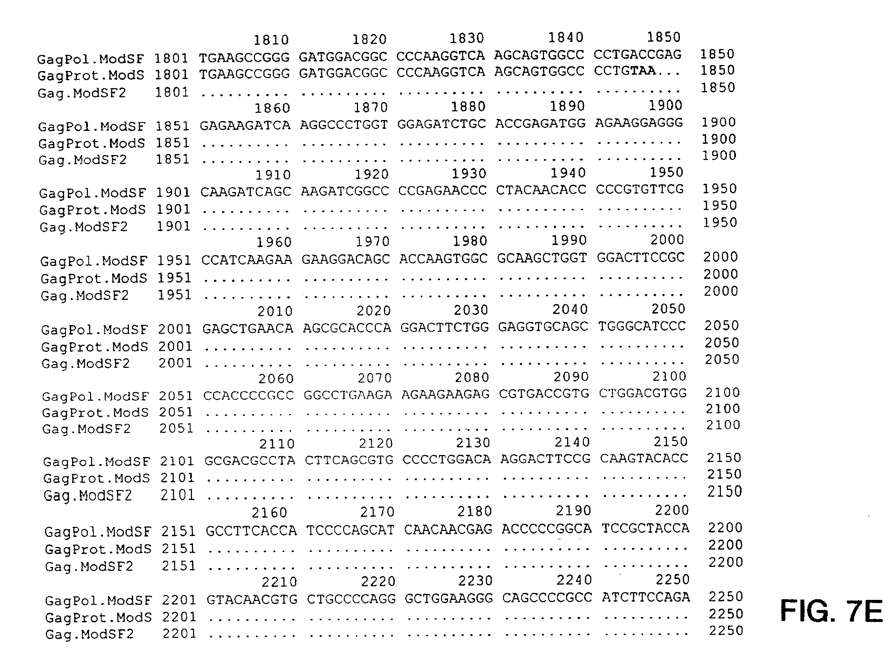

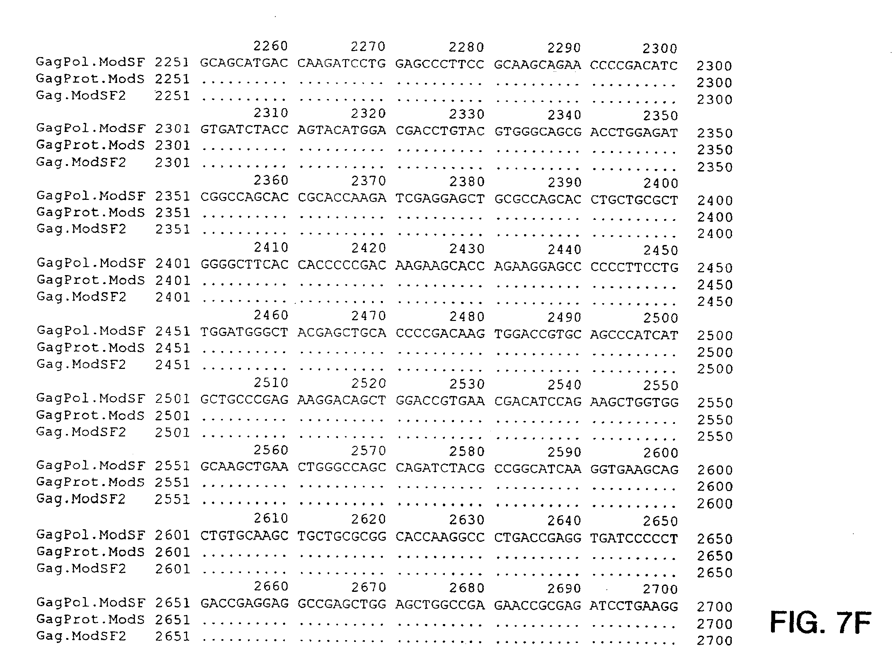

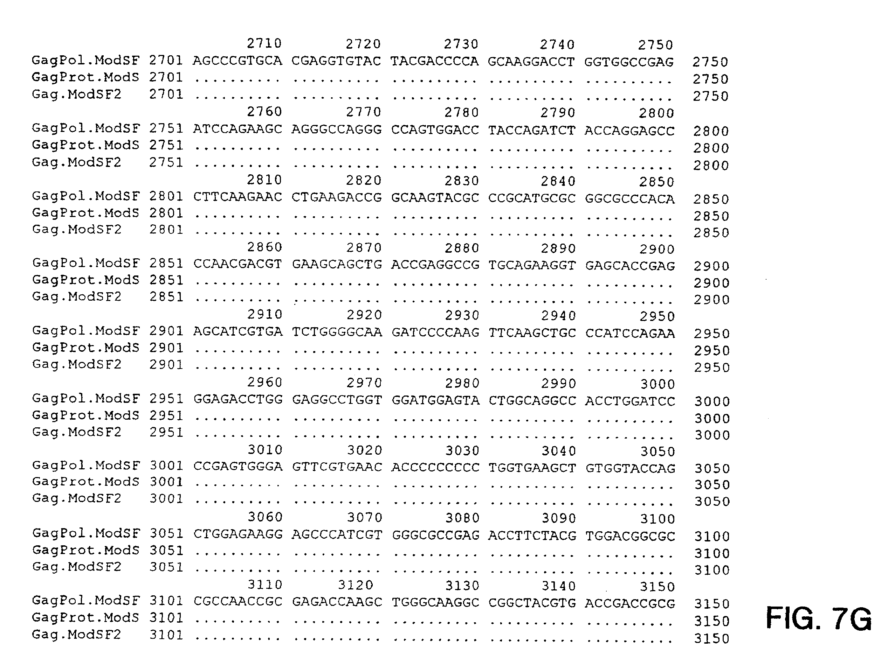

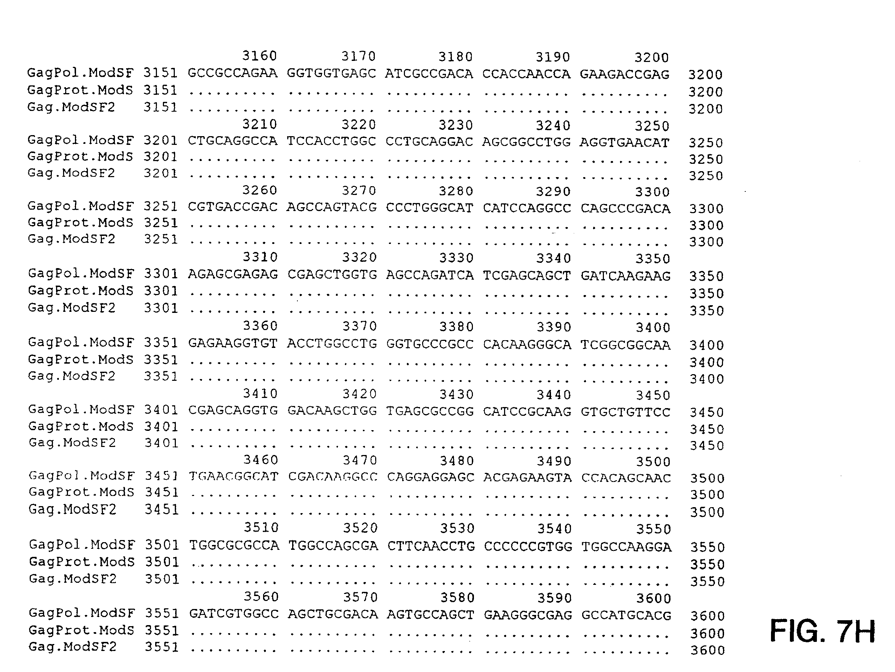

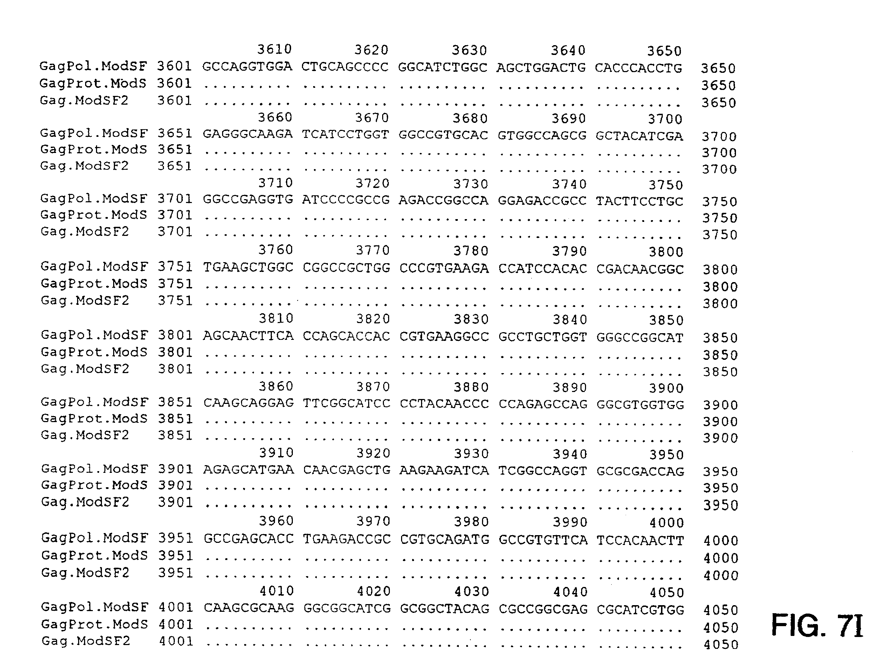

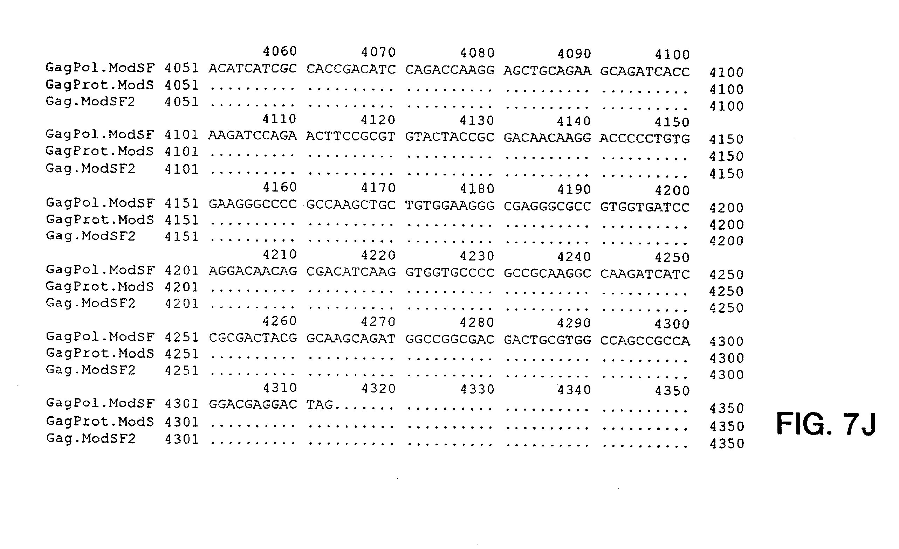

The resulting modified coding sequences are presented as a synthetic Gag expression cassette (SEQ ID NO:4), a synthetic Gag-protease expression cassette (SEQ ID NOs:5, 78 and 79), and a synthetic Gag-polymerase expression cassette (SEQ ID NO:6). Synthetic expression cassettes containing codon modifications in the reverse transcriptase region are shown in SEQ ID NOs:80 through 84. An alignment of selected sequences is presented in Figure 7. A common region (Gag-common; SEQ ID NO:9) extends from position 1 to position 1262.

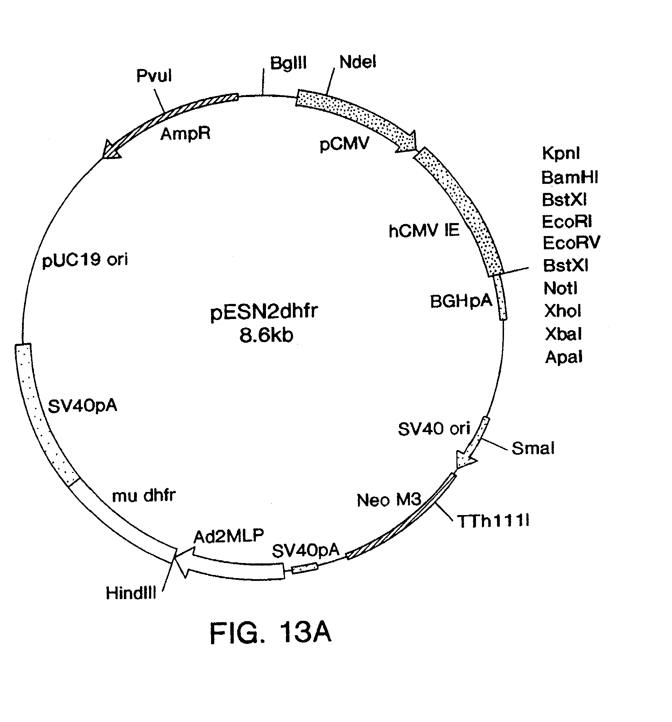

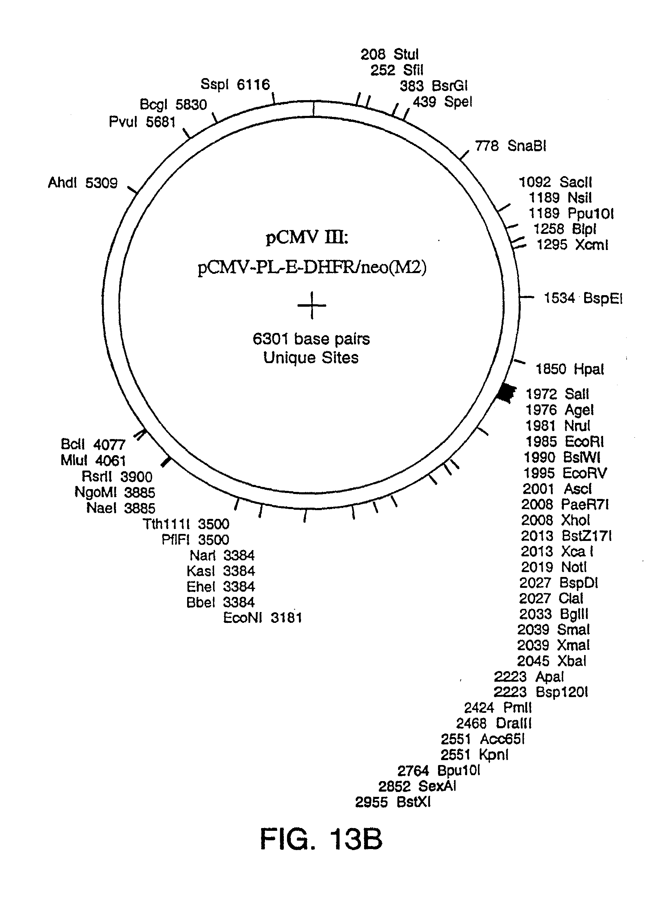

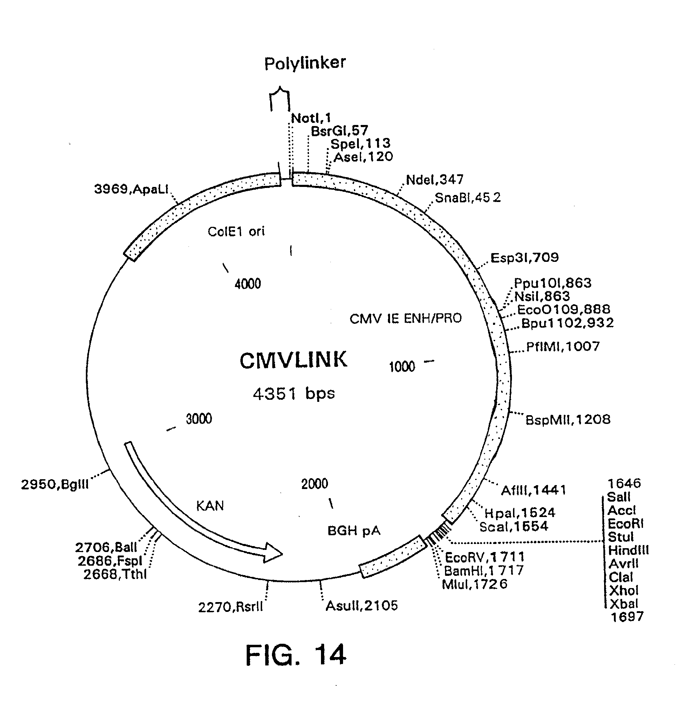

The synthetic DNA fragments for Gag and Gag-protease were cloned into the following expression vectors: pCMVKm2, for transient expression assays and DNA immunization studies, the pCMVKm2 vector was derived from pCMV6a (Chapman et al., *Nuc. Acids Res.* (1991) 19:3979-3986) and comprises a kanamycin selectable marker, a ColE1 origin of replication, a CMV promoter enhancer and Intron A, followed by an insertion site for the synthetic sequences described below followed by a polyadenylation signal derived from bovine growth hormone -- the pCMVKm2 vector differs from the pCMV-link vector only in that a polylinker site was inserted into pCMVKm2 to generate pCMV-link (Figure 14, polylinker at positions 1646 to 1697); pESN2dhfr (Figure 13A) and pCMVPLEdhfr (also known as pCMVIII as shown in Figure 13B), for expression in Chinese Hamster Ovary (CHO) cells; and, pAcC13, a shuttle vector for use in the Baculovirus expression system (pAcCl3, was derived from pAcC12 which was described by Munemitsu S., et al., *Mol Cell Biol.* 10(11):5977-5982, 1990).

A restriction map for vector pCMV-link is presented in Figure 14. In the figure, the CMV promoter (CMV IE ENH/PRO), bovine growth hormone terminator (BGH pA), kanamycin selectable marker (kan), and a ColE1 origin of replication (ColE1 ori) are indicated. A polycloning site is also indicated in the figure following the CMV promoter sequences.

A restriction map for vector pESN2dhfr is presented in Figure 13A. In the figure, the CMV promoter (pCMV, hCMVIE), bovine growth hormone terminator (BGHpA), SV40 origin of replication (SV40ori), neomycin selectable marker (Neo), SV40 polyA (SV40pA), Adenovirus 2 late promoter (Ad2VLP), and the murine dhfr gene (mu dhfr) are indicated. A polycloning site is also indicated in the figure following the CMV promoter sequences.

Briefly, construction of pCMVPLEdhfr (pCMVIII) was as follows. To construct a DHFR cassette, the EMCV IRES (internal ribosome entry site) leader was PCR-amplified from pCite-4a+ (Novagen, Inc., Milwaukee, WI) and inserted into pET-23d (Novagen, Inc., Milwaukee, WI) as an *Xba-Nco* fragment to give pET-EMCV. The *dhfr* gene was PCR-amplified from pESN2dhfr to give a product with a Gly-Gly-Gly-Ser spacer in place of the translation stop codon and inserted as an *Nco-Bam*H1 fragment to give pET-E-DHFR. Next, the attenuated neo gene was PCR amplified from a pSV2Neo (Clontech, Palo Alto, CA) derivative and inserted into the unique *Bam*H1 site of pET-E-DHFR to give pET-E-DHFR/Neo₍ₘ₂₎. Then, the bovine growth hormone terminator from pCDNA3 (Invitrogen, Inc., Carlsbad, CA) was inserted downstream of the neo gene to give pET-E-DHFR/Neo₍ₘ₂₎BGHt. The EMCV-*dhfr*/*neo* selectable marker cassette fragment was prepared by cleavage of pET-E-DHFR/Neo₍ₘ₂₎BGHt. The CMV enhancer/promoter plus Intron A was transferred from pCMV6a (Chapman et al., *Nuc. Acids Res.* (1991) 19:3979-3986) as a *Hind*III-*Sal*1 fragment into pUC19 (New England Biolabs, Inc., Beverly, MA). The vector backbone of pUC19 was deleted from the Ndel to the Sap1 sites. The above described DHFR cassette was added to the construct such that the EMCV IRES followed the CMV promoter to produce the final construct. The vector also contained an amp^{r} gene and an SV40 origin of replication.

Selected pCMVKm2 vectors containing the synthetic expression cassettes have been designated as follows: pCMVKm2.GagMod.SF2, pCMVKm2.GagprotMod.SF2, and pCMVKm2.GagpolMod.SF2, pCMVKm2.GagprotMod.SF2.GP1 (SEQ ID NO :78) and pCMVKm2.GagprotMod.SF2.GP2 (SEQ ID NO:79). Other exemplary Gag-encoding expressing cassettes are shown in the Figures and as Sequence Listings.

### B. Modification of HIV-1 Gag/Hepatitis C Core Chimeric Protein Nucleic Acid Coding Sequences Generation of Synthetic Expression Cassettes

To facilitate the ligation of the Gag and HCV core coding sequences, PCR amplification was employed. The synthetic p55Gag expression cassette was used as a PCR template with the following primers: GAG5 (SEQ ID NO:11) and P55-SAL3 (SEQ ID NO:12). The PCR amplification was conducted at 55°C for 25 cycles using Stratagene's Pfu polymerase. The resulting PCR product was rendered free of nucleotides and primers using the Promega PCR clean-up kit and then subjected to EcoRI and SalI digestions. For HCV core coding sequences, the following primers were used with an HCV template (Houghton, M., et al., U.S. Patent No. 5,714,596, issued February 3, 1998; Houghton, M., et al., U.S. Patent No. 5,712,088, issued January 27, 1998; Houghton, M., et al., U.S. Patent No. 5,683,864, issued November 4, 1997; Weiner, A.J., et al., U.S. Patent No. 5,728,520, issued March 17, 1998; Weiner, A.J., et al., U.S. Patent No. 5,766,845, issued June 16, 1998; Weiner, A.J., et al., U.S. Patent No. 5,670,152, issued September 23, 1997): CORESAL 5 (SEQ ID NO:13) and 173CORE(SEQ ID NO:14) using the conditions outlined above. The purified product was digested with SalI and BamHI restriction enzymes. The digested Gag and HCV core PCR products were ligated into the pCMVKm2 vector digested with EcoRI and BamHI. Ligation of the PCR products at the SalI site resulted in a direct fusion of the final amino acid of p55Gag to the second amino acid of HCV core, serine. Amino acid 173 of core is a serine and is followed immediately by a TAG termination codon. The sequence of the fusion clone was confirmed. The pCMVKm2 vector containing the synthetic expression cassette was designated as pCMVKm2.GagModHCVcore.

The EcoRI-BamHI fragment of p55Gag-core 173 was also cloned into EcoRI-BamHI-digested pAcC13 for baculovirus expression. Western blots confirmed expression and sucrose gradient sedimentation along with electron microscopy confirmed particle formation. To generate the above clone but containing the synthetic Gag sequences (instead of wild-type), the following steps were performed: pCMVKm2-modified p55Gag was used as template for PCR amplification with MS65 (SEQ ID NO:15) and MS66(SEQ ID NO:16) primers. The region amplified corresponds to the BspHI and SalI sites at the C-terminus of synthetic Gag sequence. The amplification product was digested with BspHI and SalI and ligated to SalI/BamHI digested pCMV-link along with the Sal/BspHI fragment from pCMV-Km-p55modGag , representing the amino terminal end of modified Gag, and the SalI/BamHI fragment from pCMV-p55Gag-core173. Thereafter, a T4-blunted-SalI partial/BamHI fragment was ligated into pAcC4-SmaI/BamHI to generate pAcC4-p55GagMod-core173 (containing the synthetic sequence presented as SEQ ID NO:7).

### C. Defining of the Major Homology Region (MHR) of HIV-1 p55Gag

The Major Homology Region (MHR) of HIV-1 p55 (Gag) is located in the p24-CA sequence of Gag. It is a conserved stretch of 20 amino acids (SEQ ID NO:19). The position in the wild type HIV-1_{SF2} Gag protein is from aa 286-305 and spans a region from nucleotides 856-915 in the native HIV-1_{SF2} Gag DNA-sequence. The position in the synthetic Gag protein is from aa 288-307 and spans a region from nucleotides 862-921 for the synthetic Gag DNA-sequence. The nucleotide sequence for the MHR in the synthetic GagMod.SF2 is presented as SEQ ID NO:20. Mutations or deletions in the amino acid sequence of the MHR can severely impair particle production (Borsetti, A., et al., *J. Virol.* 72(11):9313-9317, 1998; Mammano, F., et al., *J Virol* 68 (8) :4927-4936, 1994).

Percent identity to the MHR nucleotide sequence can be determined, for example, using the MacDNAsis program (Hitachi Software Engineering America Limited, South San Francisco, CA), Higgins algorithm, with the following exemplary parameters: gap penalty = 5, no. of top diagonals = 5, fixed gap penalty = 5, K-tuple = 2, window size = 5, and floating gap penalty = 10.

### D. Generation of Synthetic Env Expression Cassettes

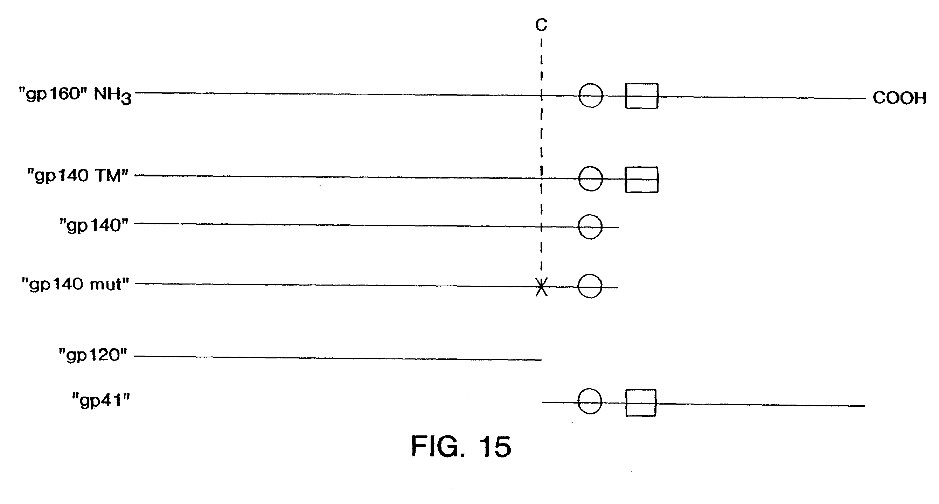

Env coding sequences of the present invention include, but are not limited to, polynucleotide sequences encoding the following HIV-encoded polypeptides: gp160, gp140, and gp120 (see, e.g., U.S. Patent No. 5,792,459 for a description of the HIV-1_{SF2} ("SF2") Env polypeptide). The relationships between these polypeptides is shown schematically in Figure 15 (in the figure: the polypeptides are indicated as lines, the amino and carboxy termini are indicated on the gp160 line; the open circle represents the oligomerization domain; the open square represents a transmembrane spanning domain (TM); and "c" represents the location of a cleavage site, in gp140.mut the "X" indicates that the cleavage site has been mutated such that it no longer functions as a cleavage site). The polypeptide gp160 includes the coding sequences for gp120 and gp41. The polypeptide gp41 is comprised of several domains including an oligomerization domain (OD) and a transmembrane spanning domain (TM). In the native envelope, the oligomerization domain is required for the non-covalent association of three gp41 polypeptides to form a trimeric structure: through non-covalent interactions with the gp41 trimer (and itself), the gp120 polypeptides are also organized in a trimeric structure. A cleavage site (or cleavage sites) exists approximately between the polypeptide sequences for gp120 and the polypeptide sequences corresponding to gp41. This cleavage site(s) can be mutated to prevent cleavage at the site. The resulting gp140 polypeptide corresponds to a truncated form of gp160 where the transmembrane spanning domain of gp41 has been deleted. This gp140 polypeptide can exist in both monomeric and oligomeric (i.e. trimeric) forms by virtue of the presence of the oligomerization domain in the gp41 moiety. In the situation where the cleavage site has been mutated to prevent cleavage and the transmembrane portion of gp41 has been deleted the resulting polypeptide product is designated "mutated" gp140 (e.g., gp140.mut). As will be apparent to those in the field, the cleavage site can be mutated in a variety of ways. The native amino acid sequence in the SF162 cleavage sites is: APTKAKRRVVQREKR (SEQ ID NO:21), where KAKRR (SEQ ID NO:22) is termed the "second" site and REKR (SEQ ID NO:23) is the "first site". Exemplary mutations include the following constructs: gp140.mut7.modSF162 which encodes the amino acid sequence APTKAISSWQSEKS (SEQ ID NO:24) in the cleavage site region; gp140.mut8.modSF162 which encodes the amino acid sequence APTIAISSVVQSEKS (SEQ ID NO:25) in the cleavage site region and gp140mut.modSF162 which encodes the amino acid sequence APTKAKRRVVQREKS (SEQ ID NO:26). Mutations are denoted in bold. The native amino acid sequence in the US4 cleavage sites is: APTQAKRRWQREKR (SEQ ID NO:27), where QAKRR (SEQ ID NO:28) is termed the "second" site and REKR (SEQ ID No:23) is the "first site". Exemplary mutations include the following construct: gp140.mut.modUS4 which encodes the amino acid sequence APTQAKRRVVQREKS (SEQ ID NO:29) in the cleavage site region. Mutations are denoted in bold.

### E. Modification of HIV-1 Env (Envelope) Nucleic Acid Coding Sequences

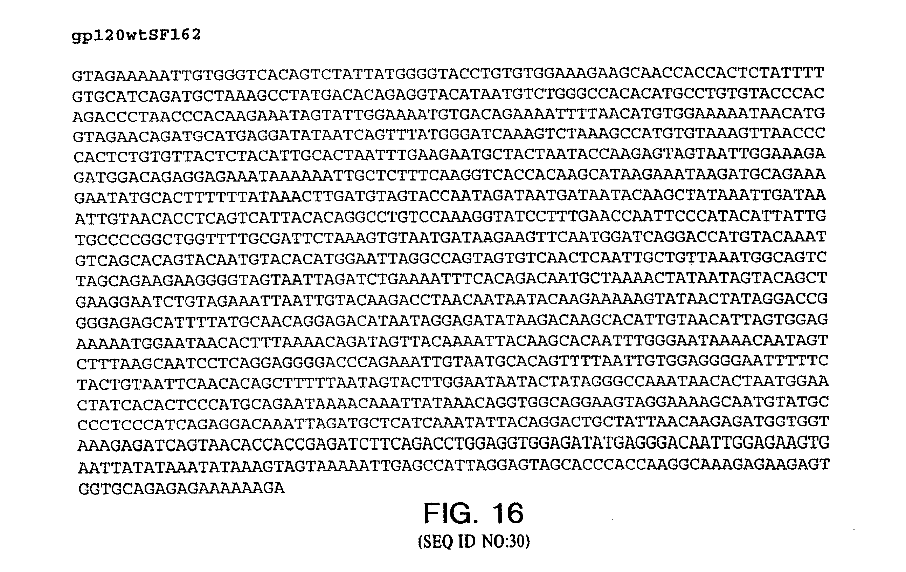

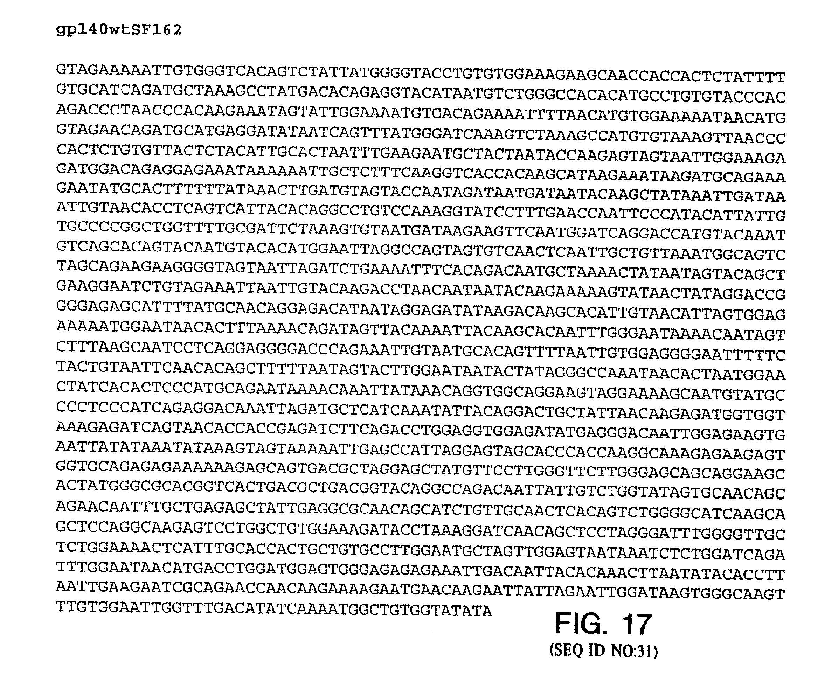

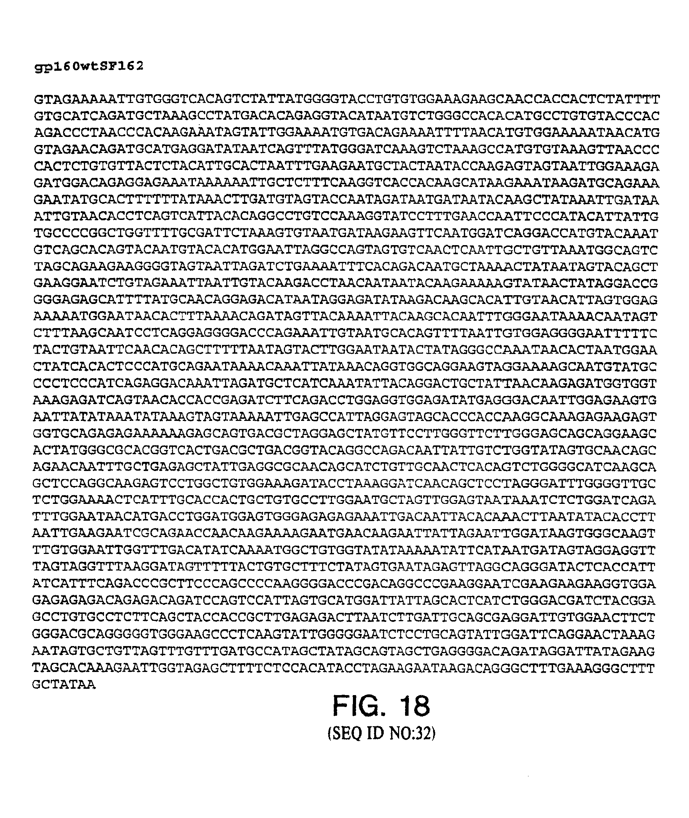

In one embodiment of the present invention, wild-type Env coding sequences were selected from the HTV-1_{SF162} ("SF162") strain (Cheng-Mayer (1989) *PNAS USA* 86:8575-8579). These SF162 sequences were as follows: gp120, SEQ ID NO:30 (Fig. 16); gp140, SEQ ID N0:31 (Fig. 17); and gp160, SEQ ID NO:32 (Fig. 18).

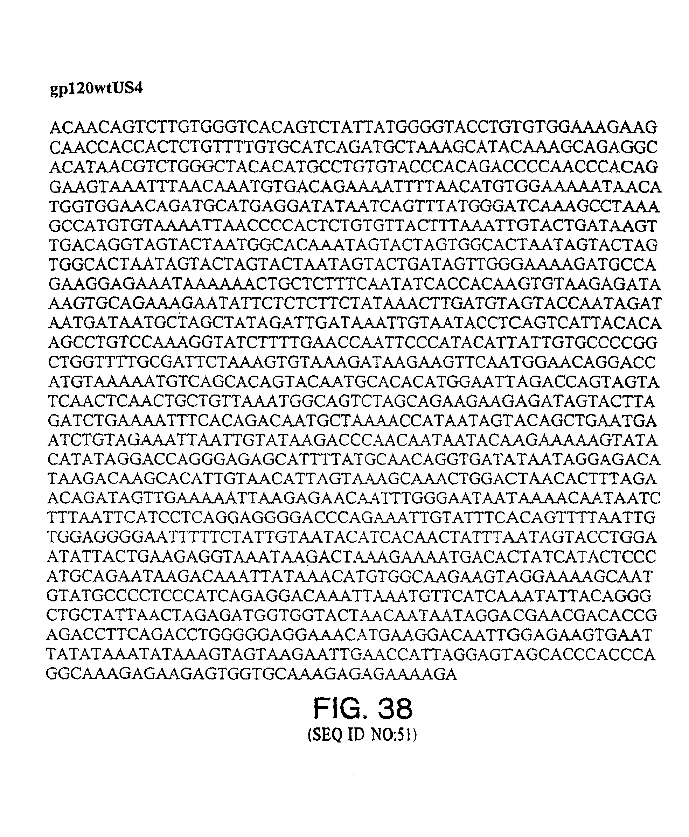

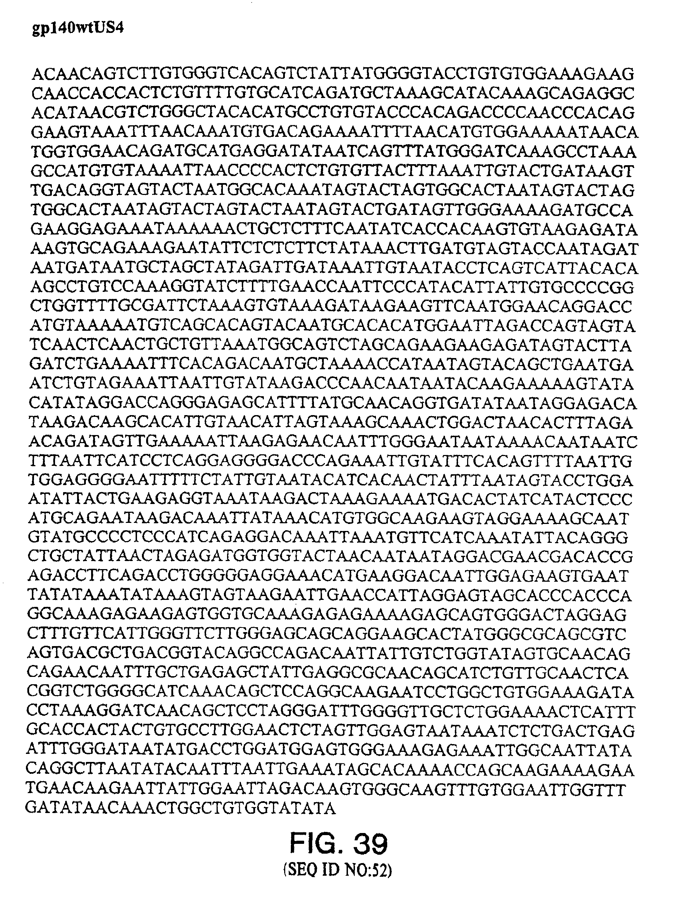

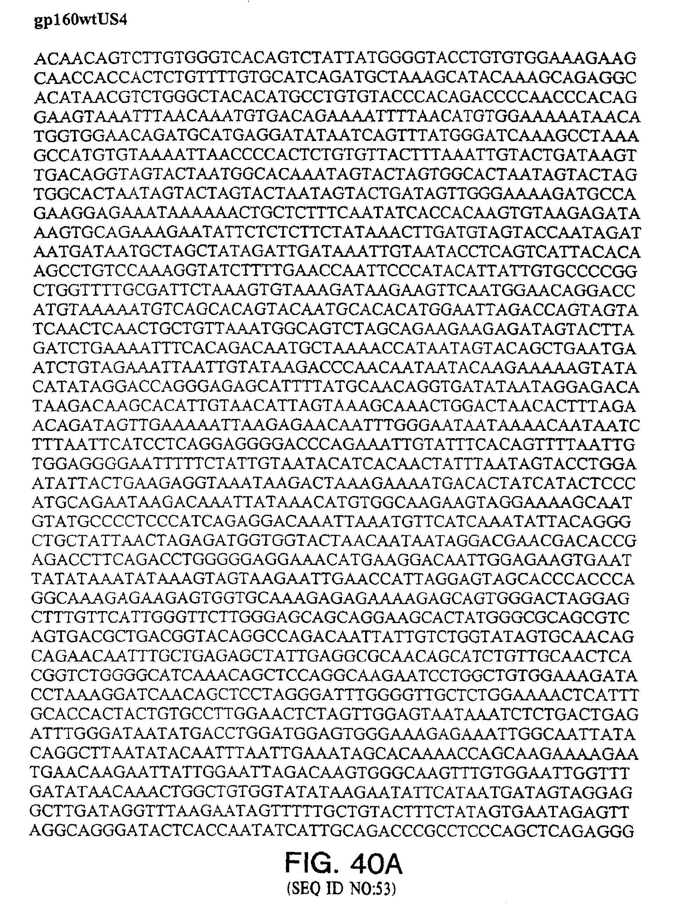

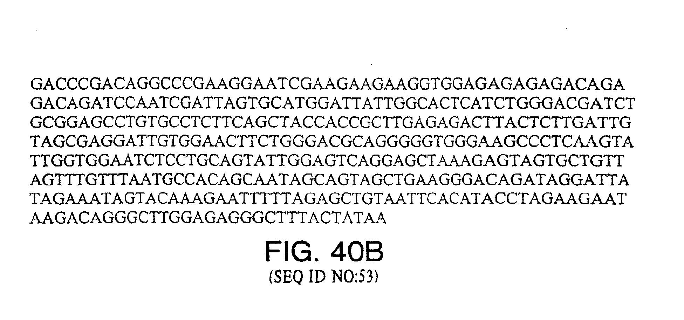

In another embodiment of the present invention, wild-type Env coding sequences were selected from the HIV-US4 strain (Mascola, et al. (1994) *J. Infect. Dis.* 169:48-54). These US4 sequences were as follows: gp120, SEQ ID NO:51 (Fig. 38); gp140, SEQ ID NO:52 (Fig. 39); and gp160, SEQ ID NO:53 (Fig. 40).

These Env coding sequences were manipulated to maximize expression of their gene products.

First, the wild-type coding region was modified in one or more of the following ways. In one embodiment, sequences encoding hypervariable regions of Env, particularly V1 and/or V2 were deleted. In other embodiments, mutations were introduced into sequences encoding the cleavage site in Env to abrogate the enzymatic cleavage of oligomeric gp140 into gp120 monomers. (See, e.g., Earl et al. (1990) *PNAS USA* 87:648-652; Earl et al. (1991) *J. Virol.* 65:31-41). In yet other embodiments, hypervariable region(s) were deleted, N-glycosylation sites were removed and/or cleavage sites mutated.

Second, the HIV-1 codon usage pattern was modified so that the resulting nucleic acid coding sequence was comparable to codon usage found in highly expressed human genes. The HIV codon usage reflects a high content of the nucleotides A or T in the codon-triplet. The effect of the HIV-1 codon usage is a high AT content in the DNA sequence that results in a decreased translation ability and instability of the mRNA. In comparison, highly expressed human codons prefer the nucleotides G or C. The Env coding sequences were modified to be comparable to codon usage found in highly expressed human genes.

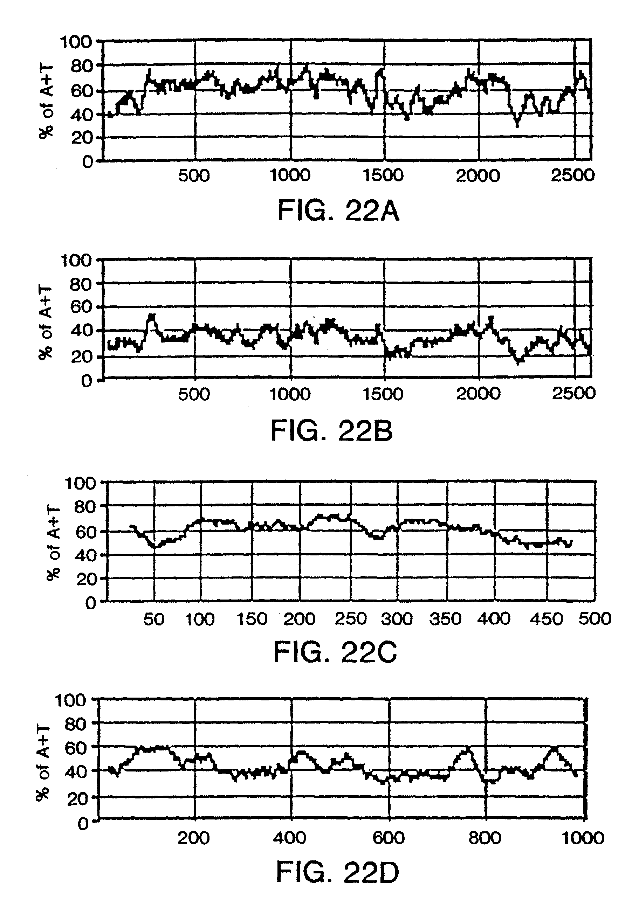

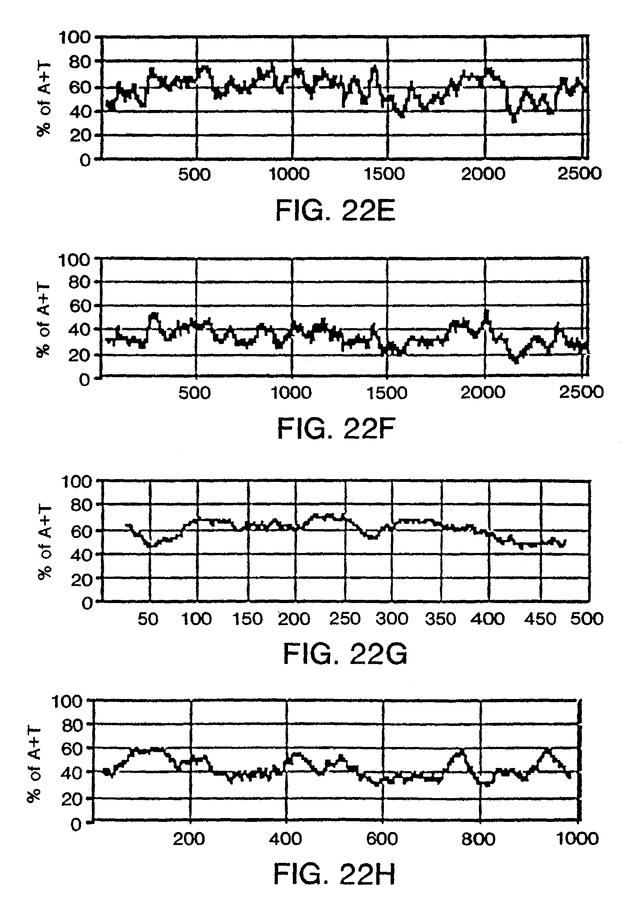

Figures 22A-22H present comparisons of the percent A-T content for the cDNAs of stable versus unstable RNAs (comparison window size = 50). Human IFNγ mRNA is known to (i) be unstable, (ii) have a short half-life, and (iii) have a high A-U content. Human GAPDH (glyceraldehyde-3-phosphate dehydrogenase) mRNA is known to (i) be a stable RNA, and (i) have a low A-U content. In Figures 22A-H, the percent A-T content of these two sequences are compared to the percent A-T content of (1) native HIV-1 US4 Env gp160 cDNA, a synthetic US4 Env gp160 cDNA sequence (i.e., having modified codons) of the present invention; and (2) native HIV-1 SF162 Env gp160 cDNA, a synthetic SF162 Env gp160 cDNA sequence (i.e., having modified codons) of the present invention. Figures 22A-H show the percent A-T content over the length of the sequences for IFNγ (Figures 22C and 22G); native gp160 Env US4 and SF162 (Figures 22A and 22E, respectively); GAPDH (Figures 22D and 22H); and the synthetic gp160 Env for US4 and SF162 (Figures 22B and 22F). Experiments performed in support of the present invention showed that the synthetic Env sequences were capable of higher level of protein production (see the Examples) than the native Env sequences. The data in Figures 22A-H suggest that one reason for this increased production is increased stability of the mRNA corresponding to the synthetic Env coding sequences versus the mRNA corresponding to the native Env coding sequences.

To create the synthetic coding sequences of the present invention the gene cassettes were designed to comprise the entire coding sequence of interest. Synthetic gene cassettes were constructed by oligonucleotide synthesis and PCR amplification to generate gene fragments. Primers were chosen to provide convenient restriction sites for subcloning. The resulting fragments were then ligated to create the entire desired sequence which was then cloned into an appropriate vector. The final synthetic sequences were (i) screened by restriction endonuclease digestion and analysis, (ii) subjected to DNA sequencing in order to confirm that the desired sequence had been obtained and (iii) the identity and integrity of the expressed protein confirmed by SDS-PAGE and Western blotting (See, Examples. The synthetic coding sequences were assembled at Chiron Corp. or by the Midland Certified Reagent Company (Midland, Texas).

Exemplary modified coding sequences are presented as synthetic Env expression cassettes in Table 1A and 1B. The following expression cassettes (i) have unique, terminal *EcoRI* and *XbaI* cloning sites; (ii) include Kozak sequences to promote optimal translation; (iii) tPA signal sequences (to direct the ENV polypeptide to the cell membrane, see, e.g., Chapman et al., infra); (iv) open reading frames optimized for expression in mammalian cells; and (v) a translational stop signal codon.

**Table 1A:**

| Exemplary Synthetic Env Expression Cassettes (SF162) | | |
|---|---|---|
| Expression Cassette | Seq Id | Further Information |
| gp120 SF162 | 30 | wild-type; Figure 16 |
| gp140 SF162 | 31 | wild-type; Figure 17 |
| gp160 SF162 | 32 | wild-type; Figure 18 |
| gp120.modSF162 | 33 | none; Figure 19 |
| gp120.modSF162.delV2 | 34 | deleted V2 loop; Figure 20 |
| gp120.modSF162.delV1/V2 | 35 | deleted V1 and V2; Figure 21 |
| gp140.modSF162 | 36 | none; Figure 23 |
| gp140.modSF162.delV2 | 37 | deleted V2 loop; Figure 24 |
| gp140.modSF162.delV1/V2 | 38 | deleted V1 and V2; Figure 25 |
| gp140.mut.modSF162 | 39 | mutated cleavage site; Fig. 26 |
| gp140.mut.modSF162.delV2 | 40 | deleted V2; mutated cleavage site; Figure 27 |
| gp140.mut.modSF162.delV1/V 2 | 41 | deleted V1 & V2; mutated cleavage site; Figure 28 |
| gp140.mut7.modSF162 | 42 | mutated cleavage site; Fig. 29 |
| gp140.mut7.modSF162.delV2 | 43 | mutated cleavage site; deleted V2; Figure 30 |
| gp140.mut7.modSF162.delV1/ V2 | 44 | mutated cleavage site; deleted V1 and V2; Figure 31 |
| gp140.mut8.modSF162 | 45 | mutated cleavage site; Fig. 32 |
| gp140.mut8.modSF162.delV2 | 46 | mutated cleavage site; deleted V2; Figure 33 |
| gp140.mut8.modSF162.delV1/ V2 | 47 | mutated cleavage site; deleted V1 and V2; Figure 34 |
| gp160.modSF162 | 48 | none; Figure 35 |
| gp160.modSF162.de1V2 | 49 | deleted V2 loop; Figure 36 |
| gp160.modSF162.delV1/V2 | 50 | deleted V1 & V2; Figure 37 |

**Table 1B:**

| Exemplary Synthetic Env Expression Cassettes (US4) | | |
|---|---|---|
| Expression Cassette | Seq Id | Further Information |
| gp120 US4 | 51 | wild-type; Figure 38 |
| gp140 US4 | 52 | wild-type; Figure 39 |
| gp160 US4 | 53 | wild-type; Figure 40 |
| gp120.modUS4 | 54 | none; Figure 41 |
| gp120.modUS4.del 128-194 | 55 | deletion in V1 and V2 regions; Figure 42 |
| gp140.modUS4 | 56 | none; Figure 43 |
| gp140.mut.modUS4 | 57 | mutated cleavage site; Figure 44 |
| gp140TM.modUS4 | 58 | native transmembrane region; Figure 45 |
| gp140.modUS4.delV1/V2 | 59 | deleted V1 and V2; Figure 46 |
| gp140.modUS4.delV2 | 60 | deleted V1; Figure 47 |
| gp140.mut.modUS4.delV1/V2 | 61 | mutated cleavage site; deleted V1 and V2; Figure 48 |
| gp140.modUS4.del 128-194 | 62 | deletion in V1 and V2 regions; Figure 49 |
| gp140.mut.modUS4.del 128-194 | 63 | mutated cleavage site; deletion in V1 and V2 regions; Figure 50 |
| gp160.modUS4 | 64 | none; Figure 51 |
| gp160.modUS4.delV1 | 65 | deleted V1; Figure 52 |
| gp160.modUS4.delV2 | 66 | deleted V2; Figure 53 |
| gp160.modUS4.delV1/V2 | 67 | deleted V1 and V2; Figure 54 |
| gp160.modUS4del 128-194 | 68 | deletion in V1 and V2 regions; Figure 55 |

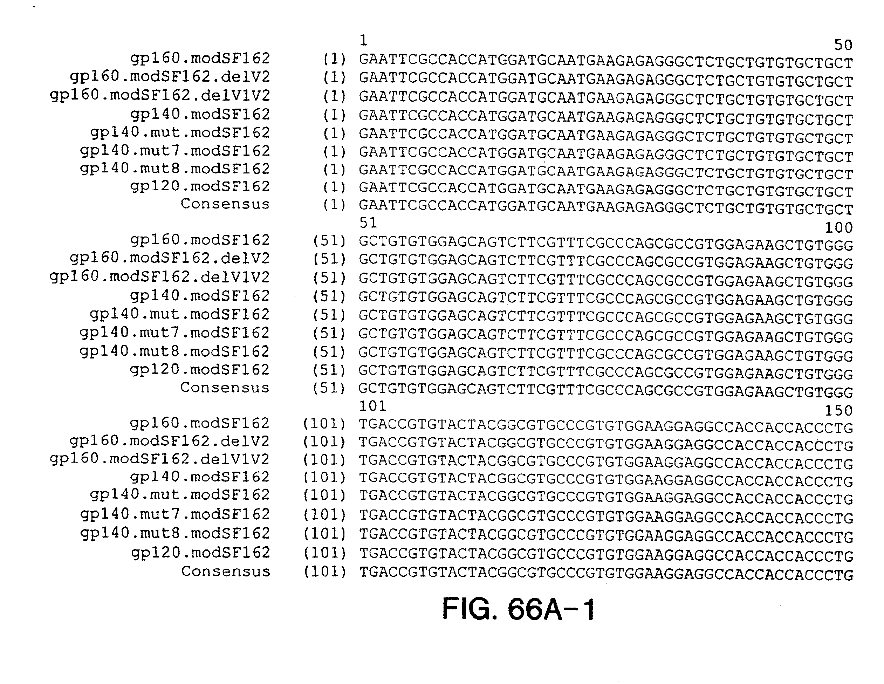

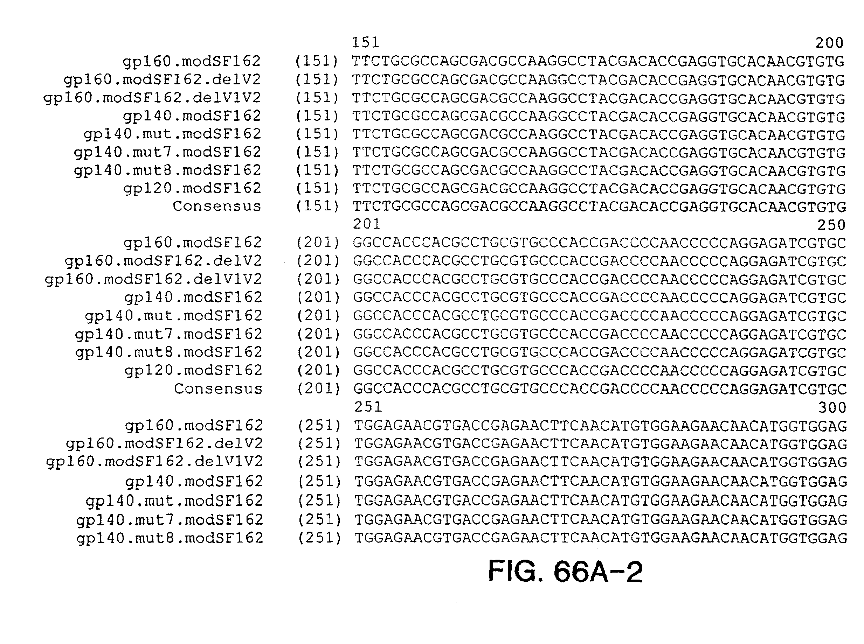

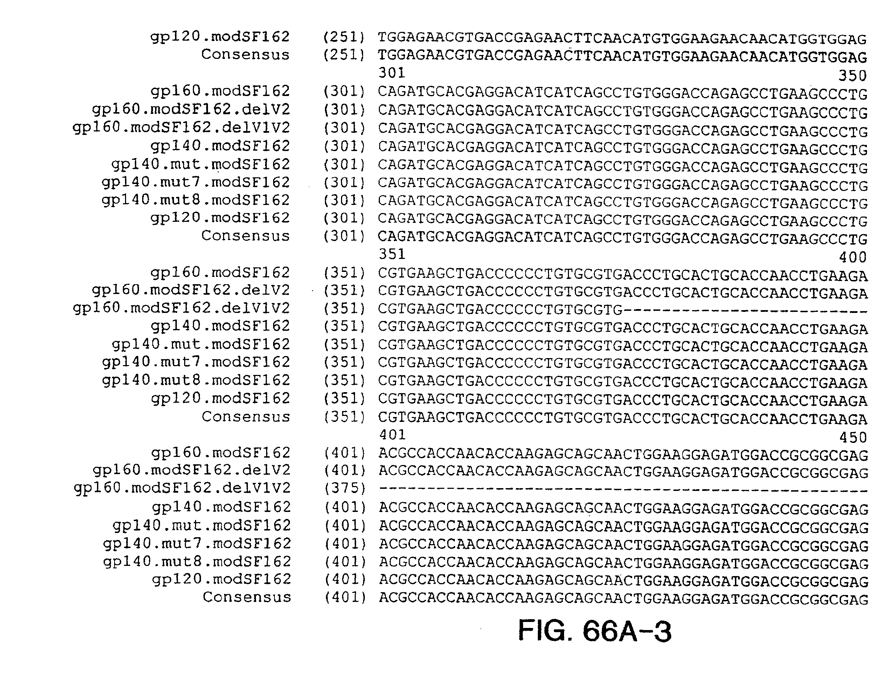

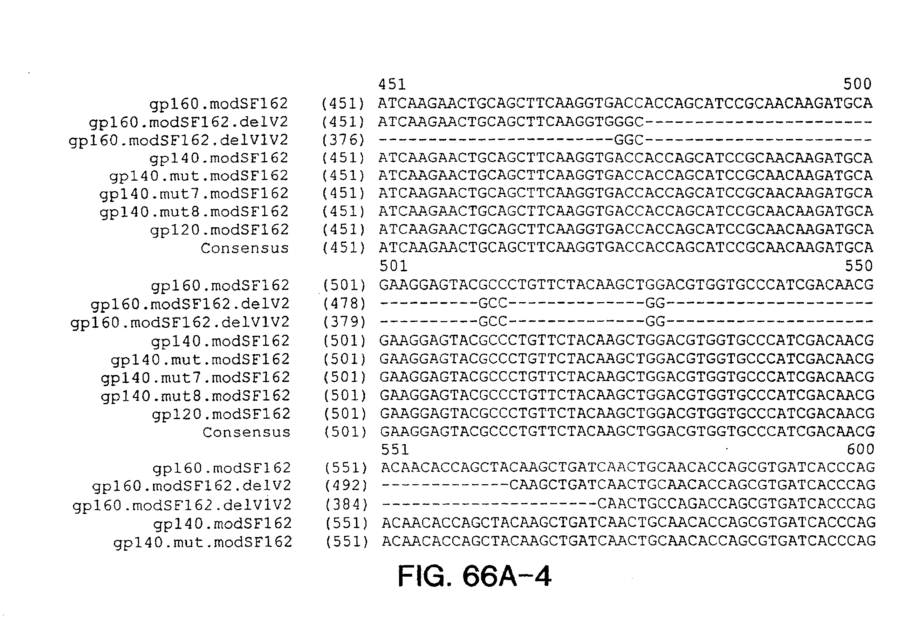

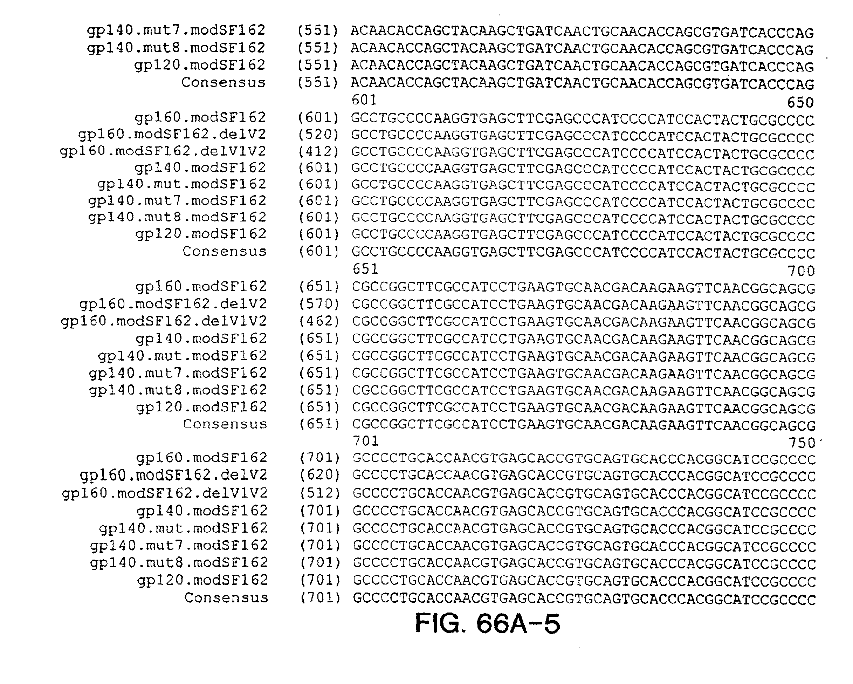

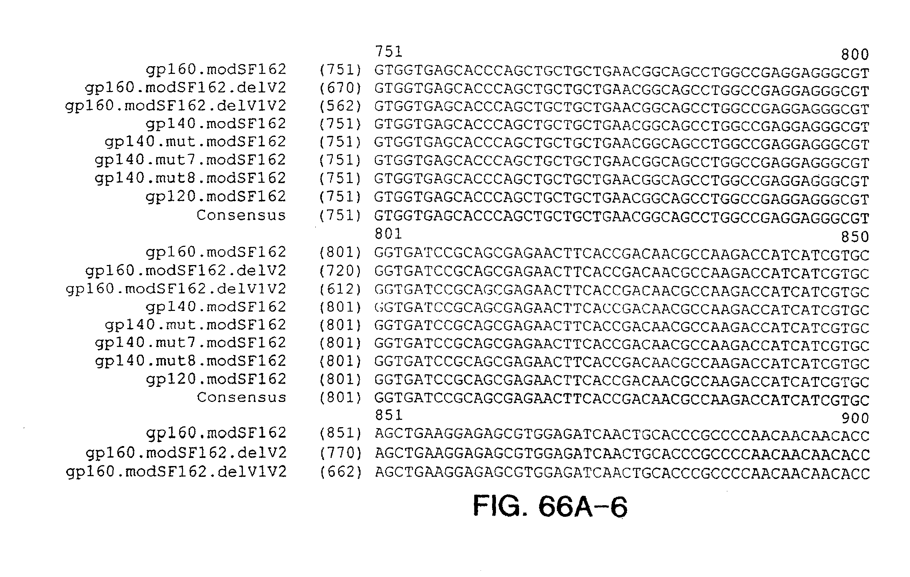

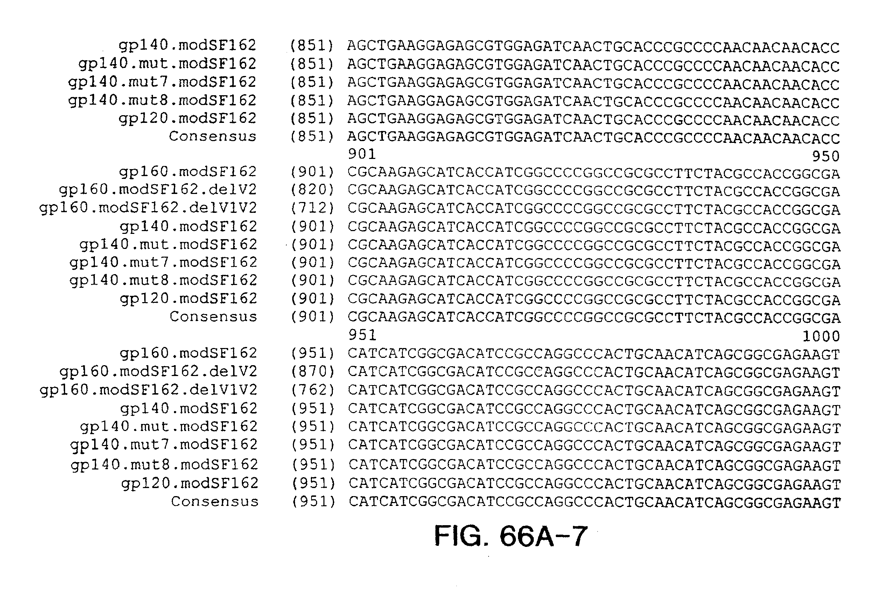

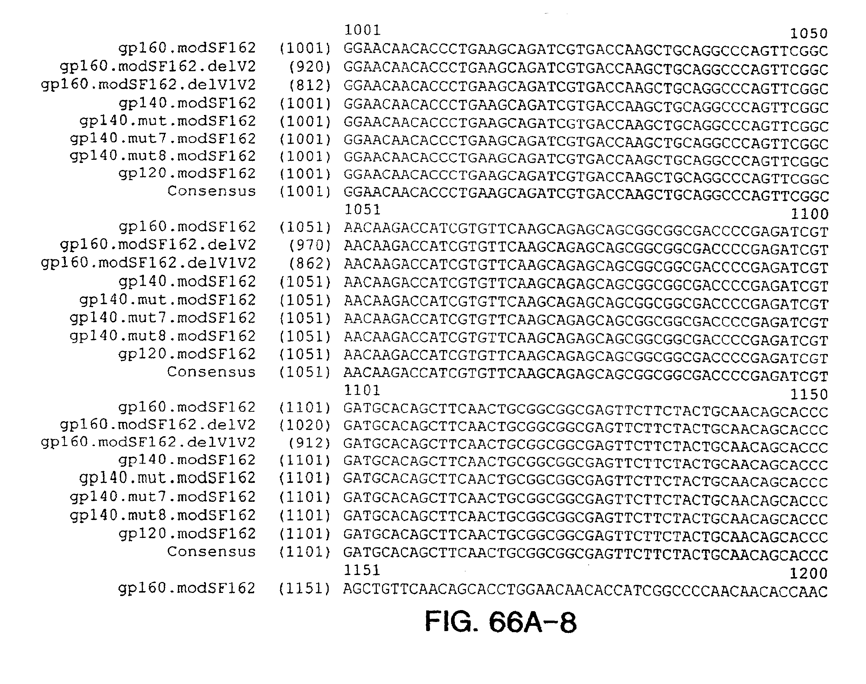

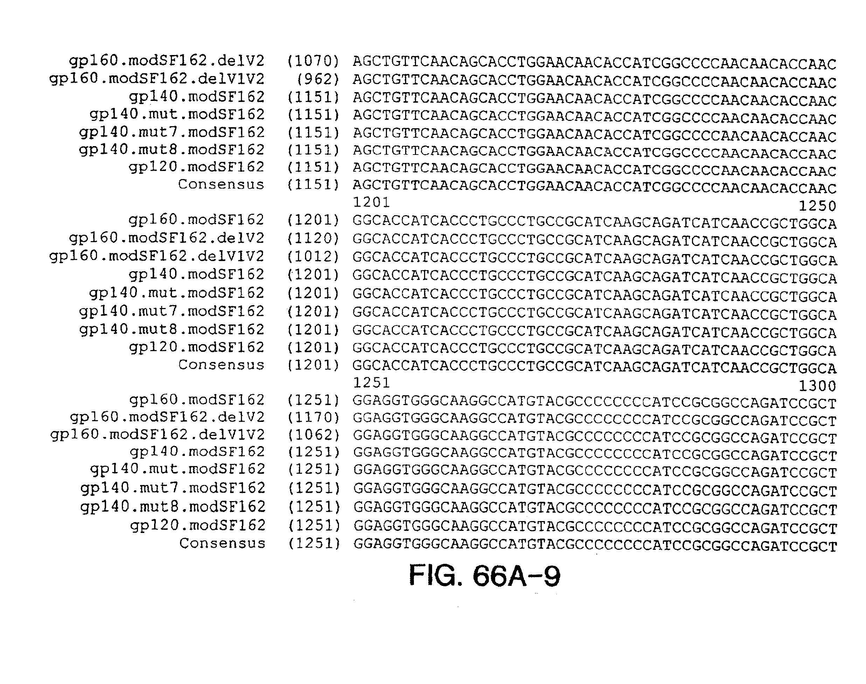

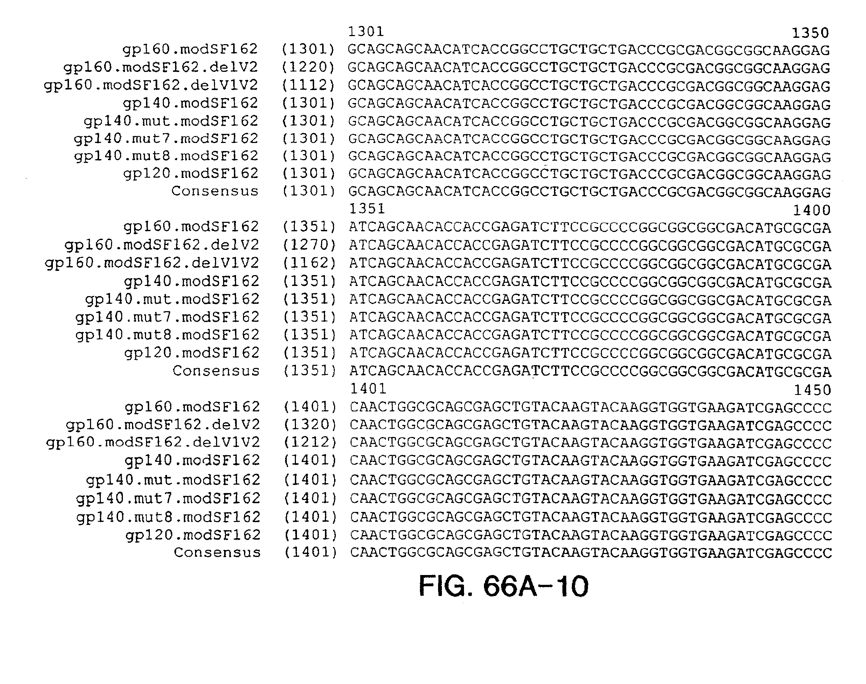

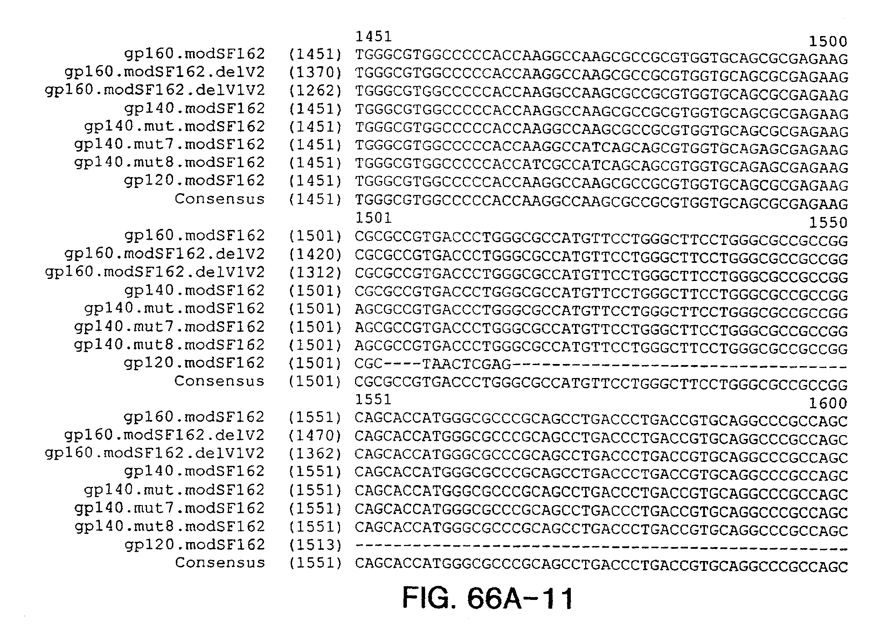

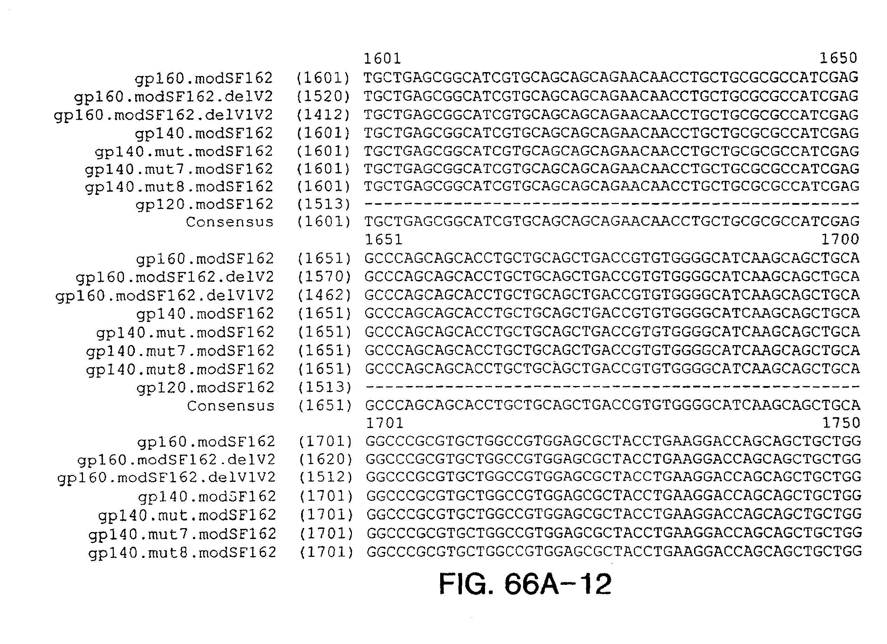

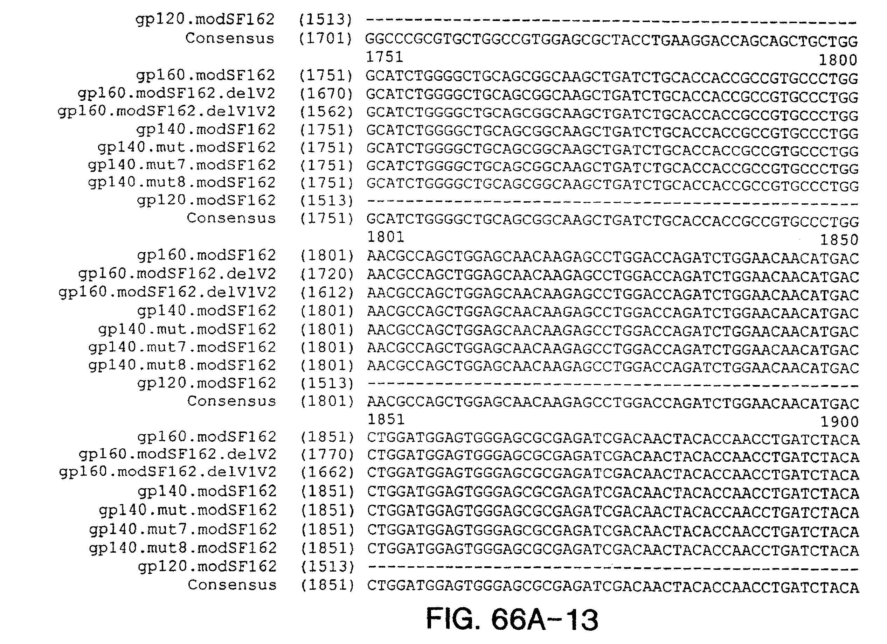

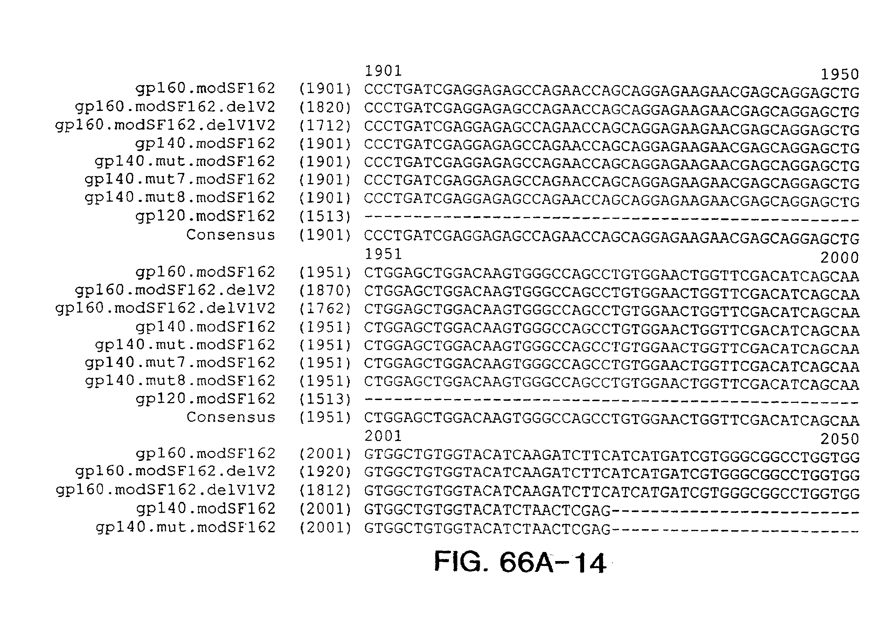

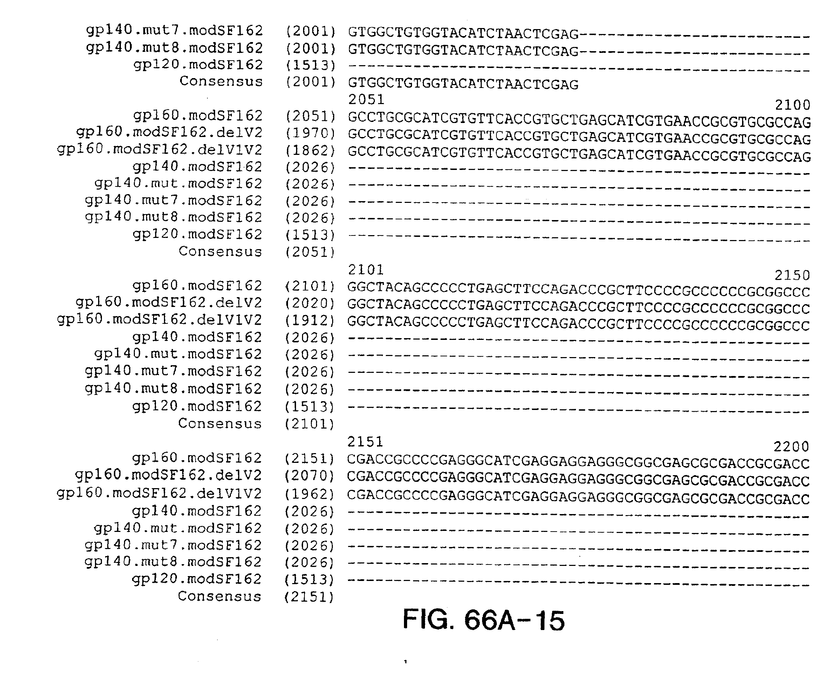

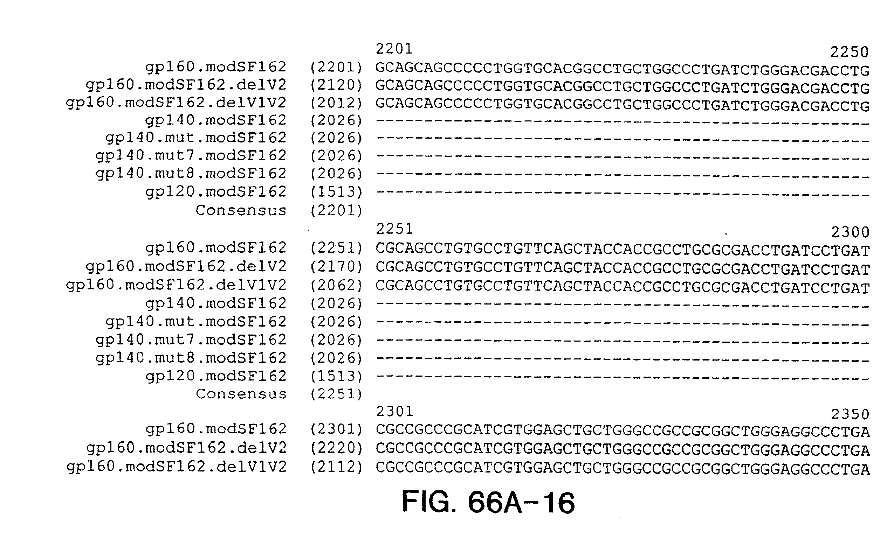

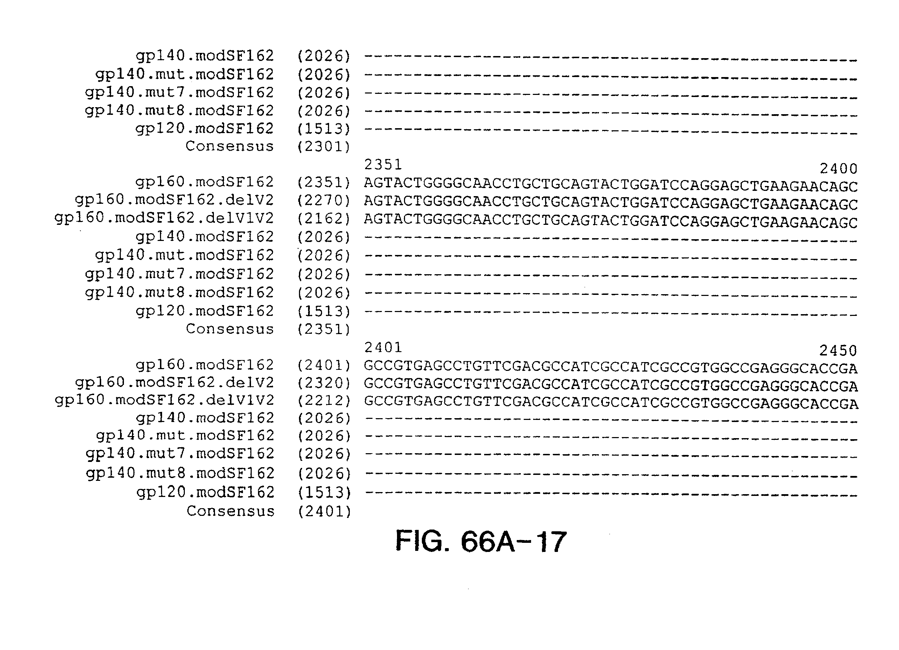

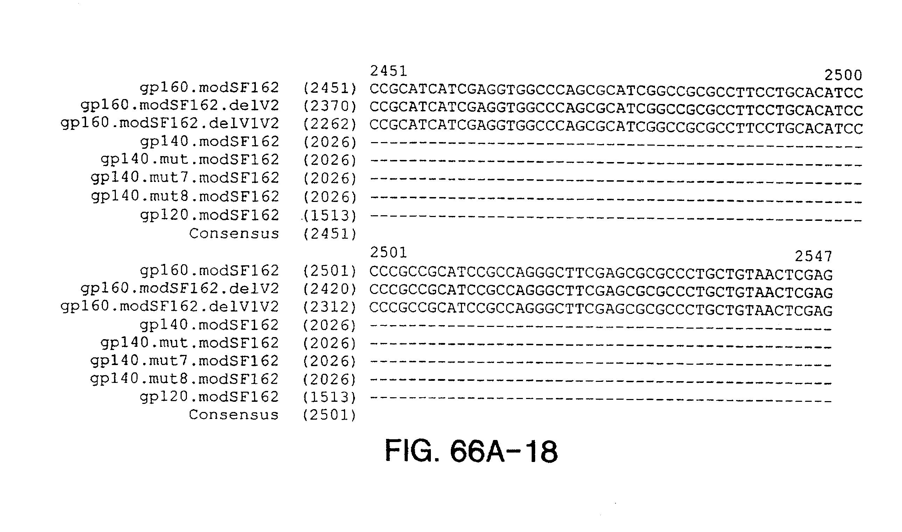

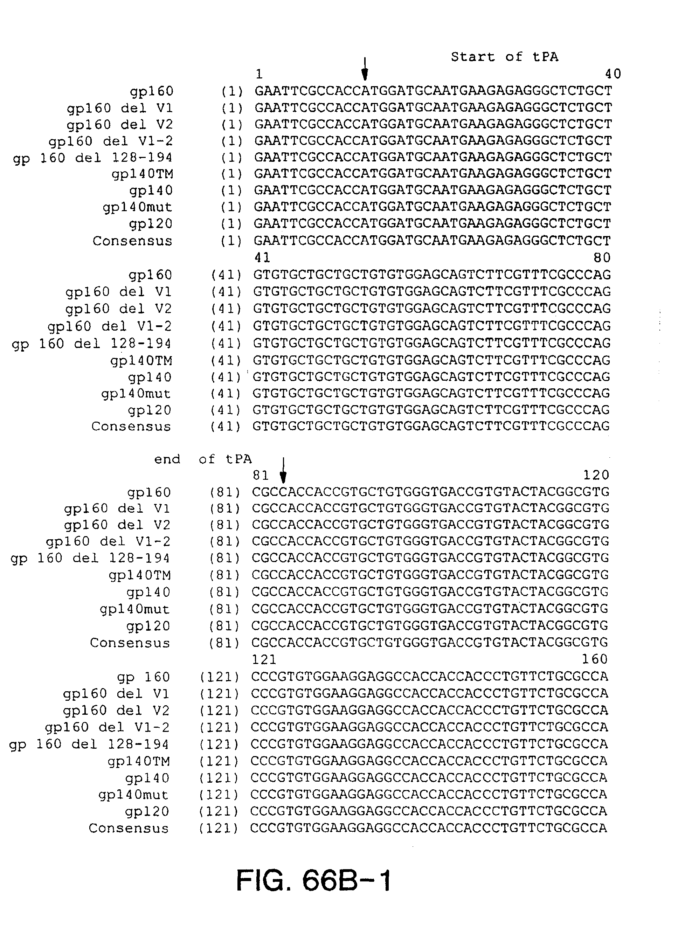

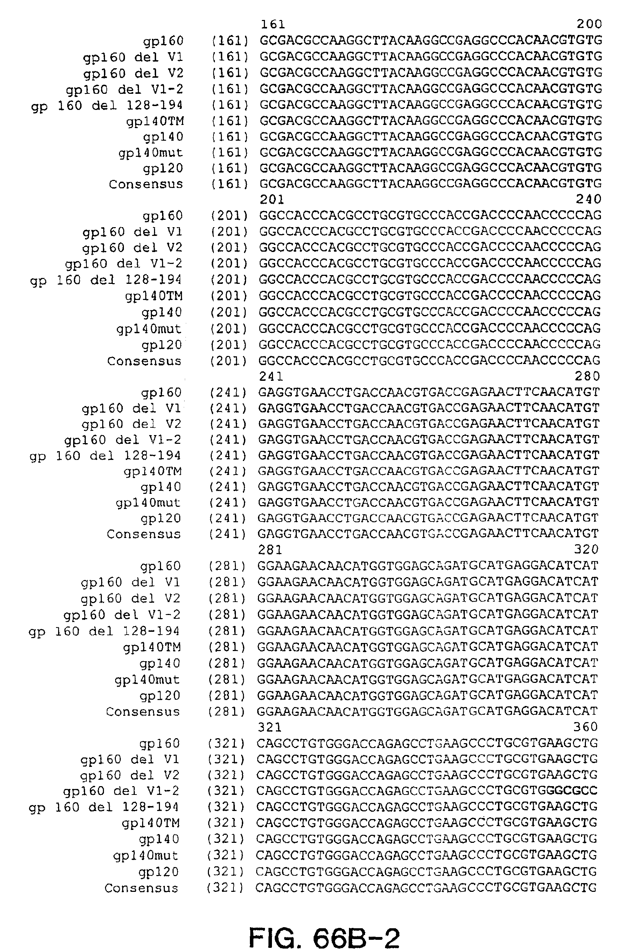

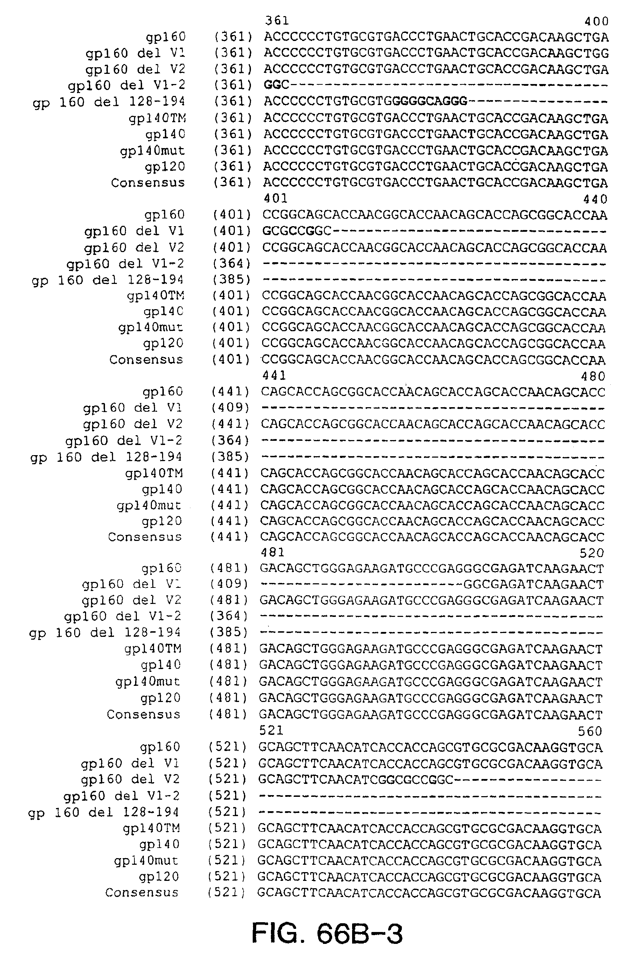

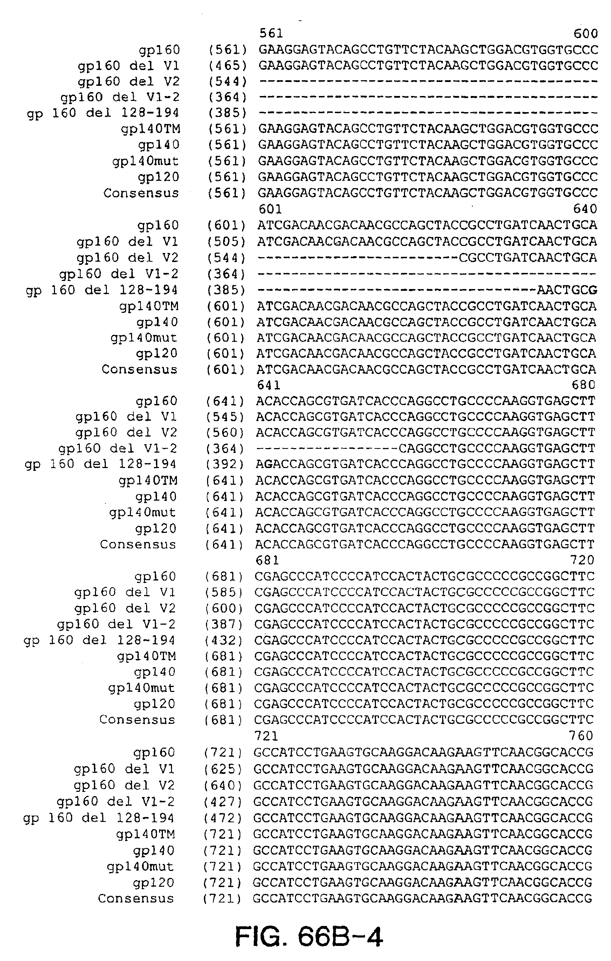

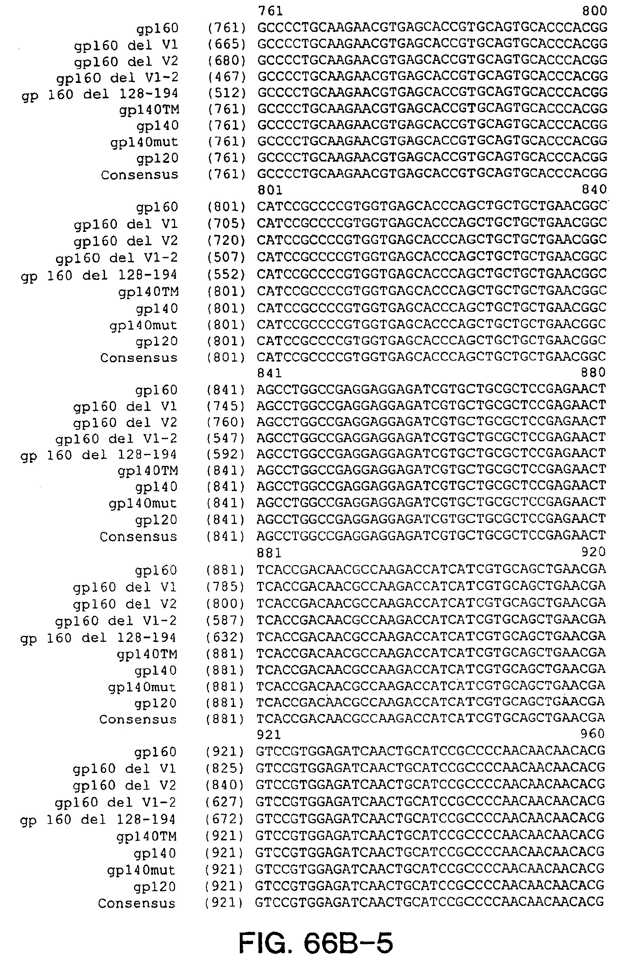

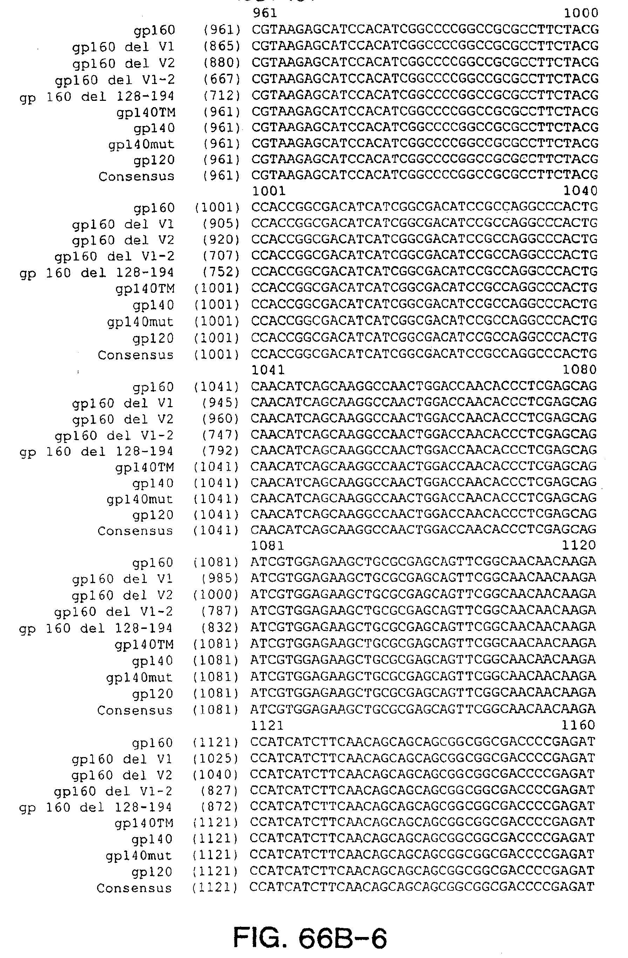

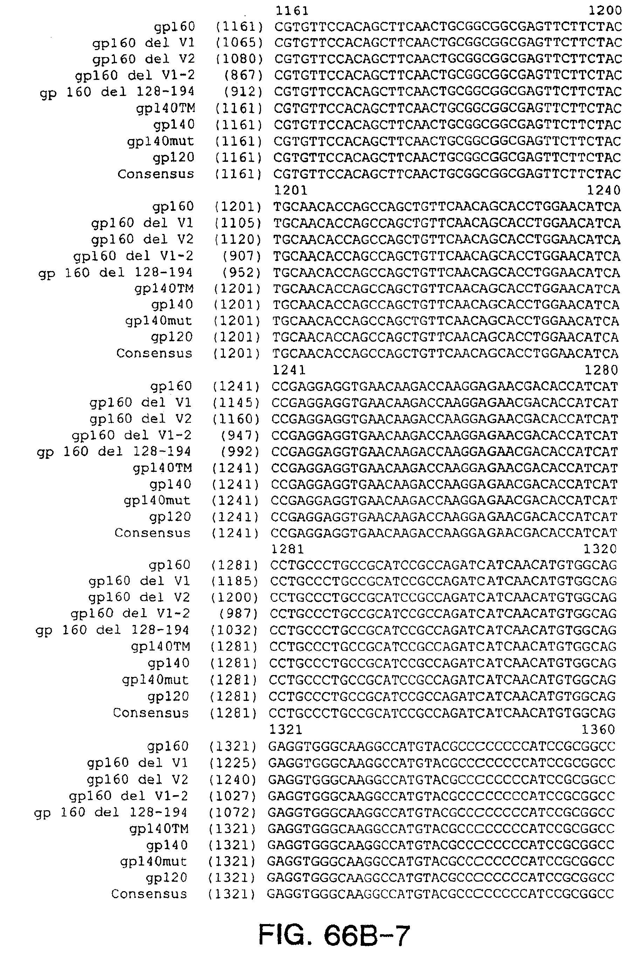

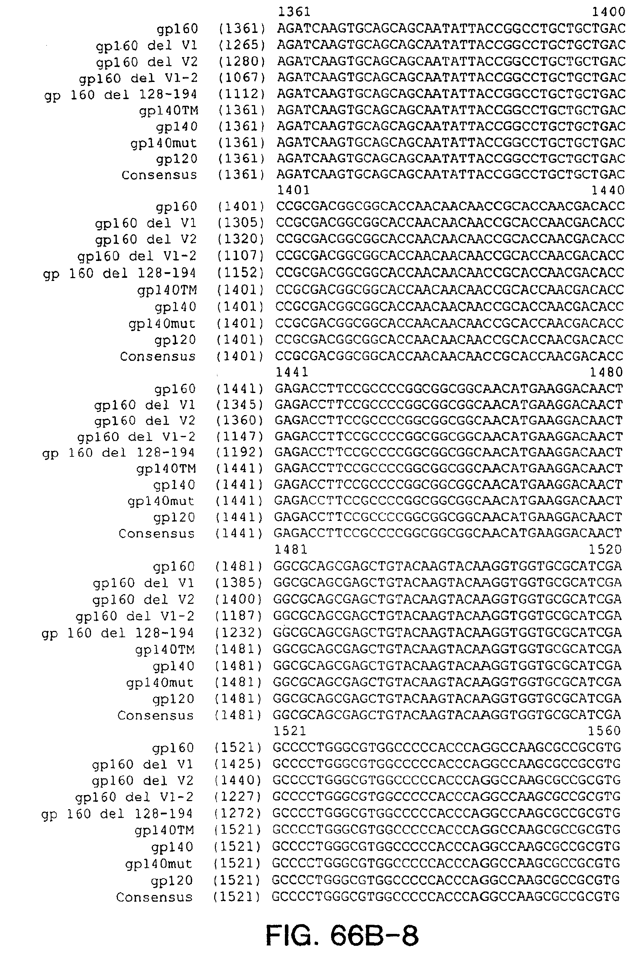

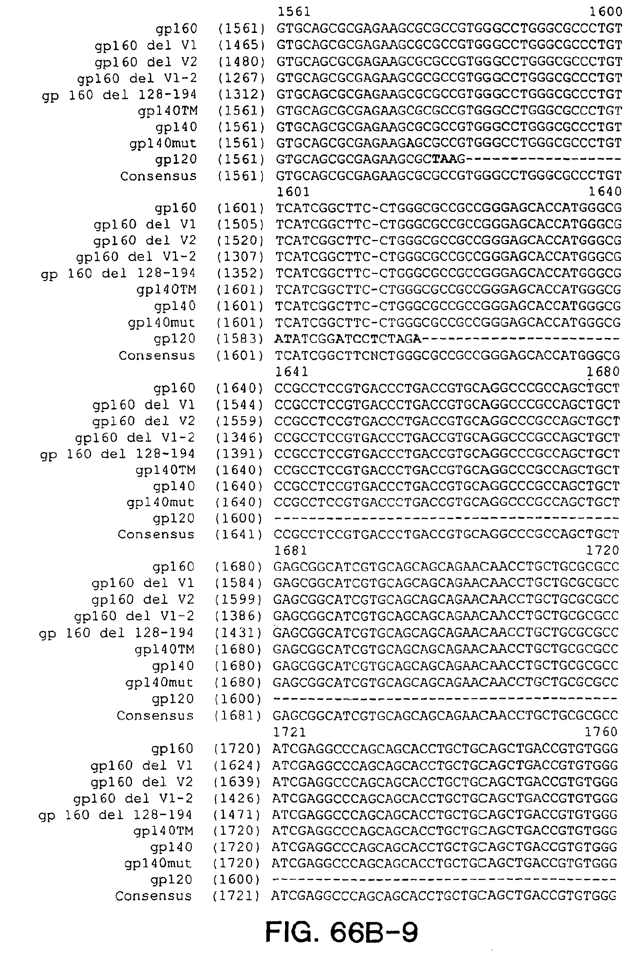

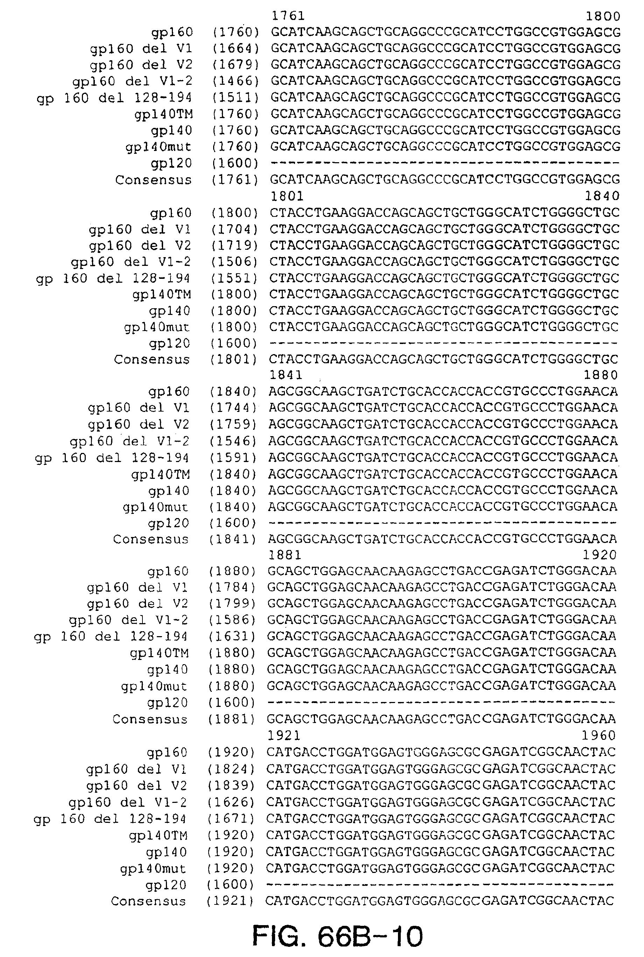

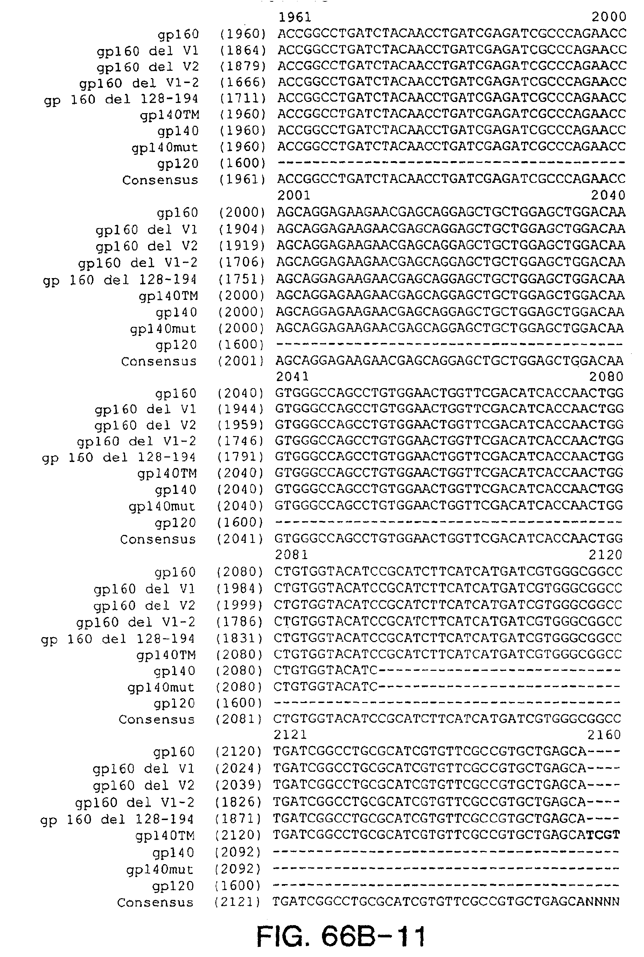

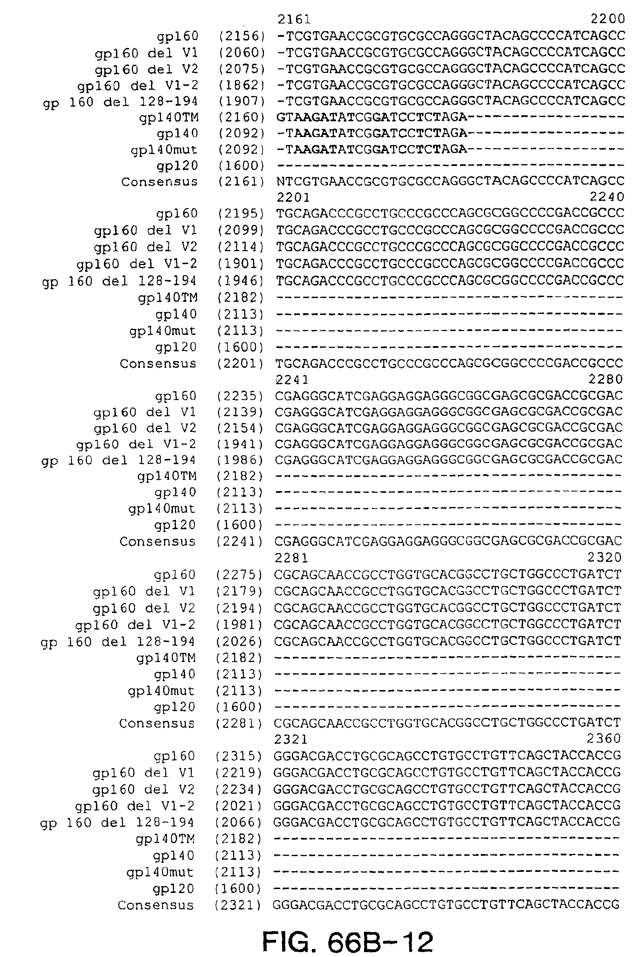

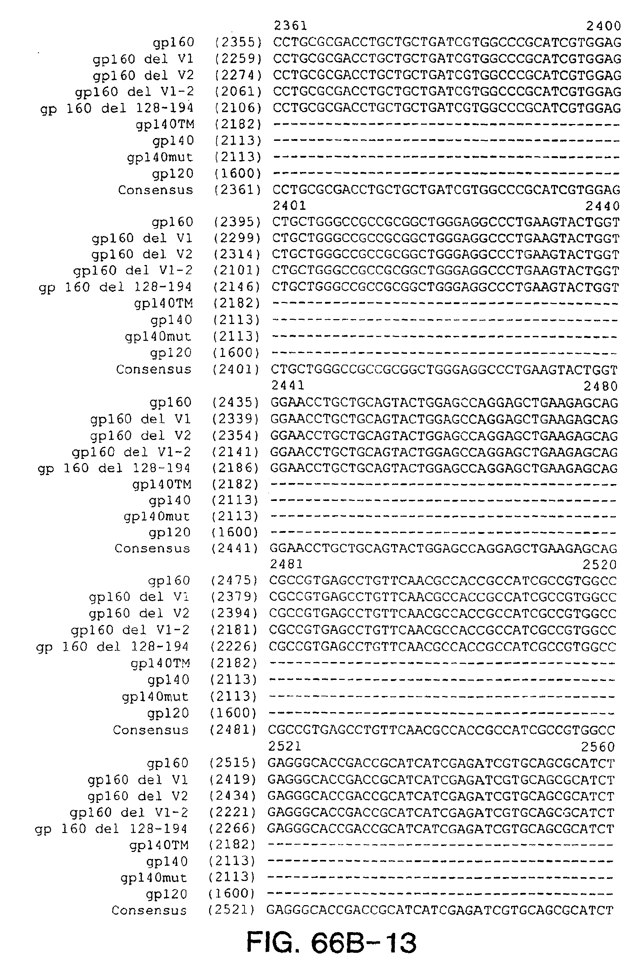

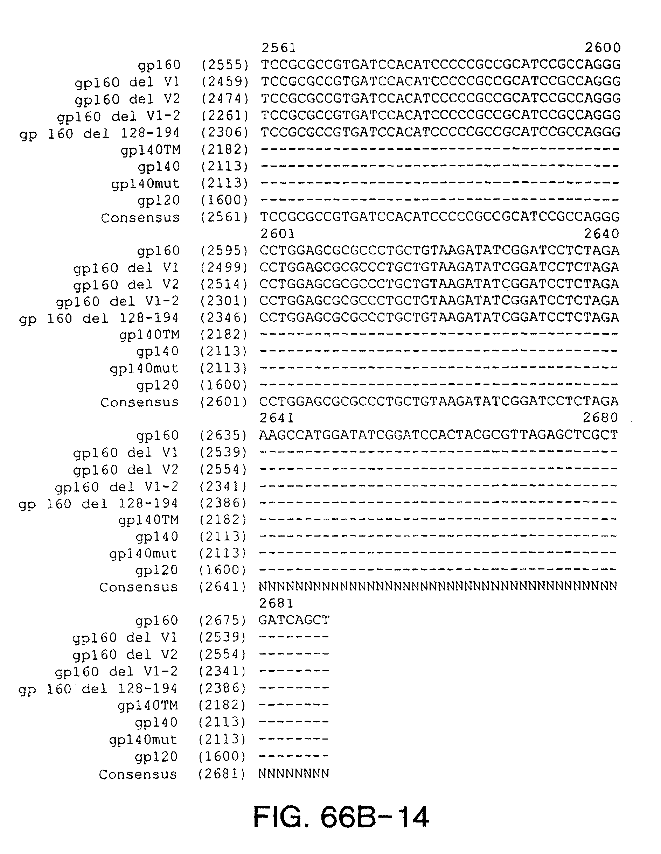

Alignments of the sequences presented in the above tables are presented in Figures 66A and 66B.

A common region (Env-common) extends from nucleotide position 1186 to nucleotide position 1329 (SEQ ID NO:69, Fig. 56) relative to the wild-type US4 sequence and from nucleotide position 1117 to position 1260 (SEQ ID NO:79, Fig. 57) relative to the wild-type SF162 sequence. The synthetic sequences of the present invention corresponding to these regions are presented, as SEQ ID NO:71 (Figure 58) for the synthetic Env US4 common region and as SEQ ID NO:72 (Figure 59) for the synthetic Env SF162 common region.

Percent identity to this sequence can be determined, for example, using the Smith-Waterman search algorithm (Time Logic, Incline Village, NV), with the following exemplary parameters: weight matrix = nuc4x4hb; gap opening penalty = 20, gap extension penalty = 5, reporting threshold = 1; alignment threshold = 20.

Various forms of the different embodiments of the present invention (e.g., constructs) may be combined.

### F. Cloning Synthetic Env Expression Cassettes of the Present Invention.

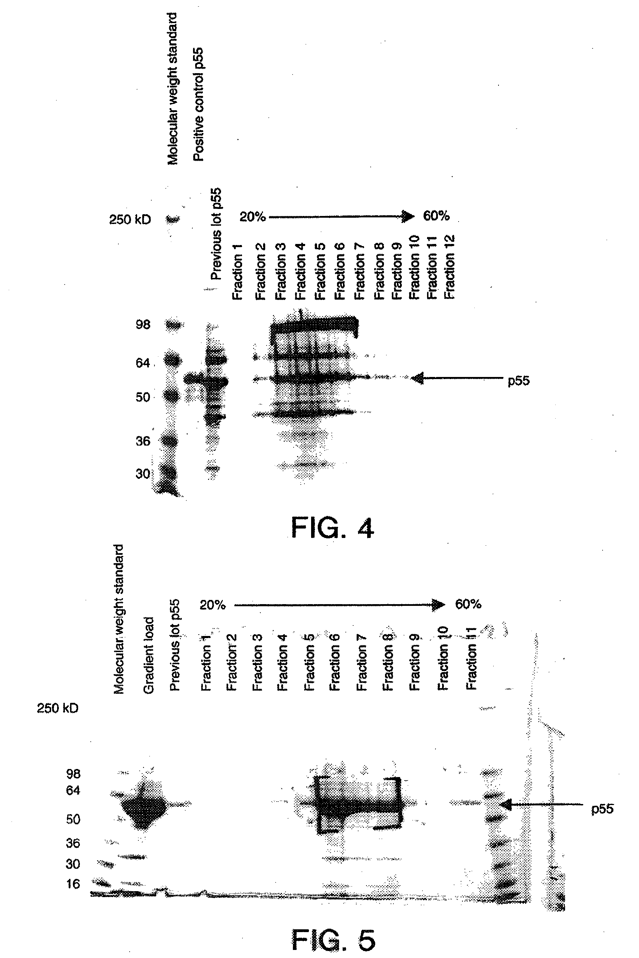

The synthetic DNA fragments encoding the Env polypeptides were typically cloned into the eucaryotic expression vectors described above for Gag, for example, pCMVKm2/pCMVlink (Figure 4), pCMV6a, pESN2dhfr (Figure 13A), pCMVIII (Figure 13B; alternately designated as the pCMV-PL-E-dhfr/neo vector).

Exemplary designations for pCMVlink vectors containing synthetic expression cassettes of the present invention are as follows: pCMVlink.gp140.modSF162; pCMVlink.gp140.-modSF162.delV2; pCMVlink.gp140.mut.modSF162; pCMVlink.gp140.mut.modSF162.delV2; pCMVKm2.gp140modUS4; pCMVKm2.gp140.modUS4.delV2; pCMVKm2.gp140.mut.modUS4; and, pCMVKm2.gp140.mut.modUS4.delV1/V2.

### G. Generation of Synthetic Tat Expression Cassettes

Tat coding sequences have also been modified according to the teachings of the present specification. The wild type nucleotide sequence encoding tat from variant SF162 is presented in Figure 76 (SEQ ID NO:85). The corresponding wild-type amino acid sequence is presented in Figure 77 (SEQ ID NO:86). Figure 81 (SEQ ID NO:89) shows the nucleotide sequence encoding the amino terminal of the tat protein and the codon encoding cystein-22 is underlined. Other exemplary constructs encoding synthetic tat polypeptides are shown in Figures 78 and 79 (SEQ ID NOs:87 and 88). In one embodiment (SEQ ID NO:88), the cystein residue at position 22 is replaced by a glycine. Caputo et al. (1996) *Gene Therapy* 3:235 have shown that this mutation affects the trans activation domain of Tat.

Various forms of the different embodiments of the invention, described herein, may be combined.

### H. Deposit of Vectors

Selected exemplary constructs shown below and described herein are deposited at Chiron Corporation, Emeryville, CA, 94662-8097, and were sent to the American Type Culture Collection, 10801 University Boulevard, Manassas, VA 20110-2209 on December 27, 1999.

| Plasmid Name | Chiron Deposit # | Date Sent to ATCC |
|---|---|---|
| pCMVgp160.modUS4 | 5094 | 27 Dec 99 |
| pCMVgp160delI.modUS4 | 5095 | 27 Dec 99 |
| pCMVgp160del2.modUS4 | 5096 | 27 Dec 99 |
| pCMVgp160del-2.modUS4 | 5097 | 27 Dec 99 |
| pCMVgp160del128-194.mod.US4 | 5098 | 27 Dec 99 |
| pCMVgp140mut.modUS4del128-194 | 5100 | 27 Dec 99 |
| pCMVgp140.mut.mod.US | 5101 | 27 Dec 99 |
| pCMVgp160.modSF162 | 5125 | 27 Dec 99 |
| pCMVgp160.modSF162.delV2 | 5126 | 27 Dec 99 |
| pCMVgp160.modSF162.delV1V2 | 5127 | 27 Dec 99 |
| pCMVgp140.mut.modSF162delV2 | 5128 | 27 Dec 99 |
| pCMVgp140.mut7.modSF162 | 5129 | 27 Dec 99 |
| pCMVgp140.mut7.modSF162delV2 | 5130 | 27 Dec 99 |
| pCMVgp140.mut8.modSF162 | 5131 | 27 Dec 99 |
| pCMVgp140.mut8.modSF162delV2 | 5132 | 27 Dec 99 |
| pCMVgp140.mut8.modSF162delV1V2 | 5133 | 27 Dec 99 |
| pCMVKm2.Gagprot.Mod.SF2.GP1 | 5150 | 27 Dec 99 |
| pCMVKm2.Gagprot.Mod.SF2.GP2 | 5151 | 27 Dec 99 |

### Example 2

### Expression Assays for the Synthetic Gag, Env and Tat Coding Sequences

### A. Gag and Gag-Protease Coding Sequences

The HIV-1SF2 wild-type Gag (SEQ ID NO:1) and Gag-protease (SEQ ID NO:2) sequences were cloned into expression vectors having the same features as the vectors into which the synthetic Gag (SEQ ID NO:4) and Gag-protease (SEQ ID NOs:5, 78 or 79)) sequences were cloned.

Expression efficiencies for various vectors carrying the HIV-1SF2 wild-type and synthetic Gag sequences were evaluated as follows. Cells from several mammalian cell lines (293, RD, COS-7, and CHO; all obtained from the American Type Culture Collection, 10801 University Boulevard, Manassas, VA 20110-2209) were transfected with 2 µg of DNA in transfection reagent LT1 (PanVera Corporation, 545 Science Dr., Madison, WI). The cells were incubated for 5 hours in reduced serum medium (Opti-MEM, Gibco-BRL, Gaithersburg, MD). The medium was then replaced with normal medium as follows: 293 cells, IMDM, 10% fetal calf serum, 2% glutamine (BioWhittaker, Walkersville, MD); RD and COS-7 cells, D-MEM, 10% fetal calf serum, 2% glutamine (Opti-MEM, Gibco-BRL, Gaithersburg, MD); and CHO cells, Ham's F-12, 10% fetal calf serum, 2% glutamine (Opti-MEM, Gibco-BRL, Gaithersburg, MD). The cells were incubated for either 48 or 60 hours. Supernatants were harvested and filtered through 0.45 µm syringe filters and, optionally, stored at -20°C.

Supernatants were evaluated using the Coulter p24-assay (Coulter Corporation, Hialeah, FL, US), using 96-well plates coated with a murine monoclonal antibody directed against HIV core antigen. The HIV-1 p24 antigen binds to the coated wells. Biotinylated antibodies against HIV recognize the bound p24 antigen. Conjugated strepavidin-horseradish peroxidase reacts with the biotin. Color develops from the reaction of peroxidase with TMB substrate. The reaction is terminated by addition of 4N H₂SO₄. The intensity of the color is directly proportional to the amount of HIV p24 antigen in a sample.

The results of these expression assays are presented in Tables 2A and 2B. Tables 2A and 2B shows data obtained using the synthetic Gag-protease expression cassette of SEQ ID NO:5. Similar results were obtained using the Gag-protease expression cassettes of SEQ ID NOs:78 and 79.

### Table 2: in vitro gag and gagprot p24 expression

**TABLE 2a.**

| Increased *in vitro* expression from modified vs. native *gag* plasmids in supernatants and lysates from transiently transfected cells | | | | | |
|---|---|---|---|---|---|
| experiment | native (nat)^{a} modified (mod)^{b} | supernatant (sup) lysate (lys) | cell line | hours post transfection | total ng p24 (fold increase) |
| 1 | nat | sup | 293 | 48 | 3.4 |
| | mod | sup | 293 | 48 | 1260(371) |
| | nat | sup | 293 | 60 | 3.2 |
| | mod | sup | 293 | 60 | 2222(694) |
| 2 | nat | sup | 293 | 60 | 1.8 |
| | mod | sup | 293 | 60 | 1740(966) |
| 3 | nat | sup | 293 | 60 | 1.8 |
| | mod | sup | 293 | 60 | 580 (322) |
| 4 | nat | lys | 293 | 60 | 1.5 |
| | mod | lys | 293 | 60 | 85 (57) |
| 1 | nat | sup | RD | 48 | 5.6 |
| | mod | sup | RD | 48 | 66(12) |
| | nat | sup | RD | 60 | 7.8 |
| | mod | sup | RD | 60 | 70.2 (9) |
| 2 | nat | lys | RD | 60 | 1.9 |
| | mod | lys | RD | 60 | 7.8 (4) |
| 1 | nat | sup | COS-7 | 48 | 0.4 |
| | mod | sup | COS-7 | 48 | 33.4 (84) |
| 2 | nat | sup | COS-7 | 48 | 0.4 |
| | mod | sup | COS-7 | 48 | 10 (25) |
| | nat | lys | COS-7 | 48 | 3 |
| | mod | lys | COS-7 | 48 | 14(5) |

| | | | | | |
|---|---|---|---|---|---|
| ^{a}pCMVLink.Gag.SF2.PRE | | | | | |
| ^{b}pCMVKm2.GagMod.SF2 | | | | | |

**TABLE 2b.**

| *In vitro* expression from modified gag and *gagprotease* plasmids in supernatants and lysates from transiently transfected cells | | | | |
|---|---|---|---|---|
| plasmid | supernatant (sup) supernatant lysate (lys) | cell line | hours post transfection | total ng p24 ^{d} |
| Gag^{a} | sup | 293 | 60 | 760 |
| GagProt(GP1)^{b} | sup | 293 | 60 | 380 |
| GagProt(GP2)^{c} | sup | 293 | 60 | 320 |
| Gag | lys | 293 | 60 | 78 |
| GagProt(GP1) | lys | 293 | 60 | 1250 |
| GagProt(GP2) | lys | 293 | 60 | 400 |
| Gag | sup | COS-7 | 72 | 40 |
| GagProt(GP1) | sup | COS-7 | 72 | 150 |
| GagProt(GP2) | sup | COS-7 | 72 | 290 |
| Gag | lys | COS-7 | 72 | 60 |
| GagProt(GP1) | lys | COS-7 | 72 | 63 |
| GagProt(GP2) | lys | COS-7 | 72 | 58 |

| | | | | |
|---|---|---|---|---|
| ^{a} pCMVKm2.GagMod.SF2 | | | | |
| ^{b} pCMVKm2.GagProtMod.SF2(GP1) *gagprotease* with codon optimization and inactivation of INS in *protease* | | | | |
| ^{c} pCMVKm2.GagProtMod.SF2(GP2) *gagprotease* with only inactivation of INS in *protease* | | | | |
| ^{d} Shown are representative results from 3 independent experiments for each cell line tested. | | | | |

The data showed that the synthetic Gag and Gag-protease expression cassettes provided dramatic increases in production of their protein products, relative to the native (HIV-1SF2 wild-type) sequences, when expressed in a variety of cell lines.

### B. Env Coding Sequences

The HIV-SF162 ("SF162") wild-type Env (SEQ ID No:1-3) and HIV-US4 ("US4") wild-type Env (SEQ ID NO:22-24) sequences were cloned into expression vectors having the same features as the vectors into which the synthetic Env sequences were cloned.

Expression efficiencies for various vectors carrying the SF162 and US4 wild-type and synthetic Env sequences were evaluated essentially as described above for Gag except that cell lysates were prepared in 40 µl lysis buffer (1.0 % NP40, 0.1 M Tris pH 7.5) and frozen at-20°C and capture ELISAs were performed as follows.

For Capture ELISAs, 250 ng of an ammonium sulfate IgG cut of goat polyclonal antibody to gp120SF2/env2-3 was used to coat each well of a 96-well plate (Corning, Corning, NY). Serial dilutions of gp120/SF2 protein (MID 167) were used to set the quantitation curve from which expression of US4 or SF162 gp120 proteins from transfection supernatant and lysates were calculated. Samples were screened undiluted and, optionally, by serial 2-fold dilutions. A human polyclonal antibody to HIV-1 gp120/SF2 was used to detect bound gp120 envelope protein, followed by horse-radish peroxidase (HRP)-labeled goat anti-human IgG conjugates. TMB (Pierce, Rockford, IL) was used as the substrate and the reaction is terminated by addition of 4N H₂SO₄. The reaction was quantified by measuring the optical density (OD) at 450 nm. The intensity of the color is directly proportional to the amount of HIV gp120 antigen in a sample. Purified SF2 gp120 protein was diluted and used as a standard.

The results of the transient expression assays are presented in Tables 3 and 4. Table 3 depicts transient expression in 293 cells transfected with a pCMVKm2 vector carrying the Env cassette of interest. Table 4 depicts transient expression in RD cells transfected with a pCMVKm2 vector carrying the Env cassette of interest.

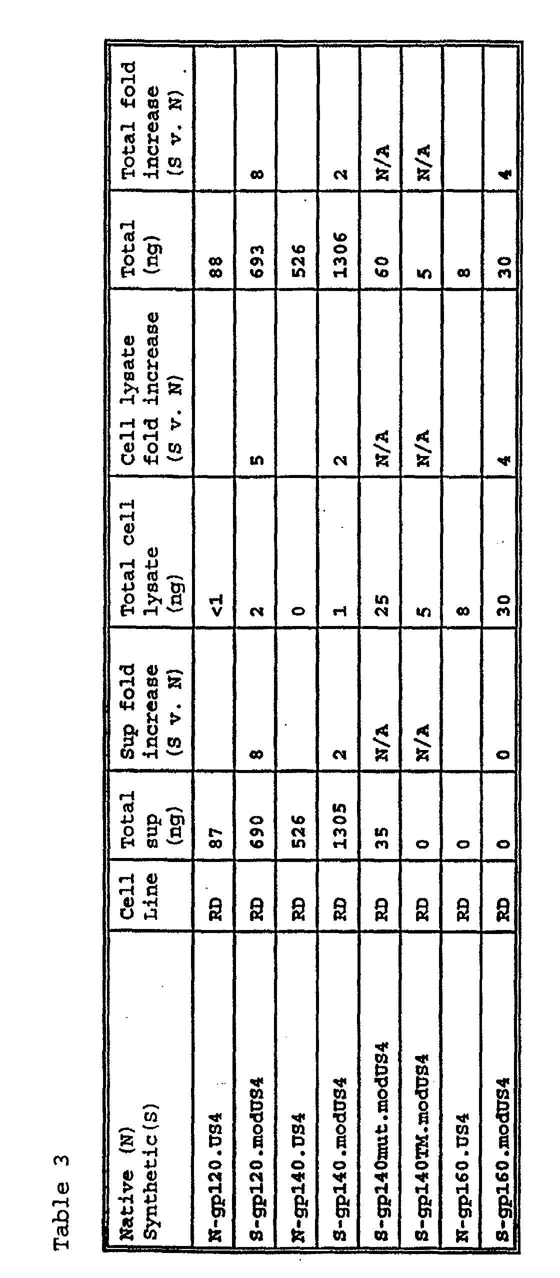

**Table 4**

| CHO Cell Lines Expression Level of US4 Envelope Constructs | | | |
|---|---|---|---|
| Constructs | CHO Clone # | MTX Level | Expression Level* (ng/ml) |
| gp120.modUS4 | 1 | 3.2µM | 250-450 |
| | 2 | 1.6µM | 350-450 |
| | 3 | 200nM | 230-580 |
| | 4 | 200nM | 300-500 |
| gp140.modUS4 | 1 | 1µM | 155-300 |
| | 2 | 1µM | 100-260 |
| | 3 | 1µM | 200-430 |
| gp140.mut. modUS4 | 1 | 1µM | 110-270 |
| | 2 | 1µM | 100-235 |
| | 3 | 1µM | 100-220 |
| gp140.modUS4 .delV1/V2 | 1 | 50nM | 313-587** |
| | 2 | 50nM | 237-667** |
| | 3 | 50nM | 492-527** |
| gp140.mut. modUS4.delV1 /V2 | 1 | 50nM | 46-328** |
| | 2 | 50nM | 82-318** |
| | 3 | 50nM | 204-385** |

| | | | |
|---|---|---|---|
| *All samples measured at T-75 flask stage unless otherwise indicated | | | |
| **at 24 well and 6 well plate stages ***in a three liter bioreactor perfusion culture this clone yielded approximately 2-5 µg/ml. | | | |

The data showed that the synthetic Env and expression cassettes provided a significant increase in production of their protein products, relative to the native (HIV-1SF162 or US4 wild-type) sequences, when expressed in a variety of cell lines.

### C. CHO Cell line Env expression data

Chinese hamster ovary (CHO) cells were transfected with plasmid DNA encoding the synthetic HIV-1 gp120 or gp140 proteins (e.g., pESN2dhfr or pCMVIII vector backbone) using Mirus TransIT-LT1 polyamine transfection reagent (Pan Vera) according to the manufacturers instructions and incubated for 96 hours. After 96 hours, media was changed to selective media (F12 special with 250 µg/ml G418) and cells were split 1:5 and incubated for an additional 48 hours. Media was changed every 5-7 days until colonies started forming at which time the colonies were picked, plated into 96 well plates and screened by gp120 Capture ELISA. Positive clones were expanded in 24 well plates and screened several times for Env protein production by Capture ELISA, as described above. After reaching confluency in 24 well plates, positive clones were expanded to T25 flasks (Corning, Corning, NY). These were screened several times after confluency and positive clones were expanded to T75 flasks.

Positive T75 clones were frozen in LN2 and the highest expressing clones amplified with 0-5 µM methotrexate (MTX)at several concentrations and plated in 100mm culture dishes. Plates were screened for colony formation and all positive closed were again expanded as described above. Clones were expanded an amplified and screened at each step by gp120 capture ELISA. Positive clones were frozen at each methotrexate level. Highest producing clones were grown in perfusion bioreactors (3L, 100L) for expansion and adaptation to low serum suspension culture conditions for scale-up to larger bioreactors.

Tables 5 and 6 show Capture ELISA data from CHO cells transfected with pCMVIII vector carrying a cassette encoding synthetic HIV-US4 and SF162 Env polypeptides (*e.g.*, mutated cleavage sites, modified codon usage and/or deleted hypervariable regions). Thus, stably transfected CHO cell lines which express Env polypeptides (*e.g.*, gp120, gp140-monomeric, and gp140-oligomeric) have been produced.

**Table 5**

| CHO Cell Lines Expression Level of US4 Envelope Constructs | | | |
|---|---|---|---|
| Constructs | CHO Clone # | MTX Level | Expression Level* (ng/ml) |
| gp120.modUS4 | 1 | 3.2µM | 250-450 |
| | 2 | 1.6µM | 350-450 |
| | 3 | 200nM | 230-580*** |
| | 4 | 200nM | 300-500 |
| gp140.modUS4 | 1 | 1µM | 155-300 |
| | 2 | 1µM | 100-260 |
| | 3 | 1µM | 200-430 |
| gp140.mut. modUS4 | 1 | 1µM | 110-270 |
| | 2 | 1µM | 100-235 |
| | 3 | 1µM | 100-220 |
| gp140.modUS4 .delV1/V2 | 1 | 50nM | 313-587** |
| | 2 | 50nM | 237-667** |
| | 3 | 50nM | 492-527** |
| gp140.mut. modUS4.delV1 /V2 | 1 | 50nM | 46-328** |
| | 2 | 50nM | 82-318** |
| | 3 | 50nM | 204-385** |

| | | | |
|---|---|---|---|
| *All samples measured at T-75 flask stage unless otherwise indicated | | | |
| **at 24 well and 6 well plate stages | | | |
| ***in a three liter bioreactor perfusion culture this clone yielded approximately 2-5 µg/ml. | | | |

**Table 6**

| CHO Cell Lines Expression Level of SF162 Envelope Constructs | | | |
|---|---|---|---|
| Constructs | CHO Clone # | MTX Level | Expression Level* (ng/ml) |
| gp120.modSF162 | 1 | 0 | 755-2705 |
| | 2 | 0 | 928-1538 |
| | 3 | 0 | 538-1609 |
| gp140.modSF162 | 1 | 20 nM | 180-350 |
| gp140.mut. modSF162 | 1 | 20 nM | 164-451 |
| | 2 | 20 nM | 188-487 |
| | 3 | 20 nM | 233-804 |
| gp120.modSF162 .delV2 | 1 | 800nM | 528-1560 |
| | 2 | 800nM | 487-1878 |
| | 3 | 800nM | 589-1212 |
| gp140.modSF162 .delV2 | 1 | 800nM | 300-600 |
| | 2 | 800nM | 200-400 |
| | 3 | 800nM | 200-500 |
| gp140.mut. modSF162.delV2 | 1 | 800nM | 300-700 |
| | 2 | 400nM | 1161 |
| | 3 | 800nM | 400-600 |
| | 4 | 400nM | 1600-2176 |

| | | | |
|---|---|---|---|
| *All samples measured at T-75 flask stage unless otherwise indicated | | | |

The results presented above demonstrate the ability of the constructs of the present invention to provide expression of Env polypeptides in CHO cells. Production of polypeptides using CHO cells provides (i) correct glycosylation patterns and protein conformation (as determined by binding to panel of MAbs); (ii) correct binding to CD4 receptor molecules; (iii) absence of non-mammalian cell contaminants (e.g., insect viruses and/or cells); and (iv) ease of purification.

### D. Tat Coding Sequences

The HIV-SF162 ("SF162") wild-type Tat (SEQ ID NO:85) sequences were cloned into expression vectors having the same features as the vectors into which the synthetic Tat sequences were cloned (SEQ ID NOs:87, 88 and 89).

Expression efficiencies for various vectors carrying the SF162 wild-type and synthetic Tat sequences are evaluated essentially as described above for Gag and Env using capture ELISAs with the appropriate anti-tat antibodies and/or CHO cell assays. Expression of the polypeptides encoded by the synthetic cassettes is improved relative to wild type.

### Example 3

### Western Blot Analysis of Expression

### A. Gag and Gag-Protease Coding Sequences

Human 293 cells were transfected as described in Example 2 with pCMV6a-based vectors containing native or synthetic Gag expression cassettes. Cells were cultivated for 60 hours post-transfection. Supernatants were prepared as described. Cell lysates were prepared as follows. The cells were washed once with phosphate-buffered saline, lysed with detergent [1% NP40 (Sigma Chemical Co., St. Louis, MO) in 0.1 M Tris-HCl, pH 7.5], and the lysate transferred into fresh tubes. SDS-polyacrylamide gels (pre-cast 8-16%; Novex, San Diego, CA) were loaded with 20 µl of supernatant or 12.5 µl of cell lysate. A protein standard was also loaded (5 µl, broad size range standard; BioRad Laboratories, Hercules, CA). Electrophoresis was carried out and the proteins were transferred using a BioRad Transfer Chamber (BioRad Laboratories, Hercules, CA) to Immobilon P membranes (Millipore Corp., Bedford, MA) using the transfer buffer recommended by the manufacturer (Millipore), where the transfer was performed at 100 volts for 90 minutes. The membranes were exposed to HIV-1-positive human patient serum and immunostained using o-phenylenediamine dihydrochloride (OPD; Sigma).

The results of the immunoblotting analysis showed that cells containing the synthetic Gag expression cassette produced the expected p55 protein at higher per-cell concentrations than cells containing the native expression cassette. The Gag p55 protein was seen in both cell lysates and supernatants. The levels of production were significantly higher in cell supernatants for cells transfected with the synthetic Gag expression cassette of the present invention. Experiments performed in support of the present invention suggest that cells containing the synthetic Gag-prot expression cassette produced the expected Gag-prot protein at comparably higher per-cell concentrations than cells containing the native expression cassette.

In addition, supernatants from the transfected 293 cells were fractionated on sucrose gradients. Aliquots of the supernatant were transferred to Polyclear™ ultracentrifuge tubes (Beckman Instruments, Columbia, MD), under-laid with a solution of 20% (wt/wt) sucrose, and subjected to 2 hours centrifugation at 28,000 rpm in a Beckman SW28 rotor. The resulting pellet was suspended in PBS and layered onto a 20-60% (wt/wt) sucrose gradient and subjected to 2 hours centrifugation at 40,000 rpm in a Beckman SW41ti rotor.

The gradient was then fractionated into approximately 10 x 1 ml aliquots (starting at the top, 20%-end, of the gradient). Samples were taken from fractions 1-9 and were electrophoresed on 8-16% SDS polyacrylamide gels. Fraction number 4 (the peak fraction) corresponds to the expected density of Gag protein VLPs. The supernatants from 293/synthetic Gag cells gave much stronger p55 bands than supernatants from 293/native Gag cells, and, as expected, the highest concentration of p55 in either supernatant was found in fraction 4.

These results demonstrate that the synthetic Gag expression cassette provides superior production of both p55 protein and VLPs, relative to the native Gag coding sequences.

### B. Env Coding Sequences

Human 293 cells were transfected as described in Example 2 with pCMVKm2-based; pCMVlink-based; p-CMVII-based or pESN2-based vectors containing native or synthetic Env expression cassettes. Cells were cultivated for 48 or 60 hours post-transfection. Cell lysates and supernatants were prepared as described (Example 2). Briefly, the cells were washed once with phosphate-buffered saline, lysed with detergent [1% NP40 (Sigma Chemical Co., St. Louis, MO)] in 0.1 M Tris-HCl, pH 7.5], and the lysate transferred into fresh tubes. SDS-polyacrylamide gels (pre-cast 8-16%; Novex, San Diego, CA) were loaded with 20 µl of supernatant or 12.5 µl of cell lysate. A protein molecular weight standard and an HIV SF2 gp120 positive control protein (5 µl, broad size range standard; BioRad Laboratories, Hercules, CA) were also loaded. Electrophoresis was carried out and the proteins were transferred using a BioRad Transfer Chamber (BioRad Laboratories, Hercules, CA) to Immobilon P membranes (Millipore Corp., Bedford, MA) using the transfer buffer recommended by the manufacturer (Millipore), where the transfer was performed at 100 volts for 90 minutes. The membranes were then reacted against polyclonal goat anti-gp120SF2/env2-3 anti-sera, followed by incubation with swine anti-goat IgG-peroxidase (POD) (Sigma, St. Louis, MO). Bands indicative of binding were visualized by adding DAB with hydrogen peroxide which deposits a brown precipitate on the membranes.

The results of the immunoblotting analysis showed that cells containing the synthetic Env expression cassette produced the expected Env gp proteins of the predicted molecular weights as determined by mobilities in SDS-polyacrylamide gels at higher per-cell concentrations than cells containing the native expression cassette. The Env proteins were seen in both cell lysates and supernatants. The levels of production were significantly higher in cell supernatants for cells transfected with the synthetic Env expression cassette of the present invention.

### C. Tat Coding Sequences

Human 293 cells are transfected as described in Example 2 with various vectors containing native or synthetic Tat expression cassettes. Cells are cultivated and isolated proteins analyzed as described above. Immunoblotting analysis shows that cells containing the synthetic Tat expression cassette produced the expected Tat proteins of the predicted molecular weights as determined by mobilities in SDS-polyacrylamide gels at higher per-cell concentrations than cells containing the native expression cassette.

### Example 4

### Purification of Env polypeptides

### A. Purification of Oligomeric gp140

Purification of oligomeric gp140 (o-gp140 US4) was conducted essentially as shown in Figure 60. For the experiments described herein, o-gp140 refers to oligomeric gp140 in either native or modified (e.g., optimized expression sequences, deleted, mutated, truncated, etc.) form. Briefly, concentrated (30-50X) supernatants obtained from CHO cell cultures were loaded onto an anion exchange (DEAE) column which removed DNA and other serum proteins. The eluted material was loaded onto a ceramic hydroxyapatite column (CHAP) which bound serum proteins but not HIV Env proteins. The flow-through from the DEAE and CHAP columns was loaded onto a Protein A column as a precautionary step to remove any remaining serum immunoglobulins. The Env proteins in the flow-through were then captured using the lectin gluvanthus navalis (GNA, Vector Labs, Burlingame, CA). GNA has high affinity for mannose rich carbohydrates such as Env. The Env proteins were then eluted with GNA substrate. To remove other highly glycosylated proteins, a cation exchange column (SP) was used to purify gp140/gp120. In a final step, which separates gp120 from o-gp140, a gel filtration column was used to separate oligomers from monomers. Sizing and chromatography analysis of the final product revealed that this strategy lead to the successful isolation of oligomeric gp140.

### B. Purification of gp120

Purification of gp120 was conducted essentially as previously described for other Env proteins. Briefly, concentrated supernatants obtained from CHO cell cultures were loaded onto an anion exchange (DEAE) column which removed DNA and other serum proteins. The eluted material was loaded onto a ceramic hydroxyapatite column (CHAP) which bound serum proteins but not HIV Env proteins. The flow-through from the CHAP column was loaded a cation exchange column (SP) where the flow-through was discarded and the bound fraction eluted with salt. The eluted fraction(s) were loaded onto a Suprose 12/Superdex 200 Tandem column (Pharmacia-Upjohn, Uppsala, Sweden) from which purified gp120 was obtained. Sizing and chromatography analysis of the final product revealed that this strategy successfully purified gp120 proteins.

### Example 5

### Analysis of Purified Env Polypeptides

### A. Analysis of o-gp140

It is well documented that HIV Env protein binds to CD4 only in its correct conformation. Accordingly, the ability of o-gp140 US4 polypeptides, produced and purified as described above, to bind CD4 cells was tested. O-gp140 US4 was incubated for 15 minutes with FITC-labeled CD4 at room temperature and loaded onto a Biosil 250 (BioRad) size exclusion column using Waters HPLC. CD4-FITC has the longest retention time (2.67 minutes), followed by CD4-FITC-gp120 (2.167 min). The shortest retention time (1.9 min) was observed for CD4-FITC-o-gp140 US4 indicating that, as expected, o-gp140 US4 binds to CD4 forming a large complex which reduces retention time on the column. Thus, the o-gp140 US4 produced and purified as described above is of the correct size and conformation.

In addition, the US4 o-gp140, purified as described above, was also tested for its ability to bind to a variety of monoclonal antibodies with known epitope specificities for the CD4 binding site, the CD4 inducible site, the V3 loop and oligomer-specific gp41 epitope. O-gp140 bound strongly to these antibodies, indicating that the purified protein retains its structural integrity.

### B. Analysis of gp120

As described above, CD4-FITC binds gp120, as demonstrated by the decreased retention time on the HPLC column. Thus, US4 gp120 purified by the above method retains its conformational integrity. In addition, the properties of purified gp120 can be tested by examining its integrity and identity on western blots, as well as, by examining protein concentration, pH, conductivity, endotoxin levels, bioburden and the like. US4 gp120, purified as described above, was also tested for its ability to bind to a variety of monoclonal antibodies with known epitope specificities for the CD4 binding site, the CD4 inducible site, the V3 loop and oligomer-specific gp41 epitope. The pattern of mAb binding to gp120 indicated that the purified protein retained its structural integrity, for example, the purified gp120 did not bind the mAb having the oligomer-specific gp41 epitope (as expected).

### Example 6

### Electron Microscopic Evaluation of VLP Production

The cells for electron microscopy were plated at a density of 50-70% confluence, one day before transfection. The cells were transfected with 10 µg of DNA using transfection reagent LT1 (Panvera) and incubated for 5 hours in serum-reduced medium (see Example 2). The medium was then replaced with normal medium (see Example 2) and the cells were incubated for 14 hours (COS-7) or 40 hours (CHO). After incubation the cells were washed twice with PBS and fixed with 2% glutaraldehyde. Electron microscopy was performed by Prof. T.S. Benedict Yen, Veterans Affairs, Medical Center, San Francisco, CA).

Electron microscopy was carried out using a transmission electron microscope (Zeiss 10c). The cells were pre-stained with osmium and stained with uranium acetate and lead citrate. The magnification was 100,000X.

Figures 3A and 3B show micrographs of CHO cells transfected with pCMVKM2 carrying the synthetic Gag expression cassette (SEQ ID NO:5) or carrying the Gag-prot expression cassette (SEQ ID NO:79). In the figure, free and budding immature virus-like-particles (VLP) of the expected size (100 nm) are seen for the Gag expression cassette (Figure 3A) and both immature and mature VLPs are seen for the Gag-prot expression cassette (Figure 3B). COS-7 cells transfected with the same vector have the same expression pattern. VLP can also be found intracellularly in CHO and COS-7 cells.

Native and synthetic Gag expression cassettes were compared for their associated levels of VLP production when used to transfect human 293 cells. The comparison was performed by density gradient ultracentrifugation of cell supernatants and Western-blot analysis of the gradient fractions. There was a clear improvement in production of VLPs when using the synthetic Gag construct.

### Example 7

### Expression of Virus-like Particles in the Baculovirus System

### A. Expression of Native HIV p55 Gag

To construct the native HIV p55 Gag baculovirus shuttle vector, the prototype SF2 HIV p55 plasmid, pTM1-Gag (Selby M.J., et al., *J Virol.* 71(10):7827-7831, 1997), was digested with restriction endonucleases *Nco*1 and *Bam*HI to extract a 1.5 Kb fragment that was subsequently subcloned into pAcC4 *(Bio*/*Technology 6*:47-55, 1988), a derivative of pAc436. Generation of the recombinant baculovirus was achieved by co-transfecting 2 µg of the HIV p55 Gag pAcC4 shuttle vector with 0.5 µg of linearized, *Autographa californica* baculovirus (AcNPV) wild-type viral DNA into *Spodoptera frugiperda* (Sf9) cells (Kitts, P.A., Ayres M.D., and Possee R.D., *Nucleic Acids Res.* 18:5667-5672, 1990). The isolation of recombinant virus expressing HIV p55 Gag was performed according to standard techniques (O'Reilly, D.R., L.K. Miller, and V. A. Luckow, *Baculovirus Expression Vector: A Laboratory Manual,* W.H. Freeman and Company, New York, 1992).

Expression of the HIV p55 Gag was achieved using a 500 ml suspension culture of Sf9 cells grown in serum-free medium (Miaorella, B., D. Inlow, A. Shauger, and D. Harano, *Bio*/*Technology* **6**:1506-1510, 1988) that had been infected with the HIV p55 Gag recombinant baculovirus at a multiplicity of infection (MOI) of 10. Forty-eight hours post-infection, the supernatant was separated by centrifugation and filtered through a 0.2 µm filter. Aliquots of the supernatant were then transferred to Polyclear™ (Beckman Instruments, Palo Alto, CA) ultracentrifuge tubes, underlaid with 20% (wt/wt) sucrose, and subjected to 2 hours centrifugation at 24,00 rpm using a Beckman SW28 rotor.

The resulting pellet was suspended in Tris buffer (20 mM Tris HCl, pH 7.5, 250 mM NaCl, and 2.5 mM ethylenediaminetetraacetic acid [EDTA]), layered onto a 20-60% (wt/wt) sucrose gradient, and subjected to 2 hours centrifugation at 40,000 rpm using a Beckman SW41*ti* rotor. The gradient was then fractionated starting at the top (20% sucrose) of the gradient into approximately twelve 0.75 ml aliquots. A sample of each fraction was electrophoresed on 8-16% SDS polyacrylamide gels and the resulting bands were visualized after commassie staining (Figure 4). Additional aliquots were subjected to refractive index analysis.

The results shown in Figure 4 indicated that the p55 Gag virus-like particles banded at a sucrose density of range of 1.15 - 1.19 g/ml with the peak at approximately 1.17 g/ml. The peak fractions were pooled and concentrated by a second 20% sucrose pelleting. The resulting pellet was suspended in 1 ml of Tris buffer (described above). The total protein yield as estimated by Bicimchrominic Acid (BCA) (Pierce Chemical, Rockford, IL) was 1.6 mg.

### B. Expression of Synthetic HIV p55 Gag

A baculovirus shuttle vector containing the synthetic p55 Gag sequence was constructed as follows. The synthetic HIV p55 expression cassette (Example 1) was digested with restriction enzyme *Sal*I followed by incubation with T4-DNA polymerase. The resulting fragment was isolated (PCR Clean-Up™, Promega, Madison, WI) and then digested with *BamHI* endonuclease. The shuttle vector pAcCl3 (Munemitsu S., et al., *Mol Cell Biol.* 10(11):5977-5982, 1990) was linearized by digestion with EcoI, followed by incubation with T4-DNA polymerase, and then isolated (PCR Clean-Up™). The linearized vector was digested with BamHI, treated with alkaline phosphatase, and isolated by size fragmentation in an agarose gel. The isolated 1.5 kb fragment was ligated with the prepared pAcCl3 vector. The resulting clone was designated pAcCl3-Modif.p55Gag.

The expression conditions for the synthetic HIV p55 VLPs differed from those of the native p55 Gag as follows: a culture volume of 1 liter used instead of 500 ml; *Trichoplusia ni* (Tn5) (Wickham, T.J., and Nermerow, G.R., *BioTechnology Progress,* 9:25-30, 1993) insect cells were used instead of Sf9 insect cells; and, an MOI of 3 was instead of an MOI of 10. Experiments performed in support of the present invention showed that there was no appreciable difference in expression level between the Sf9 and Tn5 insect cells with the native p55 clone. In terms of MOI, experience with the native p55 clone suggested that an MOI of 10 resulted in higher expression (approximately 2-fold) of VLPs than a lower MOI.

The sucrose pelleting and banding methods used for the synthetic p55 VLPs were similar to those employed for the native p55 VLPs (described above), with the following exceptions: pelleted VLPs were suspended in 4 ml of phosphate buffered saline (PBS) instead of 1.0 ml of the Tris buffer; and four, 20-60% sucrose gradients were used instead of a single gradient. Also, due to the high concentration of banded VLPs, further concentration by pelleting was not required. The peak fractions from all 4 gradients were simply dialyzed against PBS. The approximate density of the banded VLPs ranged from 1.23-1.28 g/ml. A total protein yield as estimated by BCA was 46 mg. Results from the sucrose gradient banding of the synthetic p55 are shown in Figure 5.

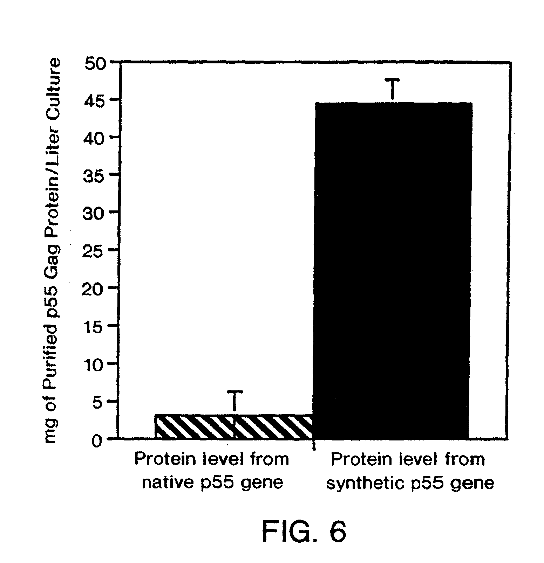

A comparison of the total amount of purified HIV p55 Gag from several preparations obtained from the two baculovirus expression cassettes has been summarized in Figure 6. The average yield from the native p55 was 3.16 mg/liter of culture (n=5, standard deviation (sd) ±1.07, range = 1.8-4.8 mg/L) whereas the average yield from the synthetic p55 was more than ten-fold higher at 44.5 mg/liter of culture (n=2, sd=±6.4).

In addition to a higher total protein yield, the final product from the synthetic p55-expressed Gag consistently contained lower amounts of contaminating baculovirus proteins than the final product from the native p55-expressed Gag. This difference can be seen in the two commassie-stained gels Figures 4 and 5.

### C. Expression of Native and Synthetic Gag-Core

Expression of the HIV p55 Gag/HCV Core 173 (SEQ ID NO:8) was achieved using a 2.5 liter suspension culture of Sf9 cells grown in serum-free medium (Miaorella, B., D. Inlow, A. Shauger, and D. Harano. 1988 Bio/Technology 6:1506-1510). The cells were infected with an HIV p55 Gag/HCV Core 173 recombinant baculovirus. Forty-eight hours post-infection, the supernatant was separated from the cells by centrifugation and filtered through a 0.2 µm filter. Aliquots of the supernatant were then transferred to a Polyclear™ (Beckman Instruments, Palo Alto, CA) ultracentrifuge tubes containing 30% (wt/wt) sucrose, and subjected to 2 hours of centrifugation at 24,000 rpm in a Beckman SW28 rotor and ultracentrifuge.

The resulting pellet was suspended in Tris buffer (50 mM Tris-HCl, pH 7.5, 500 mM NaCl) and layered onto a 30-60% (wt/wt) sucrose gradient and subjected to 2 hours centrifugation at 40,000 rpm in a Beckman SW41*ti* rotor and ultracentrifuge. The gradient was then fractionated starting at the top (30%) of the gradient into approximately 11 x 1.0 ml aliquots. A sample of each fraction was electrophoresed on 8-16% SDS polyacrylamide gels and the resulting bands were visualized after commassie staining.

A subset of aliquots were also subjected to Western blot analysis using monoclonal antibody 76C.5EG (Steimer, K.S., et al., Virology 150:283-290, 1986) which is specific for HIV p24 (a subunit of HIV p55). The peak fractions from the sucrose gradient were pooled and concentrated by a second 20% sucrose pelleting. The resulting pellet was suspended in 1 ml of buffer Tris buffer and the total protein yield as estimated by BCA (Pierce Chemical, Rockford, IL) was - 1.0 mg.

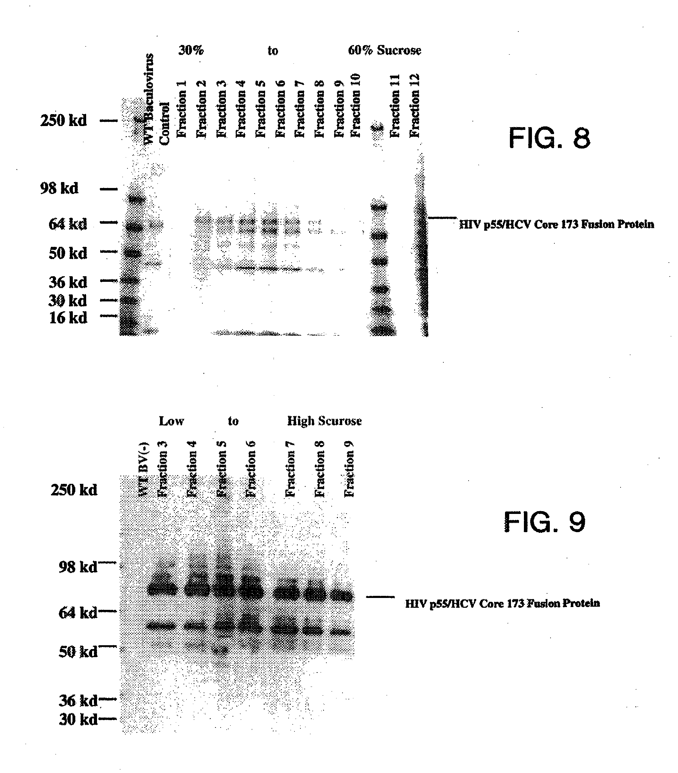

The results from the SDS PAGE are shown in Figure 8 and the anti- p24 Western blot results are shown in Figure 9. Taken together, these results indicate that the HIV p55 Gag/HCV Core 173 chimeric VLPs banded at a sucrose density similar to that of the HIV p55 Gag VLPs and the visible protein band that migrated at a molecular weight of - 72,000 kd was reactive with the HIV p24-specific monoclonal antibody. An additional immunoreactive band at approximately 55,000 kd also appeared to be reactive with the anti-p24 antibody and may be a degradation product.

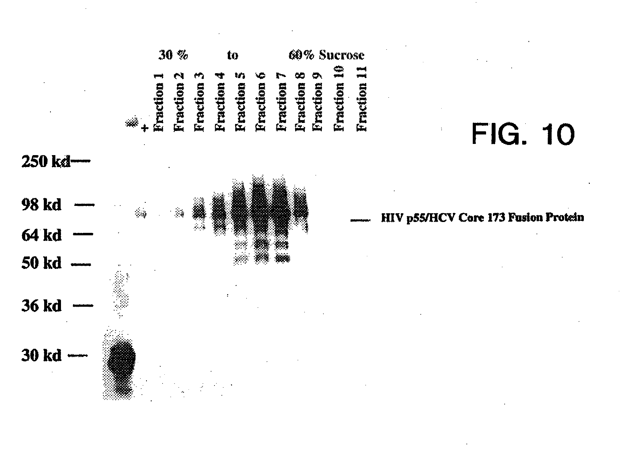

Although aliquots from the above preparation were not tested for reactivity with an HCV Core-specific antibody (an anti-CD22 rabbit serum), results from a similar preparation are shown in Figure 10 and indicate that the main HCV Core-specific reactivity migrates at an approximate molecular weight of 72,000 kd which is in accordance with the predicted molecular weight of the chimeric protein.

The expression conditions for the synthetic HIV p55 Gag/HCV Core 173 (SEQ ID NO:8) VLPs differed from those of the native p55 Gag and are as follows: a culture volume of 1 liter used instead of 2.5 liters, *Trichoplusia ni* (Tn5)(Wickham, T.J., and Nemerow, G.R. 1993 BioTechnology Progress, 9:25-30) insect cells were used instead of Sf9 insect cells and an MOI of 3 was instead of an MOI of 10. The sucrose pelleting and banding methods used for the synthetic HIV p55 Gag/HCV Core 173 VLPs were similar to those employed for the native HIV p55 Gag/HCV Core 173 VLPs. However, differences included: pelleted VLPs were suspended in 1 ml of phosphate buffered saline (PBS) instead of 1.0 ml of the Tris buffer, and a single 20-60% sucrose gradients was used. A comparison of the total amount of purified HIV p55 Gag/HCV Core 173 from multiple preparations obtained from the two baculovirus expression cassettes showed that there was an increase in expression using the synthetic HIV p55 Gag/HCV Core 173 cassette.

### D. Alternative method for the enrichment of HIV p55 Gag VLPs

In addition to purification from the media, p55 (Gag protein) expressed in baculovirus (e.g., using a synthetic expression cassette of the present invention) can also be purified as virus-like particles from the infected insect cells. For example, forty-eight hours post infection, the media and cell pellet are separated by centrifugation and the cell pellet is stored at -70°C until future use. At the time of processing, the cell pellet is suspended in 5 volumes of hypotonic lysis buffer (20 mM Tris-HCl, pH 8.2, 1 mM EGTA; 1 mM MgCl, and Complete Protease Inhibitor® (Boehringer Mannheim Corp., Indianapolis, IN]). If needed, the cells are then dounced 8-10 times to complete cell lysis.

The lysate is then centrifuged at approximately 1000-1500 x g for 20 minutes. The supernatant is decanted into UltraClear™ tubes, underlayed with 20% sucrose (w/w) and centrifuged at 24,000 rpm in SW28 buckets for 2 hours. The resulting pellet is suspended in Tris buffer (20 mM Tris HCl, pH 7.5, 250 mM NaCl, and 2.5 mM ethylene-diamine-tetraacetic acid (EDTA) with 0.1% IGEPAL detergent (Sigma Chemical, St. Louis, MO) and 250 units/ml of benzonase (American International Chemical, Inc., Natick, MA) and incubated at 4°C for at least 30 minutes. The suspension is subsequently layered onto a 20-60% sucrose gradient and spun at 40,000 rpm using an SW41ti rotor for 20-24 hours.

After ultracentrifugation, the sucrose gradient is fractionated and aliquots run on SDS PAGE to identify peak fractions. The peak fractions are dialyzed against PBS and measured for protein content. Negatively stained electron mircographs typically show non-enveloped VLPs somewhat smaller in diameter (80-120 nm) than the budded VLPs. HIV Gag VLPs prepared in this manner are also capable of generating Gag-specific CTL responses in mice.

### Example 8

### In Vivo Immunoaenicity of Synthetic Gag Expression Cassettes

### A. Immunization

To evaluate the possibly improved immunogenicity of the synthetic Gag expression cassettes, a mouse study was performed. The plasmid DNA, pCMVKM2 carrying the synthetic Gag expression cassette, was diluted to the following final concentrations in a total injection volume of 100 µl: 20 µg, 2 µg, 0.2 µg, and 0.02 µg. To overcome possible negative dilution effects of the diluted DNA, the total DNA concentration in each sample was brought up to 20 µg using the vector (pCMVKM2) alone. As a control, plasmid DNA of the native Gag expression cassette was handled in the same manner. Twelve groups of four Balb/c mice (Charles River, Boston, MA) were intramuscularly immunized (50 µl per leg, intramuscular injection into the *tibialis anterior)* according to the schedule in Table 7.

**Table 7**

| Group | Gag Expression Cassette | Concentration of Gag plasmid DNA (µg) | Immunized at time (weeks): |
|---|---|---|---|
| 1 | Synthetic | 20 | 0¹, 4 |
| 2 | Synthetic | 2 | 0, 4 |
| 3 | Synthetic | 0.2 | 0, 4 |
| 4 | Synthetic | 0.02 | 0, 4 |
| 5 | Synthetic | 20 | 0 |
| 6 | Synthetic | 2 | 0 |
| 7 | Synthetic | 0.2 | 0 |
| 8 | Synthetic | 0.02 | 0 |
| 9 | Native | 20 | 0 |
| 10 | Native | 2 | 0 |
| 11 | Native | 0.2 | 0 |
| 12 | Native | 0.02 | 0 |
| 1 = initial immunization at "week 0" | | | |

Groups 1-4 were bled at week 0 (before immunization), week 4, week 6, week 8, and week 12. Groups 5-12 were bled at week 0 (before immunization) and at week 4.

### B. Humoral Immune Response

The humoral immune response was checked with an anti-HIV Gag antibody ELISAs (enzyme-linked immunosorbent assays) of the mice sera 0 and 4 weeks post immunization (groups 5-12) and, in addition, 6 and 8 weeks post immunization, respectively, 2 and 4 weeks post second immunization (groups 1-4).

The antibody titers of the sera were determined by anti-Gag antibody ELISA. Briefly, sera from immunized mice were screened for antibodies directed against the HIV p55 Gag protein. ELISA microtiter plates were coated with 0.2 µg of HIV-1_{SF2} p24-Gag protein per well overnight and washed four times; subsequently, blocking was done with PBS-0.2% Tween (Sigma) for 2 hours. After removal of the blocking solution, 100 µl of diluted mouse serum was added. Sera were tested at 1/25 dilutions and by serial 3-fold dilutions, thereafter. Microtiter plates were washed four times and incubated with a secondary, peroxidase-coupled anti-mouse IgG antibody (Pierce, Rockford, IL). ELISA plates were washed and 100 µl of 3, 3', 5, 5'-tetramethyl benzidine (TMB; Pierce) was added per well. The optical density of each well was measured after 15 minutes. The titers reported are the reciprocal of the dilution of serum that gave a half-maximum optical density (O.D.). The ELISA results are presented in Table 8.

**Table 8**

| Group | Inoculum (µg) | Expression cassette | Sera - Week 4³ | Sera - Week 6 | Sera - Week 8 |
|---|---|---|---|---|---|
| 1 | 20 | S¹ - *gag* | 98 | 455 | 551 |
| 2 | 2 | S - *gag* | 59 | 1408 | 227 |
| 3 | 0. | S - *gag* | 29 | 186 | 61 |
| 4 | 0.02 | S - *gag* | < 20 | < 20 | < 20 |
| 5 | 20 | S - *gag* | 67 | n.a.⁴ | n.a. |
| 6 | 2 | S - *gag* | 63 | n.a. | n.a. |
| 7 | 0. | S - *gag* | 57 | n.a. | n.a. |
| 8 | 0.02 | S - *gag* | < 20 | n.a. | n.a. |
| 9 | 20 | N² - *gag* | 43 | n.a. | n.a. |
| 10 | 2 | N - *gag* | < 20 | n.a. | n.a. |
| 11 | 0. | N - *gag* | < 20 | n.a. | n.a. |
| 12 | 0.02 | N - *gag* | < 20 | n.a. | n.a. |

| | | | | | |
|---|---|---|---|---|---|
| 1 = synthetic *gag* expression cassette (SEQ ID NO: 4) | | | | | |
| 2 = native *gag* expression cassette (SEQ ID NO: 1) | | | | | |
| 3 = geometric mean antibody titer | | | | | |
| 4 = not applicable | | | | | |

The results of the mouse immunizations with plasmid-DNAs show that the synthetic expression cassettes provide a clear improvement of immunogenicity relative to the native expression cassettes. Also, the second boost immunization induced a secondary immune response after two weeks (groups 1-3).

### C. Cellular Immune Response

The frequency of specific cytotoxic T-lymphocytes (CTL) was evaluated by a standard chromium release assay of peptide pulsed Balb/c mouse CD4 cells. Gag expressing vaccinia virus infected CD-8 cells were used as a positive control (vvGag). Briefly, spleen cells (Effector cells, E) were obtained from the BALB/c mice immunized as described above (Table 8) were cultured, restimulated, and assayed for CTL activity against Gag peptide-pulsed target cells as described (Doe, B., and Walker, C.M., *AIDS* 10(7):793-794, 1996). The HIV-1_{SF2} Gag peptide used was p7g SEQ ID NO:10. Cytotoxic activity was measured in a standard ⁵¹Cr release assay. Target (T) cells were cultured with effector (E) cells at various E:T ratios for 4 hours and the average cpm from duplicate wells was used to calculate percent specific ⁵¹Cr release. The results are presented in Table 9.

Cytotoxic T-cell (CTL) activity was measured in splenocytes recovered from the mice immunized with HIV Gag DNA (compare Effector column, Table 9, to immunization schedule, Table 8). Effector cells from the Gag DNA-immunized animals exhibited specific lysis of Gag p7g peptide-pulsed SV-BALB (MHC matched) targets cells indicative of a CTL response. Target cells that were peptide-pulsed and derived from an MHC-unmatched mouse strain (MC57) were not lysed (Table 9; MC/p7g).

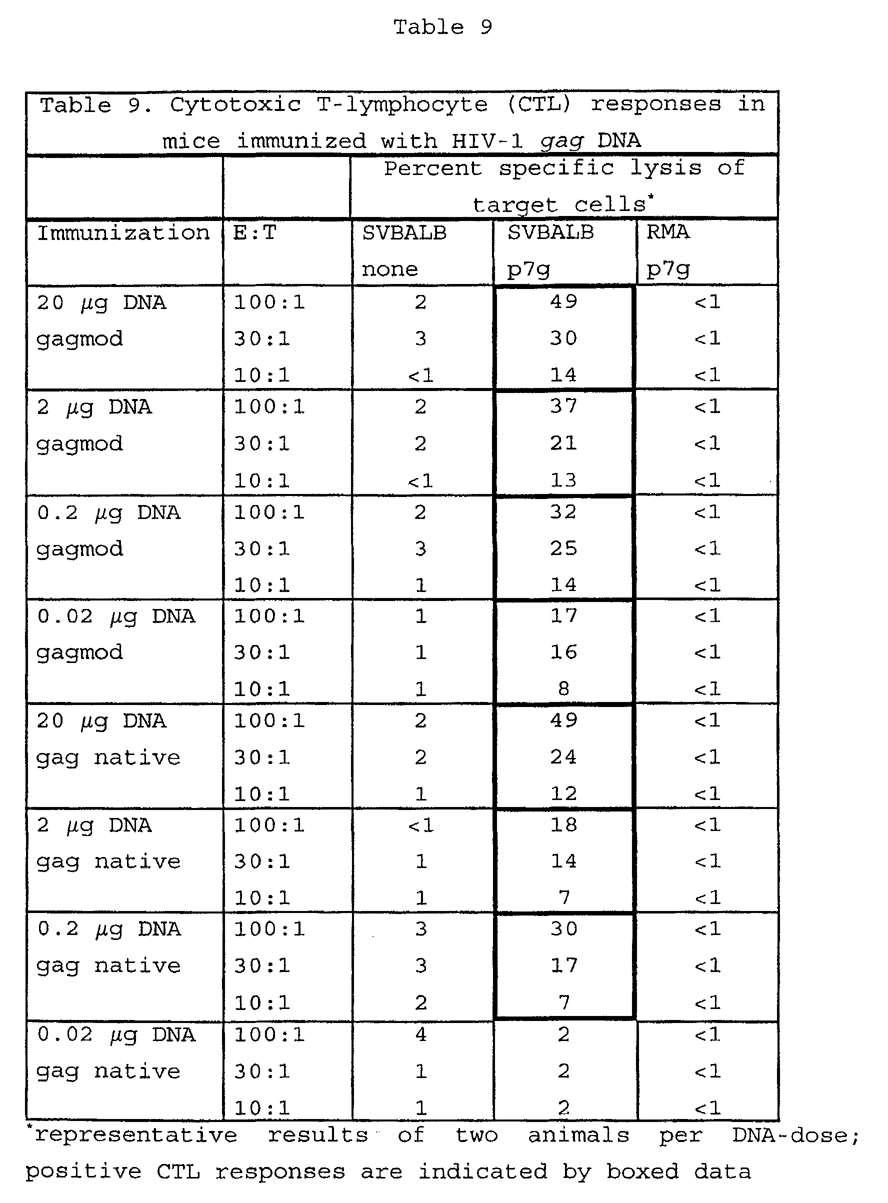

The results of the CTL assays show increased potency of synthetic Gag expression cassettes for induction of cytotoxic T-lymphocyte (CTL) responses by DNA immunization.

### Example 9

### In vivo Immunization with Env polypeptides

### A. Immunogenicity Study of US4 o-gp140 in Ras-3c Adjuvant System

Studies have been conducted using rabbits immunized with US4 o-gp140 purified as described above. Studies are also underway in animals to determine immunogenicity of US4 gp120, SF162 o-gp140 and SF162 gp120.

Two rabbits (#1 and #2) were immunized intramuscularly at 0, 4, 12 and 24 weeks with 50 µg of US4 o-gp140 in the Ribi™ adjuvant system (RAS-3c), (Ribi Immunochem, Hamilton, MT) containing 2% Squalene, 0.2% Tween 80, and one or more bacterial cell wall components from the group consisting of monophosphorylipid A (MPL, Ribi Immunochem, Hamilton, MT). In each experiment described herein, o-gp140 can be native, mutated and/or modified. Antibody responses directed against the US4 o-gp140 protein were measured by ELISA. Results are shown in Table 10.

**Table 10**

| Rabbit/sample | Approximate o-gp140 ELISA titer |
|---|---|
| pre-immunization | 0 |
| #1: post1 (0 week immuniz) | 400 |
| #1: post2 (4 week immuniz) | 15,000 |
| #1: post3 (12 week immuniz) | 50,000 |
| #1: post4 (24 week immuiz) | 100,000 |
| #2: post1 (0 week immuniz) | 600 |
| #2: post2 (4 week immuniz) | 12,000 |
| #2: post3 (12 week immuniz) | 25,000 |
| #2: post4 (24 week immuiz) | 55,000 |

The avidities of antibodies directed against the US4 o-gp140 protein were measured in a similar ELISA format employing successive washes with increasing concentrations of ammonium isothiocynate. Results are shown in Table 11.

**Table 11**

| Time of sample | Approx. Antibody avidity (NH₄HCN Conc. in M) |
|---|---|
| pre-immunization | 0.02 |
| post1 (0 week immuniz) | 1.8 |
| post2 (4 week immuniz) | 3.5 |
| post3 (12 week immuniz) | 5.5 |
| post4 (24 week immuniz) | 5.1 |

These results show that US4 o-gp140 is highly immunogenic and able to induce substantial antibody responses after only one or two immunizations.

### B. Immunoctenicity of US4 o-gp140 in MF59-based Adjuvants

Groups of 4 rabbits were immunized intramuscularly at 0, 4, 12 and 24 weeks with various doses of US4 o-gp140 protein in three different MF59-based adjuvants (MF59 is described in International Publication No. WO 90/14837 and typically contains 5% Squalene, 0.5% Tween 80, and 0.5% Span 85). Antibody titers were measured post-third by ELISA using SF2 gp120 to coat the plates. QHC is a quill-based adjuvant (Iscotek, Uppsala, Sweden). Results are shown in Table 12.

**Table 12**

| **Antigen dose (µg)** | **Adjuvant** | **Anti-gp120**_{**SF2**} **Ab GMT*** |
|---|---|---|
| 12.5 | MF59 | 7231 |
| 25 | MF59 | 8896 |
| 50 | MF59 | 12822 |
| 12.5 | MF59/MPL | 24146 |
| 25 | MF59/MPL | 27199 |
| 50 | MF59/MPL | 23059 |
| 50 | MF59/MPL/QHC | 31759 |

| | | |
|---|---|---|
| *GMT = geometric mean titer | | |

Thus, adjuvanted o-gp140 generated antigen-specific antibodies. Further, the antibodies were shown to increased in avidity over time.

### C. Neutralizing Antibodies

Neutralizing antibodies post-third immunization were measured against HIV-1 SF2 in a T-cell line adapted virus (TCLA) assay and against PBMC-grown HIV-1 variants SF2, SF162 and 119 using the CCR5+ CEMx174 LTR-GFP reporter cell line, 5.25 (provided by N. Landau, Salk Institute, San Diego, CA) as target cells. Results are shown in Table 13.

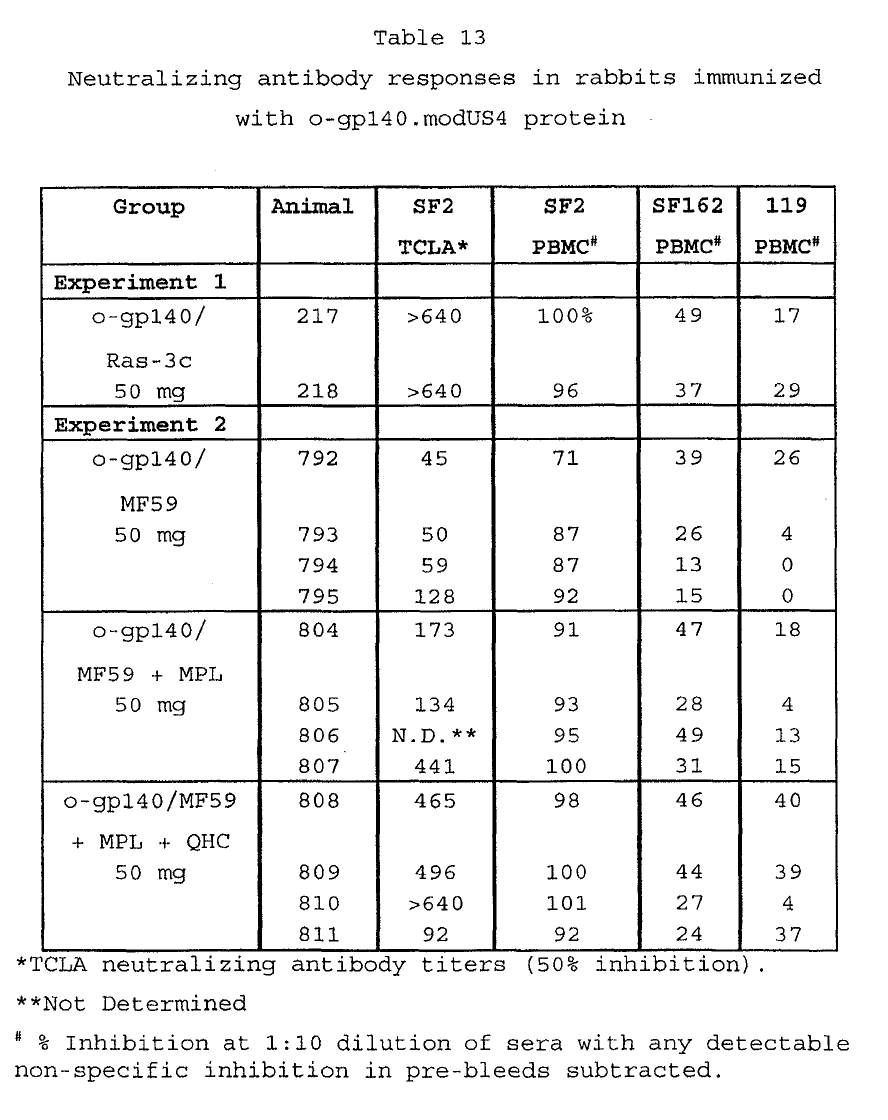

The above studies in rabbits indicate that the US4 o-gp140 protein is highly immunogenic. When administered with adjuvant, this protein was able to induce substantial antibody responses after only one or two immunizations. Moreover, the adjuvanted o-gp140 protein was able to generate antigen-specific antibodies which increased in avidity after successive immunizations, and substantial neutralizing activity against T-cell line adapted HIV-1. Neutralizing activity was also observed against PBMC-grown primary HIV strains, including the difficult to neutralize CCR5 co-receptor (R5)-utilizing isolates, SF162 and 119.

### Example 10

### In Vivo Immunogenicity of Synthetic Env Expression Cassettes

### A. General Immunization Methods

To evaluate the immunogenicity of the synthetic Env expression cassettes, studies using guinea pigs, rabbits, mice, rhesus macaques and baboons were performed. The studies were structured as follows: DNA immunization alone (single or multiple); DNA immunization followed by protein immunization (boost); DNA immunization followed by Sindbis particle immunization; immunization by Sindbis particles alone.

### B. Humoral Immune Response

The humoral immune response was checked in serum specimens from immunized animals with an anti-HIV Env antibody ELISAs (enzyme-linked immunosorbent assays) at various times post-immunization. The antibody titers of the sera were determined by anti-Env antibody ELISA as described above. Briefly, sera from immunized animals were screened for antibodies directed against the HIV gp120 or gp140 Env protein. Wells of ELISA microtiter plates were coated overnight with the selected Env protein and washed four times; subsequently, blocking was done with PBS-0.2% Tween (Sigma) for 2 hours. After removal of the blocking solution, 100 µl of diluted mouse serum was added. Sera were tested at 1/25 dilutions and by serial 3-fold dilutions, thereafter. Microtiter plates were washed four times and incubated with a secondary, peroxidase-coupled anti-mouse IgG antibody (Pierce, Rockford, IL). ELISA plates were washed and 100 µl of 3, 3', 5, 5'-tetramethyl benzidine (TMB; Pierce) was added per well. The optical density of each well was measured after 15 minutes. Titers are typically reported as the reciprocal of the dilution of serum that gave a half-maximum optical density (O.D.).

### Example 11

### DNA-immunization of Baboons Using Synthetic Gag Expression Cassettes

### A. Baboons

Four baboons were immunized 3 times (weeks 0, 4 and 8) bilaterally, intramuscular into the quadriceps using 1mg pCMVKM2.GagMod.SF2 plasmid-DNA (Example 1). The animals were bled two weeks after each immunization and a p24 antibody ELISA was performed with isolated plasma. The ELISA was performed essentially as described in Example 5 except the second antibody-conjugate was an anti-human IgG, g-chain specific, peroxidase conjugate (Sigma Chemical Co., St. Louis, MD 63178) used at a dilution of 1:500. Fifty µg/ml yeast extract was added to the dilutions of plasma samples and antibody conjugate to reduce non-specific background due to preexisting yeast antibodies in the baboons. The antibody titer results are presented in Table 14.

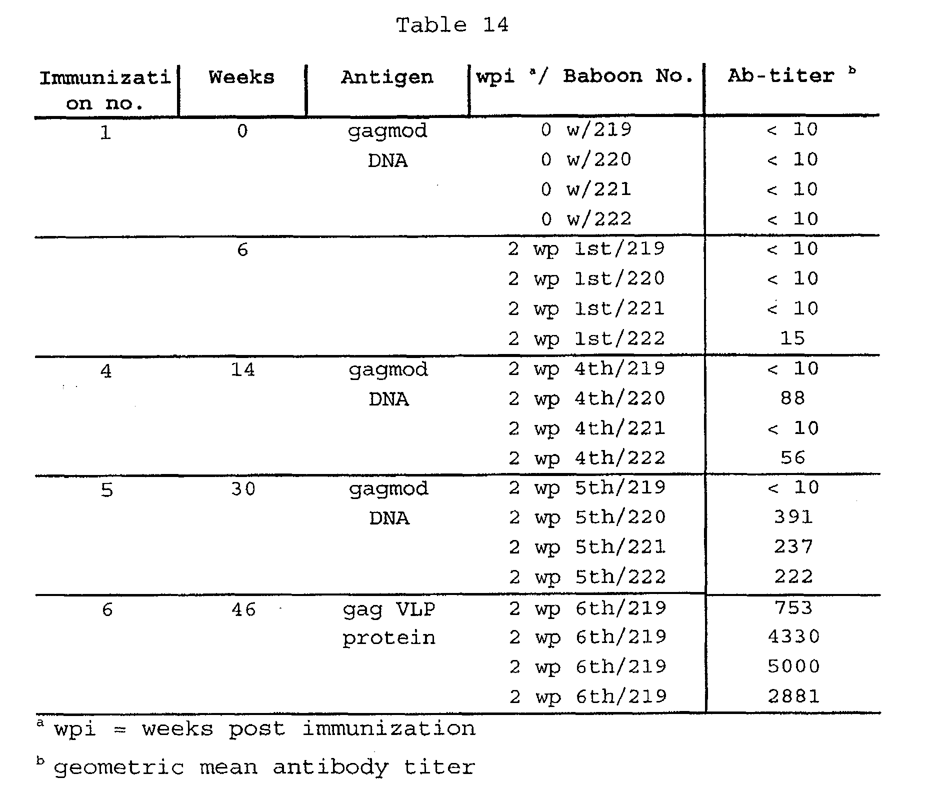

In Table 14, pre-bleed data are given as Immunization No. 0; data for bleeds taken 2 weeks post-first immunization are given as Immunization No. 1; data for bleeds taken 2 weeks post-second immunization are given as Immunization No. 2; and, data for bleeds taken 2 weeks post-third immunization are given as Immunization No. 3.

Further, lymphoproliferative responses to p24 antigen were also observed in baboons 221 and 222 two weeks post-fourth immunization (at week 14), and enhanced substantially post-boosting with VLP (at week 44 and 76). Such proliferation results are indicative of induction of T-helper cell functions.

### B. Rhesus Macaques

The improved potency of the codon-modified gag expression plasmid observed in mouse and baboon studies was confirmed in rhesus macaques. Four of four macaques had detectable Gag-specific CTL after two or three 1 mg doses of modified gag plasmid. In contrast, in a previous study, only one of four macaques given 1 mg doses of plasmid-DNA encoding the wild-type HIV-1_{SF2} Gag showed strong CTL activity that was not apparent until after the seventh immunization. Further evidence of the potency of the modified gag plasmid was the observation that CTL from two of the four rhesus macaques reacted with three nonoverlapping Gag peptide pools, suggesting that as many as three different Gag peptides are recognized and indicating that the CTL response is polyclonal. Additional quantification and specificity studies are in progress to further characterize the T cell responses to Gag in the plasmid-immunized rhesus macaques. DNA immunization of macaques with the modified gag plasmid did not result in significant antibody responses, with only two of four animals seroconverting at low titers. In contrast, in the same study the majority of macaques in groups immunized with p55Gag protein seroconverted and had strong Gag-specific antibody titers. These data suggest that a prime-boost strategy (DNA-prime and protein-boost) could be very promising for the induction of a strong CTL and antibody response.

In sum, these results demonstrate that the synthetic Gag plasmid DNA is immunogenic in non-human primates. When similar experiments were carried out using wild-type Gag plasmid DNA no such induction of anti-p24 antibodies was observed after four immunizations.

### Example 12

### DNA- and Protein Immunizations of Animals Using Env Expression Cassettes and Polypeptides

### A. Guinea Pigs

Groups comprising six guinea pigs each were immunized intramuscularly at 0, 4, and 12 weeks with plasmid DNAs encoding the gp120.modUS4, gp140.modUS4, gp140.modUS4.delV2, gp140.modUS4.delV2, gp140.modUS4.delV1/V2, or gp160.modUS4 coding sequences of the US4-derived Env. The animals were subsequently boosted at 18 weeks with a single intramuscular dose of US4 o-gp140.mut.modUS4 protein in MF59 adjuvant. Anti-gp120 SF2 antibody titers (geometric mean titers) were measured at two weeks following the third DNA immunization and at two weeks after the protein boost. Results are shown in Table 15.

**Table 15**

| Group | GMT post-DNA immuniz. | GMT post-protein boost |
|---|---|---|
| gp120.modUS4 | 2098 | 9489 |
| gp140.modUS4 | 190 | 5340 |
| gp140.modUS4.delV1 | 341 | 7808 |
| gp140.modUS4.delV2 | 386 | 8165 |
| gp140.modUS4.delV1/V 2 | 664 | 8270 |
| gp160.modUS4 | 235 | 9928 |

These results demonstrate the usefulness of the synthetic constructs to generate immune responses, as well as, the advantage of providing a protein boost to enhance the immune response following DNA immunization.

### B. Rabbits

Rabbits were immunized intramuscularly and intradermally using a Bioject needless syringe with plasmid DNAs encoding the following synthetic SF162 Env polypeptides: gp120.modSF162, gp120.modSF162.delV2, gp140.modSF162, gp140.modSF162.delV2, gp140.mut.modSF162, gp140.mut.modSF162.delV2, gp160.modSF162, and gp160.modSF162.delV2. Approximately 1 mg of plasmid DNA (pCMVlink) carrying the synthetic Env expression cassette was used to immunize the rabbits. Rabbits were immunized with plasmid DNA at 0, 4, and 12 weeks. At two weeks after the third immunization all of the constructs were shown to have generated significant antibody titers in the test animals. Further, rabbits immunized with constructs containing deletions of the V2 region generally generated similar antibody titers relative to rabbits immunized with the companion construct still containing the V2 region.

The nucleic acid immunizations are followed by protein boosting with o-gp140.modSF162.delV2 (0.1 mg of purified protein) at 24 weeks after the initial immunization. Results are shown in Table 16.

**Table 16**

| **Group** | **GMT 2wks post-2nd DNA immunization** | **GMT 2wks post-3rd DNA immunization** | **GMT 2wks post-protein boost** |
|---|---|---|---|
| gp120.modSF162 | 4573 | 5899 | 26033 |
| gp120.modSF162.delV2 | 3811 | 3122 | 29606 |
| gp140.modSF162 | 1478 | 710 | 12882 |
| gp140.modSF162.delV2 | 1572 | 819 | 11067 |
| gp140.mut.modSF162 | 1417 | 788 | 8827 |
| gp140.mut.modSF162.delV 2 | 1378 | 1207 | 13301 |
| gp160.modSF162 | 23 | 81 | 7050 |
| gp160.modSF162.delV2 | 85 | 459 | 11568 |

All constructs are highly immunogenic and generate substantial antigen binding antibody responses after only 2 immunizations in rabbits.

### C. Baboons

Groups of four baboons were immunized intramuscularly with 1 mg doses of DNA encoding different forms of synthetic US4 gp140 (see the following table) at 0, 4, 8, 12, 28, and 44 weeks. The animals were also boosted twice with US4 0-gp140 protein (gp140.mut.modUS4) at 44 and 76 weeks using MF59 as adjuvant. Results are shown in Table 17.

**Table 17**

| Animal | Treatment | 2 Wks Post 5th DNA immunization | 2 Wks post 6th DNA (plus ogp140 prot. immuniz.) | 2 Wks post 7th DNA (o-gp140 protein only) |
|---|---|---|---|---|
| CY 215 | | 8.3 | 446 | 1813 |
| CY 216 | gp140.modUS4 | 8.3 | 433 | 1236 |
| CY 217 | | 68 | 1660 | 2989 |
| CY 218 | | 101 | 2556 | 1610 |
| Geomean: | | 26.2 | 951.4 | 1812.1 |
| CY 219 | | 8.3 | 8.3 | 421 |
| CY 220 | gp140.modUS4 | 8.3 | 8.3 | 3117 |
| CY 221 | + p55gag.SF2 | 8.3 | 954 | 871 |
| CY 222 | | 8.3 | 71 | 916 |
| Geomean: | | 8.3 | 46.5 | 1011.5 |
| CY 223 | | 41.4 | 10497 | 46432 |
| CY 224 | gp140.mut. | 8.3 | 979 | 470 |
| CY 225 | modUS4 | 135 | 2935 | 3870 |
| CY 226 | | 47 | 1209 | 4009 |
| Geomean: | | 68.3 | 2457.4 | 4289.6 |
| CY 227 | | 8.3 | 56 | 5001 |
| CY 228 | gp140TM. | 8.3 | 806 | 1170 |
| CY 229 | modUS4 | 8.3 | 48 | 3402 |
| CY 230 | | 8.3 | 38 | 6520 |
| GMT*: | | 8.3 | 95.3 | 3375.3 |

| | | | | |
|---|---|---|---|---|
| *GMT = geometric mean titer | | | | |

The results in Table 17 demonstrate the usefulness of the synthetic constructs to generate immune responses in primates such as baboons. In addition, all animals showed evidence of antigen-specific (Env antigen) lymphoproliferative responses.

### D. Rhesus Macaques

Two rhesus macaques (designated H445 and J408) were immunized with 1 mg of DNA encoding SF162 gp140 with a deleted V2 region (SF162.gp140.delV2) by intramuscular (IM) and intradermal (ID) routes at 0, 4, 8, and 28 weeks. Approximately 100 µg of the protein encoded by the SF162. gp140mut.delV2 construct was also administered in MF59 by IM delivery at 28 weeks.

ELISA titers are shown in Figure 61. Neutralizing antibody activity is shown Tables 18 and 19. Neutralizing antibody activity was determined against a variety of primary HIV-1 isolates in a primary lymphocyte or "PBMC-based" assay (see the following tables). Further, the phenotypic co-receptor usage for each of the primary isolates is indicated. As can be seen in the tables neutralizing antibodies were detected against every isolate tested, including the HIV-1 primary isolates (i.e., SF128A, 92US660, 92HT593, 92US657, 92US714, 91US056, and 91US054).

**Table 18**

| | Treatment | | Bleed 0 | Bleed 1 | Bleed 2 |
|---|---|---|---|---|---|
| Animal | 1st Immunization | 2nd Immunization | 1st Imm'n | 2nd Imm'n | 2 Wks post 2nd |
| EO 456 | | | 8.3 | 45 | 309 |
| EO 457 | | | 8.3 | 254 | 460 |
| EO 458 | 25µg 120mod DNA | (None) | 8.3 | 8.3 | 93 |
| EO 459 | | | 8.3 | 43 | 45 |
| EO 460 | | | 8.3 | 8.3 | 274 |
| EO 461 | | | 8.3 | 47 | 1502 |
| EO 462 | | | 8.3 | 80 | 5776 |
| EO 463 | 25µg 120mod DNA | 25µg 120mod | 8.3 | 89 | 3440 |
| EO 464 | | DNA | 8.3 | 8.3 | 3347 |
| EO 465 | | | 8.3 | 69 | 1127 |
| EO 466 | | | 8.3 | 63 | 102 |
| EO 467 | | | 8.3 | 112 | 662 |
| EO 468 | 50µg 120mod DNA | (None) | 8.3 | 94 | 459 |
| EO 469 | | | 8.3 | 58 | 48 |
| EO 470 | | | 8.3 | 95 | 355 |
| EO 471 | | | 8.3 | 110 | 9074 |
| EO 472 | | | 8.3 | 8.3 | 4897 |
| EO 473 | 50µg 120mod DNA | 50µg 120mod | 8.3 | 49 | 4089 |
| EO 474 | | DNA | 8.3 | 59 | 5280 |
| EO 475 | | | 8.3 | 8.3 | 929 |
| EO 476 | | | 8.3 | | 653 |
| EO 477 | | | 8.3 | 87 | 22675 |
| EO 478 | 25µg 120mod DNA | Sindbis/Env | 8.3 | 76 | 3869 |
| EO 479 | | | 8.3 | | 1004 |
| EO 480 | | | 8.3 | 71 | 7080 |

**Table 19**

| | Treatment | | Bleed 0 | Bleed 1 | Bleed 2 |
|---|---|---|---|---|---|
| Animal | 1st Immunization | 2nd Immunization | 1st Imm'n | 2nd Imm'n | 2 Wks post 2nd |
| EO 481 | | | 8.3 | 8.3 | 8.3 |
| EO 482 | | | 8.3 | 8.3 | 8.3 |
| EO 483 | Sindbis/Env | (None) | 8.3 | 78 | 103 |
| EO 484 | | | 8.3 | 8.3 | 32 |
| EO 485 | | | 8.3 | 76 | 207 |
| EO 486 | | | 8.3 | 8.3 | 458 |
| EO 487 | | | 8.3 | 8.3 | 345 |
| EO 488 | Sindbis/Env | Sindbis/Env | 8.3 | 8.3 | 331 |
| EO 489 | | | 8.3 | 103 | 111 |
| EO 490 | | | 8.3 | 8.3 | 5636 |

Lymphoproliferative activity (LPA) was also determined by antigenic stimulation followed by uptake of ³H-thymidine in these animals and is shown in Table 20. Experiment 1 was performed at 14 weeks post third DNA immunization and Experiment 2 was performed at 2 weeks post fourth DNA immunization using DNA and protein. For gp120ThaiE, gp120SF2 and US4 o-gp140, appropriate background values were used to calculate Stimulation Indices (S.I.; Antigenic stimulation CPM/Background CPM).

**Table 20**

| S.I.: Calculated as Ag CPM/Background CPM | | | | |
|---|---|---|---|---|
| Animal/ exp# | gp120Thai E | gp120 SF2 | env2-3SF2 | ogp140US4 |
| J408/#1 | 2 | 1 | 1 | 5 |
| H445/#1 | 1 | 1 | 1 | 6 |
| J408/#2 | 1 | 1 | 2 | 3 |
| H445/#2 | 0 | 0 | 3 | 2 |

As can be seen by the results presented in Table 20 lymphoproliferative responses to o-gp140.US4 antigen were also in all four animals at both experimental time points. Such proliferation results are indicative of induction of T-helper cell functions.

The results presented above demonstrate that the synthetic gp140.modSF162.delV2 DNA and protein are immunogenic in non-human primates.

### Example 13

### In vitro expression of recombinant Sindbis RNA and DNA containing the synthetic Gag or Env expression cassettes

### A. Synthetic Gag expression cassettes

To evaluate the expression efficiency of the synthetic Gag expression cassette in Alphavirus vectors, the synthetic Gag expression cassette was subcloned into both plasmid DNA-based and recombinant vector particle-based Sindbis virus vectors. Specifically, a cDNA vector construct for *in vitro* transcription of Sindbis virus RNA vector replicons (pRSIN-luc; Dubensky, et al., *J Virol.* 70:508-519, 1996) was modified to contain a *Pme*I site for plasmid linearization and a polylinker for insertion of heterologous genes. A polylinker was generated using two oligonucleotides that contain the sites *Xho*I, *Pml*I*, Apa*I, *NarI, Xba*I, *and Not*I (XPANXNF, SEQ ID NO:17, and XPANXNR, SEQ ID NO:18).

The plasmid pRSIN-luc (Dubensky et al., *supra)* was digested with *Xho*I and *Not*I to remove the luciferase gene insert, blunt-ended using Klenow and dNTPs, and purified from an agarose get using GeneCleanII (Bio101, Vista, CA). The oligonucleotides were annealed to each other and ligated into the plasmid. The resulting construct was digested with *NotI* and *Sacl* to remove the minimal Sindbis 3'-end sequence and A₄₀ tract, and ligated with an approximately 0.4 kbp fragment from PKSSIN1-BV (WO 97/38087). This 0.4 kbp fragment was obtained by digestion of pKSSIN1-BV with *Not*I and *Sac*I, and purification after size fractionation from an agarose gel. The fragment contained the complete Sindbis virus 3'-end, an A₄₀ tract and a *Pme*I site for linearization. This new vector construct was designated SINBVE.

The synthetic HIV Gag coding sequence was obtained from the parental plasmid by digestion with *EcoR*I, blunt-ending with Klenow and dNTPs, purification with GeneCleanII, digestion with *Sal*I, size fractionation on an agarose gel, and purification from the agarose gel using GeneCleanII. The synthetic Gag coding fragment was ligated into the SINBVE vector that had been digested with *Xho*I and *Pm*lI. The resulting vector was purified using GeneCleanII and designated SINBVGag. Vector RNA replicons may be transcribed *in vitro* (Dubensky et al., supra) from SINBVGag and used directly for transfection of cells. Alternatively, the replicons may be packaged into recombinant vector particles by co-transfection with defective helper RNAs or using an alphavirus packaging cell line as described, for example, in U.S. Patent Numbers 5,843,723 and 5,789,245, and then administered in vivo as described..

The DNA-based Sindbis virus vector pDCMVSIN-beta-gal (Dubensky, et al., J Virol. 70:508-519, 1996) was digested with *Sal*I and *Xba*I, to remove the beta-galactosidase gene insert, and purified using GeneCleanII after agarose gel size fractionation. The HIV Gag gene was inserted into the the pDCMVSIN-beta-gal by digestion of SINBVGag with *Sal*I and *Xho*I, purification using GeneCleanII of the Gag-containing fragment after agarose gel size fractionation, and ligation. The resulting construct was designated pDSIN-Gag, and may be used directly for in vivo administration or formulated using any of the methods described herein.

BHK and 293 cells were transfected with recombinant Sindbis vector RNA and DNA, respectively. The supernatants and cell lysates were tested with the Coulter p24 capture ELISA (Example 2).

BHK cells were transfected by electroporation with recombinant Sindbis RNA. The expression of p24 (in ng/ml) is presented in Table 21. In the table, SINGag#1 and 2 represent duplicate measurements, and SINβgal represents a negative control. Supernatants and lysates were collected 24h post transfection.

**Table 21**

| Construct | Supernatant | Lysate |
|---|---|---|
| SINβgal RNA | 0 | 0 |
| SINGag#1 RNA | 7 ng | Max (approx. 1 µg) |
| SINGag#2 RNA | 1 ng | 700 ng |

293 cells were transfected using LT-1 (Example 2) with recombinant Sindbis DNA. Synthetic pCMVKM2GagMod.SF2 was used as a positive control. Supernatants and lysates were collected 48h post transfection. The expression of p24 (in ng/ml) is presented in Table 22.

**Table 22**

| Construct | Supernatant | Lysate |
|---|---|---|
| SINGag DNA | 3 | 30 |
| pCMVKM2.GagMod.SF2 DNA | 32 | 42 |

The results presented in Tables 21 and 22 demonstrate that Gag proteins can be efficiently expressed from both DNA and RNA-based Sindbis vector systems using the synthetic Gag expression cassette (p55Gag.mod).

### B. Synthetic Env expression cassettes

To evaluate the expression efficiency of the synthetic Env expression cassette in Alphavirus vectors, synthetic Env expression cassettes were subcloned into both plasmid DNA-based and recombinant vector particle-based Sindbis virus vectors as described above for Gag.

The synthetic HIV Env coding sequence was obtained from the parental plasmid by digestion with *Sal*I and *Xba*I, size fractionation on an agarose gel, and purification from the agarose gel using GeneCleanII. The synthetic Env coding fragment was ligated into the SINBVE vector that had been digested with *Xho*I and *Xba*I. The resulting vector was purified using GeneCleanII and designated SINBVEnv. Vector RNA replicons may be transcribed in vitro (Dubensky et al., supra) from SINBVEnv and used directly for transfection of cells. Alternatively, the replicons may be packaged into recombinant vector particles by co-transfection with defective helper RNAs or using an alphavirus packaging cell line and administered as described above for Gag.

The DNA-based Sindbis virus vector pDCMVSIN-beta-gal (Dubensky, et al., *J Virol.* 70:508-519, 1996) was digested with *Sal*I and *Xba*I, to remove the beta-galactosidase gene insert, and purified using GeneCleanII after agarose gel size fractionation. The HIV Env gene was inserted into the the pDCMVSIN-beta-gal by digestion of SINBVEnv with *Xba*I and *Xho*I, purification using GeneCleanII of the Env-containing fragment after agarose gel size fractionation, and ligation. The resulting construct was designated pDSIN-Env, and may be used directly for in vivo administration or formulated using any of the methods described herein.

BHK and 293 cells were transfected with recombinant Sindbis vector RNA and DNA, respectively. The supernatants and cell lysates were tested by capture ELISA.

BHK cells were transfected by electroporation with recombinant Sindbis RNA. The expression of Env (in ng/ml) is presented in Table 23. In the table, the Sindbis RNA containing synthetic Env expression cassettes are indicated and βgal represents a negative control. Supernatants and lysates were collected 24h post transfection.

**Table 23**

| Construct | Supernatant (Neat)ng/ml | Lysate (1:10 dilution)ng/ml |
|---|---|---|
| βgal RNA | 0 | 0 |
| gp140.modUS4 | 726 | 7147 |
| gp140.modSF162 | 3529 | 7772 |
| gp140.modUS4.delV1/V2 | 1738 | 6526 |
| gp140.modUS4.delV2 | 960 | 3023 |
| gp140.modSF162.delV2 | 2772 | 3359 |

293 cells were transfected using LT-1 mediated transfection (PanVera) with recombinant Sindbis DNA containing synthetic expression cassettes of the present invention and βgal sequences as a negative control. Supernatants and lysates were collected 48h post transfection. The expression of Env (in ng/ml) is presented in Table 24.

**Table 24**

| Construct | Supernatant (Neat)ng/ml | Lysate (1:10 dilution)ng/ml |
|---|---|---|
| βgal | 0 | 0 |
| gp140.modSF162.delV2 | 1977 | 801 |
| gp140.modSF162 | 949 | 746 |

The results presented in Tables 23 and 24 demonstrated that Env proteins can be efficiently expressed from both DNA and RNA-based Sindbis vector systems using the synthetic Env expression cassettes of the present invention.

### Example 14

### A. In vivo Immunization with Gag-containing DNA and/or Sindbis particles

CB6F1 mice were immunized intramuscularly at 0 and 4 weeks with plasmid DNA and/or Sindbis vector RNA-containing particles each containing GagMod.SF2 sequences as indicated in Table 25. Animals were challenged with recombinant vaccinia expressing SF2 Gag at 3 weeks post second immunization (at week 7). Spleens were removed from the immunized and challenged animals 5 days later for a standard ⁵¹C release assay for CTL activity. Values shown in Table 25 indicate the results from the spleens of three mice from each group. The boxed values in Table 25 indicate that all groups of mice receiving immunizations with pCMVKm2.GagMod.SF2 DNA and/or SindbisGagMod.SF2 virus particles either alone or in combinations showed antigen-specific CTL activity.

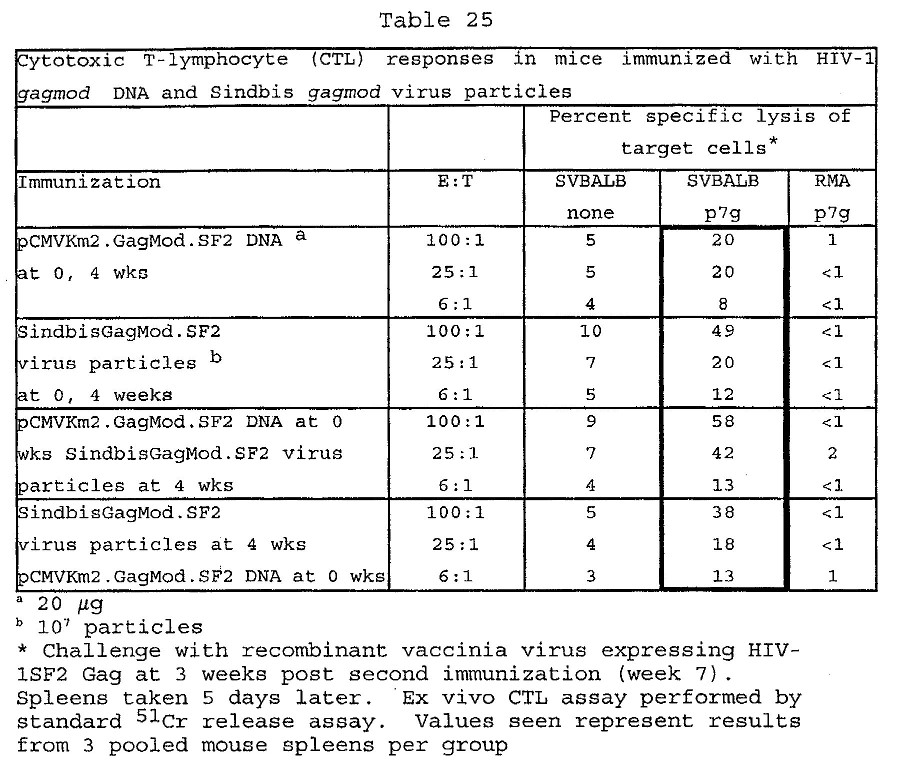

### B. In vivo Immunization with Env-containing DNA and/or Sindbis particles

Balb/C mice were immunized intramuscularly at 0 and 4 weeks(as shown in the following table) with plasmid DNA and/or Sindbis-virus RNA-containing particles each containing gp120.modUS4 sequences. Treatment regimes and antibody titers are shown in Table 26. Antibody titers were determined by ELISA using gp120 SF2 protein to coat the plates.

**Table 26**

| | Treatment | | Bleed 0 | Bleed 1 (8 wks) | Bleed 2 (10 wks) |
|---|---|---|---|---|---|
| Animal | 1st Immunization | 2nd Immunization | 1st Imm'n | 2nd Imm'n | 2 Wks post 2nd |
| EO 456 | | | 8.3 | 45 | 309 |
| EO 457 | | | 8.3 | 254 | 460 |
| EO 458 | 25µg 120mod | (None) | 8.3 | 8.3 | 93 |
| EO 459 | DNA | | 8.3 | 43 | 45 |
| EO 460 | | | 8.3 | 8.3 | 274 |
| EO 461 | | | 8.3 | 47 | 1502 |
| EO 462 | | | 8.3 | 80 | 5776 |
| EO 463 | 25µg 120mod | 25µg 120mod | 8.3 | 89 | 3440 |
| EO 464 | DNA | DNA | 8.3 | 8.3 | 3347 |
| EO 465 | | | 8.3 | 69 | 1127 |
| EO 466 | | | 8.3 | 63 | 102 |
| EO 467 | | | 8.3 | 112 | 662 |
| EO 468 | 50µg 120mod | (None) | 8.3 | 94 | 459 |
| EO 469 | DNA | | 8.3 | 58 | 48 |
| EO 470 | | | 8.3 | 95 | 355 |
| EO 471 | | | 8.3 | 110 | 9074 |
| EO 472 | | | 8.3 | 8.3 | 4897 |
| EO 473 | 50µg 120mod | 50µg 120mod | 8.3 | 49 | 4089 |
| EO 474 | DNA | DNA | 8.3 | 59 | 5280 |
| EO 475 | | | 8.3 | 8.3 | 929 |
| EO 476 | | | 8.3 | | 653 |
| EO 477 | | | 8.3 | 87 | 22675 |
| EO 478 | 25µg 120mod DNA | Sindbis/Env | 8.3 | 76 | 3869 |
| EO 479 | | | 8.3 | | 1004 |
| EO 480 | | | 8.3 | 71 | 7080 |
| EO 481 | | | 8.3 | 8.3 | 8.3 |
| EO 482 | | | 8.3 | 8.3 | 8.3 |
| EO 483 | Sindbis/Env | (None) | 8.3 | 78 | 103 |
| EO 484 | | | 8.3 | 8.3 | 32 |
| EO 485 | | | 8.3 | 76 | 207 |
| EO 486 | | | 8.3 | 8.3 | 458 |
| EO 487 | | | 8.3 | 8.3 | 345 |
| EO 488 | Sindbis/Env | Sindbis/Env | 8.3 | 8.3 | 331 |
| EO 489 | | | 8.3 | 103 | 111 |
| EO 490 | | | 8.3 | 8.3 | 5636 |

As can be seen from the data presented above, all of the mice generally demonstrated substantial immunological responses by bleed number 2. For Env, the best results were obtained using either (i) 50 µg of gp120.modUS4 DNA for the first immunization followed by a second immunization using 50 µg of gp120.modUS4 DNA, or (ii) 25 µg of gp120.modUS4 DNA for the first immunization followed by a second immunization using 10⁷ pfus of Sindbis.

The results presented above demonstrate that the Env and Gag proteins of the present invention are effective to induce an immune response using Sindbis vector systems which include the synthetic Env (e.g., gp120.modUS4) or Gag expression cassettes.

### Example 15

### Co-Transfection of Env and Gag as Monocistronic and Bicistronic Constructs

DNA constructs encoding (i) wild-type US4 and SF162 Env polypeptides, (ii) synthetic US4 and SF162 Env polypeptides (gp160.modUS4, gp160.modUS4.delV1/V2, gp160.modSF162, and gp120.modSF162.delV2), and (iii) SF2gag polypeptide (i.e., the Gag coding sequences obtained from the SF2 variant or optimized sequences corresponding to the gagSF2 -- gag.modSF2) were prepared. These monocistronic constructs were co-transfected into 293T cells in a transient transfection protocol using the following combinations: gp160.modUS4; gp160.modUS4 and gag.modSF2; gp160.modUS4.delV1/V2; gp160.modUS4.delV1/V2 and gag.modSF2; gp160.modSF162 and gag.modSF2; gp120.modSF162.delV2 and gag.modSF2; and gag.modSF2 alone.

Further several bicistronic constructs were made where the coding sequences for Env and Gag were under the control of a single CMV promoter and, between the two coding sequences, an IRES (internal ribosome entry site (EMCV IRES); Kozak, M., Critical Reviews in Biochemistry and Molecular Biology 27(45):385-402, 1992; Witherell, G.W., et al., Virology 214:660-663, 1995) sequence was introduced after the Env coding sequence and before the Gag coding sequence. Those constructs were as follows: gp160.modUS4.gag.modSF2, SEQ ID NO:73 (Figure 61); gp160.modUSF162.gag.modSF2, SEQ ID NO:74 (Figure 62); gp160.modUS4.delV1/V2.gag.modSF2, SEQ ID NO:75 (Figure 63); and gp160.modSF162.delV2.gag.modSF2, SEQ ID NO:76 (Figure 64).

Supernatants from cell culture were filtered through 0.45 µm filters then ultracentrifuged for 2 hours at 24,000 rpm (140,000Xg) in an SW28 rotor through a 20% sucrose cushion. The pelleted materials were suspended and layered on a 20-60% sucrose gradient and spun for 2 hours at 40,000 rpm (285,000Xg) in an SW41Ti rotor. Gradients were fractionated into 1.0 ml samples. A total of 9-10 fractions were typically collected from each DNA transfection group.

The fractions were tested for the presence of the Env and Gag proteins (across all fractions). These results demonstrated that the appropriate proteins were expressed in the transfected cells (i.e., if an Env coding sequence was present the corresponding Env protein was detected; if a Gag coding sequence was present the corresponding Gag protein was detected).

Virus like particles (VLPs) were known to be present through a selected range of sucrose densities. Chimeric virus like particles (VLPs) were formed using all the tested combinations of constructs containing both Env and Gag. Significantly more protein was found in the supernatant collected from the cells transfected with "gp160.modUS4.delV1/V2 and gag.modSF2" than in all the other supernatants.

Western blot analysis was also performed on sucrose gradient fractions from each transfection. The results show that bicistronic plasmids gave lower amounts of VLPs than the amounts obtained using co-transfection with monocistronic plasmids.

In order to verify the production of chimeric VLPs by these cell lines the following electron microscopic analysis was carried out.

293T cells were plated at a density of 60-70% confluence in 100 mm dishes on the day before transfection. The cells were transfected with 10 µg of DNA in transfection reagent LT1 (Panvera Corporation, 545 Science Dr., Madison, WI). The cells were incubated overnight in reduced serum medium (opti-MEM, Gibco-BRL, Gaithersburg, MD). The medium was replaced with 10% fetal calf serum, 2% glutamine in IMDM in the morning of the next day and the cells were incubated for 65 hours. Supernatants and lysates were collected for analysis as described above (see Example 2).

The fixed, transfected 293T cells and purified ENV-GAG VLPs were analyzed by electron microscopy. The cells were fixed as follows. Cell monolayers were washed twice with PBS and fixed with 2% glutaraldehyde. For purified VLPs, gradient peak fractions were collected and concentrated by ultracentrifugation (24,000 rpm) for 2 hours. Electron microscopic analysis was performed by Prof. T.S. Benedict Yen (Veterans Affairs, Medical Center, San Francisco, CA).

Electron microscopy was carried out using a transmission electron microscope (Zeiss 10c). The cells were pre-stained with osmium and stained with uranium acetate and lead citrate. Immunostaining was performed to visualize envelope on the VLP. The magnification was 200,000X.

Figures 65A-65F show micrographs of 293T cells transfected with the following constructs: Figure 65A, gag.modSF2; Figure 65B, gp160.modUS4; Figure 65C, gp160.modUS4.delV1/V2.gag.modSF2 (bicistronic Env and Gag); Figures 65D and 65E, gp160.modUS4.delV1/V2 and gag.modSF2; and Figure 65F, gp120.modSF162.delV2 and gag.modSF2. In the figures, free and budding immature virus-like-particles (VLPs) of the expected size (approximately 100 nm) decorated with the Env protein were seen. In sum, gp160 polypeptides incorporate into Gag VLPs when constructs were co-transfected into cells. The efficiency of incorporation is 2-3 fold higher when constructs encoding V-deleted Env polypeptides from high synthetic expression cassettes are used.

Although preferred embodiments of the subject invention have been described in some detail, it is understood that obvious variations can be made without departing from the spirit and the scope of the invention as defined by the appended claims.

## Claims

1. An expression cassette comprising a polynucleotide sequence encoding a polypeptide comprising an HIV env polypeptide, wherein the polynucleotide sequence encoding said HIV env polypeptide comprises a contiguous sequence of at least 144 nucleotides having at least 90% sequence identity to SEQ ID NO:71 (Figure 58) or SEQ ID NO:72 (Figure 59).

2. An expression cassette comprising a polynucleotide sequence encoding a polypeptide comprising an HIV env polypeptide, wherein the polynucleotide sequence encoding said env polypeptide comprises (A) X contiguous nucleotides, wherein (i) the X contiguous nucleotides have at least 90% identity to Y contiguous nucleotides of SEQ ID NO:71, (ii) X equals Y, and (iii) X is 144 nucleotides; or (B) X contiguous nucleotides, wherein (i) the X contiguous nucleotides have at least 90% identity to Y contiguous nucleotides of SEQ ID NO:72, (ii) X equals Y, and (iii) X is 144 nucleotides.

3. The expression cassette of claim 1 or 2, wherein said *env* polypeptide further comprises sequences flanking a V1 region but has a deletion in the V1 region itself.

4. The expression cassette of either claim 1 or 2, wherein the polynucleotide sequence encoding the polypeptide comprises the sequence presented as SEQ ID NO:65 (Figure 52).

5. The expression cassette of either claim 1 or 2, wherein said *env* polypeptide further comprises sequences flanking a V2 region but has a deletion hi the V2 region itself.

6. The expression cassette of claim 5, wherein the polynucleotide sequence encoding the polypeptide is selected from the group consisting of: SEQ ID NO:60 (Figure 47); and SEQ ID NO:66 (Figure 53).

7. The expression cassette of claim 5, wherein the polynucleotide sequence encoding the polypeptide is selected from the group consisting of: SEQ ID NO:34 (Figure 20 gp120); SEQ ID NO:37 (Figure 24); SEQ ID NO:40 (Figure 27); SEQ ID NO:43 (Figure 30); SEQ ID NO:46 (Figure 33); SEQ ID NO:49 (Figure 36); and SEQ ID NO:76 (Figure 64).

8. The expression cassette of either claim 1 or 2, wherein said *env* polypeptide further encodes sequences flanking a V1/V2 region but has a deletion in the V1/V2 region.

9. The expression cassette of claim 8, wherein said polynucleotide sequence encoding said env polypeptide is selected from the group consisting of: SEQ ID NO:59 (Figure 46); SEQ ID NO:61 (Figure 48); SEQ ID NO:67 (Figure 54); and SEQ ID NO:75 (Figure 63).

10. The expression cassette of claim 8, wherein said polynucleotide sequence encoding said *env* polypeptide is selected from the group consisting of: SEQ ID NO:35 (Figure 21); SEQ ID NO:38 (Figure 25); SEQ ID NO:41 (Figure 28); SEQ ID NO:44 (Figure 31); SEQ ID NO:47 (Figure 34) and SEQ ID NO:50 (Figure 37).

11. The expression cassette of either claim 1 or 2, wherein said *env* polypeptide has a mutated cleavage site that prevents the cleavage of a gp140 polypeptide into a gp120 polypeptide and a gp41 polypeptide.

12. The expression cassette of claim 11, wherein the polynucleotide sequence encoding the polypeptide is selected from the group consisting of: SEQ ID NO:57 (Figure 44); SEQ ID NO:61 (Figure 48); SEQ ID NO:63 (Figure 50); SEQ ID NO:39 (Figure 26); SEQ ID NO:40 (Figure 27); SEQ ID NO:41 (Figure 28); SEQ ID NO:42 (Figure 29); SEQ ID NO:4 (Figure 30); SEQ ID NO:4 (Figure 31); SEQ ID NO:45 (Figure 32); SEQ ID NO:46 (Figure 33); and SEQ ID NO:47 (Figure 34).

13. The expression cassette of claim 1 or 2, wherein said *env* polypeptide encodes a gp160 env polypeptide or an immunogenic fragment thereof.

14. The expression cassette of claim 13, wherein the polynucleotide sequence encoding the polypeptide is selected from the group consisting of: SEQ ID NO:64 (Figure 51); SEQ ID NO:65 (Figure 52); SEQ ID NO:66 (Figure 53); SEQ ID NO:67 (Figure 54); SEQ ID NO:68 (Figure 55); SEQ ID NO:75 (Figure 63); SEQ ID NO:73 (Figure 61); SEQ ID NO:48 (Figure 35); SEQ ID NO:49 (Figure 36); SEQ ID NO:50 (Figure 37); SEQ ID NO:76 (Figure 64); and SEQ ID NO:74 (Figure 62).

15. The expression cassette of either claim 1 or 2, wherein said *env* polypeptide encodes a gp140 *env* polypeptide or an immunogenic fragment thereof.

16. The expression cassette of claim 15, wherein the polynucleotide sequence encoding the polypeptide is selected from the group consisting of: SEQ ID NO:56 (Figure 43); SEQ ID NO:57 (Figure 44); SEQ ID NO:58 (Figure 45); SEQ ID NO:59 (Figure 46); SEQ ID NO:60 (Figure 47); SEQ ID NO:61 (Figure 48); SEQ ID NO:62 (Figure 49); SEQ ID NO:63 (Figure 50); SEQ ID NO:36 (Figure 23); SEQ ID NO:37 (Figure 24); SEQ ID NO:38 (Figure 25); SEQ ID NO:39 (Figure 26); SEQ ID NO:40 (Figure 27); SEQ ID NO:41 (Figure 28); SEQ ID NO:4 (Figure 29); SEQ ID NO:43 (Figure 30); SEQ ID NO:44 (Figure 31); SEQ ID NO:45 (Figure 32); SEQ ID NO: (Figure 33); and SEQ ID NO:4 (Figure 34).

17. The expression cassette of claim 1 or 2, wherein said *env* polypeptide encodes a gp120 *env* polypeptide or an immunogenic fragment thereof.

18. The expression cassette of claim 17, wherein the polynucleotide sequence encoding the env polypeptide is selected from the group consisting of: SEQ ID NO:54 (Figure 41); SEQ ID NO:55 (Figure 42); SEQ ID NO:33 (Figure 19); SEQ ID NO:34 (Figure 20); and SEQ ID NO:35 (Figure 21).

19. The expression cassette of claim 1 or 2, wherein the polynucleotide sequence encoding the polypeptide is selected from the group consisting of: SEQ ID NO:55 (Figure 42 gp120); SEQ ID NO:62 (Figure 49); SEQ ID NO:63 (Figure 50); and SEQ ID NO:68 (Figure 55).

20. A recombinant expression system for use in a selected host cell comprising, an expression cassette of any of claims 1-19, and wherein said polynucleotide sequence is operably linked to control elements compatible with expression in the selected host cell.

21. The recombinant expression system of claim 20, wherein said control elements are selected from the group consisting of a transcription promoter, a transcription enhancer element, a transcription termination signal, polyadenylation sequences, sequences for optimization of initiation of translation, and translation termination sequences.

22. The recombinant expression system of claim 21, wherein, said transcription promoter is selected from the group consisting of CMV, CMV+intron A, SV40, RSV, HIVLtr, MMLV-Itr, and metallothionein.

23. A cell comprising an expression cassette of any of claims 1-19, wherein said polynucleotide sequence is operably linked to control elements compatible with expression in the selected cell.

24. The cell of claim 23, wherein the cell is a mammalian cell.

25. The cell of claim 24, wherein the cell is selected from the group consisting of BHK, VERO, HT1080, 293, RD, COS-7, and CHO cells.

26. The cell of claim 25, wherein said cell is a CHO cell.

27. The cell of claim 23, wherein the cell is selected from the group consisting of an insect cell, a bacterial cell, a yeast cell, a plant cell, a primary cell, an immortalized cell, a tumor-derived cell, a macrophage, a monocyte, a dendritic cell, a B-cell, a T-cell, a stem cell, and a progenitor cell.

28. A cell line useful for packaging lentivirus vectors comprising suitable host cells that have been transfected with an expression vector containing an expression cassette of any of claims 1-19, and wherein said polynucleotide sequence is operably linked to control elements compatible with expression in the host cell.

29. The cell line of claim 28, wherein suitable host cells have been transfected with an expression vector containing the expression cassette of any of claims 1-19.

30. A gene delivery vector for use in a subject, wherein the vector comprises an expression cassette of any of claims 1-19, and wherein said polynucleotide sequence is operably linked to control elements compatible with expression in the subject.

31. The gene delivery vector of claim 30, wherein said gene delivery vector is selected from the group consisting of a nonviral vector, a viral vector and a bacterial plasmid vector.

32. The viral vector of claim 31, selected from the group consisting of: a retroviral vector, a lentiviral vector, an adenoviral vector, an alphavirus vector, and a pox virus derived vector.

33. The alphavirus vector of claim 32, wherein the vector comprises a eukaryotic layered vector initiation system.

34. The pox virus vector of claim 32, wherein said pox virus derived vector is a vaccinia virus.

35. The gene delivery vehicle of any one of claims 30-34, wherein the vector is associated with a particulate carrier.

36. The vector of any one of claims 30-35, wherein said vector is encapsulated in a liposome preparation.

37. *In vitro* use of an expression cassette of any one of claims 1-19 to stimulate an immune response.

38. Use of the expression cassette according to any one of claims 1-19 in the manufacture of a medicament.

39. Use of the medicament of claim 38 for inducing an immune response.

40. A method for producing a polypeptide expressed by an expression cassette according to any one of claims 1-19, said method comprising culturing a host cell under conditions which result in the expression of the polypeptide, and recovering the polypeptide.

41. Use of the polypeptide recovered from claim 40, in the manufacture of a medicament.

42. Use of the medicament of claim 41 for inducing an immune response.

43. The use of a polypeptide according to claim 41, further comprising an adjuvant.

44. *In vitro* use of a polynucleotide sequence of any one of claims 1-19 to stimulate an immune response, wherein said polynucleotide sequence operatively encodes an HIV polypeptide when introduced directly into the tissue of a subject.

45. The use of a polynucleotide sequence according to either claim 43 or 44, wherein said polynucleotide sequence is introduced into tissue by a gene delivery vector.

46. A composition comprising the expression cassette of any one of claims 1-19 for use in the manufacture of a medicament.

47. Use of the medicament of claim 46 for inducing an immune response.

48. The use of the composition of claim 46, wherein said immune response is a humoral immune response.

49. The use of the composition of claim 46, wherein said immune response is a cellular immune response.

50. Use of a composition comprising an expression cassette according to any one of claims 1-19 in the manufacture of a medicament wherein said medicament is administered in a priming step and is followed by a boosting step.

51. Use of a composition comprising an expression cassette according to any one of claims 1-19 in the manufacture of a medicament wherein said medicament is administered in a boosting step and is preceded by a priming step.

52. The use of a composition of claims 50 or 51 wherein the compositions of the priming or the boosting step further comprises an adjuvant.

53. The use of a composition of claims 50 or 51 wherein the compositions of both the priming and the boosting steps further comprise an adjuvant.

54. The composition of claim 46, further comprising a gag polypeptide.

55. The composition of claim 46, further comprising an additional *env* polypeptide encoded by at least one of the expression cassettes of any of claims 1-19.

56. Use of a composition comprising an expression cassette encoding an *env* polypeptide according to any one of claims 1-19 for the manufacture of a medicament wherein said medicament is administered before, after or simultaneously with another expression cassette.

57. The composition of any one of claims 46-54 for use in the treatment of HIV.

58. The composition of any one of claims 46-55, further comprising an adjuvant.

59. A method for producing virus-like particles (VLPs) comprising, incubating the cells of claims 23-28, under conditions for producing said VLPs.

60. A method for producing a composition of virus-like particles (VLPs) comprising, (a) incubating the cells of claims 23-28, under conditions for producing said VLPs; and (b) substantially purifying said VLPs to produce a composition of VLPs.
